(19) Europäisches Patentamt / European Patent Office / Office européen des brevets

(11) **EP 4 523 704 A1**

(12) **EUROPEAN PATENT APPLICATION**
published in accordance with Art. 153(4) EPC


(43) Date of publication:
**19.03.2025 Bulletin 2025/12**

(21) Application number: **23802996.1**

(22) Date of filing: **11.05.2023**

(51) International Patent Classification (IPC):
***A61K 47/68*** *(2017.01)* ***A61P 35/00*** *(2006.01)*
***C07D 491/22*** *(2006.01)* ***C07K 16/28*** *(2006.01)*

(52) Cooperative Patent Classification (CPC):
**A61K 47/68; A61P 35/00; C07D 491/22; C07K 16/28**

(86) International application number:
**PCT/CN2023/093503**

(87) International publication number:
**WO 2023/217227 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.05.2022 CN 202210515898**
**19.08.2022 CN 202210999585**
**14.04.2023 CN 202310406469**



(71) Applicant: **Hainan Simcere Zaiming Pharmaceutical Co., Ltd.**
**Hainan 570311 (CN)**

(72) Inventors:
- **FU, Yayuan**
  **Shanghai 201318 (CN)**
- **LI, Zhen**
  **Shanghai 201318 (CN)**
- **CHEN, Changyan**
  **Shanghai 201318 (CN)**
- **CHAI, Xiaojuan**
  **Shanghai 201318 (CN)**
- **YU, Zhiyong**
  **Shanghai 201318 (CN)**
- **TANG, Feng**
  **Shanghai 201318 (CN)**
- **CAO, Zhuoxiao**
  **Shanghai 201318 (CN)**
- **TANG, Renhong**
  **Shanghai 201318 (CN)**

(74) Representative: **Richly & Ritschel Patentanwälte PartG mbB**
**Sattlerweg 20**
**51429 Bergisch Gladbach (DE)**


Remarks:
The complete document including Reference Table(s) and the Sequence Listing(s) can be downloaded from the EPO website

(54) **CAMPTOTHECIN DERIVATIVE AND LIGAND-DRUG CONJUGATE**


(57) The present disclosure relates to a ligand-drug conjugate of a novel structure or a pharmaceutically acceptable salt thereof. Specifically, the present disclosure provides a ligand-drug conjugate having a structural general formula represented by Pc-(L-D)n or a pharmaceutically acceptable salt thereof, a method for preparing same, a pharmaceutical composition comprising the conjugate, and use thereof in treating a tumor.


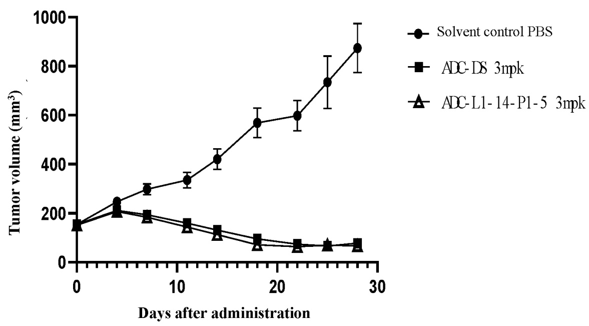


FIG. 2

EP 4 523 704 A1

## Description

### CROSS-REFERENCE TO RELATED APPLICATIONS

The present application claims priority to the Chinese Patent Application No. 202210515898.0 filed on May 12, 2022, the Chinese Patent Application No. 202210999585.7 filed on August 19, 2022, and the Chinese Patent Application No. 202310406469.4 filed on April 14, 2023, which are incorporated herein by reference in their entirety for all purposes.

### TECHNICAL FIELD

The present disclosure belongs to the field of biomedicine, and relates to a ligand-drug conjugate with a novel structure, a preparation method therefor, a pharmaceutical composition containing the conjugate, and use thereof as an anti-tumor drug.

### BACKGROUND

Antibody-drug conjugate (ADC), as a novel targeted drug, combines the tumor targeting of an antibody with the efficient killing effect of a toxin molecule by linking a monoclonal antibody specifically binding to a tumor cell surface antigen to a toxin molecule with biological activity, and at the same time avoids the poor therapeutic effect of the former and the serious toxic and side effects, poor druggability, and the like of the latter. Compared with the traditional chemotherapeutic agents, the ADC drug can accurately target tumor cells and reduce the effect on normal cells so as to achieve a safer and more effective anti-tumor effect.

The first antibody-drug conjugate, Mylotarg (gemtuzumab ozogamicin), was approved by the FDA in the United States in 2000 for the treatment of adult acute myelocytic leukemia (AML). A new targeted ADC drug, Adcetris (bretuximab vedotin), was approved by the FDA in the United States in 2011 for the treatment of hodgkin's lymphoma and systemic anaplastic large cell lymphoma. Both Mylotarg and Adcetris are therapeutics against hematologic tumors. Kadcyla (ado-trastuzumab emtansine, T-DM1) was approved by the FDAin the United States in 2013 for the treatment of HER2-positive advanced or metastatic breast cancer resistant to trastuzumab and paclitaxel, which was the first approved ADC drug for the treatment of solid tumors.

An ADC generally comprises three parts: an antibody, a linker, and a toxin. Camptothecin derivatives are toxins applied to ADC development, and achieve an anti-tumor effect by inhibiting topoisomerase I. Daiichi Sankyo developed the ADC drug, Enhertu (trastuzumab deruxtecan, DS-8201), for the HER2 target using the camptothecin derivative exatecan as a toxin, which was approved by the FDA in the United States in 2019. Clinical studies have shown that Enhertu has good therapeutic effect on HER2-positive breast cancer, gastric cancer, non-small cell lung cancer, and the like.

Despite the availability of numerous ADC drugs on the market currently, there still remain some safety and drug resistance issues and there is still a great unmet clinical need. Therefore, there is a need in the art to further develop more effective and safer ADC drugs.

### SUMMARY

The present disclosure provides a ligand-drug conjugate having a structural general formula of Pc-(L-D)$_n$ or a pharmaceutically acceptable salt thereof,
wherein

Pc is a ligand unit;
L is a linker unit;
D is a drug unit of the following formula (D-I):

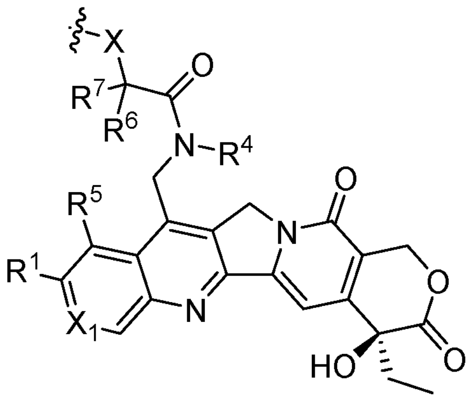

(D-I)

wherein

X is selected from NH or O;

$R^1$ is selected from halogen, CN, $C_1$-$C_6$ alkyl, $C_3$-$C_6$ cycloalkyl, or $C_2$-$C_6$ alkynyl, wherein the $C_1$-$C_6$ alkyl, $C_3$-$C_6$ cycloalkyl, or $C_2$-$C_6$ alkynyl is optionally substituted with one or more $R^{a1}$;

$X_1$ is selected from $CR^2$ or N;

$R^2$ is selected from H, halogen, and CN, or $R^1$ and $R^2$, together with the atom linked thereto, form 5-6 membered heterocyclyl, wherein the 5-6 membered heterocyclyl is optionally substituted with one or more $R^{a2}$;

$R^4$ is selected from H, $C_1$-$C_3$ alkyl, $C_3$-$C_6$ cycloalkyl, or 4-7 membered heterocyclyl, wherein the $C_1$-$C_3$ alkyl, $C_3$-$C_6$ cycloalkyl, or 4-7 membered heterocyclyl is optionally substituted with one or more $R^{a4}$;

$R^5$ is selected from H, halogen, CN, $NH_2$, or $NO_2$, or $R^1$ and $R^5$, together with the atom linked thereto, form 5-6 membered heterocyclyl, 5-6 membered heteroaryl, or $C_5$-$C_7$ cycloalkenyl, wherein the 5-6 membered heterocyclyl, 5-6 membered heteroaryl, or $C_5$-$C_7$ cycloalkenyl is optionally substituted with one or more $R^{a5}$;

$R^6$ is selected from H or $C_1$-$C_3$ alkyl;

$R^7$ is selected from H, $C_1$-$C_3$ alkyl, or $C_3$-$C_6$ cycloalkyl, or $R^6$ and $R^7$, together with the C atom linked thereto, form $C_3$-$C_6$ cycloalkyl, wherein the $C_3$-$C_6$ cycloalkyl is optionally substituted with one or more $R^{a7}$;

each $R^{a1}$, $R^{a2}$, $R^{a4}$, $R^{a5}$, $R^{a7}$ is independently selected from D, halogen, CN, =O, OH, $NH_2$, $C_1$-$C_3$ alkyl, $C_3$-$C_6$ cycloalkyl, or 4-7 membered heterocyclyl, wherein the OH, $NH_2$, $C_1$-$C_3$ alkyl, $C_3$-$C_6$ cycloalkyl, or 4-7 membered heterocyclyl is optionally substituted with one or more $R^b$;

each $R^b$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$;

provided that: i) when $R^1$ is selected from methyl and $R^2$ is selected from F, $R^6$ and $R^7$, together with the C atom linked thereto, form cyclopropyl; ii) when X is selected from NH, $R^5$ is not selected from H; iii) the compound of formula (D-I) does not include

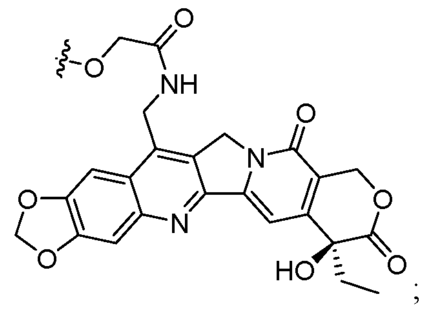

;

moreover, n is a real number of 1-16.

In some embodiments, each $R^{a1}$, $R^{a2}$, $R^{a4}$, $R^{a5}$, $R^{a7}$ is independently selected from D, halogen, CN, =O, OH, $NH_2$, $C_1$-$C_3$ alkyl, $C_3$-$C_6$ cycloalkyl, or 4-7 membered heterocyclyl.

In some embodiments, each $R^{a2}$ and $R^{a7}$ is independently selected from D.

In some embodiments, $R^1$ is selected from halogen, $C_1$-$C_3$ alkyl, $C_3$-$C_6$ cycloalkyl, or $C_2$-$C_3$ alkynyl.

In some embodiments, $R^1$ is selected from Cl, Br, methyl, cyclopropyl, or ethynyl.

In some embodiments, $R^1$ is selected from Cl, Br, or methyl.

In some embodiments, $R^2$ is selected from H, halogen, and CN, or $R^1$ and $R^2$, together with the atom linked thereto, form 5-6 membered heterocyclyl, wherein the 5-6 membered heterocyclyl comprises 1 or 2 oxygen atoms as a ring atom, and the 5-6 membered heterocyclyl is optionally substituted with one or more D atoms.

In some embodiments, $R^2$ is selected from H, halogen, and CN, or $R^1$ and $R^2$, together with the atom linked thereto, form 5-6 membered heterocyclyl, wherein the 5-6 membered heterocyclyl comprises 1 or 2 oxygen atoms as a ring atom.

In some embodiments, $R^2$ is selected from H, F, or Cl, or $R^1$ and $R^2$, together with the atom linked thereto, form

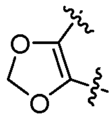, 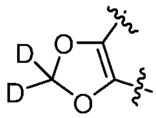, or 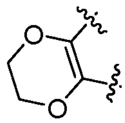.

In some embodiments, $R^2$ is selected from H, F, or Cl, or $R^1$ and $R^2$, together with the atom linked thereto, form

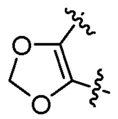 and 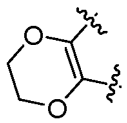.

In some embodiments, $R^5$ is selected from H, halogen, $NH_2$, or $NO_2$, or $R^1$ and $R^5$, together with the atom linked thereto, form 5-6 membered heteroaryl or $C_5$-$C_6$ cycloalkenyl, wherein the 5-6 membered heteroaryl or $C_5$-$C_6$ cycloalkenyl is optionally substituted with one or more $R^{a5}$.

In some embodiments, $R^5$ is selected from H, Cl, F, $NH_2$, or $NO_2$, or $R^1$ and $R^5$, together with the atom linked thereto, form

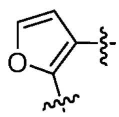 or 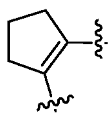.

In some embodiments, $R^4$ is selected from H or $C_1$-$C_3$ alkyl.

In some embodiments, $R^4$ is selected from H.

In some embodiments, $R^6$ is selected from H or methyl.

In some embodiments, $R^7$ is selected from H, $C_1$-$C_3$ alkyl, or $C_3$-$C_6$ cycloalkyl optionally substituted with one or more D, or $R^6$ and $R^7$, together with the C atom linked thereto, form $C_3$-$C_6$ cycloalkyl.

In some embodiments, $R^7$ is selected from H, methyl, isopropyl, or cyclopropyl optionally substituted with one or more D, or $R^6$ and $R^7$, together with the C atom linked thereto, form cyclopropyl.

In some embodiments, $R^1$ and $R^2$, together with the atom linked thereto, form

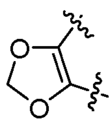

or

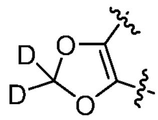,

$R^6$ is selected from H or methyl, $R^7$ is selected from H, methyl, isopropyl, or cyclopropyl optionally substituted with one or more D, or $R^6$ and $R^7$, together with the C atom linked thereto, form cyclopropyl.

In some embodiments, structural unit

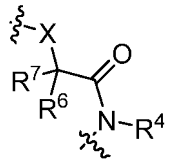

is selected from

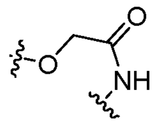 , 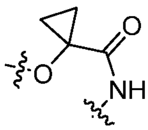 ,

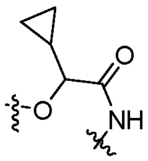 , 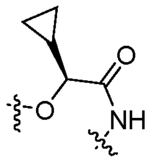 , 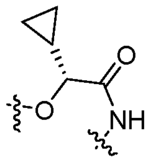 , 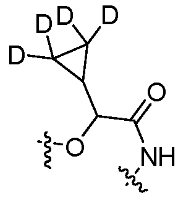 , 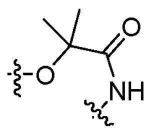 , 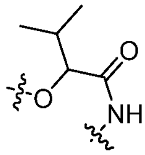 , 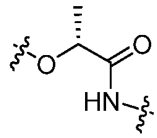 ,

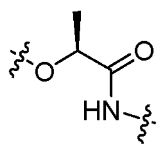 , or 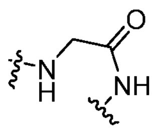 .

In some embodiments, $R^1$ is selected from methyl, $R^2$ is selected from F, and $R^6$ and $R^7$, together with the C atom linked thereto, form cyclopropyl.

In some embodiments, X is selected from NH, and $R^5$ is selected from Cl, F, $NH_2$, or $NO_2$.

In some embodiments, the drug unit of formula (D-I) is selected from the drug unit of formula (D-Ia):

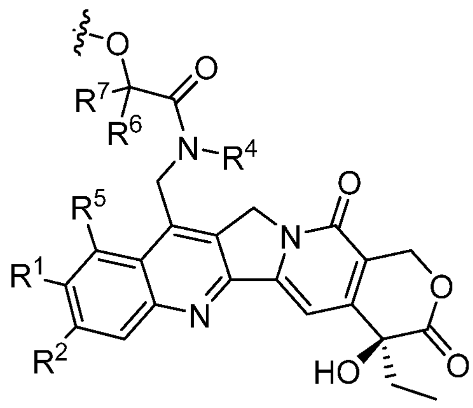

(D-Ia)

wherein $R^1$, $R^2$, $R^4$, $R^5$, $R^6$, and $R^7$ are as defined above.

In some embodiments, the compound of formula (D-I) is selected from the following compounds:

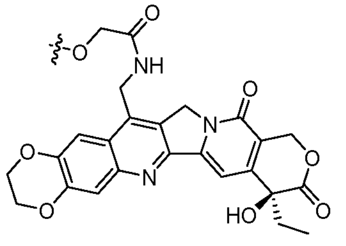

1'

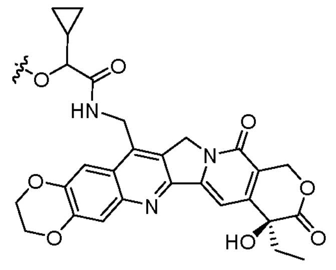

2'

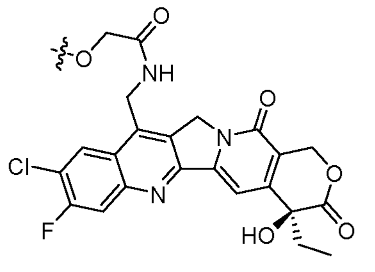

3'

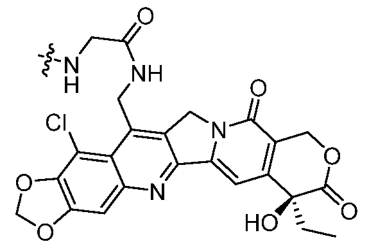

4'

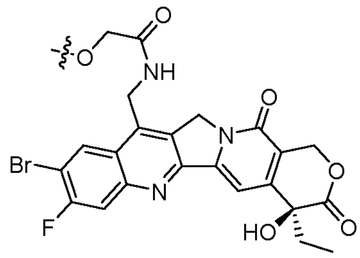

5'

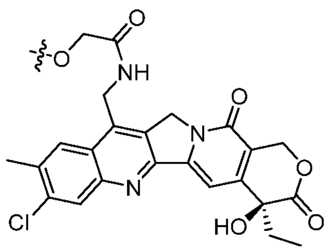

6'

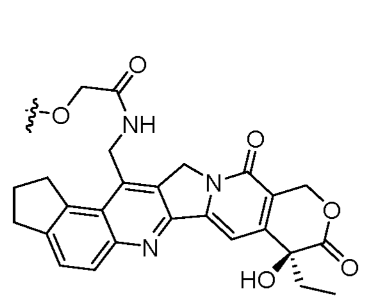

7'

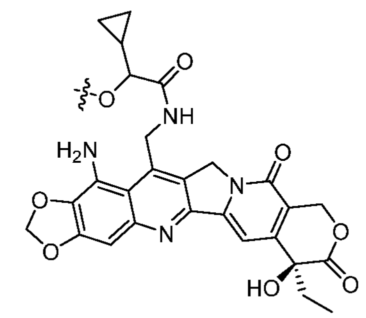

8'

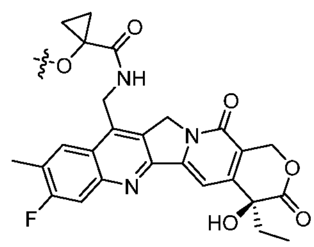

9'

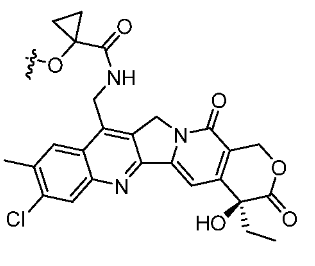

10'

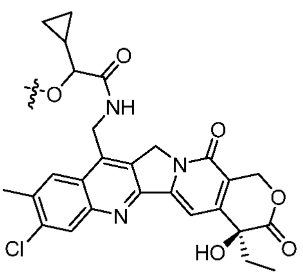

11'

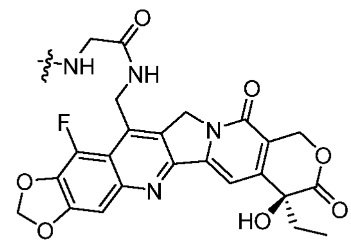

12'

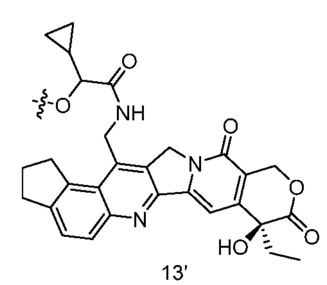

13'

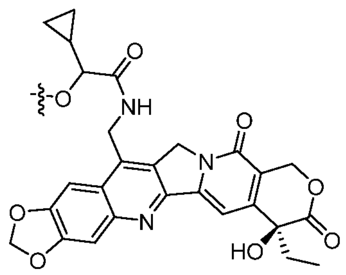

14'

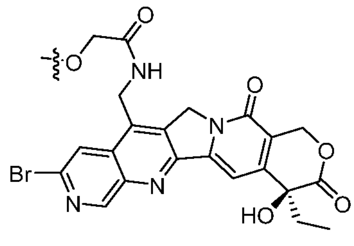

15'

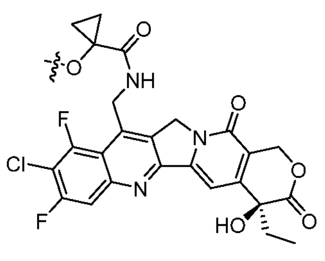
16'

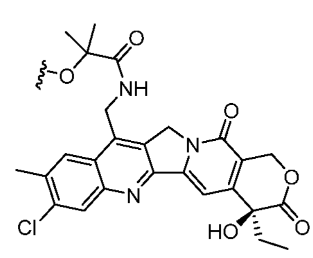
17'

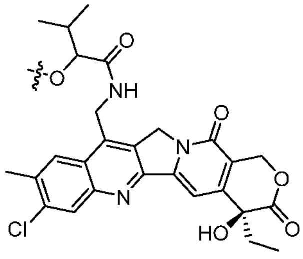
18'

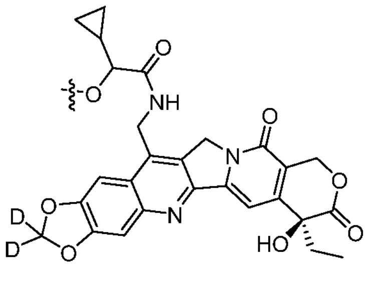
19'

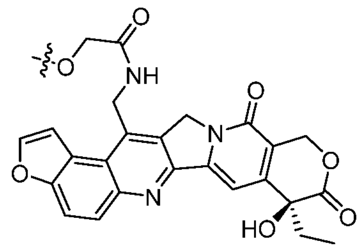
20'

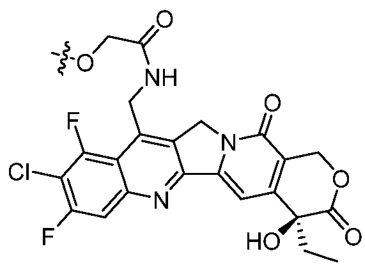
21'

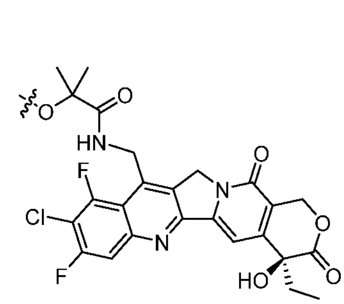
22'

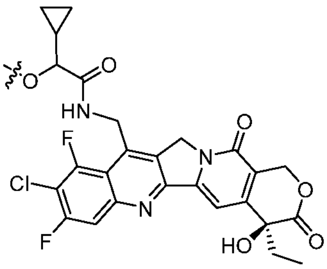
23'

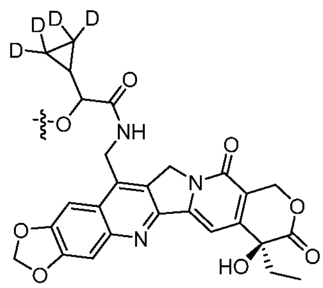
24'

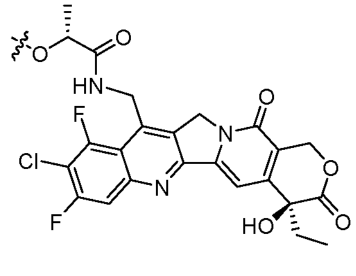
25'

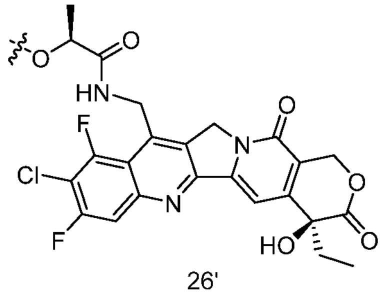
26'

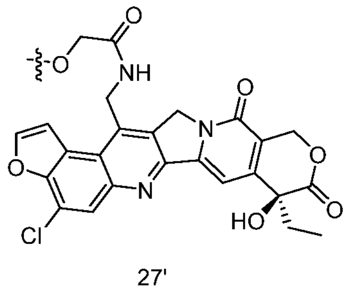
27'

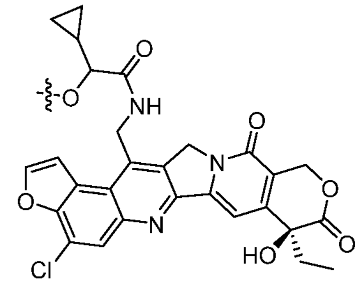
28'

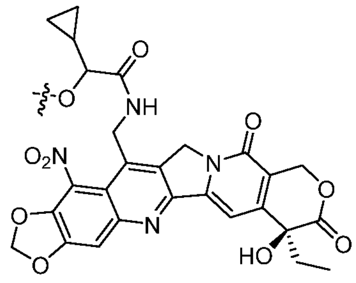
29'

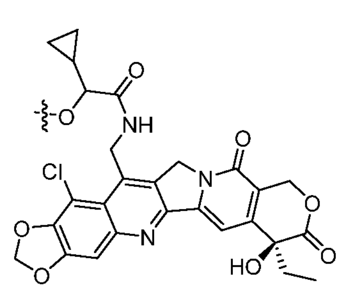
30'

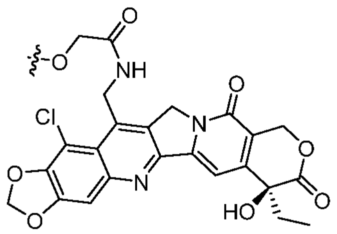

31'

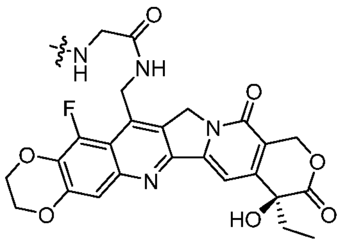

32'

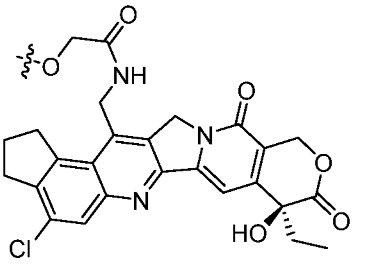

33'

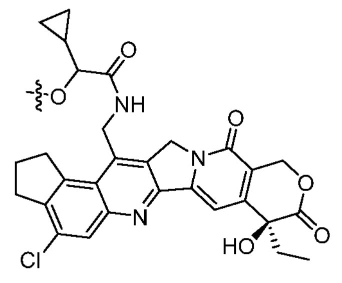

34'

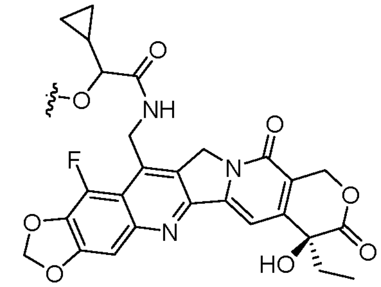

35'

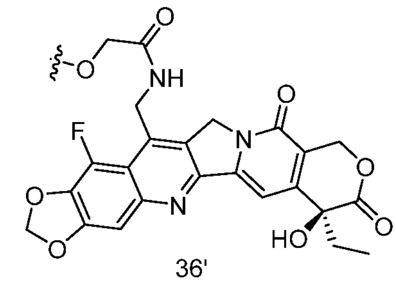

36'

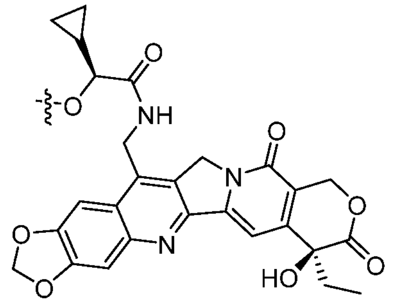

14-P1'

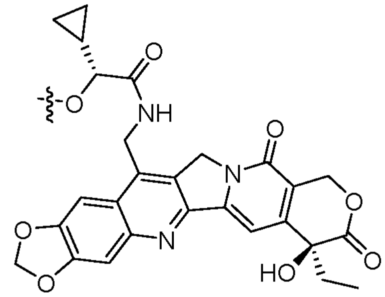

14-P2'

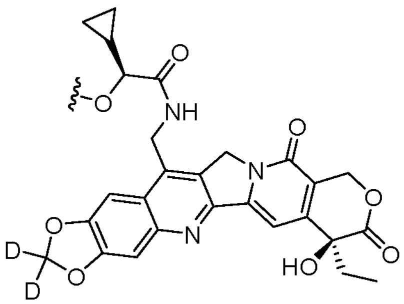

19-P1'

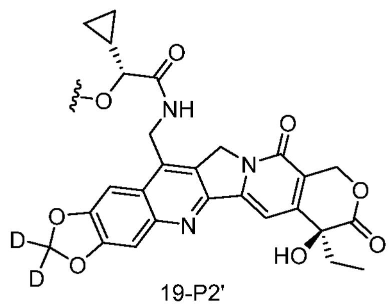

19-P2'

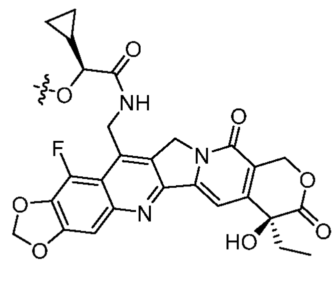

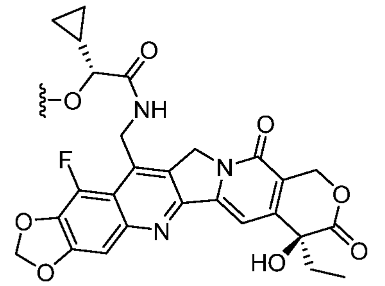

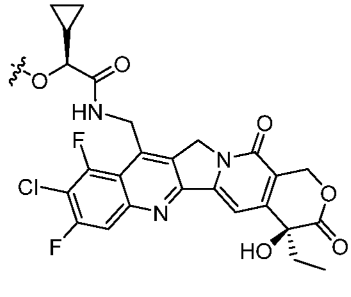

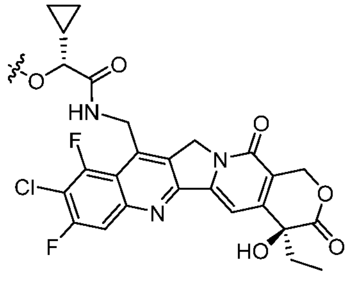

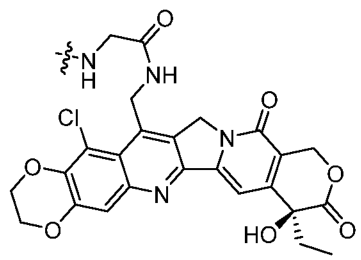

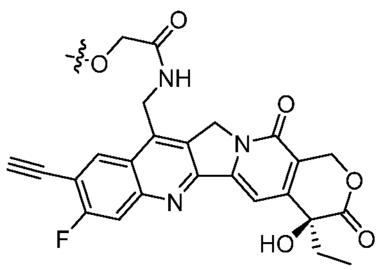

or

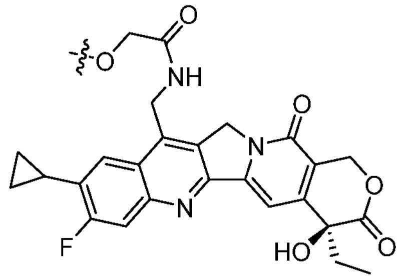

.

In some embodiments, the present disclosure provides a ligand-drug conjugate having a structural general formula of Pc-$(L\text{-}D)_n$ or a pharmaceutically acceptable salt thereof,
wherein

Pc, L, and n are as defined above;
D is selected from one of the following compounds:

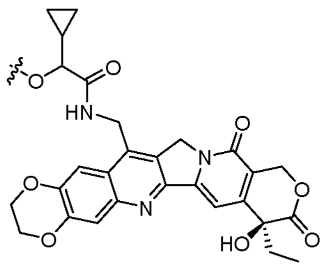

2'

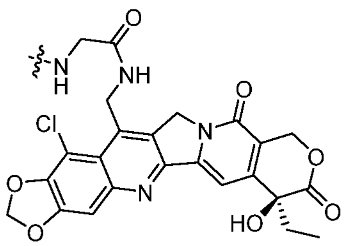

4'

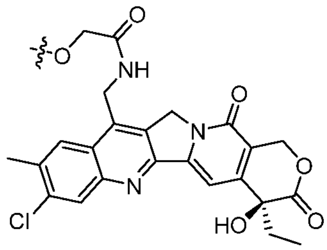

6'

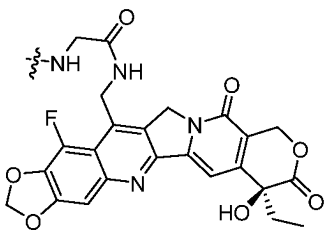

12'

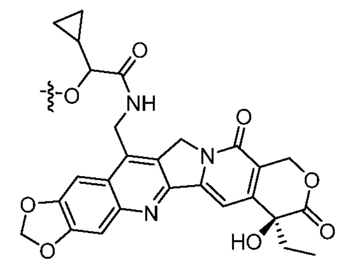

14'

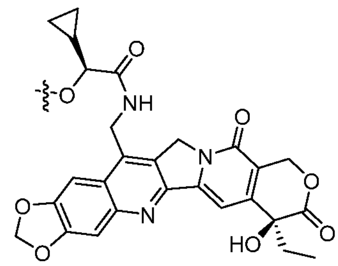

14-P1'

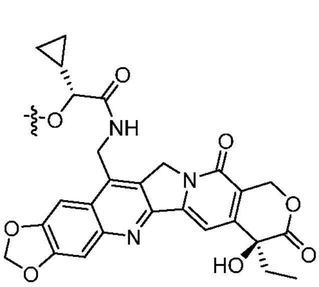

14-P2'

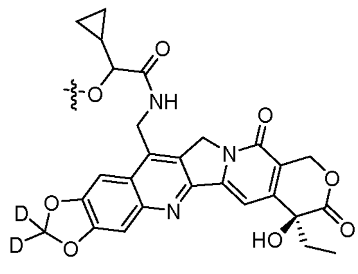

19'

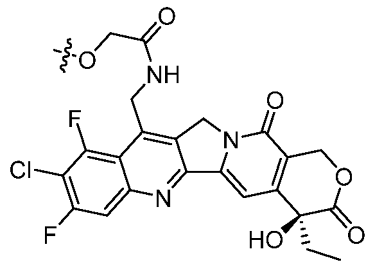

21'

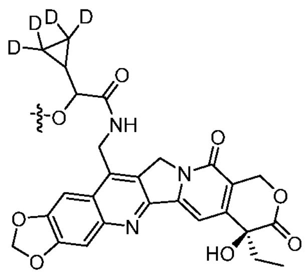

24'

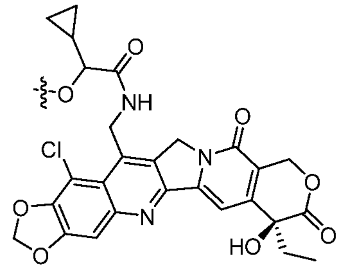

30'

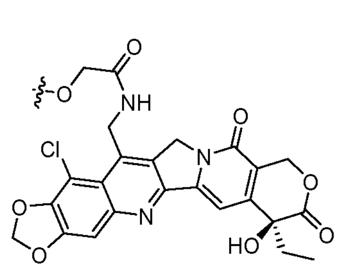

31'

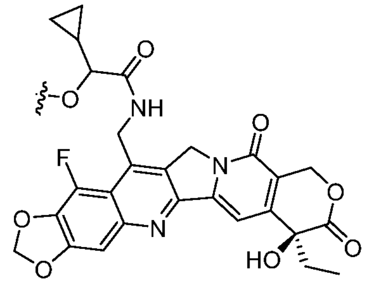

35'

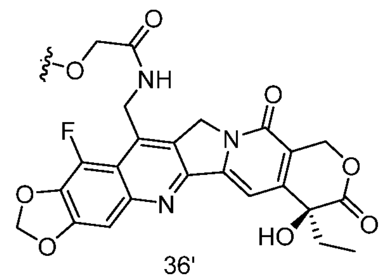

36'

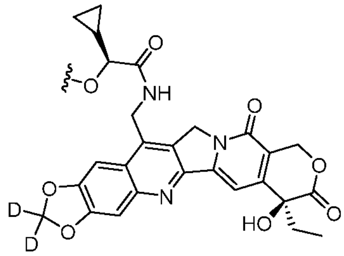

19-P1'

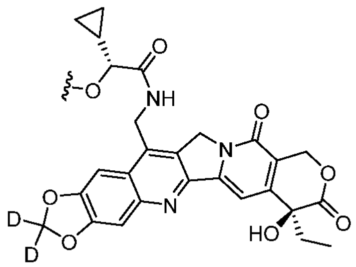

19-P2'

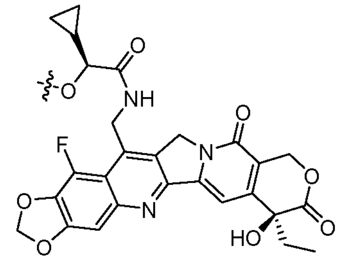

or

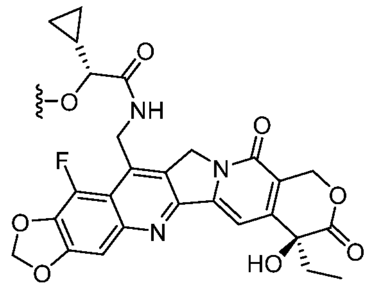

.

In some embodiments, linker unit L is selected from

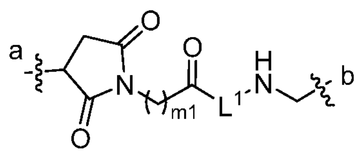

or

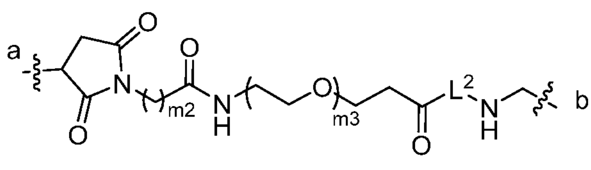

,

the end a of which is covalently linked to ligand unit Pc, and the end b is covalently linked to drug unit D, wherein m1 and m2 are each independently selected from integers of 2-8, m3 is selected from integers of 1-16, and $L^1$ and $L^2$ are each independently selected from peptide residues consisting of 1 to 8 amino acids, wherein the peptide residue is further optionally substituted with one or more substituents of halogen, CN, =O, $C_1$-$C_6$ alkyl, OH, O($C_1$-$C_6$ alkyl), $NH_2$, NH($C_1$-$C_6$ alkyl), N($C_1$-$C_6$ alkyl)$_2$, $C_3$-$C_6$ cycloalkyl, and 4-7 membered heterocyclyl.

In some embodiments, $L^1$ and $L^2$ are each independently selected from peptide residues consisting of 2, 3, or 4 amino acids, wherein the peptide residue is further optionally substituted with one or more substituents of halogen, CN, =O, $C_1$-$C_6$ alkyl, OH, O($C_1$-$C_6$ alkyl), $NH_2$, NH($C_1$-$C_6$ alkyl), N($C_1$-$C_6$ alkyl)$_2$, $C_3$-$C_6$ cycloalkyl, and 4-7 membered heterocyclyl.

In some embodiments, $L^1$ is a Gly-Gly-Phe-Gly tetrapeptide residue or an Ala-Ala-Ala tripeptide residue.

In some embodiments, $L^2$ is a Gly-Gly-Phe-Gly tetrapeptide residue or a Val-Lys dipeptide residue. In some embodiments, m1 is selected from 5.

In some embodiments, m2 is selected from 2, and m3 is selected from 8.

In some embodiments, linker unit L is selected from the following chemical structures:

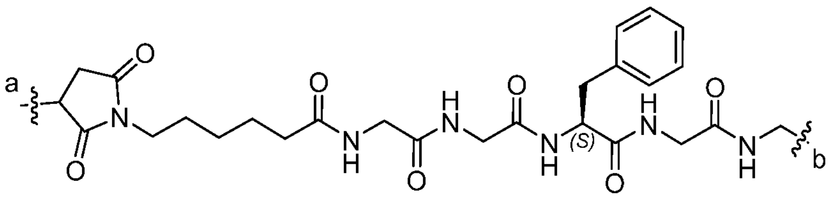

**L1**

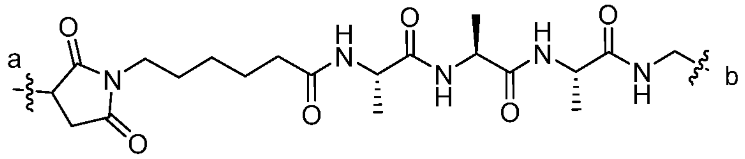

**L2**

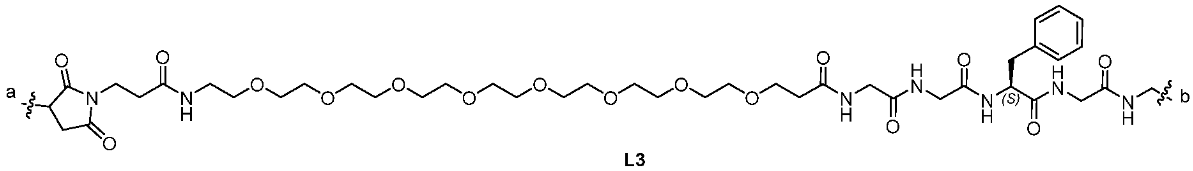

**L3**

or

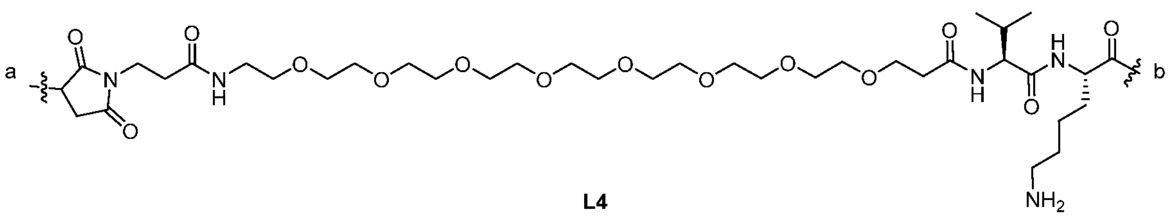

**L4**

,

the end a of which is covalently linked to ligand unit Pc, and the end b is covalently linked to drug unit D.

In some embodiments, the ligand-drug conjugate having the general formula of Pc-(L-D)$_n$ or the pharmaceutically acceptable salt thereof of the present disclosure is selected from the following compounds or pharmaceutically acceptable salts thereof:

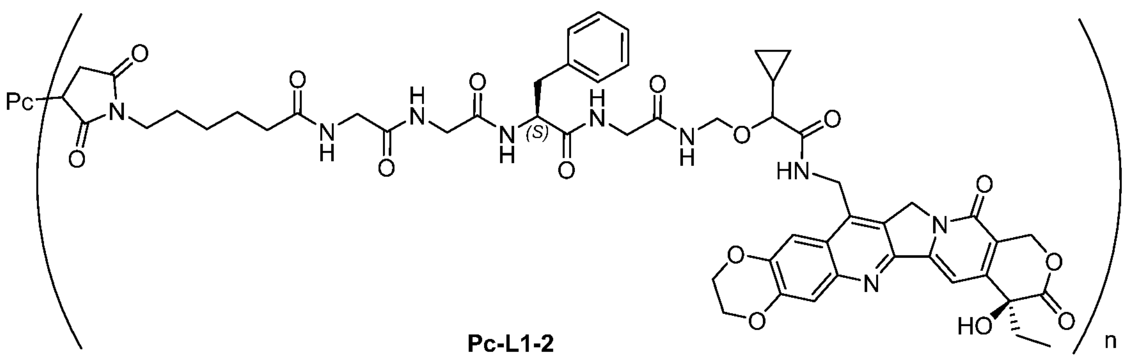

**Pc-L1-2**

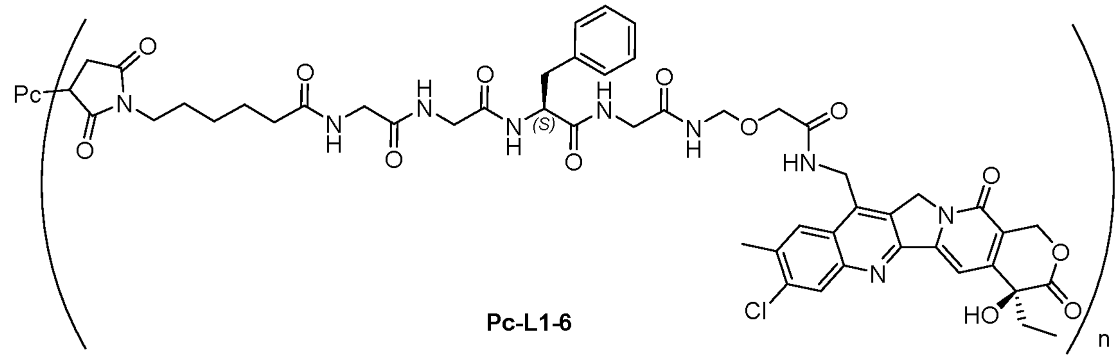

**Pc-L1-6**

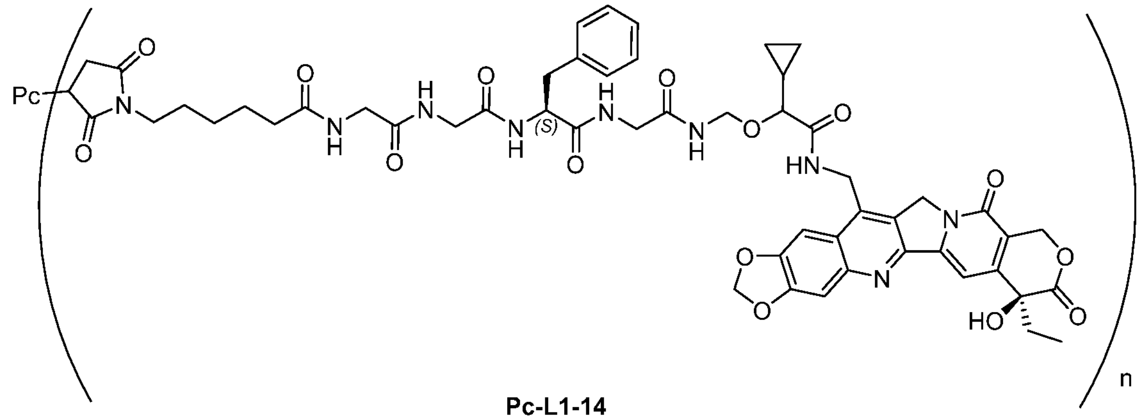

**Pc-L1-14**

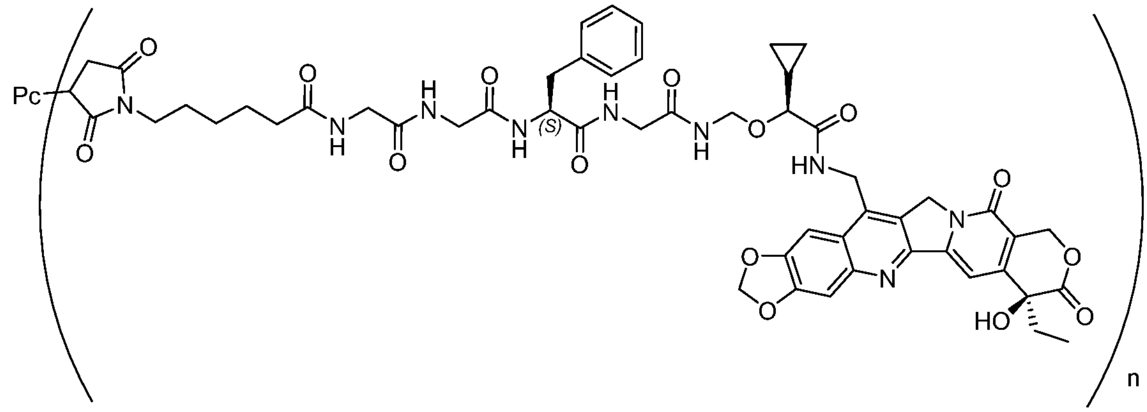

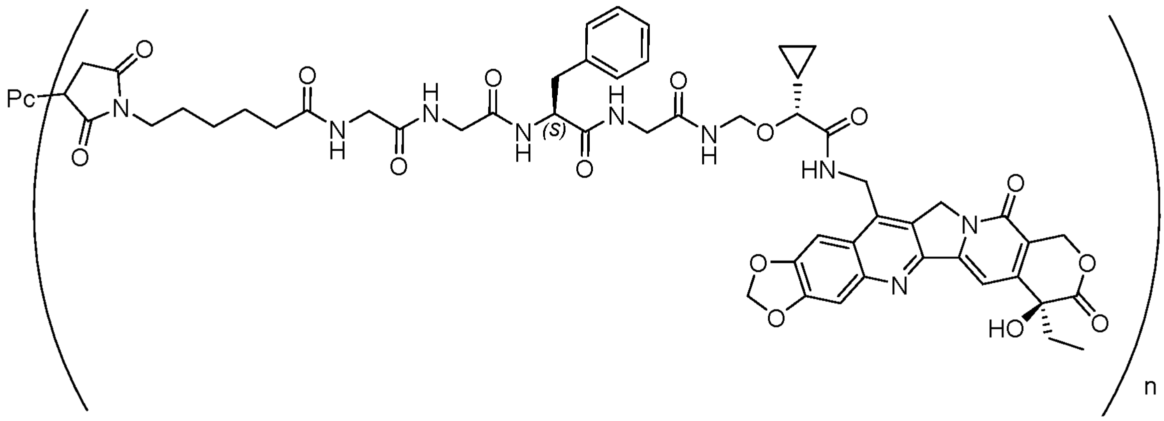

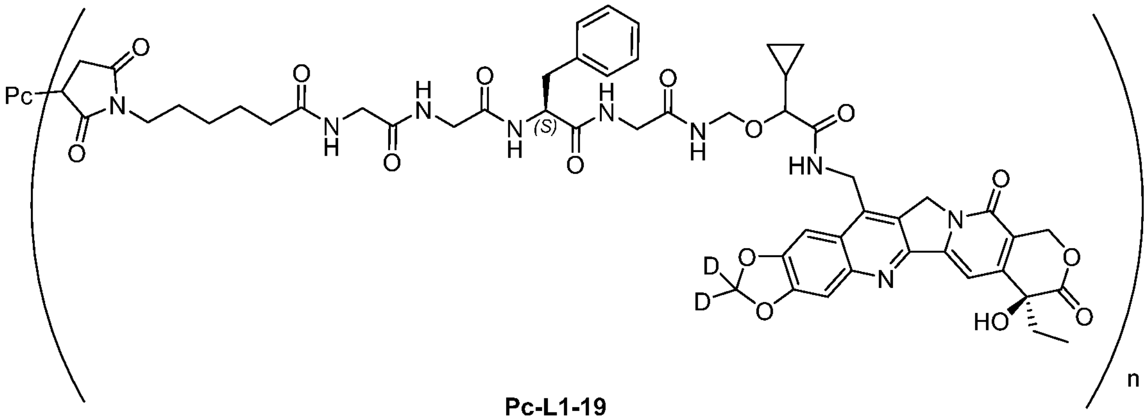

**Pc-L1-19**

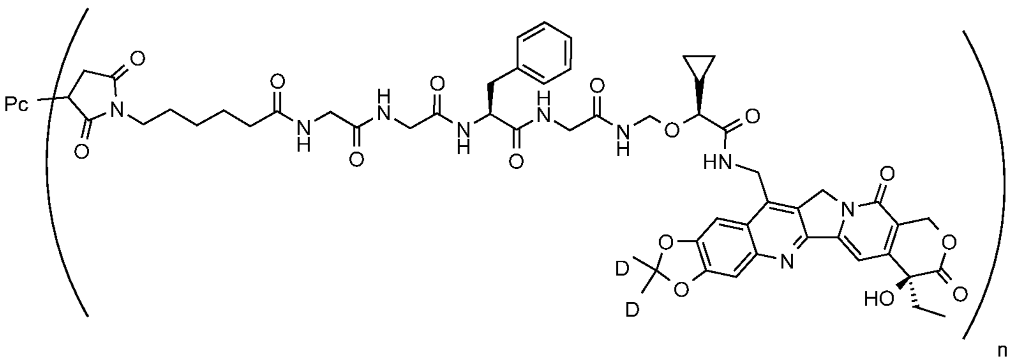

**Pc-L1-19-P1**

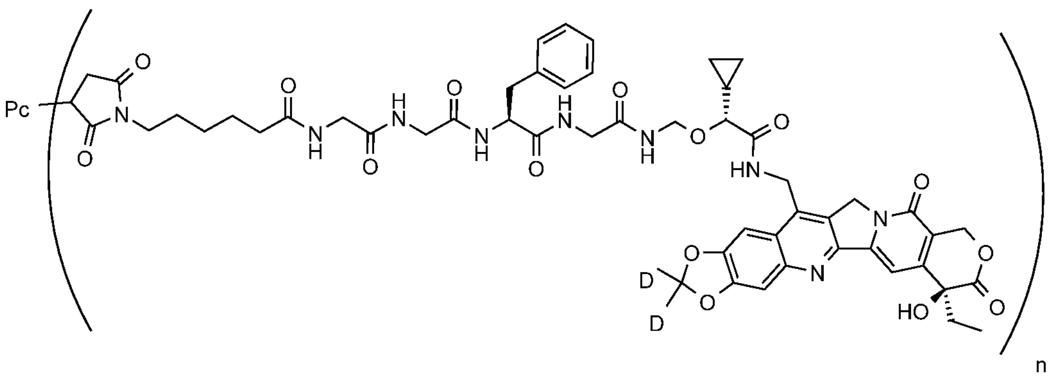

**Pc-L1-19-P2**

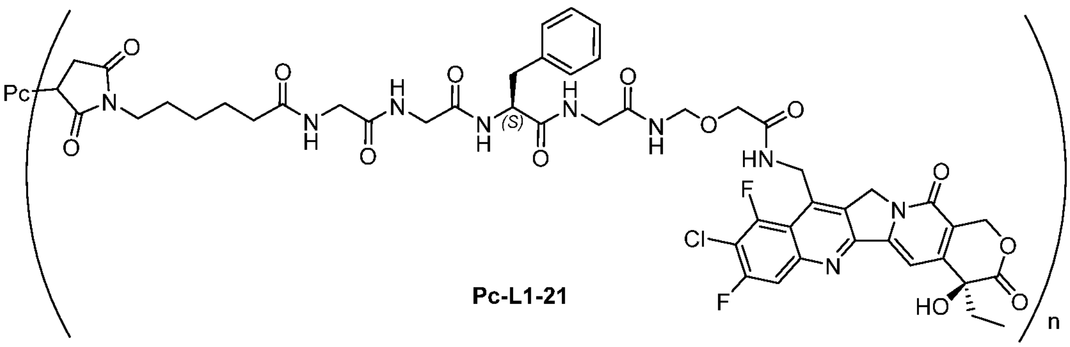

**Pc-L1-21**

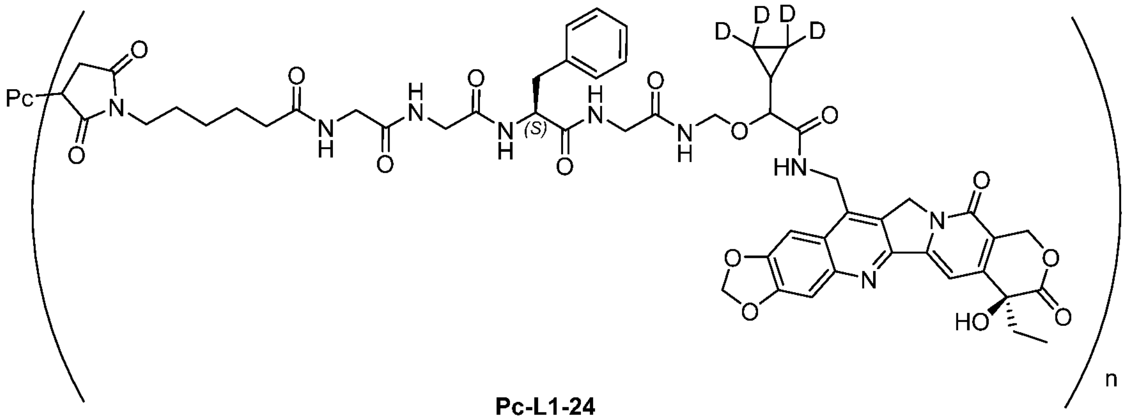

**Pc-L1-24**

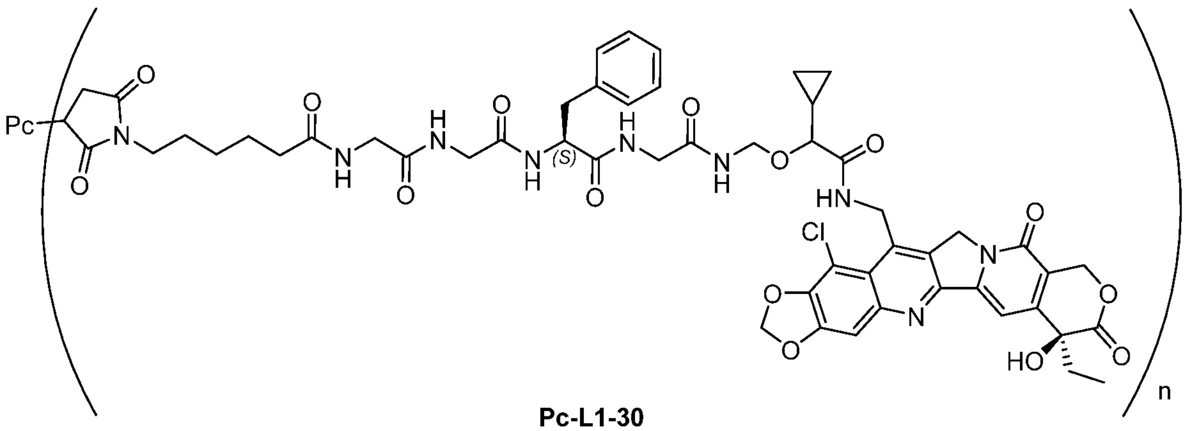

**Pc-L1-30**

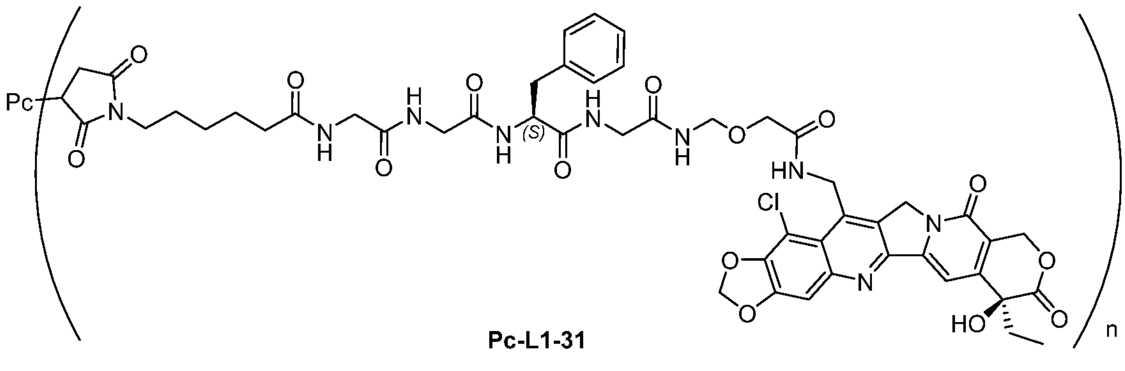

**Pc-L1-31**

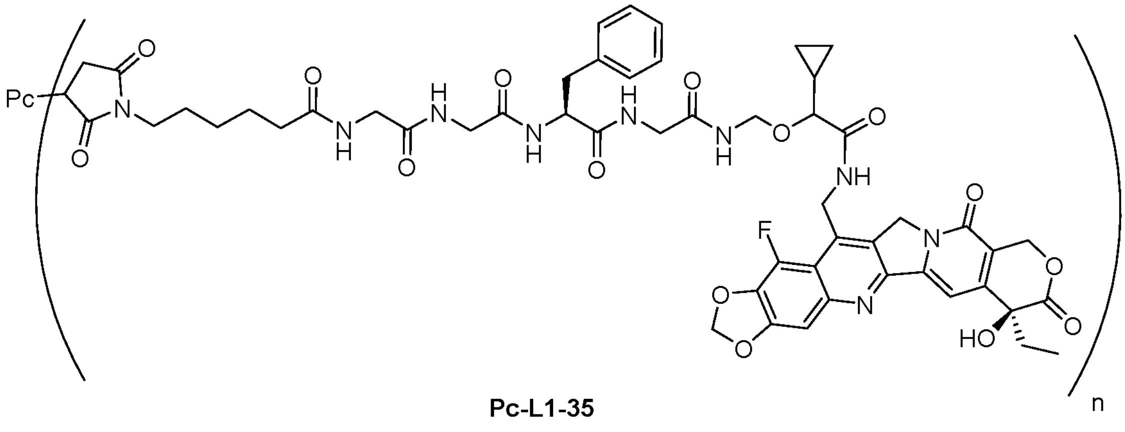

**Pc-L1-35**

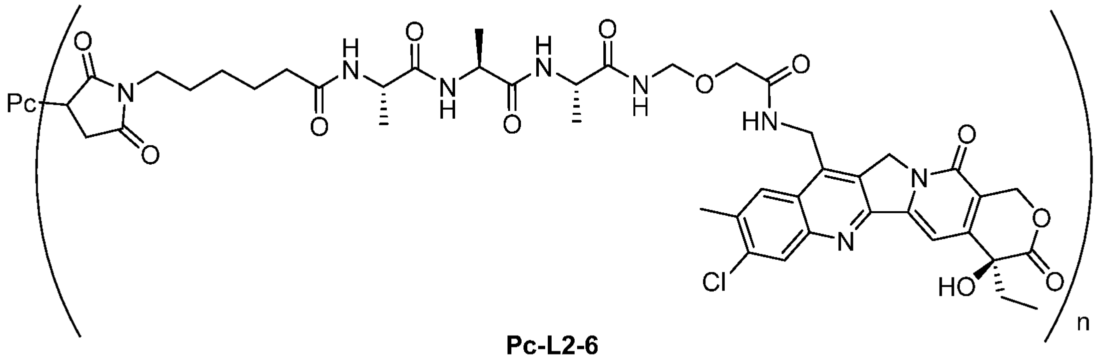

Pc-L2-6

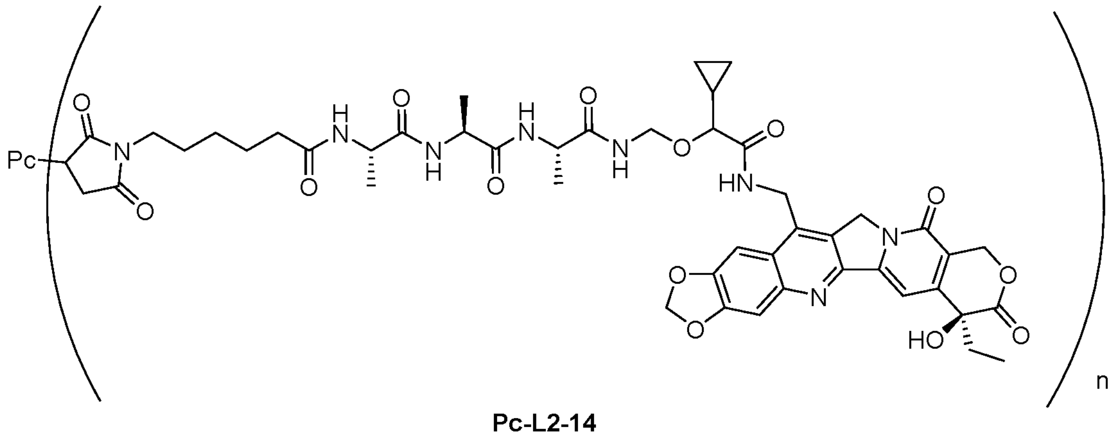

Pc-L2-14

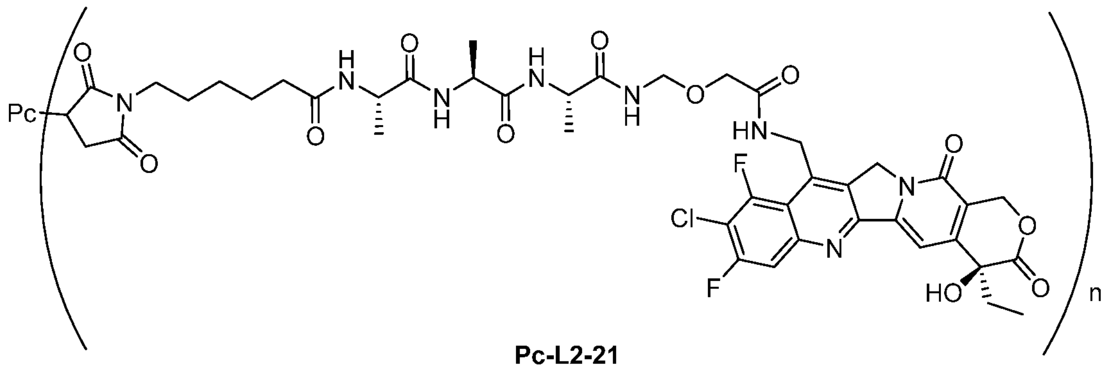

Pc-L2-21

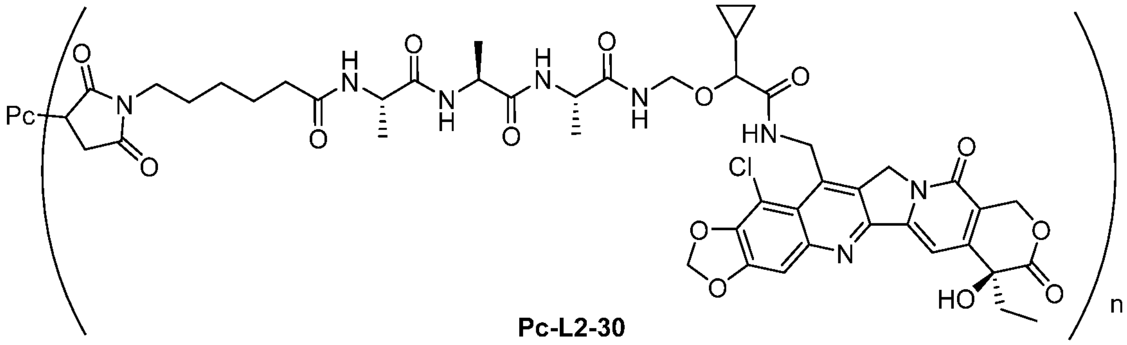

Pc-L2-30

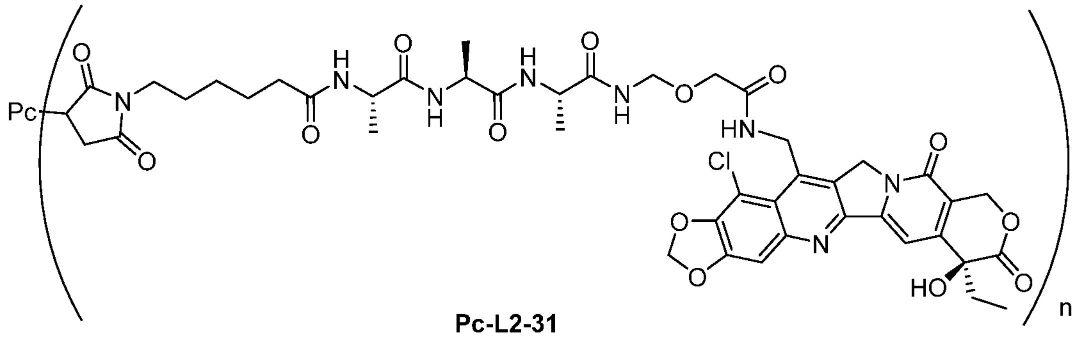

Pc-L2-31

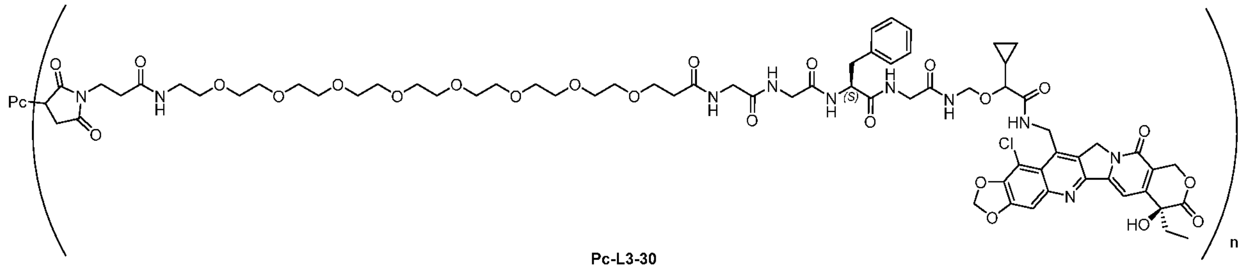

Pc-L3-30

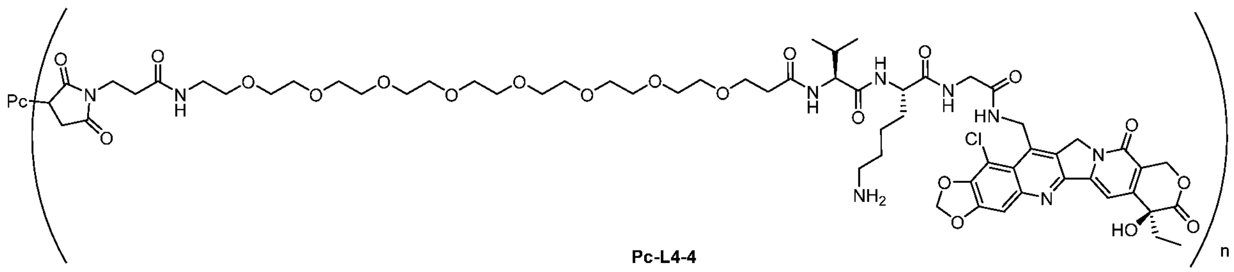

Pc-L4-4

or 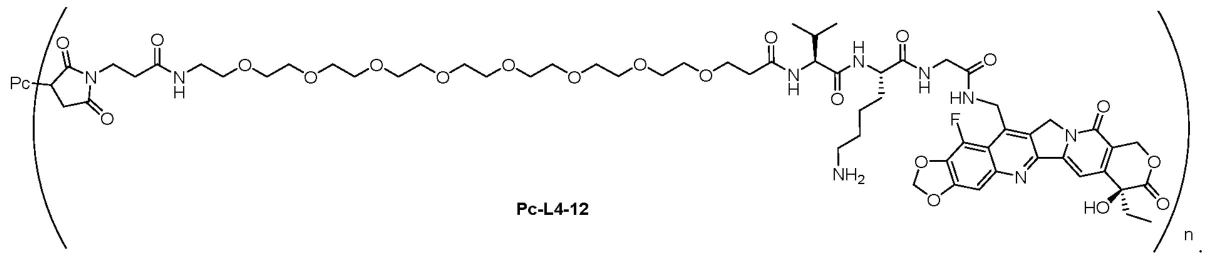

Pc-L4-12

Pc and n are as defined above.

In some embodiments, in the aforementioned ligand-drug conjugate having the general formula of Pc-$(L-D)_n$ or the pharmaceutically acceptable salt thereof, ligand unit Pc may be selected from a polypeptide, an antibody, or an antigen-binding fragment thereof.

In some embodiments, ligand unit Pc can specifically bind to one or more antigens selected from the group consisting of: HER2, p95HER2, HER3, CD3, CD16, ROR1, DLL3, CDH6, CD70, CD5, CD20, BCMA, EGFR, VEGF, and LIV-1.

In some embodiments, the antibody or the antigen-binding fragment thereof is monospecific, bispecific, trispecific, or tetraspecific.

In some embodiments, the antibody is selected from trastuzumab, pertuzumab, or rituximab.

In some embodiments, Pc is an antibody or an antigen-binding fragment thereof that specifically binds to HER2, p95HER2, CDH6, ROR1, or LIV-1; the antibody or the antigen-binding fragment thereof comprises a heavy chain variable region (VH) or/and a light chain variable region (VL).

In some embodiments, (1) the heavy chain variable region comprises HCDR1, HCDR2, and HCDR3 contained in the VH set forth in SEQ ID NO: 1, 3, 19, 21, 37, 46, 54, 56, 71, 80, 82, or 84; or/and the light chain variable region comprises LCDR1, LCDR2, and LCDR3 contained in the VL set forth in SEQ ID NO: 2, 4, 20, 22, 38, 47, 55, 57, 72, 81, 83, or 85; or (2) the heavy chain variable region or/and the light chain variable region comprises an amino acid sequence having at least

80% identity or having at most 3 insertion, deletion, or substitution mutations compared with each CDR of the HCDR1-3 or/and the LCDR1-3 in group (1); further, the at least 80% identity is 85%, 90%, 91%, 92%, 93%, 94%, 95%, 96%, 97%, 98%, 99%, or 100% identity. Optionally, the HCDR1-3 or/and the LCDR1-3 are determined according to the Kabat numbering scheme, the Chothia numbering scheme, or the IMGT numbering scheme.

In some embodiments, the heavy chain variable region comprises HCDR1, HCDR2, and HCDR3, or/and the light chain variable region comprises LCDR1, LCDR2, and LCDR3, wherein the HCDR1-3 or/and the LCDR1-3 are selected from the following:

(1) the HCDR1-3 are SEQ ID NOs: 7-9; or/and the LCDR1-3 are SEQ ID NOs: 10-12;
(2) the HCDR1-3 are SEQ ID NOs: 13-15; or/and the LCDR1-3 are SEQ ID NOs: 16-18;
(3) the HCDR1-3 are SEQ ID NOs: 23-25; or/and the LCDR1-3 are SEQ ID NOs: 26-28;
(4) the HCDR1-3 are SEQ ID NOs: 29-31; or/and the LCDR1-3 are SEQ ID NOs: 32-34;
(5) the HCDR1-3 are SEQ ID NOs: 40-42; or/and the LCDR1-3 are SEQ ID NOs: 43-45;
(6) the HCDR1-3 are SEQ ID NOs: 48-50; or/and the LCDR1-3 are SEQ ID NOs: 51-53;
(7) the HCDR1-3 are SEQ ID NOs: 58-60; or/and the LCDR1-3 are SEQ ID NOs: 61-63;
(8) the HCDR1-3 are SEQ ID NOs: 64-66; or/and the LCDR1-3 are SEQ ID NOs: 67-69;
(9) the HCDR1-3 are SEQ ID NOs: 74-76; or/and the LCDR1-3 are SEQ ID NOs: 77-79;
(10) the HCDR1-3 are SEQ ID NOs: 86-88; or/and the LCDR1-3 are SEQ ID NOs: 89-91;
(11) the HCDR1-3 are SEQ ID NOs: 92-94; or/and the LCDR1-3 are SEQ ID NOs: 95-97;
(12) the HCDR1-3 are SEQ ID NOs: 98-100; or/and the LCDR1-3 are SEQ ID NOs: 101-103; or
(13) the HCDR1-3 or/and the LCDR1-3 have an amino acid sequence having at least 80% identity or having at most 3 insertion, deletion, or substitution mutations compared with each CDR in the HCDR1-3 or/and the LCDR1-3 of any one of groups (1) - (12); preferably, the HCDR1-3 or/and the LCDR1-3 have an amino acid sequence having at least 80% identity compared with each CDR in the HCDR1-3 or/and the LCDR1-3 of any one of groups (1) - (12); further, the at least 80% identity is 85%, 90%, 91%, 92%, 93%, 94%, 95%, 96%, 97%, 98%, 99%, or 100% identity.

In some embodiments, the antibody or the antigen-binding fragment thereof comprises a heavy chain variable region (VH) or/and a light chain variable region (VL), wherein the heavy chain variable region comprises the amino acid sequence set forth in SEQ ID NO: 1, 3, 19, 21, 37, 46, 54, 56, 71, 80, 82, or 84, or/and the light chain variable region comprises the amino acid sequence set forth in SEQ ID NO: 2, 4, 20, 22, 38, 47, 55, 57, 72, 81, 83, or 85; alternatively, the heavy chain variable region or/and the light chain variable region comprises an amino acid sequence having at least 80% identity compared with any one of the heavy chain variable regions or/and the light chain variable regions described above, respectively; further, the at least 80% identity is 85%, 90%, 91%, 92%, 93%, 94%, 95%, 96%, 97%, 98%, 99%, or 100% identity.

In some embodiments, the antibody or the antigen-binding fragment thereof comprises a heavy chain constant region sequence and/or a light chain constant region sequence, the heavy chain constant region and/or the light chain constant region is selected from an intact constant region sequence or a fragment thereof, and the constant region fragment comprises CH1, hinge region, CH2, CH3, or Fc; optionally, the heavy chain constant region is selected from a human or murine IgG1, IgG2, IgG3, or IgG4 constant region, and the light chain constant region is selected from a human or murine kappa constant region or lamda constant region; optionally, the antibody or the antigen-binding fragment thereof comprises an intact heavy chain and light chain, the heavy chain consists of the VH and the heavy chain constant region, the heavy chain constant region has the amino acid sequence set forth in SEQ ID NO: 5, 39, or 73, the light chain consists of the VL and the light chain constant region, and the light chain constant region has the amino acid sequence set forth in SEQ ID NO: 6.

In some embodiments, in the aforementioned ligand-drug conjugate having the general formula of Pc-(L-D)$_n$ or the pharmaceutically acceptable salt thereof, n is selected from real numbers of 1-16, such as real numbers of 2-12, such as real numbers of 4-10, such as real numbers of 5-9, such as real numbers of 6-8.

In some embodiments, n is selected from real numbers of 5-9. For example, n is 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0.

The present disclosure further provides a drug-linker compound having a structural general formula of L'-D or a pharmaceutically acceptable salt thereof, wherein

drug unit D is as defined in any one of the preceding;
L' is selected from

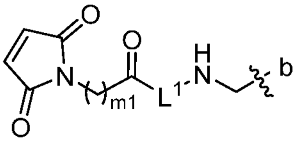

or

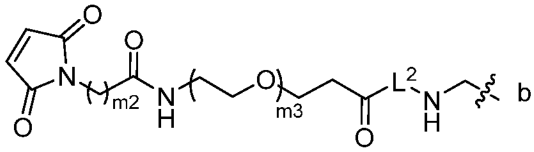

,

the end b of which is covalently linked to drug unit D, and $L^1$, $L^2$, m1, m2, and m3 are as defined in any one of the preceding.

In some embodiments, L' is selected from

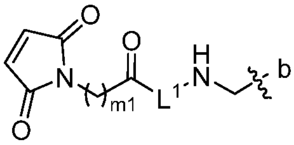

,

the end b of which is covalently linked to drug unit D, m1 is selected from 5, and $L^1$ is selected from Gly-Gly-Phe-Gly tetrapeptide residue or Ala-Ala-Ala tripeptide residue.

In some embodiments, L' is selected from

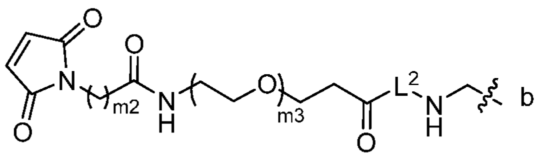

,

the end b of which is covalently linked to drug unit D, m2 is selected from 2, m3 is selected from 8, and $L^2$ is selected from Gly-Gly-Phe-Gly tetrapeptide residue or Val-Lys dipeptide residue.

In some embodiments, L' is selected from one of the following chemical structures:

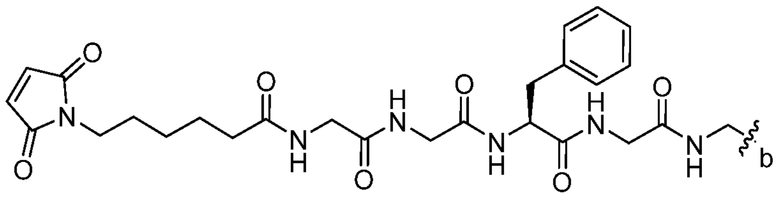

**L'1**

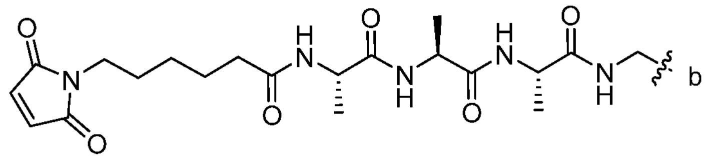

**L'2**

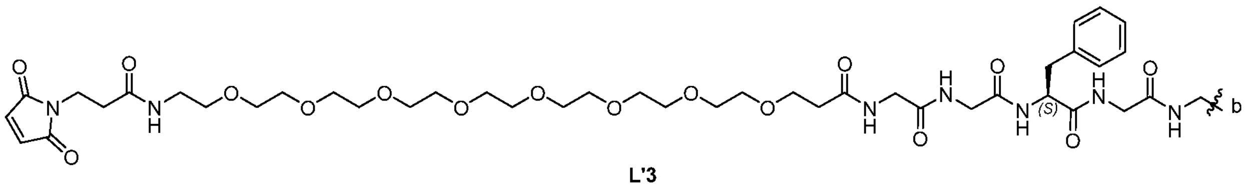

**L'3**

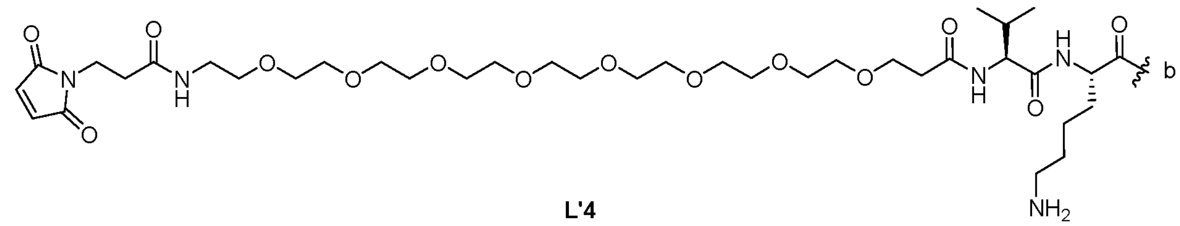

or

L'4

,

the end b of which is covalently linked to drug unit D.

In some embodiments, the drug-linker compound having the general formula of L'-D or the pharmaceutically acceptable salt thereof of the present disclosure is selected from the following compounds or pharmaceutically acceptable salts thereof:

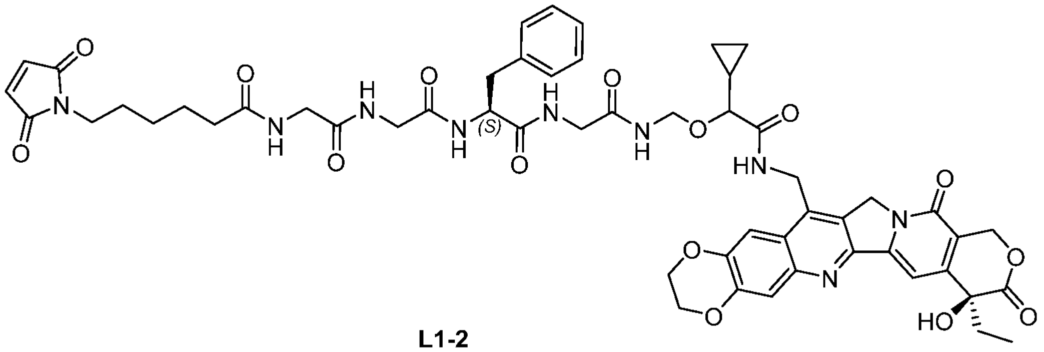

**L1-2**

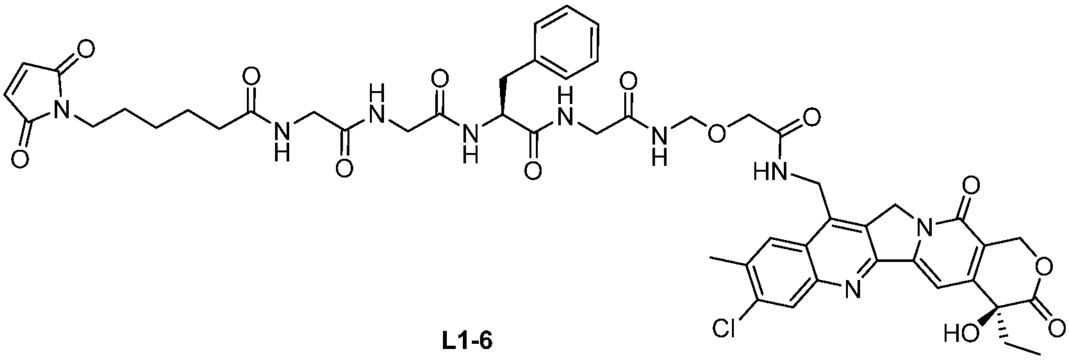

**L1-6**

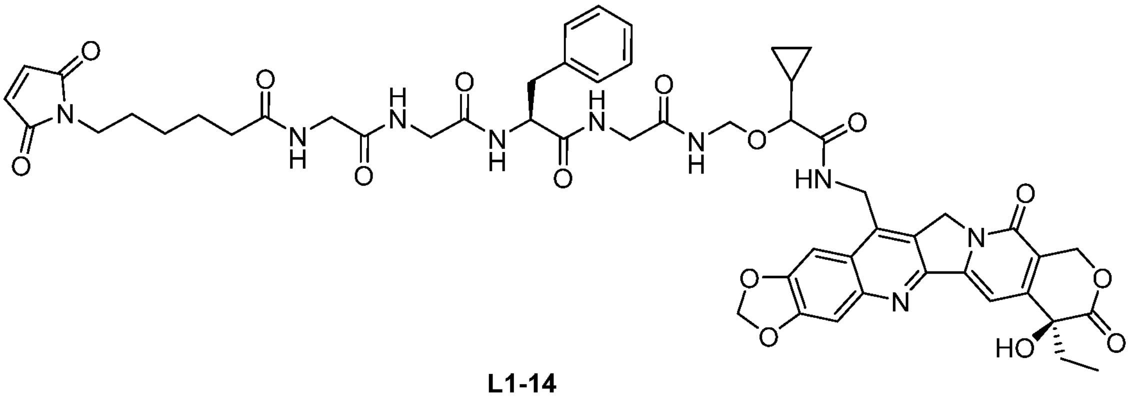

**L1-14**

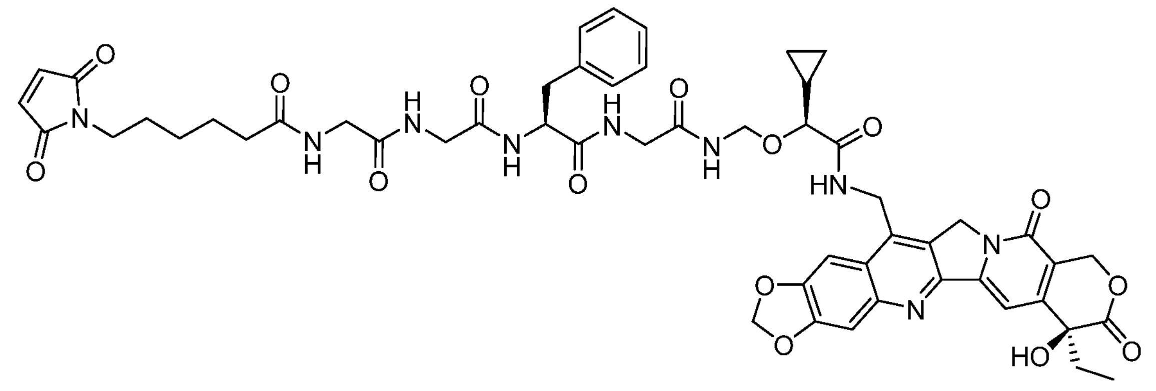

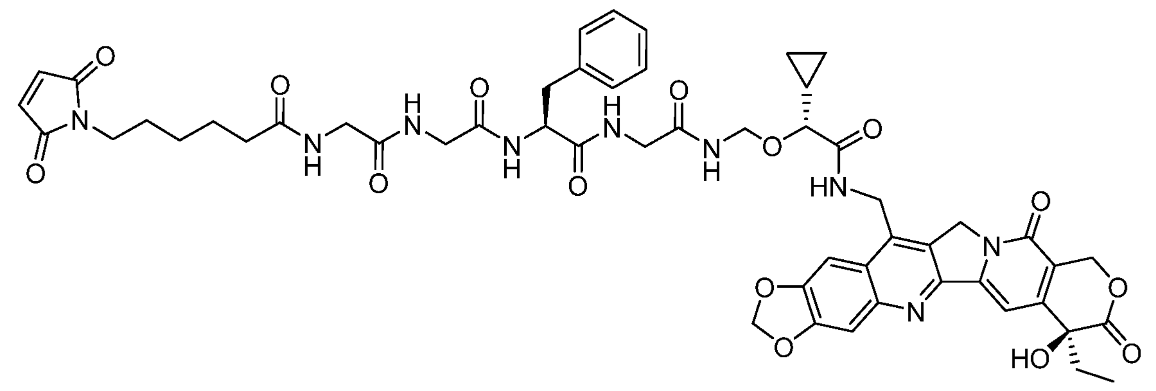

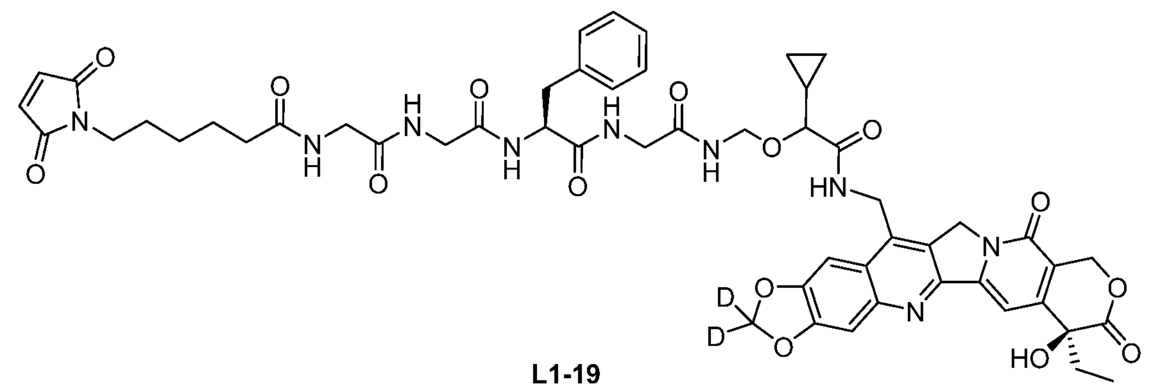

**L1-19**

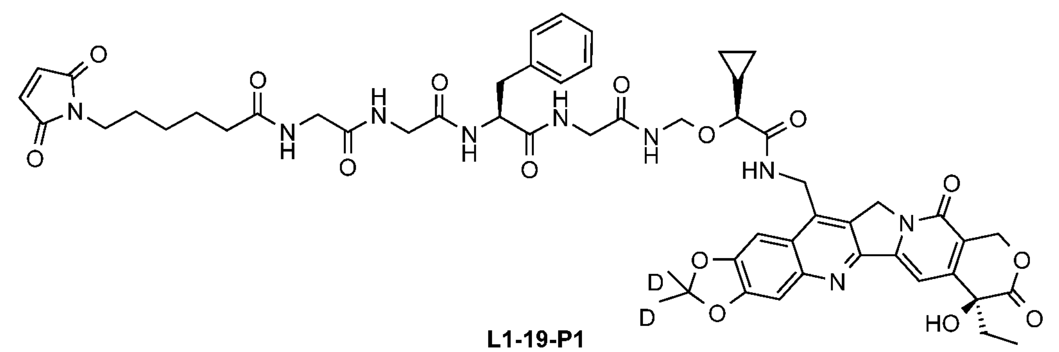

**L1-19-P1**

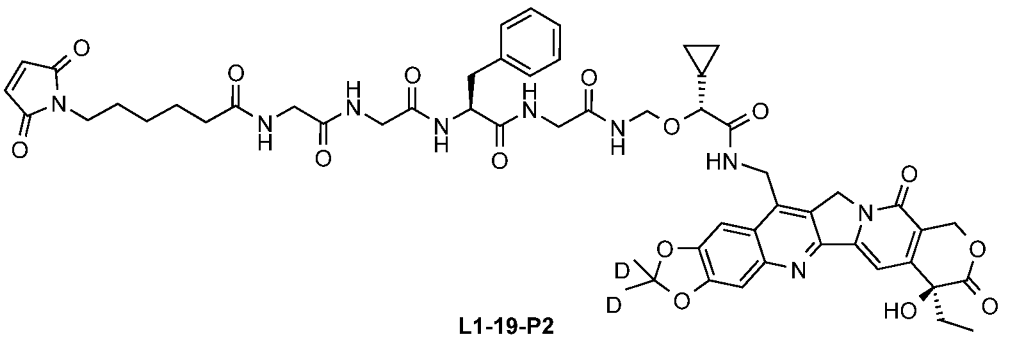

L1-19-P2

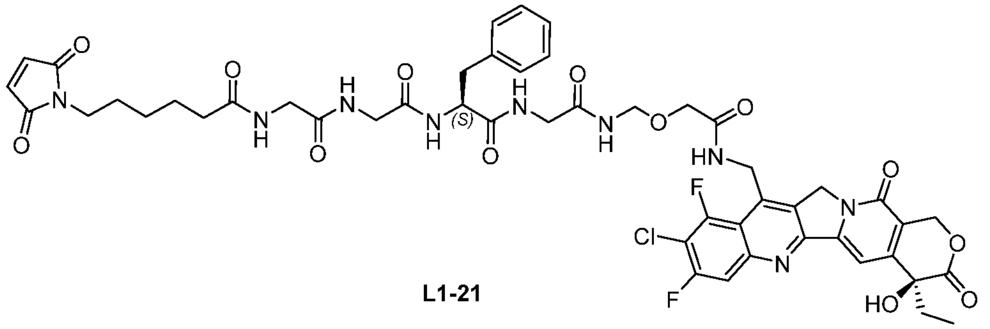

L1-21

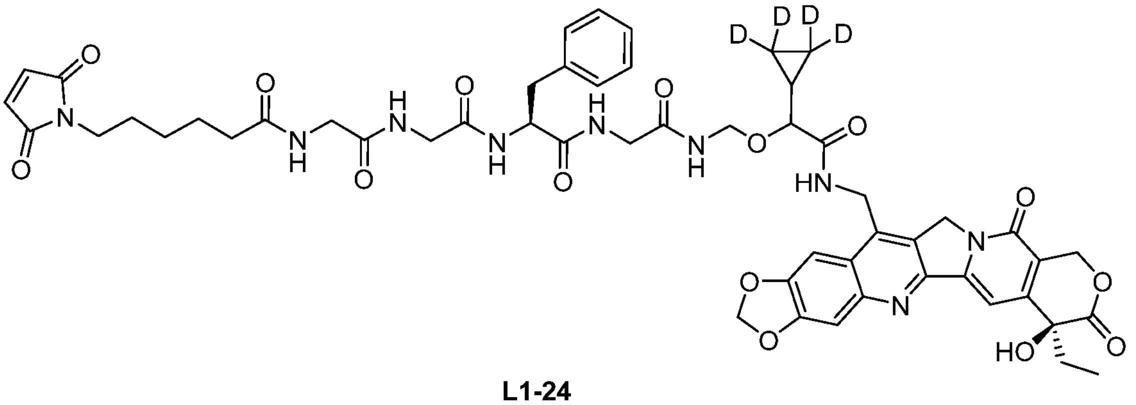

L1-24

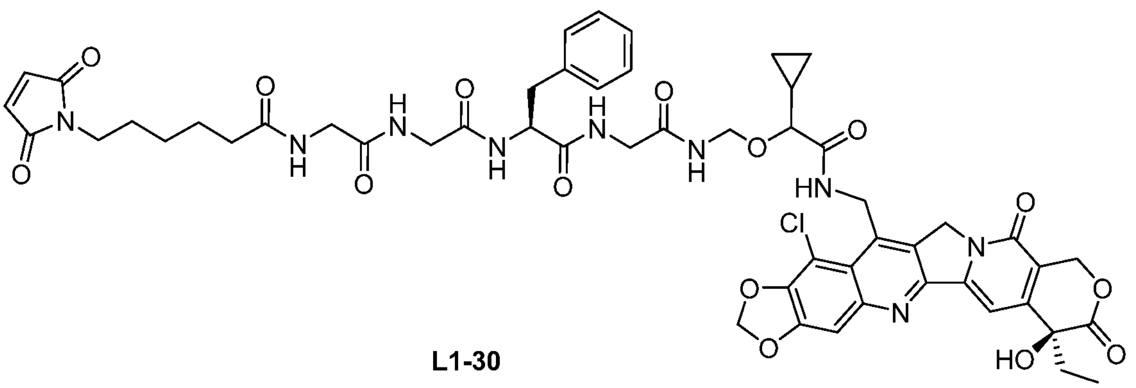

L1-30

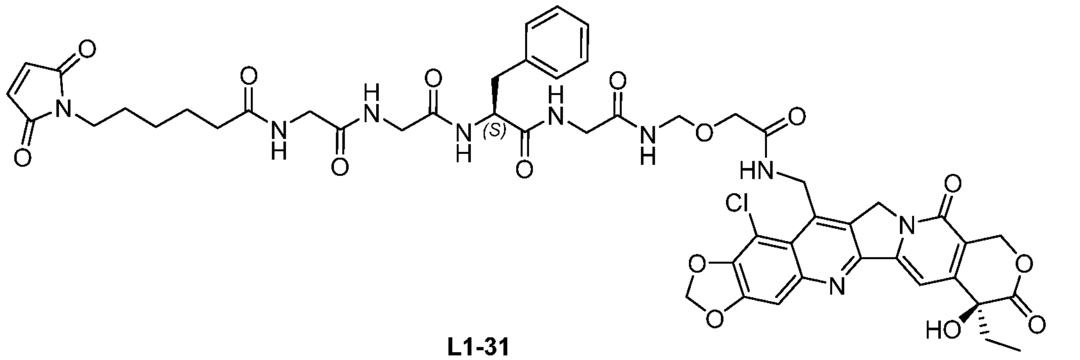

**L1-31**

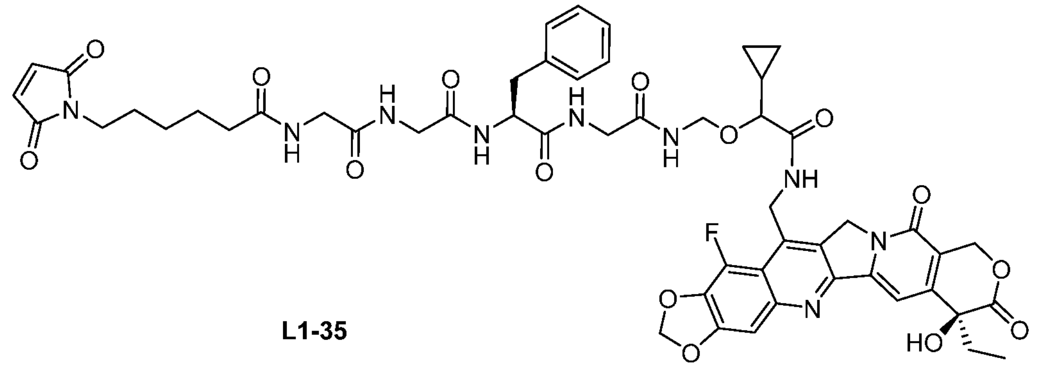

**L1-35**

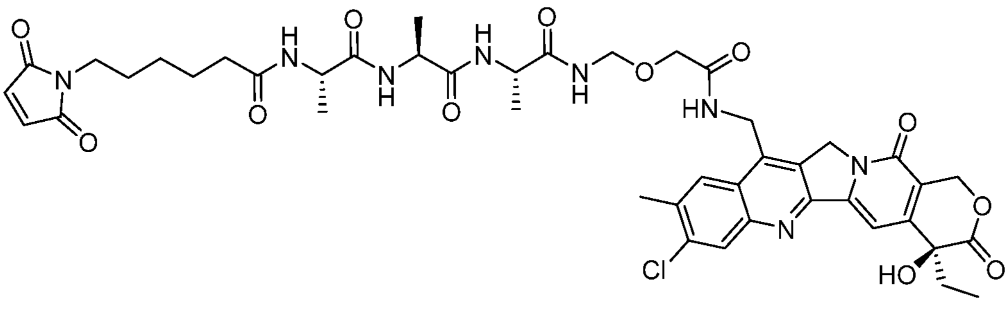

**L2-6**

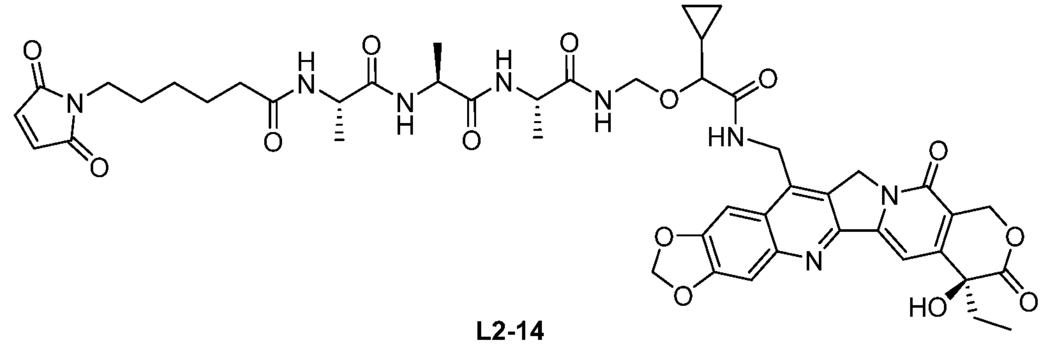

**L2-14**

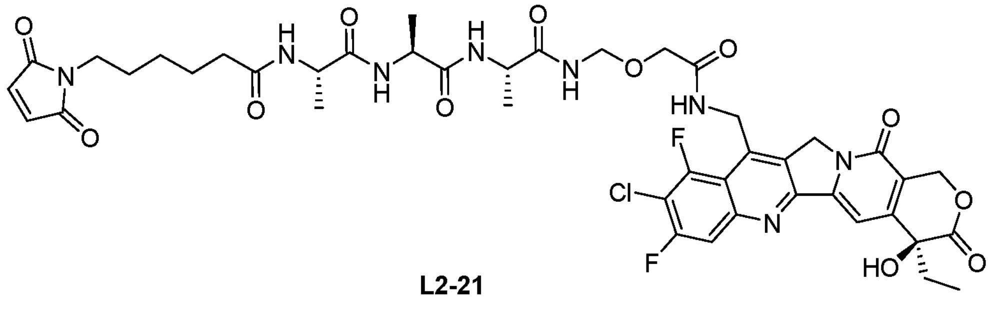

L2-21

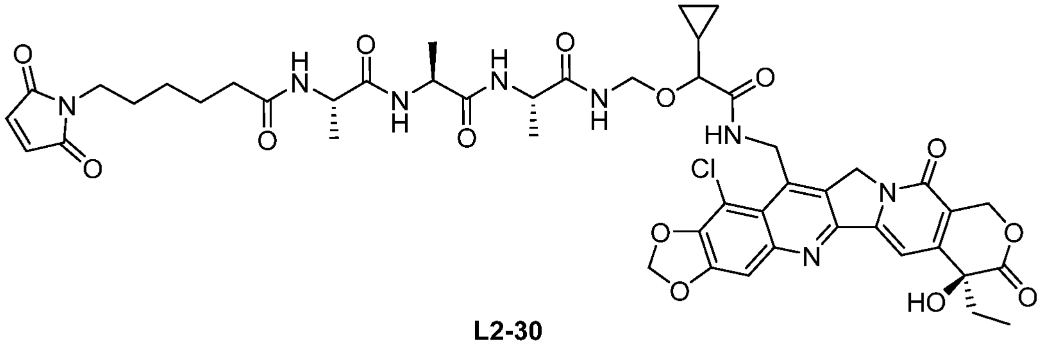

L2-30

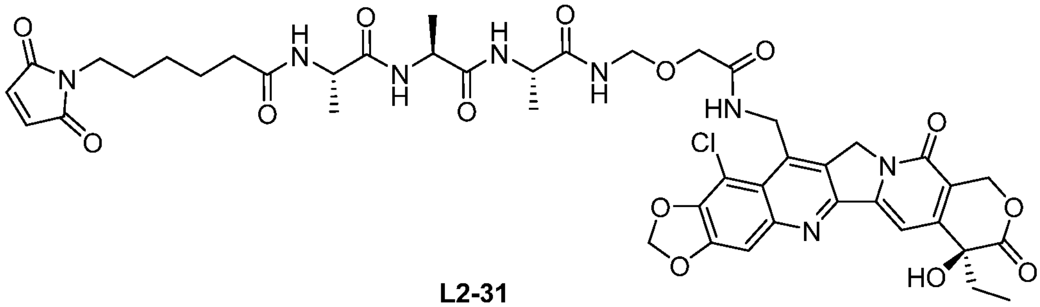

L2-31

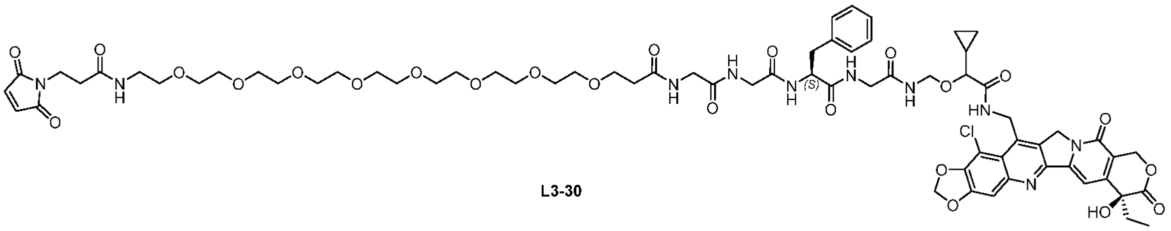

L3-30

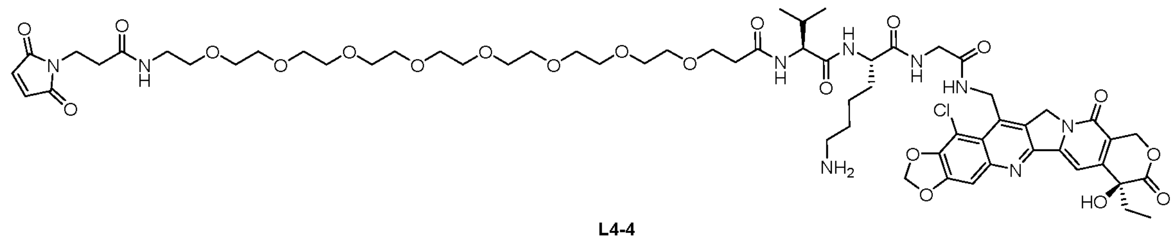

L4-4

or

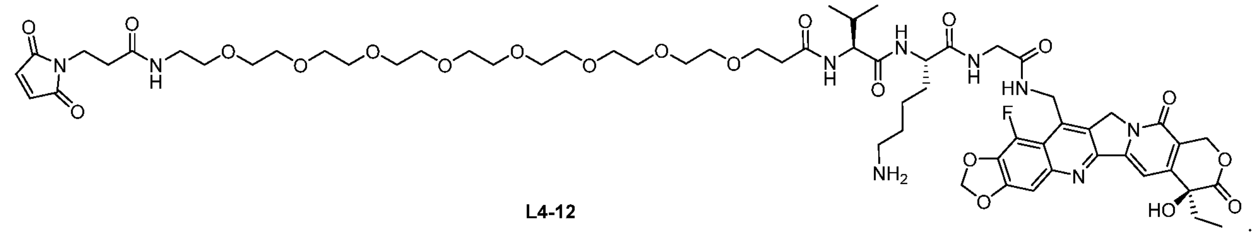

L4-12

The present disclosure further provides a compound of the following formula (D-H) or a pharmaceutically acceptable salt thereof:

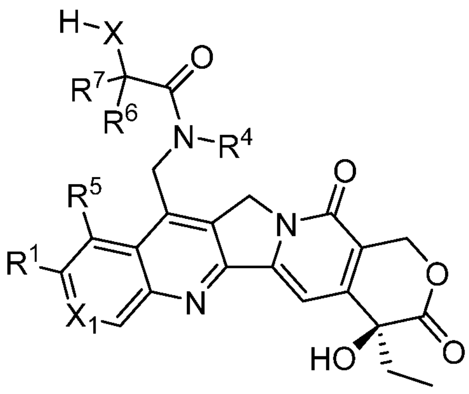

(D-H)

wherein
$R^1$, $X_1$, X, $R^4$, $R^5$, $R^6$, and $R^7$ are as defined in any one of the preceding.

In some embodiments, the compound of formula (D-H) or the pharmaceutically acceptable salt thereof is selected from the following compounds or pharmaceutically acceptable salts thereof:

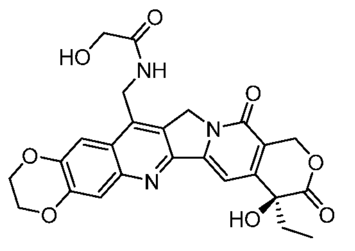

1

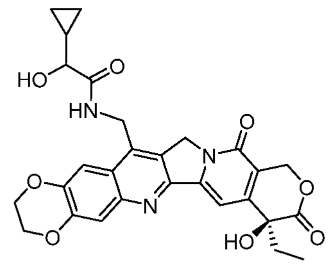

2

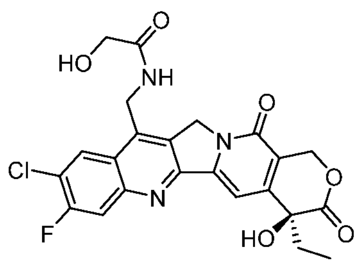

3

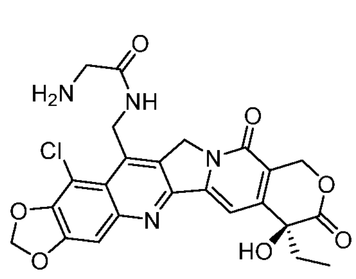

4

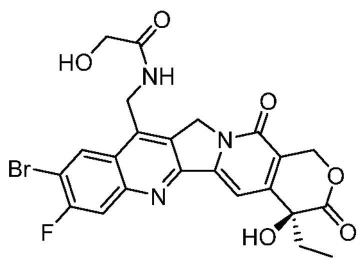

5

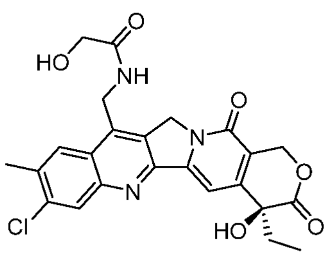

6

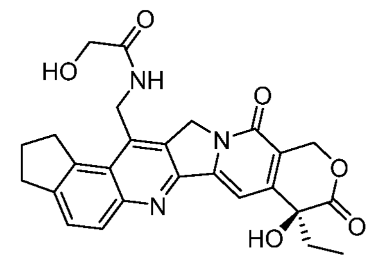
7

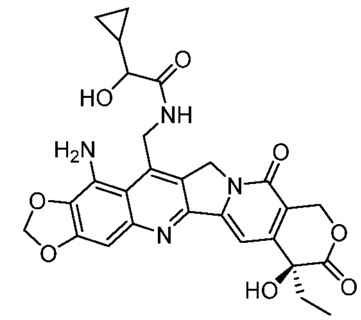
8

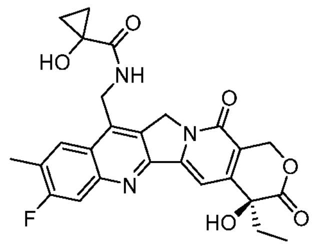
9

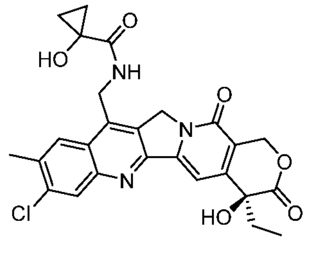
10

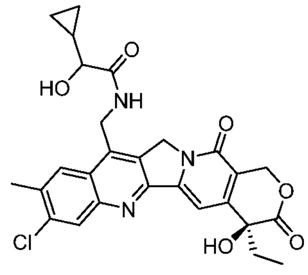
11

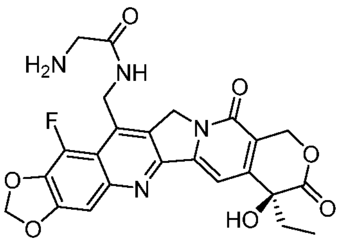
12

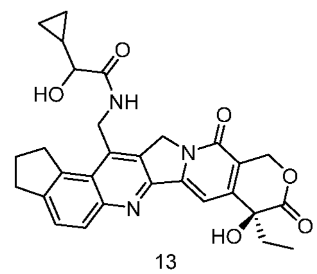
13

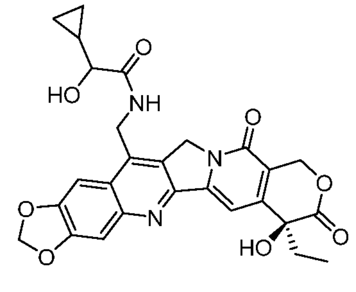
14

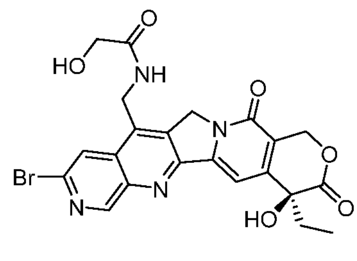
15

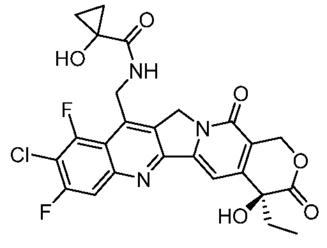
16

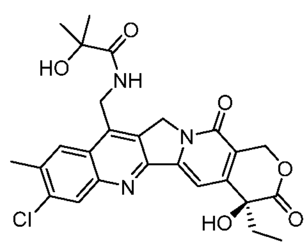
17

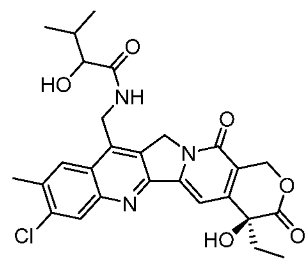
18

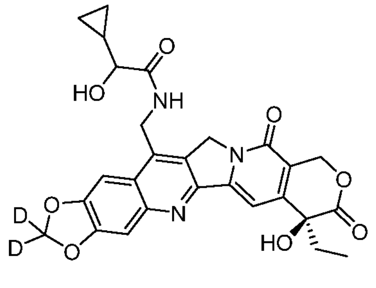
19

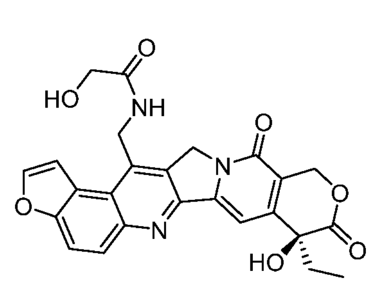
20

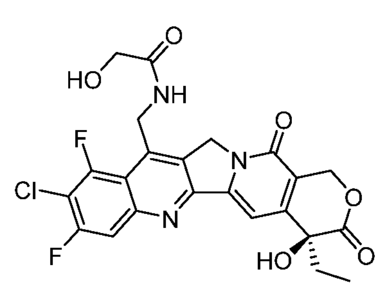
21

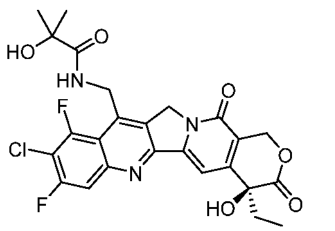
22

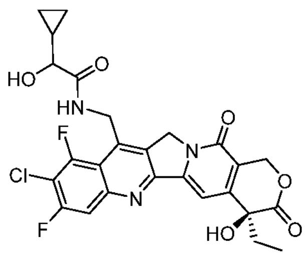
23

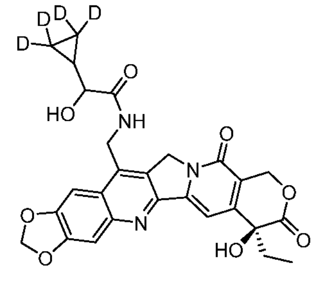
24

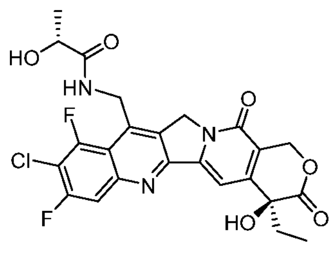
25

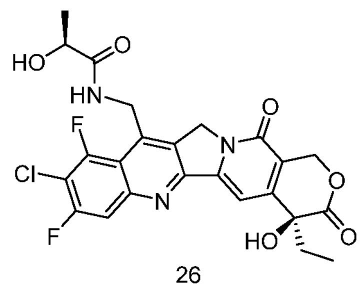
26

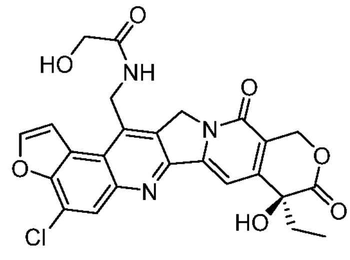
27

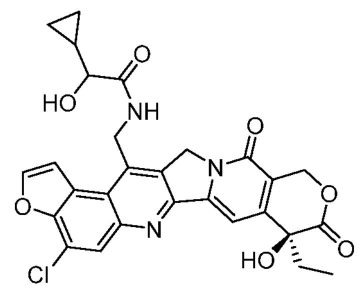
28

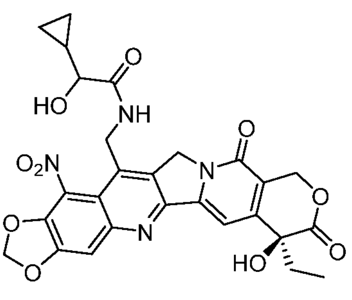
29

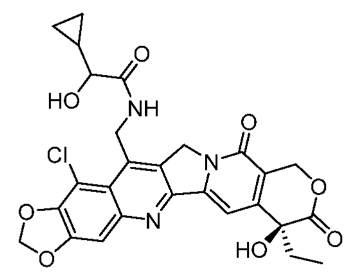
30

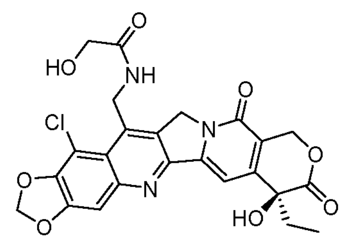
31

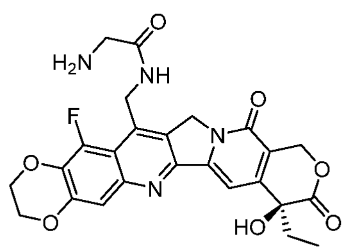
32

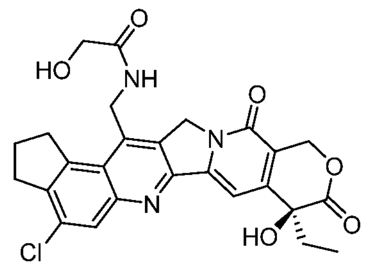
33

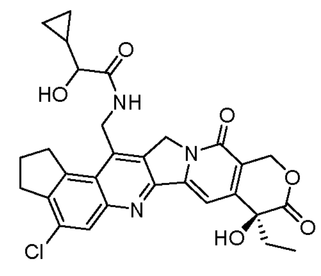
34

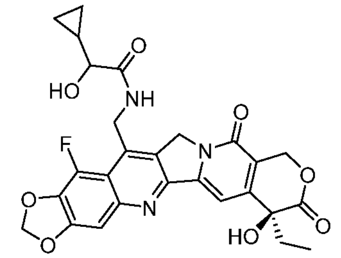
35

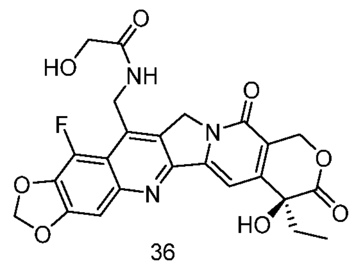
36

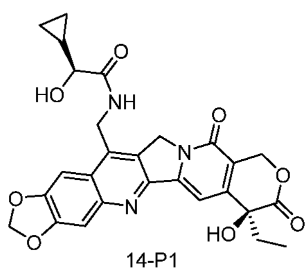
14-P1

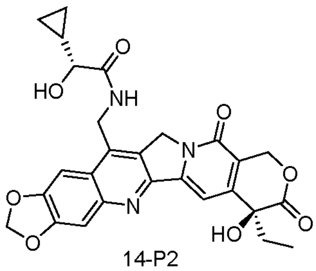
14-P2

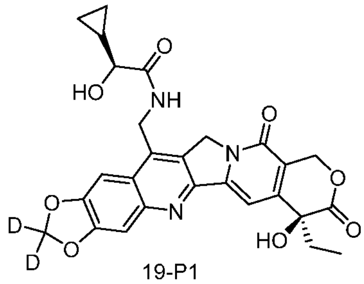
19-P1

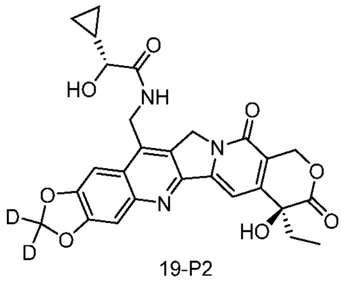
19-P2

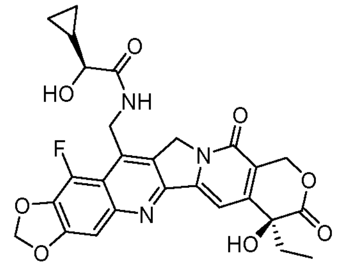

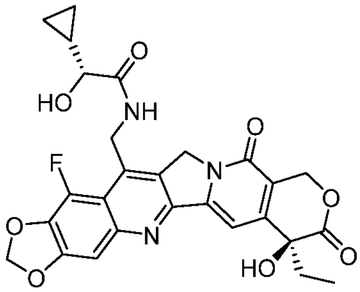

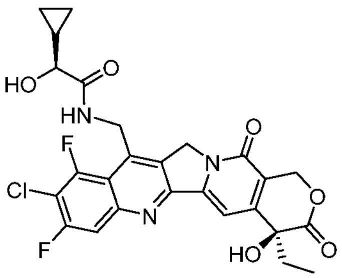

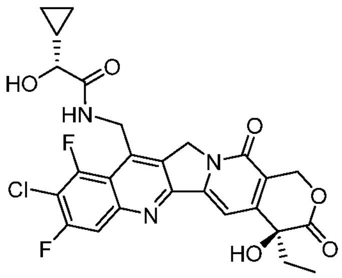

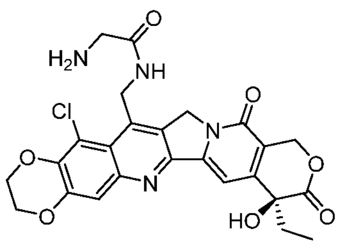

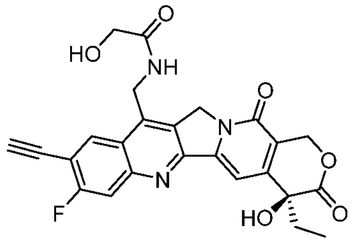
or
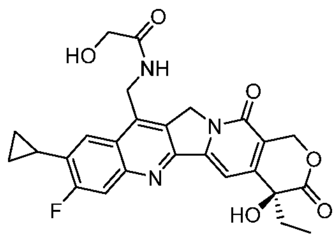
.

In another aspect, the present disclosure provides a pharmaceutical composition comprising the aforementioned ligand-drug conjugate having the general formula of Pc-(L-D)$_n$ or the pharmaceutically acceptable salt thereof of the present disclosure, and a pharmaceutically acceptable excipient.

In another aspect, the present disclosure provides a method for treating a tumor in a mammal, comprising administering to a mammal, preferably a human, in need of such treatment a therapeutically effective amount of the aforementioned ligand-drug conjugate having the general formula of Pc-(L-D)$_n$ or the pharmaceutically acceptable salt thereof, or a pharmaceutical composition thereof.

In another aspect, the present disclosure provides use of the aforementioned ligand-drug conjugate having the general formula of Pc-(L-D)$_n$ or the pharmaceutically acceptable salt thereof, or a pharmaceutical composition thereof in the manufacture of a medicament for treating a tumor.

In another aspect, the present disclosure provides use of the aforementioned ligand-drug conjugate having the general formula of Pc-(L-D)$_n$ or the pharmaceutically acceptable salt thereof, or a pharmaceutical composition thereof in treating a tumor.

In another aspect, the present disclosure provides the aforementioned ligand-drug conjugate having the general formula of Pc-(L-D)$_n$ or the pharmaceutically acceptable salt thereof, or a pharmaceutical composition thereof for use in treating a tumor.

In another aspect, the present disclosure provides a pharmaceutical composition comprising the aforementioned compound of the following formula (D-H) or the pharmaceutically acceptable salt thereof of the present disclosure,

and a pharmaceutically acceptable excipient.

In another aspect, the present disclosure provides a method for treating a tumor in a mammal, comprising administering to a mammal, preferably a human, in need of such treatment a therapeutically effective amount of the aforementioned compound of the following formula (D-H) or the pharmaceutically acceptable salt thereof, or a pharmaceutical composition thereof.

In another aspect, the present disclosure provides use of the aforementioned compound of the following formula (D-H) or the pharmaceutically acceptable salt thereof, or a pharmaceutical composition thereof in the manufacture of a medicament for treating a tumor.

In another aspect, the present disclosure provides use of the aforementioned compound of the following formula (D-H) or the pharmaceutically acceptable salt thereof, or a pharmaceutical composition thereof in treating a tumor.

In another aspect, the present disclosure provides the aforementioned compound of the following formula (D-H) or the pharmaceutically acceptable salt thereof, or a pharmaceutical composition thereof for use in treating a tumor.

In another aspect, the present disclosure provides a method for preparing the ligand-drug conjugate having the general formula of $Pc\text{-}(L\text{-}D)_n$ or the pharmaceutically acceptable salt thereof, comprising the step of conjugating the aforementioned drug-linker compound having the general formula of L'-D of the present disclosure to the aforementioned ligand, wherein optionally, the ligand is an antibody or an antigen-binding fragment thereof.

In another aspect, the present disclosure provides a method for preparing the ligand-drug conjugate having the general formula of $Pc\text{-}(L\text{-}D)_n$ or the pharmaceutically acceptable salt thereof, comprising the step of linking the aforementioned drug unit D to the aforementioned ligand unit Pc of the present disclosure, optionally via the aforementioned linker unit L, wherein optionally, the ligand is an antibody or an antigen-binding fragment thereof.

In another aspect, the present disclosure provides use of the aforementioned compound of formula (D-H) or the pharmaceutically acceptable salt thereof in the manufacture of the aforementioned ligand-drug conjugate having the general formula of $Pc\text{-}(L\text{-}D)_n$ or the pharmaceutically acceptable salt thereof, and/or use of the aforementioned compound of formula (D-H) or the pharmaceutically acceptable salt thereof in the manufacture of the aforementioned drug-linker compound having the general formula of L'-D or the pharmaceutically acceptable salt thereof.

The ligand-drug conjugate provided by the present disclosure has significant anti-inflammatory activity and/or reduced toxic and side effects.

**BRIEF DESCRIPTION OF THE DRAWINGS**

FIG. 1 shows the results of X-ray single-crystal diffraction analysis of compound 19-P1.
FIG. 2 shows the tumor growth curves of the OVCAR3 subcutaneous tumor model.
FIG. 3 shows the tumor growth curves of the OVCAR3 subcutaneous tumor model.
FIG. 4 shows the tumor growth curves of the OVCAR3 subcutaneous tumor model.
FIG. 5 shows the mouse body weight change curves of the OVCAR3 subcutaneous tumor model.
FIG. 6 shows the tumor growth curves of the H838 subcutaneous tumor model.
FIG. 7 shows the mouse body weight change curves of the H838 subcutaneous tumor model.

TERMINOLOGY AND DEFINITIONS

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered uncertain or unclear, but construed according to its common meaning in the field. When referring to a trade name, it is intended to refer to its corresponding commercial product or its active ingredient.

The term "ligand" refers to a macromolecular compound capable of recognizing and binding to an antigen or receptor associated with a target cell. The role of the ligand is to present the drug to a target cell population to which the ligand binds, and the ligands include, but are not limited to, protein hormones, lectins, growth factors, antibodies, or other molecules capable of binding to the cells. In an embodiment of the present disclosure, the ligand or ligand unit is denoted as Pc, and the ligand may form a linking bond with the linker unit through a heteroatom on the ligand. In some embodiments of the present disclosure, the ligand is selected from an antibody or an antigen-binding fragment, wherein the antibody is selected from a chimeric antibody, a humanized antibody, a fully human antibody, or a murine antibody; in some embodiments of the present disclosure, the antibody is a monoclonal antibody.

The term "linker" or "linker unit" refers to a chemical structural fragment or chemical bond, which is linked to a ligand at one end and linked to a drug at the other end.

The term "drug" refers to a small molecule compound that has biological activity in an organism. In some embodiments of the present disclosure, a drug is a glucocorticoid receptor agonist with anti-inflammatory function or its corresponding phosphate molecule.

The term "ligand-drug conjugate" means that a ligand is linked to a biologically active drug by a stable linker unit. In some embodiments of the present disclosure, a "ligand-drug conjugate" is an antibody-drug conjugate (ADC), which means that a monoclonal antibody or an antibody fragment is linked to a biologically active drug via a stable linker unit.

The term "DAR" or "drug/antibody ratio" refers to the average number of small-molecule glucocorticoid receptor agonist drugs linked to each antibody molecule. In the antibody-drug conjugates of the present disclosure, the DAR is defined by the variable "n", which may be either an integer or a decimal.

The term "antibody" is used in its broadest sense and refers to a polypeptide or a combination of polypeptides that comprises sufficient sequence from an immunoglobulin heavy chain variable region and/or sufficient sequence from an immunoglobulin light chain variable region to be capable of specifically binding to an antigen. "Antibody" herein encompasses various forms and various structures as long as they exhibit the desired antigen-binding activity. "Antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20: 639-654 (2004). Such scaffolds may also include non-antibody derived scaffolds, such as scaffold proteins known in the art to be useful for grafting CDRs, including, but not limited to tenascin, fibronectin, peptide aptamers, and the like.

"Antibody" herein includes a typical "tetrabody", which belongs to an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the antibody is of IgE isotype, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are linked to each other and to the light chains through disulfide bonds to form a Y-shaped structure. The heavy chain constant regions of immunoglobulins differ in their amino acid composition and arrangement, and thus in their antigenicity. Accordingly, "immunoglobulin" herein can be divided into five classes, or isotypes of immunoglobulins, i.e., IgM, IgD, IgG, IgA, and IgE, with their corresponding heavy chains being $\mu$, $\delta$, $\gamma$, $\alpha$, and $\varepsilon$ chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. The light chains can be divided into $\kappa$ or $\lambda$ chains according to the differences in the constant regions. Each of the five classes of Ig may have a $\kappa$ chain or a $\lambda$ chain.

"Antibody" herein also includes antibodies that do not comprise a light chain, e.g., heavy chain antibodies (HCAbs) produced by *Camelus dromedarius, Camelus bactrianus, Lama glama, Lama guanicoe, Vicugna pacos,* and the like, as well as immunoglobulin new antigen receptors (IgNARs) found in *Chondrichthyes,* e.g., shark.

"Antibody" herein may be derived from any animal, including, but not limited to, human and non-human animals, wherein the non-human animals may be selected from primates, mammals, rodents, and vertebrates, such as Camelidae species, *Lama glama, Lama guanicoe, Vicugna pacos,* sheep, rabbits, mice, rats, or *Chondrichthyes* (e.g., shark).

"Antibody" herein includes but is not limited to, monoclonal antibodies, polyclonal antibodies, monospecific antibodies, multispecific antibodies (e.g., bispecific antibodies), monovalent antibodies, multivalent antibodies, intact antibodies, fragments of an intact antibody, naked antibodies, conjugated antibodies, chimeric antibodies, humanized antibodies, or fully human antibodies.

The term "monoclonal antibody" refers to an antibody obtained from a population of substantially homogeneous antibodies, that is, the individual antibodies constituting the population are identical and/or bind to the same epitope, except for possible variants (e.g., containing naturally occurring mutations or arising during the production of the formulation, such variants typically being present in minor amounts). In contrast to polyclonal antibody formulations, which generally comprise different antibodies directed against different determinants (epitopes), each monoclonal antibody in a monoclonal antibody formulation is directed against a single determinant on the antigen. The modifier "monoclonal" herein is not to be construed as requiring the production of the antibody or the antigen-binding molecule by any particular method. For example, monoclonal antibodies can be prepared by a variety of techniques, including (but not limited to) a hybridoma technique, a recombinant DNA method, a phage library display technique, methods that utilize transgenic animals containing all or part of human immunoglobulin loci, and other methods known in the art.

The term "natural antibody" refers to an antibody that is produced and paired by the immune system of a multicellular organism. The term "engineered antibody" herein refers to a non-natural antibody obtained by genetic engineering, antibody engineering, and the like. Illustratively, "engineered antibody" includes humanized antibodies, small

molecule antibodies (e.g., scFv and the like), bispecific antibodies, and the like.

The term "monospecific" means having one or more binding sites, each of which binds to the same epitope of the same antigen.

The term "multispecific antibody" means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

The term "valent" refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

The "full-length antibody", "complete antibody", and "intact antibody" are used interchangeably herein and refer to an antibody having a structure substantially similar to that of a natural antibody.

"Antigen-binding fragment" and "antibody fragment" herein are used interchangeably and do not have the entire structure of an intact antibody, but comprise only a portion of the intact antibody or a variant of the portion that has the ability to bind to an antigen. "Antigen-binding fragment" or "antibody fragment" herein includes but is not limited to, a Fab, a Fab', a Fab'-SH, a F(ab')2, an Fv, a VHH, and an scFv.

An intact antibody is digested by papain to produce two identical antigen-binding fragments, called "Fab" fragments, each of which contains a heavy chain variable domain and a light chain variable domain, as well as a light chain constant domain and a first heavy chain constant domain (CH1). Thus, the term "Fab fragment" herein refers to an antibody fragment comprising a light chain fragment comprising the VL domain and the constant domain (CL) of a light chain, and the VH domain and the first constant domain (CH1) of a heavy chain. A Fab' fragment differs from the Fab fragment by the addition of a few residues (including one or more cysteines from an antibody hinge region) at the carboxyl terminus of the heavy chain CH1 domain. Fab'-SH is a Fab' fragment in which the cysteine residue in the constant domain carries a free thiol group. Pepsin treatment produces an F(ab')2 fragment having two antigen-binding sites (two Fab fragments) and a portion of the Fc region. An "Fv fragment" is the smallest fragment produced by IgG and IgM, comprising the intact antigen-binding site. An Fv fragment has the same binding properties and similar three-dimensional binding properties as Fab. The VH and VL chains of the Fv fragment are bound together by a non-covalent interaction.

The term "scFv" (single-chain variable fragment) refers to a single polypeptide chain comprising VL and VH domains, wherein the VL and VH are linked through a linker (see, e.g., Bird et al., Science 242: 423-426 (1988); Huston et al., Proc. Natl. Acad. Sci. USA 85: 5879-5883 (1988); and Pluckthun, The Pharmacology of monoclonal Antibodies, Vol. 113, Roseburg and Moore Ed., Springer-Verlag, New York, pp 269-315 (1994)). Such scFv molecules may have a general structure: NH2-VL-linker-VH-COOH or NH2-VH-linker-VL-COOH. An appropriate linker in the prior art consists of GGGGS amino acid sequence repeats or a variant thereof. For example, a linker having the amino acid sequence (GGGGS)4 can be used, and variants thereof can also be used (Holliger et al. (1993), Proc. Natl. Acad. Sci. USA 90: 6444-6448). Other linkers that can be used in the present disclosure are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol. In some cases, there may also be disulfide bonds between the VH and VL of the scFv, forming a disulfide-linked Fv (dsFv).

The term "diabody" refers to an antibody having VH and VL domains that are expressed on a single polypeptide chain, but using a linker that is too short to allow the pairing of the two domains on the same chain, thereby forcing the domains to pair with the complementary domains of the other chain and generating two antigen-binding sites (see, e.g., Holliger P. et al., Proc. Natl. Acad. Sci. USA 90: 6444-6448 (1993), and Poljak R.J. et al., Structure 2: 1121-1123 (1994)).

The term "chimeric antibody" refers to an antibody in which a portion of the light chain or/and heavy chain is derived from one antibody (which may be derived from a particular species or belong to a particular antibody class or subclass) and another portion of the light chain or/and heavy chain is derived from another antibody (which may be derived from the same or a different species or belong to the same or a different antibody class or subclass), but which nevertheless retains binding activity to a target antigen (U.S.P 4,816,567 to Cabilly et al.; Morrison et al., Proc. Natl. Acad. Sci. USA, 81: 6851 6855(1984)). For example, the term "chimeric antibody" can include an antibody (e.g., a human-murine chimeric antibody) in which the heavy and light chain variable regions of the antibody are derived from a first antibody (e.g., a murine antibody) and the heavy and light chain constant regions of the antibody are derived from a second antibody (e.g., a human antibody).

The term "humanized antibody" refers to a genetically engineered non-human antibody that has an amino acid sequence modified to increase the homology to the sequence of a human antibody. Generally, all or part of the CDRs of a humanized antibody is derived from a non-human antibody (donor antibody), and all or part of the non-CDRs (e.g., FRs and/or constant regions in variable regions) is derived from a human immunoglobulin (receptor antibody). The humanized antibody generally retains or partially retains the desired properties of the donor antibody, including, but not limited to, antigen specificity, affinity, reactivity, the ability to increase the activity of immune cells, the ability to enhance immune response, and the like.

The term "fully human antibody" refers to an antibody having variable regions in which both the FRs and CDRs are derived from human germline immunoglobulin sequences. Furthermore, if the antibody comprises constant regions, the constant regions are also derived from human germline immunoglobulin sequences. The "fully human antibody" herein may include amino acid residues that are not encoded by human germline immunoglobulin sequences (e.g., mutations introduced by random or site-specific mutagenesis *in vitro* or by somatic mutations *in vivo*). However, "fully human antibody" herein does not include antibodies in which CDR sequences derived from the germline of another mammalian species (e.g., mouse) have been grafted onto human framework sequences. The term "variable region" refers to a region contained in a heavy or light chain of an antibody enabling the binding of the antibody to an antigen. "Heavy chain variable region" is used interchangeably with "VH" and "HCVR", and "light chain variable region" is used interchangeably with "VL" and "LCVR". Heavy and light chain variable domains (VH and VL, respectively) of natural antibodies generally have similar structures, each of which contains four conservative framework regions (FRs) and three hypervariable regions (HVRs). See, e.g., Kindt et al., Kuby Immunology, 6th ed., W. H. Freeman and Co., p.91 (2007). A single VH or VL domain may be sufficient to provide antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region because it is precisely complementary to an epitope in a spatial structure, wherein the heavy chain variable region CDR may be abbreviated as HCDR and the light chain variable region CDR may be abbreviated as LCDR. The term "framework region" or "FR region" herein is used interchangeably and refers to those amino acid residues of an antibody heavy chain variable region or light chain variable region, other than CDRs. Generally, a typical antibody variable region consists of 4 FRs and 3 CDRs in the following order: FR1-CDR1-FR2-CDR2-FR3-CDR3-FR4.

"CDR" herein may be labeled and defined in a manner well known in the art, including, but not limited to, Kabat numbering scheme, Chothia numbering scheme, or IMGT numbering scheme; the tool sites used include, but are not limited to, AbRSA site (http://cao.labshare.cn/AbRSA/cdrs.php), abYsis site (www.abysis.org/abysis/sequence_input/-key_annotation/key_annotation.cgi), and IMGT site (http://www.imgt.org/3Dstructure-DB/cgi/DomainGapAlign.cgi#results). The CDR herein includes overlaps and subsets of amino acid residues defined in different ways.

The term "heavy chain constant region" used herein refers to the carboxyl-terminal portion of an antibody heavy chain that is not directly involved in the binding of the antibody to an antigen, but exhibits effector functions, such as interaction with an Fc receptor, which has a more conserved amino acid sequence relative to the variable domain of the antibody. The "heavy chain constant region" at least comprises: a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region, while the "heavy chain constant region fragment" includes only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; and when the antibody is a heavy chain antibody, it does not comprise a CH1 domain. Illustratively, a typical "heavy chain constant region fragment" may be selected from CH1, Fc, and CH3 domains.

The term "light chain constant region" herein refers to the carboxyl-terminal portion of an antibody light chain that is not directly involved in the binding of the antibody to an antigen. The light chain constant region may be selected from a constant $\kappa$ domain and a constant $\lambda$ domain.

The term "Fc" herein refers to the carboxyl-terminal portion of an antibody that is formed by the hydrolysis of an intact antibody by papain, which typically comprises the CH3 and CH2 domains of the antibody. The Fc region includes, for example, an Fc region of native sequences, a recombinant Fc region, and a variant Fc region. Although the boundaries of the Fc region of an immunoglobulin heavy chain may vary slightly, the human IgG heavy chain Fc region is generally defined to stretch from an amino acid residue at position Cys226, or from Pro230, to the carboxyl terminus thereof. The C-terminal lysine of the Fc region (residue 447 according to the Kabat numbering scheme) may be removed, for example, during production or purification of the antibody, or by recombinant engineering of the nucleic acid encoding the heavy chain of the antibody, and thus, the Fc region may or may not include Lys447.

The term "identity" herein can be obtained by calculating as follows: to determine the percent "identity" of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the

sequences. For example, the percent identity between two amino acid sequences is determined with the Needlema and Wunsch algorithm ((1970) J. Mol. Biol., 48: 444-453; available at www.gcg.com) which has been integrated into the GAP program of the GCG software package, using the Blossum 62 matrix or PAM250 matrix and gap weight of 16, 14, 12, 10, 8, 6, or 4 and length weight of 1, 2, 3, 4, 5, or 6. For another example, the percent identity between two nucleotide acid sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossum62 scoring matrix with a gap penalty of 12, a gap extension penalty of 4, and a frameshift gap penalty of 5.

"n is a real number of 1-16" herein means that n is any real number greater than or equal to 1 and less than or equal to 16.

Herein,

"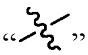"

represents a connection site.

The illustration of the pure compounds of the racemate or the enantiomer herein is from Maehr, J. Chem. Ed. 1985, 62: 114-120. Unless otherwise stated, the absolute configuration of a stereogenic center is represented by a wedged bond and a wedged dashed bond ( 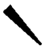 and 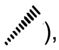 ), and the relative configuration of a stereogenic center (e.g., *cis-* or trans-configuration of alicyclic compounds) is represented by a black solid bond and a dashed bond ( 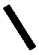 and 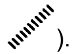 ).

The term "tautomer" refers to functional isomers resulting from the rapid movement of an atom in a molecule between two positions. The compounds of the present disclosure may exhibit the tautomerism. Tautomeric compounds may exist in two or more interconvertible forms. Tautomers generally exist in an equilibrium form. Trying to separate a single tautomer usually leads to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. In the present disclosure, all tautomeric forms of the compound are included.

The term "stereoisomer" refers to isomers resulting from different spatial arrangements of atoms in a molecule, including *cis-trans* isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may exist in the form of a particular geometric isomer or stereoisomer. The form of a particular geometric isomer or stereoisomer may be *cis* and *trans* isomers, E and Z geometric isomers, (-)- and (+)- enantiomers, (R)- and (S)-enantiomers, diastereoisomers, (D)-isomers, (L)-isomers, and racemic mixtures or other mixtures thereof, such as an enantiomer or diastereoisomer enriched mixture, and all of the above isomers as well as mixtures thereof are encompassed within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof referred to in the substituents are also encompassed within the definition scope of the compound of the present disclosure. The compound with asymmetric atoms of the present disclosure can be separated in an optically active pure form or in a racemic form. The optically active pure form can be isolated from a racemic mixture or synthesized by using chiral starting materials or chiral reagents.

The term "substituted" means that any one or more hydrogen atoms on a specific atom are substituted by substituents, as long as the valence of the specific atom is normal and the resulting compound is stable. When the substituent is oxo (namely =O), it means that two hydrogen atoms are substituted, and oxo is not available on an aromatic group.

The term "optional" or "optionally" means that the subsequently described event or circumstance may, but not necessarily, occur. The description includes instances where the event or circumstance occurs and instances where the event or circumstance does not. For example, an ethyl "optionally" substituted with halogen, means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). It will be understood by those skilled in the art that for any group comprising one or more substituents, no substitution or substituting pattern that is spatially impossible and/or cannot be synthesized will be introduced.

When any variable (e.g., $R^a$ or $R^b$) occurs more than once in the constitution or structure of a compound, the variable is independently defined in each case. For example, if a group is substituted with 2 $R^b$, the definition of each $R^b$ is independent.

When the number of a linking group is 0, such as $-(CH_2)_0-$, it means that the linking group is a bond. When one of variables is selected from a chemical bond or is absent, it means that the two groups which it links are linked directly. For

example, when L in A-L-Z represents a bond, it means that the structure is actually A-Z.

When the linking direction of the linking group referred to herein is not specified, the linking direction is arbitrary. For example, when X in the structural unit

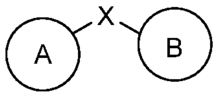

is selected from "$C_1$-$C_3$ alkylene-O", then X may link ring A and ring B in a direction from left to right to constitute "ring A-$C_1$-$C_3$ alkylene-O-ring B", or link ring A and ring B in a direction from right to left to constitute "ring A-O-$C_1$-$C_3$ alkylene-ring B".

$C_m$-$C_n$ used herein means that the portion has an integer number of carbon atoms in the range of m-n.

The term "alkyl" refers to hydrocarbyl with a general formula of $C_nH_{2n+1}$. The alkyl may be linear or branched. The term "$C_1$-$C_6$ alkyl" should be understood to represent a linear or branched saturated hydrocarbyl having 1, 2, 3, 4, 5, or 6 carbon atoms. The alkyl includes, but is not limited to, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "$C_1$-$C_3$ alkyl" refers to alkyl containing 1 to 3 carbon atoms, such as methyl, ethyl, *n*-propyl, and isopropyl.

The "$C_1$-$C_6$ alkyl" described herein may further include "$C_1$-$C_3$ alkyl".

The term "alkynyl" refers to a linear or branched unsaturated aliphatic hydrocarbon group consisting of carbon atoms and hydrogen atoms and having at least one triple bond. For example, the term "$C_2$-$C_6$ alkynyl" should be understood to preferably represent a linear or branched hydrocarbyl group comprising one or more triple bonds and having 2, 3, 4, 5, or 6 carbon atoms. Examples of "$C_2$-$C_6$ alkynyl" include, but are not limited to, ethynyl ($-C\equiv CH$), prop-1-ynyl (1-propynyl, $-C\equiv CCH_3$), prop-2-ynyl ($-CH_2C\equiv CH$), but-1-ynyl, but-2-ynyl, or but-3-ynyl. "$C_2$-$C_6$ alkynyl" may include "$C_2$-$C_3$ alkynyl", and examples of "$C_2$-$C_3$ alkynyl" include ethynyl ($-C\equiv CH$), prop-1-ynyl (1-propynyl, $-C\equiv CCH_3$), and prop-2-ynyl ($-CH_2C\equiv CH$).

The term "cycloalkyl" refers to a fully saturated carbon ring that exists in the form of a monocycle, fused cycle, bridged cycle, spiro cycle, and the like. The term "$C_3$-$C_6$ cycloalkyl" should be understood as a saturated monocyclic, fused, spiro, or bridged ring having 3-6 carbon atoms. Specific examples include, but are not limited to, cyclopropyl, cyclobutyl, cyclopentyl, cyclohexyl, and the like.

The term "cycloalkenyl" refers to a non-aromatic carbon ring group that is not fully saturated and exists in the form of a monocyclic ring, a fused ring, a bridged ring, a spiro ring, or the like. Unless otherwise specified, the carbon ring is generally a 5-8 membered ring. The term "$C_5$-$C_7$ cycloalkenyl" refers to cycloalkenyl with 5, 6, or 7 ring atoms. Specific examples include, but are not limited to, cyclopentenyl, cyclopentadienyl, cyclohexenyl, cyclohexadienyl, cycloheptenyl, cycloheptadienyl, or the like. The term "$C_5$-$C_7$ cycloalkenyl" may include the ranges of "$C_5$-$C_6$ cycloalkenyl" and the like. The term "$C_5$-$C_6$ cycloalkenyl" refers to cycloalkenyl with 5 or 6 ring atoms. Specific examples include, but are not limited to, cyclopentenyl, cyclopentadienyl, cyclohexenyl, cyclohexadienyl, and the like.

The term "heterocyclyl" refers to a fully saturated or partially saturated monocyclic, fused cyclic, spiro cyclic, or bridged cyclic group, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), $-S(=O)_2-$, -S(=O)-, $-P(=O)_2-$, -P(=O)-, -NH-, -S(=O)(=NH)-, -C(=O)NH-, -NHC(=O)NH-, and the like. The term "4-7 membered heterocyclyl" refers to a heterocyclyl group having a ring atom number of 4, 5, 6, or 7, and ring atoms of the heterocyclyl group include 1-3 heteroatoms or heteroatom groups independently selected from those described above. Examples of 4-membered heterocyclyl include, but are not limited to, azetidinyl or oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1H-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridinyl, or 4H-[1,3,4]thiadiazinyl; examples of 7-membered heterocyclyl include, but are not limited to, diazepanyl. Preferably, "4-7 membered heterocyclyl" may include the ranges of "4-7 membered heterocycloalkyl", "5-6 membered heterocyclyl", "5-6 membered heterocycloalkyl", and the like. The term "5-6 membered heteroaryl" refers to an aromatic ring group which has 5 or 6 ring atoms, and comprises 1-3, preferably 1-2, heteroatoms independently selected from N, O, and S. In particular, 5-6 membered heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, triazinyl, or the like.

The term "halo-" or "halogen" refers to fluorine, chlorine, bromine and iodine.

The term "treatment" refers to surgical or therapeutic treatment for the purpose of preventing, slowing (reducing) the progression of an undesired physiological or pathological change, e.g., cancer, an autoimmune disease, and virus

infection, in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc. The term "effective amount" refers to an amount of a therapeutic agent that is effective to prevent or alleviate symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously. The term "subject" refers to an organism that receives treatment for a particular disease or disorder described herein. Examples of subjects and patients include mammals, such as humans, primates (e.g., monkey), or non-primate mammals, that receive treatment for a disease or disorder.

The amount of the compound disclosed herein constituting the "therapeutically effective amount" varies dependently on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio. The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipients" refers to those which do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The word "comprise" and variations thereof such as "comprises" or "comprising" may be understood in an open, non-exclusive sense, i.e., "including but not limited to".

The present disclosure also comprises isotopically-labeled compounds which are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively, and the like.

Certain isotopically-labeled compounds of the present disclosure (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$ and $^{18}F$ can be used in positron emission tomography (PET) studies to determine substrate occupancy. Isotopically-labeled compounds of the present disclosure can generally be prepared by following procedures analogous to those disclosed in the schemes and/or examples below while substituting a non-isotopically labeled reagent with an isotopically-labeled reagent.

The pharmaceutical compositions of the present disclosure may be suitable for parenteral administration, such as sterile solutions, suspensions or lyophilized products in suitable unit dosage forms. For example, the pharmaceutical composition of the present disclosure may be in the form of a sterile injectable aqueous solution for intramuscular or subcutaneous administration. Other vehicles or solvents such as water, Ringer's solution or isotonic sodium chloride solution are acceptable when the pharmaceutical composition of the present disclosure is used.

In all of the administration methods of the compound described herein, the daily dose administered is from 0.001 mg/kg to 600 mg/kg body weight, preferably from 0.05 mg/kg to 200 mg/kg body weight, and more preferably from 0.1 mg/kg to 100 mg/kg body weight, given in individual or separated doses.

The compounds disclosed herein can be prepared by a variety of synthetic methods well known to those skilled in

the art, including the specific embodiments listed below, embodiments formed by combinations thereof with other chemical synthetic methods, and equivalents thereof well known to those skilled in the art. The preferred embodiments include, but are not limited to, the examples disclosed herein.

The chemical reactions of the specific embodiments disclosed herein are conducted in a suitable solvent that must be suitable for the chemical changes in the present disclosure and the reagents and materials required. In order to obtain the compounds disclosed herein, it is sometimes necessary for those skilled in the art to modify or select a synthesis procedure or a reaction process based on the existing embodiments.

An important consideration in synthetic route planning in the art is the selection of suitable protective groups for reactive functional groups (e.g., amino and carboxy in the present disclosure). For example, reference may be made to Greene's Protective Groups in Organic Synthesis (4th Ed.) Hoboken, New Jersey: John Wiley & Sons, Inc. All references cited in the present disclosure are incorporated herein in their entirety.

## DETAILED DESCRIPTION

The present disclosure is described in detail below by way of examples. However, this is by no means disadvantageously limiting the scope of the present disclosure. Although the present disclosure has been described in detail herein and specific examples have also been disclosed, it will be apparent to those skilled in the art that various modifications can be made to the specific embodiments of the present disclosure without departing from the spirit and scope of the present disclosure. All reagents used in the present disclosure are commercially available and can be used without further purification. Unless otherwise stated, the ratio represented by a mixed solvent is a volume mixing ratio.

Unless otherwise stated, % refers to wt%.

Compounds are named either manually or by ChemDraw® software, and supplier's catalog names are given for commercially available compounds.

The structures of the compounds are determined by nuclear magnetic resonance (NMR) and/or mass spectrometry (MS). NMR shifts are given in $10^{-6}$ (ppm). Solvents for NMR determination are deuterated dimethyl sulfoxide, deuterated chloroform, deuterated methanol or the like, and internal standard is tetramethylsilane (TMS); "$IC_{50}$" refers to the half maximal inhibitory concentration, which is the concentration at which half of the maximal inhibitory effect is achieved; "$EC_{50}$" refers to the half maximal effect concentration, a concentration that causes 50% of the maximal effect.

### Example 1. (*S*)-N-((8-ethyl-8-hydroxy-9,12-dioxo-2,3,8,9,12,14-hexahydro-11*H*-[1,4]dioxino [2,3-g]pyrano [3',4':6,7]indolizino[1,2-*b*]quinolin-15-yl)methyl)-2-hydroxyacetamide (Compound 1)

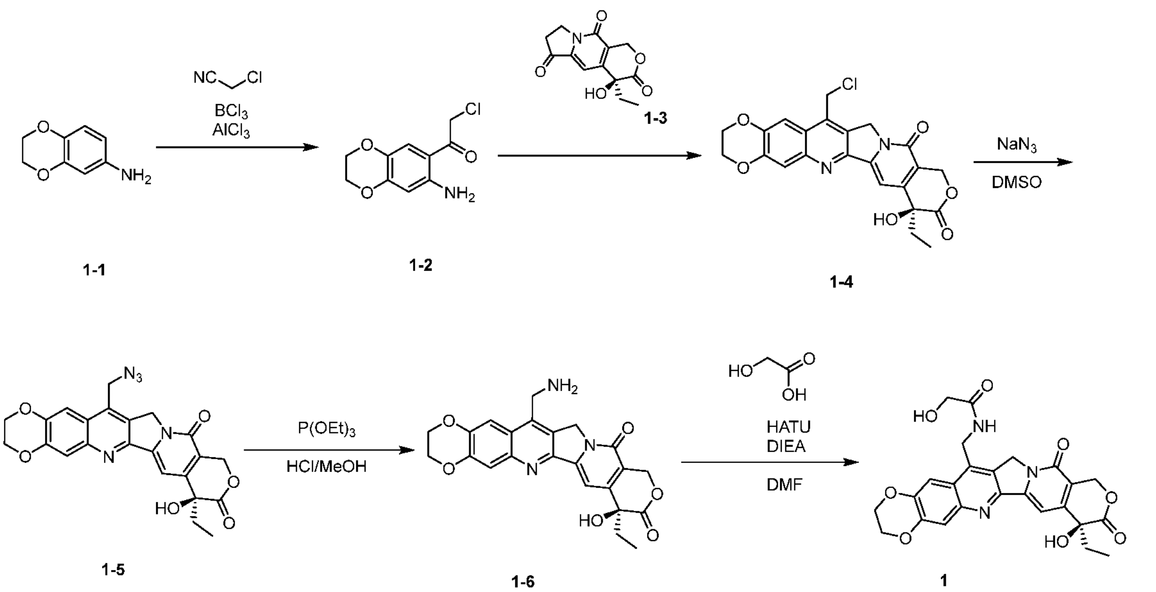


1-1 1-2 1-4 1-5 1-6 1

### Step 1: synthesis of 1-(7-amino-2,3-dihydrobenzo[*b*][1,4]dioxin-6-yl)-2-chloroethane-1-one (intermediate 1-2)

**Reactant 1-1** (500 mg, 3.31 mmol) was dissolved in 1,2-dichloroethane (3 mL). The reaction mixture was cooled to 0 °C, and boron trichloride (1 M, 2.65 mL) and aluminum trichloride (573.36 mg, 4.30 mmol) were added thereto.

Chloroacetonitrile (299.67 mg, 3.97 mmol) was added to the reaction mixture at 0 °C under nitrogen atmosphere, and the reaction mixture was stirred at 90 °C for 16 h under nitrogen atmosphere. The reaction was completed as detected by LC-MS. After the reaction mixture was cooled to room temperature, ice water (30 mL) and 1 N HCl (10 mL) were added sequentially and stirred for 30 min. Dichloromethane (30 mL × 3) was added to the reaction mixture, and the mixture was extracted 3 times. The organic phases were combined and washed with saturated brine (30 mL), and the washed organic phase was dried over an appropriate amount of anhydrous sodium sulfate. The organic phase was concentrated to dryness under reduced pressure, and the crude product was purified by preparative thin-layer chromatography (silica, petroleum ether:ethyl acetate = 9:1) to give the title compound (210 mg).

MS m/z (ESI): 228.0 $[M+H]^+$.

**Step 2: synthesis of (*S*)-15-(chloromethyl)-8-ethyl-8-hydroxy-2,3,11,14-tetrahydro-12*H*-** [1,4] **dioxino[2,3-g]pyrano[3',4':6,7]indolizino[1,2-*b*]quinoline-9,12(8H)-dione (intermediate 1-4)**

**Intermediate 1-2** (100 mg, 439.28 μmol) and **intermediate 1-3** (115.64 mg, 439.28 μmol) were dissolved in anhydrous toluene (3 mL), and pyridinium p-toluenesulfonate (22.08 mg, 87.86 μmol) was added thereto. The reaction mixture was stirred at 100 °C for 16 h under nitrogen atmosphere. The reaction was completed as detected by LC-MS. After the reaction mixture was cooled to room temperature, the reaction mixture was filtered, and the filter cake was washed with ethanol (5 mL × 2) to give a crude product of the title compound (130 mg).

MS m/z (ESI): 455.1 $[M+H]^+$.

**Step 3: synthesis of (*S*)-15-(azidomethyl)-8-ethyl-8-hydroxy-2,3,11,14-tetrahydro-12*H*-[1,4] dioxino[2,3-*g*]pyrano[3',4':6,7]indolizino[1,2-*b*]quinoline-9,12(8*H*)-dione (intermediate 1-5)**

Intermediate 1-4 (120 mg, 263.82 μmol) was dissolved in dimethyl sulfoxide (1 mL), and sodium azide (25.73 mg, 395.73 μmol) was added thereto. The reaction mixture was stirred at 25 °C for 3 h under nitrogen atmosphere. The reaction was completed as detected by LC-MS. Ice water (2 mL) was added thereto. The mixture was stirred for 0.5 h and filtered to give a crude product of the title compound (90 mg).

MS m/z (ESI): 462.1 $[M+H]^+$.

**Step 4: synthesis of (*S*)-15-(aminomethyl)-8-ethyl-8-hydroxy-2,3,11,14-tetrahydro-12*H*-[1,4] dioxino[2,3-*g*]pyrano[3',4':6,7]indolizino[1,2-*b*]quinoline-9,12(8*H*)-dione (intermediate 1-6)**

**Intermediate 1-5** (90 mg, 195.05 μmol) was dissolved in anhydrous toluene (1 mL), and triethyl phosphite (81.02 mg, 487.62 μmol) was added thereto. The reaction mixture was stirred at 100 °C for 3 h under nitrogen atmosphere. The reaction mixture was cooled to 25 °C, and a hydrochloric acid methanol solution (0.5 mL) was added thereto. The reaction mixture was stirred at 85 °C for 16 h under nitrogen atmosphere. The reaction was completed as detected by LC-MS. After the reaction mixture was cooled to room temperature, the reaction mixture was filtered to give the title compound (18 mg).

MS m/z (ESI): 436.1 $[M+H]^+$.

**Step 5: synthesis of (*S*)-*N*-((8-ethyl-8-hydroxy-9,12-dioxo-2,3,8,9,12,14-hexahydro-11*H*-[1,4] dioxino[2,3-*g*]pyrano[3',4':6,7]indolizino[1,2-*b*]quinolin-15-yl)methyl)-2-hydroxyacetamide (compound 1)**

**Intermediate 1-6** (18 mg, 41.34 μmol) and 2-hydroxyacetic acid (15.72 mg, 206.69 μmol) were dissolved in anhydrous *N*,*N*-dimethylformamide (1 mL), and 2-(7-azabenzotriazole)-*N*,*N*,*N*,*N*-tetramethyluronium hexafluorophosphate (HATU) (23.58 mg, 62.01 μmol) and *N*,*N*-diisopropylethylamine (DIEA) (16.03 mg, 124.02 μmol) were added thereto. The reaction mixture was stirred at 25 °C for 3 h. The reaction was completed as detected by LC-MS. The reaction mixture was filtered and purified by preparative high performance liquid chromatography (YMC-Actus Triart C18 column, 5 μm silica, 25 mm diameter, 100 mm length; a mixture of water (containing 0.05% formic acid) and acetonitrile with decreasing polarity was used as the eluent; acetonitrile gradient: 10%-30%, elution time: 12 min) to give the title compound (7 mg).

MS m/z (ESI): 494.1 $[M+H]^+$.

**$^1$H NMR (400MHz, DMSO-$d_6$)** δ = 8.69 (t, *J* = 6.0 Hz, 1H), 7.92 (s, 1H), 7.56 (s, 1H), 7.26 (s, 1H), 6.49 (s, 1H), 5.57 (t, *J* = 5.7 Hz, 1H), 5.46 (s, 2H), 5.43 (s, 2H), 4.74 (d, *J* = 6.0 Hz, 2H), 4.44 (s, 4H), 3.82 (d, *J* = 5.6 Hz, 2H), 1.94-1.80 (m, 2H), 0.88 (m, 3H).

**Example 3. (*S*)-*N*-((9-chloro-4-ethyl-8-fluoro-4-hydroxy-3,14-dioxo-3,4,12,14-tetrahydro-1*H*-pyrano[3',4':6,7]indolizino[1,2-*b*]quinolin-11-yl)methyl)-2-hydroxyacetamide (Compound 3)**

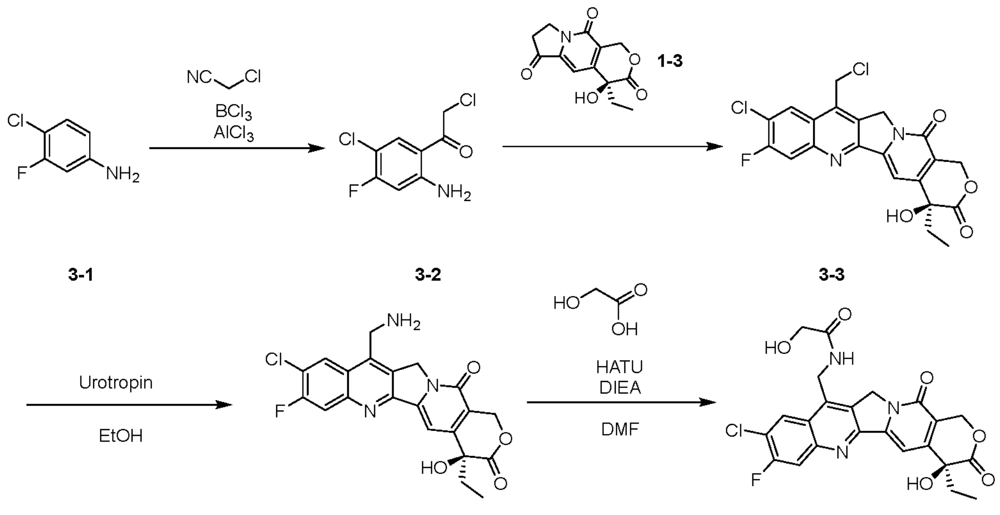


3-1 3-2 3-3 3-4 3

**Step 1: synthesis of 1-(2-amino-5-chloro-4-fluorophenyl)-2-chloroethane-1-one (intermediate 3-2)**

Boron trichloride (1 M, 13.74 mL) was dissolved in 1,2-dichloroethane (24 mL). The reaction mixture was cooled to 0 °C, and **reactant 3-1** (2 g, 13.74 mmol) and chloroacetonitrile (1.56 g, 20.61 mmol) were added thereto. The reaction was stirred at 0 °C for 10 min, and aluminum trichloride (2.38 g, 17.86 mmol) was added thereto. Then the reaction mixture was warmed to 25 °C and stirred for 10 min under nitrogen atmosphere. The reaction mixture was stirred at 90 °C for 18 h under nitrogen atmosphere. The reaction was completed as detected by LC-MS. After the reaction mixture was cooled to room temperature, ice water (50 mL) and 5% HCl (10 mL) were added sequentially and slowly and stirred at 25 °C for 30 min, and dichloromethane (50 mL) was added. The organic phase was washed with water (2 mL × 2), and the washed organic phase was dried over an appropriate amount of anhydrous sodium sulfate. The organic phase was concentrated to dryness under reduced pressure, and the crude product was purified by preparative high performance liquid chromatography (YMC-Actus Triart C18 column, 5 μm silica, 25 mm diameter, 100 mm length; a mixture of water (containing 0.05% formic acid) and acetonitrile with decreasing polarity was used as the eluent; acetonitrile gradient: 40%-60%, elution time: 10 min) to give the title compound (320 mg).

MS m/z (ESI): 222.0 $[M+H]^+$.

**Step 2: synthesis of (*S*)-9-chloro-11-(chloromethyl)-4-ethyl-8-fluoro-4-hydroxy-1,12-dihydro-14*H*-pyrano [3',4':6,7]indolizino[1,2-*b*]quinoline-3,14(4*H*)-dione (intermediate 3-3)**

**Intermediate 3-2** (220 mg, 990.80 μmol) and **intermediate 1-3** (273.86 mg, 1.04 mmol) were dissolved in toluene (2 mL), and pyridinium *p*-toluenesulfonate (24.90 mg, 99.08 μmol) was added thereto. The reaction mixture was stirred at 100 °C for 18 h. The reaction was completed as detected by LC-MS. After the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) to give a crude product of the title compound (270 mg).

MS m/z (ESI): 449.0 $[M+H]^+$.

**Step 3: synthesis of (*S*)-11-(aminomethyl)-9-chloro-4-ethyl-8-fluoro-4-hydroxy-1,12-dihydro-14*H*-pyrano [3',4':6,7]indolizino[1,2-*b*]quinoline-3,14(4*H*)-dione (3-4)**

**Intermediate 3-3** (50 mg, 111.29 μmol) was dissolved in ethanol (1 mL), and urotropin (23.40 mg, 166.94 μmol) was added thereto. The reaction mixture was stirred at 90 °C for 1.5 h. The reaction was completed as detected by LC-MS. After the reaction mixture was cooled to room temperature, the reaction mixture was concentrated to dryness under reduced pressure and purified by preparative high performance liquid chromatography (YMC-Actus Triart C18 column, 5 μm silica, 25 mm diameter, 100 mm length; a mixture of water (containing 0.225% formic acid) and methanol with

decreasing polarity was used as the eluent; methanol gradient: 0%-30%, elution time: 12 min) to give the title compound (22 mg).

MS m/z (ESI): 430.1 $[M+H]^+$.

**$^1$H NMR (400MHz, DMSO-$d_6$)** $\delta$ = 8.71 (d, *J* = 8.0 Hz, 1H), 8.23 (d, *J* = 10.3 Hz, 1H), 8.14 (s, 0.3H, HCOOH), 7.36 (s, 1H), 6.57 (s, 1H), 5.52 (s, 2H), 5.46 (s, 2H), 4.55 (s, 2H), 1.93-1.84 (m, 2H), 0.90-0.85 (m, 3H).

**Step 4: synthesis of (*S*)-*N*-((9-chloro-4-ethyl-8-fluoro-4-hydroxy-3,14-dioxo-3,4,12,14-tetrahydro-1*H*-pyrano[3',4':6,7]indolizino[1,2-*b*]quinolin-11-yl)methyl)-2-hydroxyacetamide (compound 3)**

**3-4** (22 mg, 51.18 μmol) and 2-hydroxyacetic acid (19.46 mg, 255.92 μmol) were dissolved in anhydrous *N*,*N*-dimethylformamide (1 mL), and HATU (29.19 mg, 76.77 μmol) and *N,N*-diisopropylethylamine (19.84 mg, 153.55 μmol) were added thereto. The reaction mixture was stirred at 25 °C for 1.5 h. The reaction was completed as detected by LC-MS. The reaction mixture was filtered and concentrated to dryness under reduced pressure, and the crude product was purified by preparative high performance liquid chromatography (YMC-Actus Triart C18 column, 5 μm silica, 25 mm diameter, 100 mm length; a mixture of water (containing 0.05% formic acid) and acetonitrile with decreasing polarity was used as the eluent; acetonitrile gradient: 10%-40%, elution time: 12 min) to give the title compound (2.20 mg).

MS m/z (ESI): 488.1 $[M+H]^+$.

**$^1$H NMR (400MHz, DMSO-$d_6$)** $\delta$ = 8.90-8.85 (m, 2H), 8.20 (d, *J* = 10.3 Hz, 1H), 7.35 (s, 1H), 6.55 (s, 1H), 5.60 (t, *J* = 5.7 Hz, 1H), 5.56 (s, 2H), 5.45 (s, 2H), 4.83 (d, *J* = 6.0 Hz, 2H), 3.83 (d, *J* = 5.8 Hz, 2H), 1.93-1.81 (m, 2H), 0.88 (m, 3H).

**Example 5. (*S*)-*N*-((9-bromo-4-ethyl-8-fluoro-4-hydroxy-3,14-dioxo-3,4,12,14-tetrahydro-1*H*-pyrano[3',4':6,7]indolizino[1,2-*b*]quinolin-11-yl)methyl)-2-hydroxyacetamide (Compound 5)**

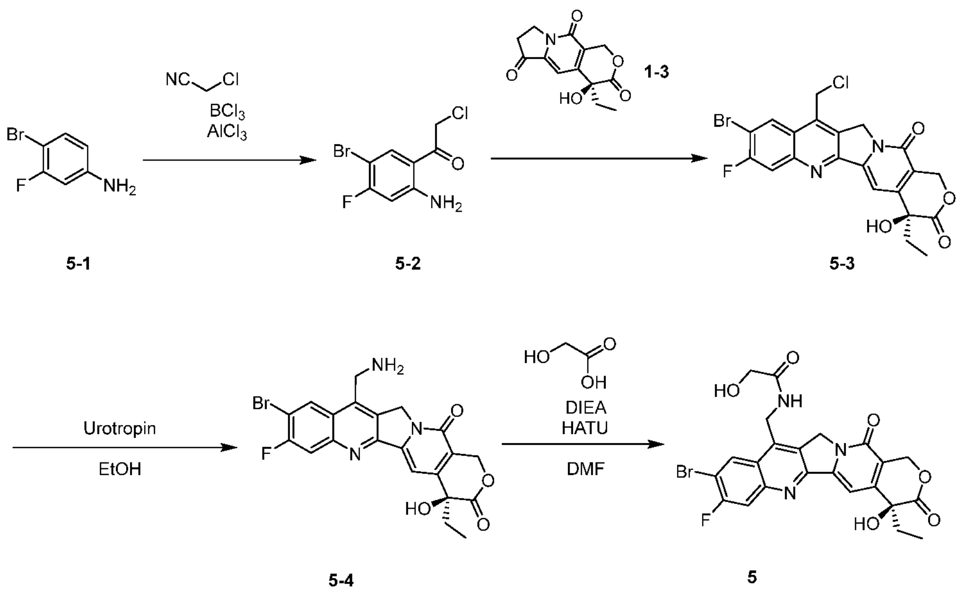


5-1 5-2 1-3 5-3 5-4 5

**Step 1: synthesis of 1-(2-amino-5-bromo-4-fluorophenyl)-2-chloroethane-1-one (intermediate 5-2)**

Boron trichloride (1 M, 10.53 mL) was dissolved in 1,2-dichloroethane (24 mL). The reaction mixture was cooled to 0 °C, and **intermediate 5-1** (2 g, 10.53 mmol) and chloroacetonitrile (1.19 g, 15.79 mmol) were added thereto. The reaction was stirred at 0 °C for 10 min, and aluminum trichloride (1.82 g, 13.68 mmol) was added thereto. The reaction mixture was stirred at 25 °C for 10 min under nitrogen atmosphere. Then the reaction mixture was warmed to 90 °C and stirred for 18 h. The reaction was completed as detected by LC-MS. After the reaction mixture was cooled to room temperature, ice water (50 mL) and 5% HCl (10 mL) were added sequentially and slowly and stirred at 25 °C for 30 min, and dichloromethane (50 mL) was added. The organic phase was washed with water (2 mL × 2), and the washed organic phase was dried over an appropriate amount of anhydrous sodium sulfate. The organic phase was purified by preparative high performance liquid chromatography (YMC-Actus Triart C18 column, 5 μm silica, 25 mm diameter, 100 mm length; a mixture of water (containing 0.05% formic acid) and acetonitrile with decreasing polarity was used as the eluent; acetonitrile gradient: 39%-49%, elution time: 12 min) to give the title compound (380 mg).

MS m/z (ESI): 265.9 $[M+H]^+$.

**Step 2: synthesis of (*S*)-9-bromo-11-(chloromethyl)-4-ethyl-8-fluoro-4-hydroxy-1,12-dihydro-14*H*-pyrano [3',4':6,7]indolizino[1,2-*b*]quinoline-3,14(4H)-dione (intermediate 5-3)**

**Intermediate 5-2** (200 mg, 750.48 μmol) and **intermediate 1-3** (207.44 mg, 788.01 μmol) were dissolved in anhydrous toluene (4 mL), and pyridinium *p*-toluenesulfonate (22.63 mg, 90.06 μmol) was added thereto. The reaction mixture was stirred at 100 °C for 18 h. The reaction was completed as detected by LC-MS. After the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) to give a crude product of the title compound (200 mg).

MS m/z (ESI): 493.0 $[M+H]^+$.

**Step 3: synthesis of (*S*)-11-(aminomethyl)-9-bromo-4-ethyl-8-fluoro-4-hydroxy-1,12-dihydro-14*H*-pyrano [3',4':6,7]indolizino[1,2-*b*]quinoline-3,14(4*H*)-dione (intermediate 5-4)**

**Intermediate 5-3** (200 mg, 405.10 μmol) was dissolved in ethanol (4 mL), and urotropin (113.58 mg, 810.19 μmol) was added thereto. The reaction mixture was stirred at 90 °C for 1.5 h. The reaction was completed as detected by LC-MS. After the reaction mixture was cooled to room temperature, the reaction mixture was concentrated to dryness under reduced pressure, and the crude product was purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 6%-26%, elution time: 12 min) to give the title compound (30 mg).

MS m/z (ESI):476.0 $[M+H]^+$.

**$^1$H NMR (400MHz, DMSO-$d_6$)** δ = 8.83 (d, *J* = 7.4 Hz, 1H), 8.18 (d, *J* = 9.8 Hz, 1H), 8.14 (s, 0.4H, HCOOH), 7.36 (s, 1H), 6.57 (s, 1H), 5.52 (s, 2H), 5.46 (s, 2H), 4.53 (s, 2H), 1.92-1.85 (m, 2H), 0.88 (t, *J* = 7.3 Hz, 3H).

**Step 4: synthesis of (*S*)-*N*-((9-bromo-4-ethyl-8-fluoro-4-hydroxy-3,14-dioxo-3,4,12,14-tetrahydro-1*H*-pyrano [3',4':6,7]indolizino[1,2-*b*]quinolin-11-yl)methyl)-2-hydroxyacetamide (compound 5)**

**Intermediate 5-4** (15 mg, 26.88 μmol) and 2-hydroxyacetic acid (10.22 mg, 134.41 μmol) were dissolved in anhydrous *N,N*-dimethylformamide (1 mL), and HATU (15.33 mg, 40.32 μmol) and*N,N*-diisopropylethylamine (10.42 mg, 80.65 μmol) were added thereto. The reaction mixture was stirred at 25 °C for 1 h. The reaction was completed as detected by LC-MS. The reaction mixture was filtered and concentrated to dryness under reduced pressure, and the crude product was purified by preparative high performance liquid chromatography (YMC-Actus Triart C18 column, 5 μm, 25 mm diameter, 100 mm length; a mixture of water (containing 0.05% formic acid) and acetonitrile with decreasing polarity was used as the eluent; acetonitrile gradient: 6%-36%, elution time: 12 min) to give the title compound (2.09 mg).

MS m/z (ESI): 532.0 $[M+H]^+$.

**$^1$H NMR (400MHz, DMSO-$d_6$)** δ = 9.00 (d, *J* = 7.5 Hz, 1H), 8.86 (t, *J* = 5.9 Hz, 1H), 8.15 (d, *J* = 9.8 Hz, 1H), 7.35 (s, 1H), 6.54 (s, 1H), 5.60 (t, *J* = 5.7 Hz, 1H), 5.56 (s, 2H), 5.45 (s, 2H), 4.83 (d, *J* = 5.8 Hz, 2H), 3.83 (d, *J* = 5.9 Hz, 2H), 1.94-1.82 (m, 2H), 0.88 (t, *J* = 7.4 Hz, 3H).

**Example 6. (*S*)-*N*-((8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide (Compound 6)**

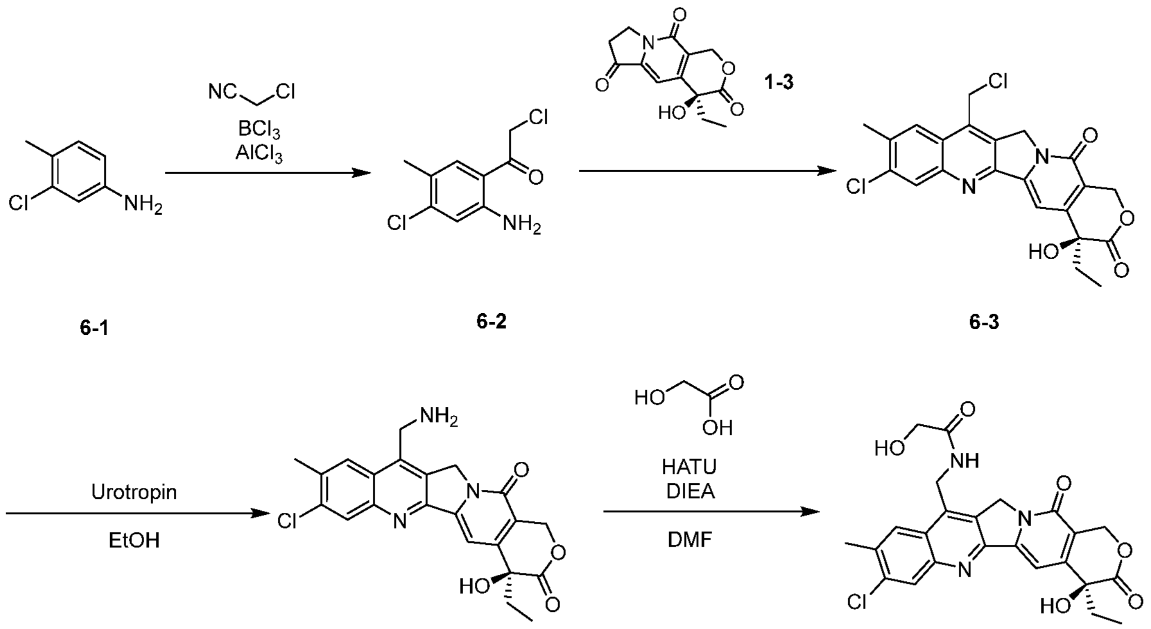


6-1 6-2 6-3

6-4 6

**Step 1: synthesis of 1-(2-amino-4-chloro-5-methylphenyl)-2-chloroethane-1-one (intermediate 6-2)**

Boron trichloride (1 M, 2.82 mL) was dissolved in 1,2-dichloroethane (8 mL). The reaction mixture was cooled to 0 °C, and **reactant 6-1** (0.5 g, 3.53 mmol) and chloroacetonitrile (319.91 g, 4.24 mmol) were added thereto. The reaction was stirred at 0 °C for 10 min, and aluminum trichloride (612.09 mg, 4.59 mmol) was added thereto. The reaction mixture was stirred at 25 °C for 10 min under nitrogen atmosphere. Then the reaction mixture was warmed to 90 °C and stirred for 18 h. The reaction was completed as detected by LC-MS. After the reaction mixture was cooled to room temperature, ice water (25 mL) and 5% HCl (5 mL) were added sequentially and slowly and stirred at 25 °C for 30 min, and dichloromethane (20 mL) was added. The organic phase was washed with water (20 mL × 2), and the washed organic phase was dried over an appropriate amount of anhydrous sodium sulfate. The organic phase was purified by preparative thin-layer chromatography (silica, petroleum ether:ethyl acetate = 9:1) to give the title compound (500 mg).

MS m/z (ESI): 218.0 $[M+H]^+$

**Step 2: synthesis of (*S*)-8-chloro-11-(chloromethyl)-4-ethyl-4-hydroxy-9-methyl-1,12-dihydro-14*H*-pyrano [3',4':6,7]indolizino[1,2-*b*]quinoline-3,14(4*H*)-dione (intermediate 6-3)**

**Intermediate 6-2** (250 mg, 1.15 mmol) and **intermediate 1-3** (316.87 mg, 1.20 mmol) were dissolved in toluene (5 mL), and pyridinium p-toluenesulfonate (34.57 mg, 137.56 μmol) was added thereto. The reaction mixture was stirred at 100 °C for 18 h. The reaction was completed as detected by LC-MS. After the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) to give a crude product of the title compound (230 mg).

MS m/z (ESI): 445.1 $[M+H]^+$.

**Step 3: synthesis of (*S*)-11-(aminomethyl)-8-chloro-4-ethyl-4-hydroxy-9-methyl-1,12-14*H*-pyrano[3',4':6,7]indolizino[1,2-*b*]quinoline-3,14(4*H*)-dione (intermediate 6-4)**

**Intermediate 6-3** (49.56 mg, 111.29 μmol) was dissolved in ethanol (0.5 mL), and urotropin (23.40 mg, 166.94 μmol) was added thereto. The reaction mixture was stirred at 90 °C for 1.5 h. The reaction was completed as detected by LC-MS. After the reaction mixture was cooled to room temperature, the reaction mixture was concentrated to dryness under reduced pressure and purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 2%-32%, elution time: 12 min) to give the title compound (11.0 mg).

MS m/z (ESI): 426.2 $[M+H]^+$.

**Step 4: synthesis of (*S*)-*N*-((8-chloro-4-ethyl-4-hydroxy-9-methyl-3,14-dioxo-3,4,12,14-tetrahydro-1*H*-pyrano [3',4':6,7]indolizino[1,2-*b*]quinolin-11-yl)methyl)-2-hydroxyacetamide (compound 6)**

**Intermediate 6-4** (11 mg, 25.83 μmol) and 2-hydroxyacetic acid (9.82 mg, 129.15 μmol) were dissolved in anhydrous *N*,*N*-dimethylformamide (0.5 mL), and HATU (14.73 mg, 38.74 μmol) and *N*,*N*-diisopropylethylamine (10.01 mg, 77.49 μmol) were added thereto. The reaction mixture was stirred at 25 °C for 1 h. The reaction was completed as detected by LC-MS. The reaction mixture was filtered and concentrated to dryness under reduced pressure, and the crude product was purified by preparative high performance liquid chromatography (YMC-Actus Triart C18 column, 5 μm, 25 mm diameter, 100 mm length; a mixture of water (containing 0.05% formic acid) and acetonitrile with decreasing polarity was used as the eluent; acetonitrile gradient: 10%-40%, elution time: 12 min) to give the title compound (3.00 mg).

MS m/z (ESI): 484.1 $[M+H]^+$.

**$^1$H NMR (400MHz, DMSO-$d_6$)** δ = 8.77 (t, *J* = 6.1 Hz, 1H), 8.52 (s, 1H), 8.26 (s, 1H), 7.32 (s, 1H), 6.54 (s, 1H), 5.59 (t, *J* = 5.8 Hz, 1H), 5.52 (s, 2H), 5.44 (s, 2H), 4.85 (d, *J* = 6.0 Hz, 2H), 3.84 (d, *J*= 5.6 Hz, 2H), 2.60 (s, 3H), 1.91-1.82 (m, 2H), 0.88 (t, *J* = 7.3 Hz, 3H)

**Example 7. (*S*)-*N*-((8-ethyl-8-hydroxy-9,12-dioxo-2,3,8,9,12,14-hexahydro-1*H*,11*H*-cyclopenta [*f*]pyrano [3',4':6,7]indolizino[1,2-*b*]quinolin-15-yl)methyl)-2-hydroxyacetamide (Compound 7)**

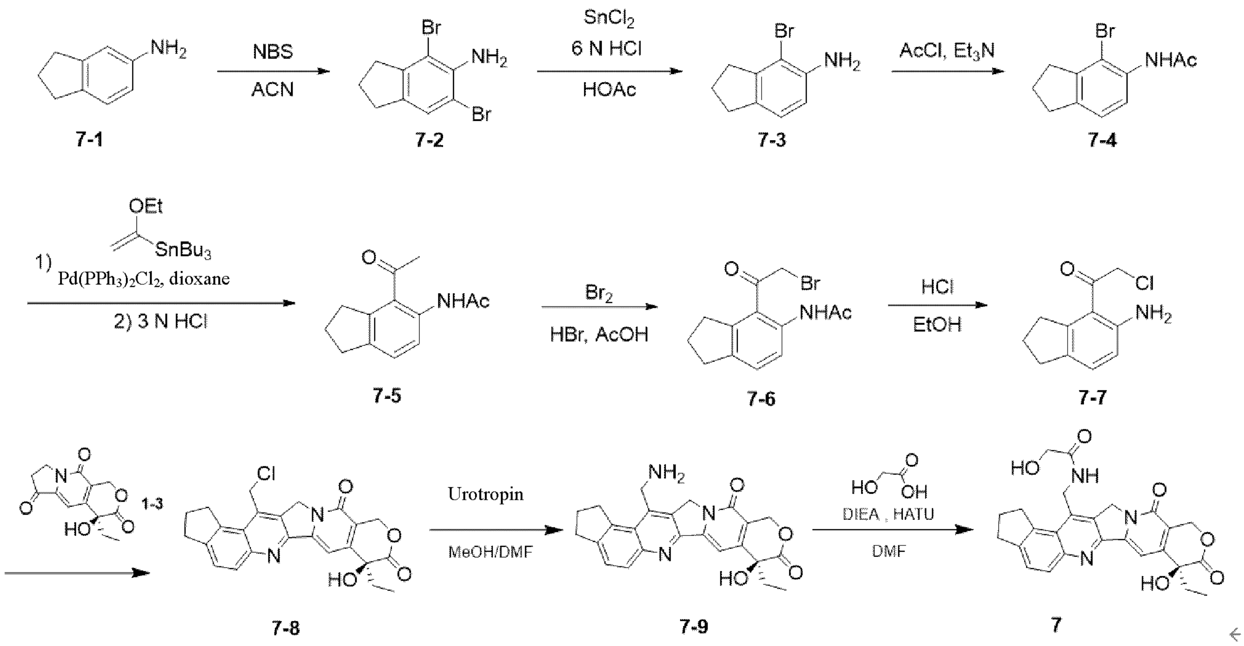


**Step 1: synthesis of 4,6-dibromo-2,3-dihydro-1*H*-inden-5-amine (intermediate 7-2)**

**Intermediate 7-1** (10.0 g) was dissolved in anhydrous acetonitrile, and N-bromosuccinimide (NBS) (27.5 g) was added in batches at 0 °C and then stirred at room temperature overnight. The reaction mixture was filtered, the filtrate was concentrated under reduced pressure, and the residue was dissolved in 200 mL of ethyl acetate and washed with water (100 mL × 2). The resulting organic phase was dried over anhydrous sodium sulfate, mixed with silica gel, then placed on celite, and rinsed with 500 mL of petroleum ether, and the filtrate was concentrated to give the title compound (17.0 g).

MS m/z (ESI): 289.9 $[M+H]^+$.

**Step 2: synthesis of 4-bromo-2,3-dihydro-1*H*-inden-5-amine (intermediate 7-3)**

**Intermediate 7-2** (15.0 g) and stannous chloride (15.0 g) were dissolved in 75 mL of acetic acid, 140 mL of 6 N concentrated hydrochloric acid was added, and the mixture was reacted at 90 °C for 3 h. The reaction system was cooled to room temperature and concentrated to remove acetic acid. The residue was dissolved in 100 mL of ethyl acetate, the pH was adjusted to about 8 with a saturated aqueous sodium carbonate solution, and the mixture was filtered. The organic phase of the filtrate was removed, and the aqueous phase was then extracted with ethyl acetate (50 mL × 3). The organic

phases were combined and dried over anhydrous sodium sulfate and filtered, and the filtrate was concentrated under reduced pressure to give the title compound (10 g).

MS m/z (ESI): 212.0 $[M+H]^+$.

**Step 3: synthesis of *N*-(4-bromo-2,3-dihydro-1*H*-inden-5-yl)acetamide (intermediate 7-4)**

**Intermediate 7-3** (10.0 g) was dissolved in 100 mL of anhydrous dichloromethane, triethylamine (10.6 g) was added, acetyl chloride (5.5 g) was slowly added dropwise at 0 °C, and then the reaction system was stirred at room temperature overnight. The reaction mixture was washed with water (100 mL × 2), and the organic phase was dried over anhydrous sodium sulfate and filtered. The filtrate was concentrated under reduced pressure, and the residue was purified by silica gel column chromatography (petroleum ether:ethyl acetate = 90:10) to give the title compound (7.1 g).

MS m/z (ESI): 254.0 $[M+H]^+$.

**Step 4: synthesis of *N*-(4-acetyl-2,3-dihydro-1*H*-inden-5-yl)acetamide (intermediate 7-5)**

**Intermediate 7-4** (6.7 g) and tributyl-(2-ethoxyvinyl)tin (10.4 g) were dissolved in 100 mL of anhydrous 1,4-dioxane under nitrogen atmosphere, then bis(triphenylphosphine)palladium dichloride (1.8 g) was added, and the reaction system was stirred at 100 °C overnight. The reaction mixture was cooled to room temperature, and 30 mL of 3 N hydrochloric acid was added and stirred at room temperature for 1 h. The reaction mixture was filtered through celite, and the resulting filtrate was diluted with 100 mL of ethyl acetate and washed with water (100 mL × 2). The organic phase was dried over anhydrous sodium sulfate and filtered, the filtrate was concentrated under reduced pressure, and the residue was purified by silica gel column chromatography (petroleum ether:ethyl acetate = 85:15) to give the title compound (4.7 g).

MS m/z (ESI): 218.1 $[M+H]^+$.

**Step 5: synthesis of *N*-(4-(2-bromoacetyl)-2,3-dihydro-1*H*-inden-5-yl)acetamide (intermediate 7-6)**

**Intermediate 7-5** (4.7 g) was dissolved in 50 mL of acetic acid, 7.3 g of a 33% hydrobromic acid-acetic acid solution was added, bromine (2.85 g) was slowly added dropwise at room temperature, and then the mixture was stirred for reaction at room temperature for another 3 h. After the reaction was completed, the reaction mixture was poured into ice water, stirred until a large amount of solid precipitated, and filtered. The filter cake was washed with petroleum ether, and the resulting solid was dried to give the title compound (5.0 g).

MS m/z (ESI): 296.0 $[M+H]^+$.

**Step 6: synthesis of 1-(5-amino-2,3-dihydro-1*H*-inden-4-yl)-2-chloroethan-1-one (intermediate 7-7)**

**Intermediate 7-6** (5.0 g) was dissolved in 30 mL of ethanol, 35 mL of 6 N concentrated hydrochloric acid was added, and the reaction system was stirred at 80 °C for 2 h. The reaction system was cooled to room temperature and concentrated under reduced pressure to remove the solvent. The residue was dissolved in 100 mL of dichloromethane, the pH was adjusted to about 7 with a saturated aqueous sodium bicarbonate solution, the organic phase was removed, and the aqueous phase was then extracted with dichloromethane (50 mL × 2). The organic phases were combined, dried over anhydrous sodium sulfate and filtered, the filtrate was concentrated under reduced pressure, and the residue was purified by silica gel column chromatography (petroleum ether:ethyl acetate = 75:25) to give the title compound (840 mg).

MS m/z (ESI): 210.0 $[M+H]^+$.

**Step 7: synthesis of (*S*)-15-(chloromethyl)-8-ethyl-8-hydroxy-1,2,3,8,11,14-hexahydro-9*H*,12*H*-cyclopenta[*f*]pyrano[3',4':6,7]indolizino[1,2-*b*]quinoline-9,12-dione (intermediate 7-8)**

**Intermediate 7-7** (100 mg) and **intermediate 1-3** (125.55 mg) were dissolved in toluene (5 mL), and pyridiniump-toluenesulfonate (5.99 mg) was added thereto. The reaction mixture was stirred at 90 °C for 18 h. After the reaction mixture was cooled to room temperature, ethanol (1 mL) was added, and the reaction mixture was stirred at 25 °C for 0.5 h. The reaction mixture was filtered, and the filter cake was washed with petroleum ether (2 mL × 2) to give the title compound (180 mg).

MS m/z (ESI): 437.0 $[M+H]^+$.

**Step 8: synthesis of (*S*)-15-(aminomethyl)-8-ethyl-8-hydroxy-1,2,3,8,11,14-hexahydro-9*H*,12*H*-cyclopenta[*f*]pyrano[3',4':6,7]indolizino[1,2-*b*]quinoline-9,12-dione (intermediate 7-9)**

**Intermediate 7-8** (50 mg) was dissolved in a mixed solution of methanol (1 mL) and *N,N*-dimethylformamide (1

mL), and urotropin (483.13 mg) was added thereto. The reaction mixture was stirred at 50 °C for 4 h. After the reaction was completed, the reaction mixture was cooled to room temperature. Concentrated hydrochloric acid (0.5 mL) was added, and the mixture was stirred for 0.5 h and then concentrated to dryness under reduced pressure. The residue was purified by preparative high performance liquid chromatography (Waters Xbridge C18 column, 5 μm, 25 mm diameter, 100 mm length; a mixture of water (containing 0.225% formic acid) and acetonitrile with decreasing polarity was used as the eluent; acetonitrile gradient: 20%-40%, elution time: 12 min) to give the title compound (22.0 mg).

MS m/z (ESI): 418.2 $[M+H]^+$.

**Step 9: synthesis of (*S*)-*N*-((8-ethyl-8-hydroxy-9,12-dioxo-2,3,8,9,12,14-hexahydro-1*H*,11*H*-cyclopenta[*f*]pyrano [3',4':6,7]indolizino[1,2-*b*]quinolin-15-yl)methyl)-2-hydroxyacetamide (compound 7)**

**Intermediate 7-9** (15 mg) and hydroxyacetic acid (10.93 mg) were dissolved in anhydrous *N,N*-dimethylformamide (0.5 mL), HATU (27.32 mg) and diisopropylethylamine (4.64 mg) were added thereto, and the reaction mixture was stirred at 25 °C for 1 h. After the reaction was completed, the reaction mixture was filtered, and the filtrate was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 30%-50%, elution time: 12 min) to give the title compound (9.0 mg).

MS m/z (ESI): 476.2$[M+H]^+$.

**$^1$H NMR (400MHz, DMSO-$d_6$)** δ = 8.28 (t, *J* = 5.1 Hz, 1H), 8.02 (d, *J* = 8.5 Hz, 1H), 7.78 (d, *J*= 8.3 Hz, 1H), 7.30 (s, 1H), 6.53 (s, 1H), 5.49-5.45 (m, 1H), 5.43 (s, 2H), 5.36 (s, 2H), 4.97 (d, *J* = 5.0 Hz, 2H), 3.88 (d, *J* = 5.5 Hz, 2H), 3.57 (t, *J* = 7.2 Hz, 2H), 3.09 (t, *J* = 7.4 Hz, 2H), 2.23-2.15 (m, 2H), 1.95-1.80 (m, 2H), 0.88 (t, *J* = 7.3 Hz, 3H).

**Example 9. (*S*)-*N*-((4-ethyl-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)-1-hydroxycyclopropane-1-carboxamide (Compound 9)**

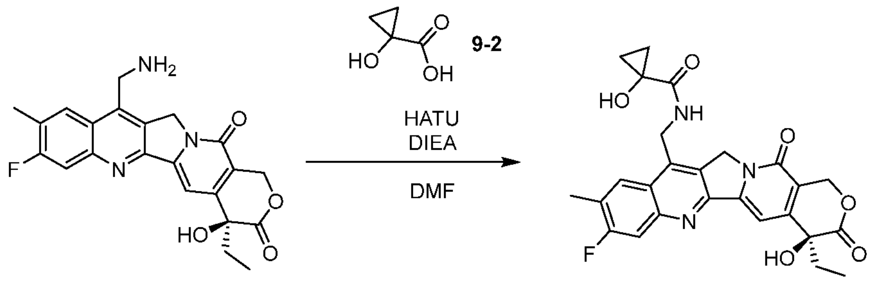


9-1 9

**Intermediate 9-1** (6.00 mg, 14.66 μmol, which can be synthesized according to the method reported in Patent Document WO2020219287) and **intermediate 9-2** (4.49 mg, 43.97 μmol) were dissolved in anhydrous *N,N*-dimethylformamide (0.5 mL), and HATU (8.36 mg, 21.98 μmol) and *N,N*-diisopropylethylamine (5.68 mg, 43.97 μmol) were added thereto. The reaction mixture was stirred at 25 °C for 1 h. The reaction was completed as detected by LC-MS. The reaction mixture was filtered and purified by preparative high performance liquid chromatography (YMC-Actus Triart C18 column, 5 μm silica, 25 mm diameter, 100 mm length; a mixture of water (containing 0.05% formic acid) and acetonitrile with decreasing polarity was used as the eluent; acetonitrile gradient: 6%-36%, elution time: 12 min) to give the title compound (3.00 mg).

MS m/z (ESI): 494.2 $[M+H]^+$.

**$^1$H NMR (400MHz, DMSO-$d_6$)** δ = 8.96 (t, *J* = 6.0 Hz, 1H), 8.50 (d, *J* = 8.3 Hz, 1H), 7.90 (d, *J* = 10.9 Hz, 1H), 7.32 (s, 1H), 6.53 (s, 1H), 6.30 (s, 1H), 5.52 (s, 2H), 5.44 (s, 2H), 4.85 (d, *J* = 5.9 Hz, 2H), 2.53 (s, 3H), 1.92-1.83 (m, 2H), 1.05-1.00 (m, 2H), 0.88 (t, *J* = 7.3 Hz, 3H), 0.85-0.81 (m, 2H).

**Example 10. (*S*)-*N*-((8-chloro-4-ethyl-4-hydroxy-9-methyl-3,14-dioxo-3,4,12,14-tetrahydro-1*H*-pyrano[3',4':6,7] indolizino[1,2-*b*]quinolin-11-yl)methyl)-1-hydroxycyclopropane-1-carboxamide (Compound 10)**

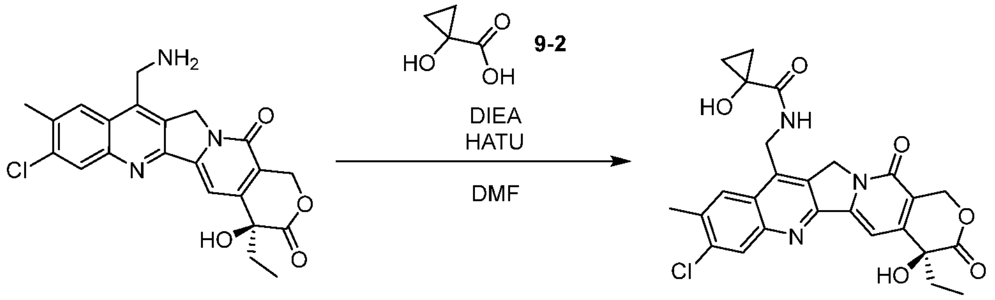


6-4

10

**Intermediate 6-4** (6.24 mg, 14.66 μmol) and **intermediate 9-2** (4.49 mg, 43.97 μmol) were dissolved in *N*,*N*-dimethylformamide (1 mL), and HATU (8.36 mg, 21.98 μmol) and DIEA (5.68 mg, 43.97 μmol) were added thereto. The reaction mixture was stirred at 25 °C for 1 h. The reaction was completed as detected by LC-MS. The reaction mixture was filtered and concentrated to dryness under reduced pressure. The crude product was purified by preparative high performance liquid chromatography (YMC-Actus Triart C18 column, 5 μm silica, 25 mm diameter, 100 mm length; a mixture of water (containing 0.225% formic acid) and acetonitrile with decreasing polarity was used as the eluent; acetonitrile gradient: 16%-46%, elution time: 12 min) to give the title compound (2.20 mg).

MS m/z (ESI): 510.1 $[M+H]^+$.

**1H NMR (400MHz, DMSO-$d_6$)** δ = 8.96 (t, *J* = 5.9 Hz, 1H), 8.54 (s, 1H), 8.26 (s, 1H), 7.32 (s, 1H), 6.54 (s, 1H), 6.29 (s, 1H), 5.52 (s, 2H), 5.44 (s, 2H), 4.84 (d, *J* = 6.0 Hz, 2H), 2.59 (s, 3H), 1.94-1.80 (m, 2H), 1.01 (d, *J* = 3.0 Hz, 2H), 0.88 (t, *J* = 7.3 Hz, 3H), 0.83 (d, *J* = 3.0 Hz, 2H).

**Example 11. *N*-(((*S*)-8-chloro-4-ethyl-4-hydroxy-9-methyl-3,14-dioxo-3,4,12,14-tetrahydro-1*H*-pyrano[3',4':6,7] indolizino[1,2-*b*]quinolin-11-yl)methyl)-2-cyclopropyl-2-hydroxyacetamide (Compound 11)**

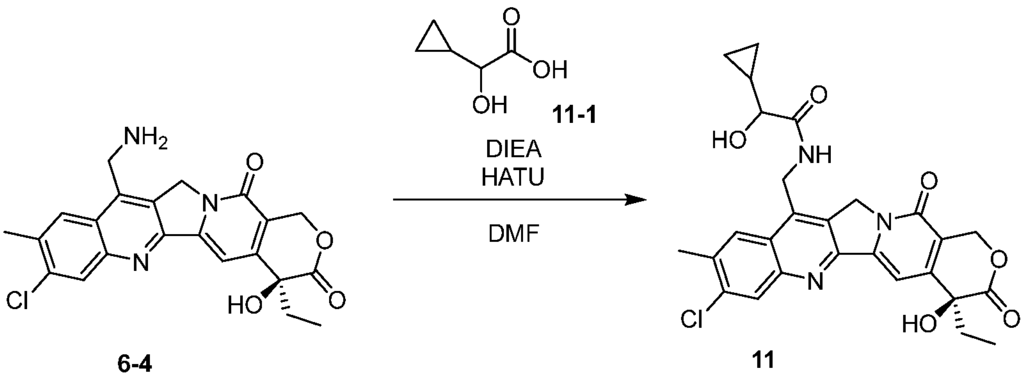


6-4

11

**Intermediate 6-4** (6.0 mg, 14.09 μmol) and **intermediate 11-1** (8.18 mg, 70.45 μmol) were dissolved in N,N-dimethylformamide (0.5 mL), and HATU (8.04 mg, 21.13 μmol) and DIEA (5.46 mg, 42.27 μmol) were added thereto. The reaction mixture was stirred at 25 °C for 1 h. The reaction was completed as detected by LC-MS. The reaction mixture was filtered and concentrated to dryness under reduced pressure, and the crude product was purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 16%-46%, elution time: 12 min) to give the title compound (3.0 mg).

MS m/z (ESI): 524.1 $[M+H]^+$.

**$^1$H NMR (400MHz, DMSO-$d_6$)** δ = 8.66 (t, *J* = 5.9 Hz, 1H), 8.47 (s, 1H), 8.26 (s, 1H), 7.32 (s, 1H), 6.54 (s, 1H), 5.53 (d, *J* = 5.0 Hz, 1H), 5.50 (s, 2H), 5.44 (s, 2H), 4.91-4.76 (m, 2H), 3.61-3.55 (m, 1H), 2.59 (s, 3H), 1.94-1.82 (m, 2H), 1.05-0.97 (m, 1H), 0.88 (t, *J* = 7.3 Hz, 3H), 0.34- 0.31 (m, 2H), 0.29-0.20 (m, 2H).

**Example 12. (*S*)-2-amino-*N*-((7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)acetamide (Compound 12)**

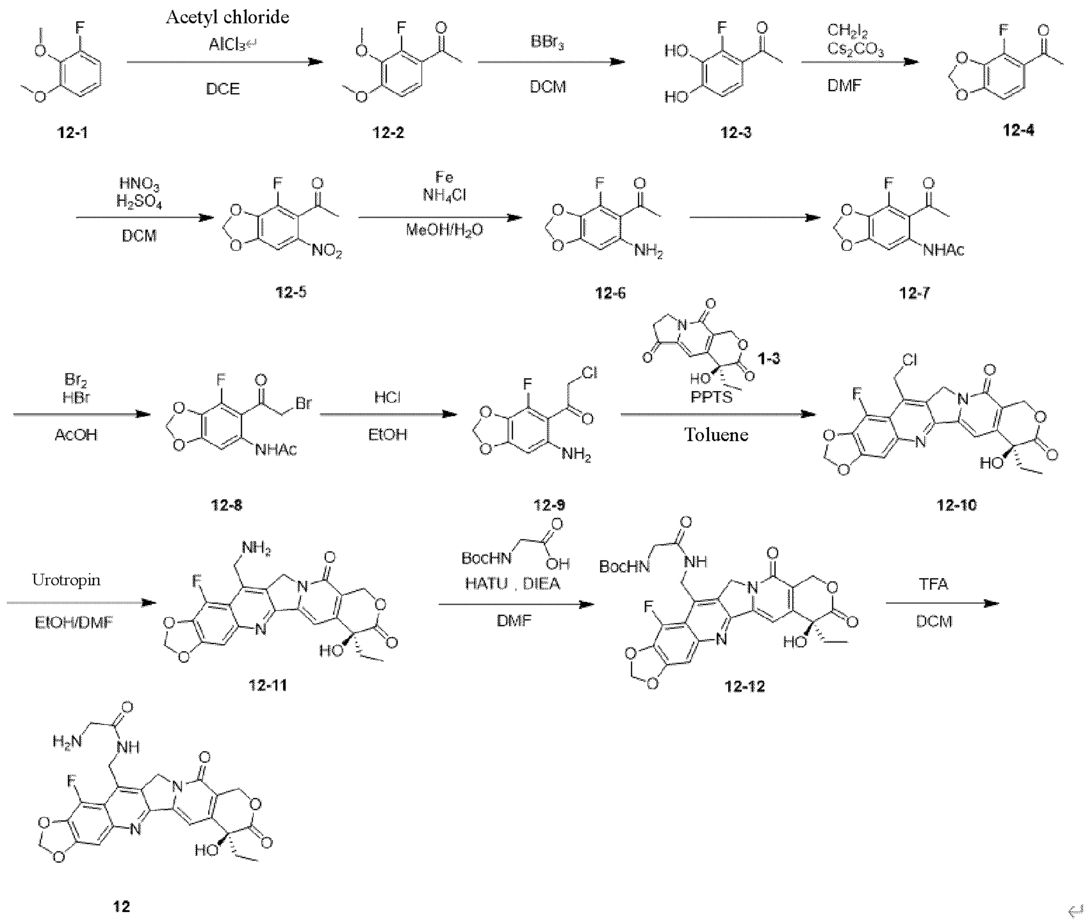


**Step 1: synthesis of 1-(2-fluoro-3,4-dimethoxyphenyl)ethane-1-one (intermediate 12-2)**

**Intermediate 12-1** (25.0 g) was dissolved in 1,2-dichloroethane (DCE, 250 mL). The reaction mixture was cooled to 0 °C, aluminum trichloride (64.04 g) was slowly added thereto, and acetyl chloride (64.04 g) was added dropwise to the reaction mixture. The reaction mixture was stirred at 0 °C for 2 h under nitrogen atmosphere. After the reaction was completed, water (300 mL) was added to the reaction mixture. The mixture was extracted with ethyl acetate (150 mL × 3 times). The organic phases were combined and dried over anhydrous sodium sulfate. The organic phase was filtered and concentrated under reduced pressure. The residue was purified by silica gel column chromatography (ISCO®; 120 g of SepaFlash® flash silica gel column, gradient: 0-50% petroleum ether/ethyl acetate, flow rate: 70 mL/min) to give the title compound (21.0 g).

MS m/z (ESI): 199.0 $[M+H]^+$.

**Step 2: synthesis of 1-(2-fluoro-3,4-dihydroxyphenyl)ethane-1-one (intermediate 12-3)**

**Intermediate 12-2** (15.00 g) was dissolved in anhydrous dichloromethane (DCM, 150 mL). The reaction mixture was cooled to -78 °C, boron tribromide (56.88 g) was slowly added dropwise thereto, and the reaction mixture was stirred at -78 °C for 2 h under nitrogen atmosphere, then warmed to 0 °C and reacted for 4 h. After the reaction was completed, the reaction mixture was slowly added dropwise into ice water to quench the reaction. After the reaction was quenched, the mixture was extracted with ethyl acetate (150 mL × 3 times). The organic phases were combined and dried over anhydrous sodium sulfate. The organic phase was filtered and concentrated under reduced pressure. The residue was purified by silica gel column chromatography (ISCO®; 120 g of SepaFlash® flash silica gel column, gradient: 0-50% petroleum ether/ethyl acetate, flow rate: 70 mL/min) to give the title compound (8.50 g).

MS m/z (ESI): 171.0 $[M+H]^+$.

**Step 3: synthesis of 1-(4-fluorobenzo[*d*][1,3]dioxol-5-yl)ethane-1-one (intermediate 12-4)**

**Intermediate 12-3** (4.0 g) was dissolved in anhydrous *N*,*N*-dimethylformamide (40 mL). Cesium carbonate

(11.49 g) and 1,2-diiodomethane (18.89 g) were added thereto. The reaction mixture was stirred at 100 °C for 8 min under nitrogen atmosphere. After the reaction was completed, the reaction mixture was slowly poured into water and extracted with ethyl acetate (50 mL × 3 times). The organic phases were combined and dried over anhydrous sodium sulfate. The organic phase was filtered and concentrated under reduced pressure. The residue was purified by silica gel column chromatography (ISCO®; 24 g of SepaFlash® flash silica gel column, gradient: 0-15% petroleum ether/ethyl acetate, flow rate: 60 mL/min) to give the title compound (2.0 g).

MS m/z (ESI): 183.0 $[M+H]^+$.

**Step 4: synthesis of 1-(4-fluoro-6-nitrobenzo[*d*][1,3]dioxol-5-yl)ethane-1-one (intermediate 12-5)**

**Intermediate 12-4** (2.0 g) was dissolved in anhydrous dichloromethane (15 mL), concentrated sulfuric acid (5.38 g, 98% mass fraction) was added thereto, and the reaction mixture was cooled to 0 °C. Concentrated nitric acid (3.46 g, 68% mass fraction) was then slowly added dropwise to the reaction mixture. The reaction mixture was stirred at 25 °C for 2 h. After the reaction was completed, the reaction mixture was slowly added dropwise into ice water (50 mL), ethyl acetate (50 mL) was then added, the organic phase was washed with water (50 mL × 2), and the washed organic phase was dried over an appropriate amount of anhydrous sodium sulfate. The organic phase was concentrated to dryness under reduced pressure. The residue was purified by silica gel column chromatography (ISCO®; 24 g of SepaFlash® flash silica gel column, gradient: 0-40% petroleum ether/ethyl acetate, flow rate: 60 mL/min) to give the title compound (1.8 g).

**$^1$H NMR (400MHz, deuterated chloroform)** δ = 7.51 (s, 1H), 6.26 (s, 2H), 2.63 (s, 3H).

**Step 5: synthesis of 1-(6-amino-4-fluorobenzo[*d*][1,3]dioxol-5-yl)ethane-1-one (intermediate 12-6)**

**Intermediate 12-5** (1.8 g) was dissolved in anhydrous methanol (18 mL) and water (9 mL), and ammonium chloride (635.83 mg) and iron powder (2.21 mg) were added. The reaction mixture was stirred at 80 °C for 2 h under nitrogen atmosphere. After the reaction was completed, the reaction mixture was cooled to room temperature. The reaction mixture was filtered, the filtrate was diluted with ethyl acetate (50 mL), the organic phase was washed with water (50 mL × 2), and the washed organic phase was dried over an appropriate amount of anhydrous sodium sulfate. The organic phase was concentrated to dryness under reduced pressure to give the title compound (1.5 g).

MS m/z (ESI): 198.0 $[M+H]^+$.

**Step 6: synthesis of *N*-(6-acetyl-7-fluorobenzo[*d*][1,3]dioxol-5-yl)acetamide (intermediate 12-7)**

**Intermediate 12-6** (500.0 mg) was dissolved in anhydrous dichloromethane (5 mL), pyridine (601.79 mg) was added thereto, and acetyl chloride (398.14 mg) was added dropwise to the reaction under nitrogen atmosphere. The reaction mixture was stirred at 25 °C for 1.5 h under nitrogen atmosphere. After the reaction was completed, the organic phase was concentrated to dryness under reduced pressure. The residue was purified by silica gel column chromatography (ISCO®; 12 g of SepaFlash® flash silica gel column, gradient: 0-40% petroleum ether/ethyl acetate, flow rate: 60 mL/min) to give the title compound (380.0 mg).

**$^1$H NMR (400MHz, deuterated chloroform)** δ = 11.72 (s, 1H), 8.18 (d, *J* = 1.0 Hz, 1H), 6.10 (s, 2H), 2.64 (d, *J* = 8.4 Hz, 3H), 2.22 (s, 3H).

**Step 7: synthesis of *N*-(6-(2-bromoacetyl)-7-fluorobenzo[*d*][1,3]dioxol-5-yl)acetamide (intermediate 12-8)**

**Intermediate 12-7** (380.0 mg) was dissolved in acetic acid (3 mL), a solution of hydrogen bromide in acetic acid (1.95 g, 33% content) was added thereto, and liquid bromine (256.42 mg) was slowly added dropwise to the reaction mixture. The reaction mixture was stirred at 25 °C for 1 h. After the reaction was completed, the reaction mixture was slowly poured into ice water, stirred for 0.5 h, and filtered. The filter cake was washed with water (20 mL × 2), and the filter cake was dried to give the title compound (400.0 mg).

MS m/z (ESI): 317.8 $[M+H]^+$.

**Step 8: synthesis of 1-(6-amino-4-fluorobenzo[*d*][1,3]dioxol-5-yl)-2-chloroethane-1-one (intermediate 12-9)**

**Intermediate 12-8** (400.0 mg) was dissolved in anhydrous ethanol (2 mL) and concentrated hydrochloric acid (2 mL), and the reaction mixture was stirred at 60 °C for 3 h. After the reaction was completed, the reaction mixture was cooled to room temperature, ice water (20 mL) was added slowly, the pH was adjusted to 8 with saturated sodium bicarbonate, and ethyl acetate (40 mL) was added sequentially. The organic phase was washed with water (20 mL × 2), and the washed organic phase was dried over an appropriate amount of anhydrous sodium sulfate. The organic phase was concentrated to dryness under reduced pressure to give the title compound (220.0 mg).

MS m/z (ESI): 232.0 $[M+H]^+$.

**Step 9: synthesis of (*S*)-14-(chloromethyl)-7-ethyl-15-fluoro-7-hydroxy-10,13-dihydro-11*H*-[1,3]dioxolo[4,5-*g*] pyrano[3',4':6,7]indolizino[1,2-*b*]quinoline-8,11(7*H*)-dione (intermediate 12-10)**

**Intermediate 12-9** (200.0 mg) and **intermediate 1-3** (227.32 mg) were dissolved in toluene (3 mL), and pyridinium p-toluenesulfonate (21.70 mg) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (5 mL × 2) to give the title compound (320.0 mg).

MS m/z (ESI): 459.0 $[M+H]^+$.

**Step 10: synthesis of (*S*)-14-(aminomethyl)-7-ethyl-15-fluoro-7-hydroxy-10,13-dihydro-11*H*-[1,3]dioxolo[4,5-*g*] pyrano[3',4':6,7]indolizino[1,2-*b*]quinoline-8,11(7*H*)-dione (intermediate 12-11)**

**Intermediate 12-10** (260.00 mg) was dissolved in anhydrous methanol (1 mL) and anhydrous *N,N*-dimethylformamide (1 mL), and urotropin (238.32 mg) was added thereto. The reaction mixture was stirred at 50 °C for 3 h. After the reaction was completed, the reaction mixture was cooled to room temperature, concentrated to dryness under reduced pressure and purified by preparative high performance liquid chromatography (Boston Prime C18 column, 5 μm silica, 30 mm diameter, 150 mm length; a mixture of water (containing 0.225% formic acid) and acetonitrile with decreasing polarity was used as the eluent (acetonitrile gradient: 0%-30%, elution time: 14 min)) to give the title compound (85.0 mg).

MS m/z (ESI): 440.0 $[M+H]^+$.

**$^1$H NMR (400MHz, DMSO-$d_6$)** $\delta$ = 7.49 (s, 1H), 7.26 (s, 1H), 6.53 (s, 1H), 6.38 (s, 2H), 5.44 (s, 4H), 4.27 (s, 2H), 1.94-1.79 (m, 2H), 0.88 (t, *J* = 7.3 Hz, 3H).

**Step 11: synthesis of *tert-butyl* (*S*)-(2-(((7-ethyl-15-fluoro-7-hydroxy-8,11-dioxo-8,10,11,13-tetrahydro-10*H*-[1,3] dioxolo[4,5-*g*]pyrano[3',4':6,7]indolizino[1,2-*b*]quinolin-14-yl)methyl)amino)-2-oxoethyl)carbamate (intermediate 12-12)**

**Intermediate 12-11** (7 mg) and 2-((*tert*-butoxycarbonyl)amino)acetic acid (5.58 mg) were dissolved in anhydrous *N*,*N*-dimethylformamide (1 mL), and 2-(7-azabenzotriazole)-*N*,*N*,*N*,*N*-tetramethyluronium hexafluorophosphate (12.11 mg) and diisopropylethylamine (2.06 mg) were added thereto. The reaction mixture was stirred at 25 °C for 1 h. After the reaction was completed, the reaction mixture was concentrated to dryness to give the title compound (8.00 mg).

MS m/z (ESI): 597.3 $[M+H]^+$.

**Step 12: synthesis of (*S*)-2-amino-*N*-((7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)acetamide (compound 12)**

**Intermediate 12-12** (6 mg) was dissolved in dichloromethane (0.5 mL), trifluoroacetic acid (902.27 mg) was added thereto, and the reaction mixture was stirred at 25 °C for 1 h. After the reaction was completed, the reaction mixture was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent (acetonitrile gradient: 2%-32%, elution time: 12 min)) to give the title compound (1.3 mg).

MS m/z (ESI): 497.1 $[M+H]^+$.

**$^1$H NMR (400MHz, DMSO-$d_6$)** $\delta$ = 8.80-8.58 (m, 1H), 7.51 (s, 1H), 7.26 (s, 1H), 6.52 (s, 1H), 6.40 (s, 2H), 5.50 (s, 2H), 5.43 (s, 2H), 4.85 (s, 2H), 3.09-2.75 (m, 2H), 1.92-1.79 (m, 2H), 0.87 (t, *J* = 7.1 Hz, 3H).

**Example 13. 2-cyclopropyl-*N*-(((*S*)-8-ethyl-8-hydroxy-9,12-dioxo-2,3,8,9,12,14-hexahydro-1*H*,11*H*-cyclopenta[*f*] pyrano[3',4':6,7]indolizino[1,2-*b*]quinolin-15-yl)methyl)-2-hydroxyacetamide (Compound 13)**

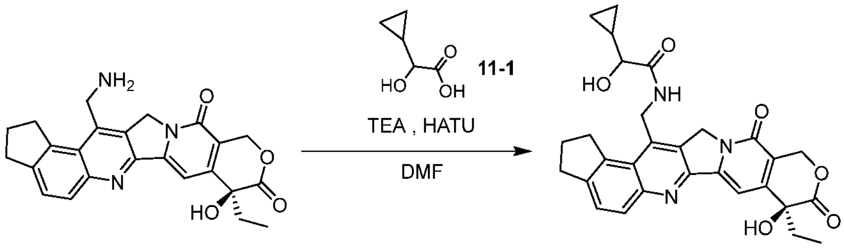


7-9

13

**Intermediate 7-9** (10 mg) and **intermediate 11-1** (8.34 mg) were dissolved in anhydrous *N,N*-dimethylformamide (0.5 mL), HATU (13.66 mg) and diisopropylethylamine (3.10 mg) were added thereto, and the reaction mixture was stirred at 25 °C for 1 h. After the reaction was completed, the reaction mixture was filtered, and the filtrate was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 34%-54%, elution time: 12 min) to give the title compound (0.8 mg).

MS m/z (ESI): 516.2 $[M+H]^+$.

**$^1$H NMR (400MHz, DMSO-$d_6$)** δ = 8.25 (t, *J* = 5.0 Hz, 1H), 8.01 (d, *J* =8.5 Hz, 1H), 7.78 (d, *J* = 8.5 Hz, 1H), 7.30 (s, 1H), 6.53 (s, 1H), 5.43 (s, 2H), 5.36 (s, 2H), 4.99-4.85 (m, 2H), 3.59 (s, 1H), 3.56 (d, *J* = 6.3 Hz, 2H), 3.08 (t, *J* = 7.7 Hz, 2H), 2.23-2.16 (m, 2H), 1.92-1.72 (m, 2H), 1.15-1.01 (m, 1H), 0.88 (t, *J* = 7.4 Hz, 3H), 0.46-0.19 (m, 4H)

**Example 14-1. 2-cyclopropyl-*N*-(((*S*)-7-ethyl-7-hydroxy-8,11-dioxo-7,8,11,13-tetrahydro-10H-[1,3]dioxolo[4,5-*g*] pyrano[3',4':6,7]indolizino[1,2-*b*]quinolin-14-yl)methyl)-2-hydroxyacetamide (Compound 14)**

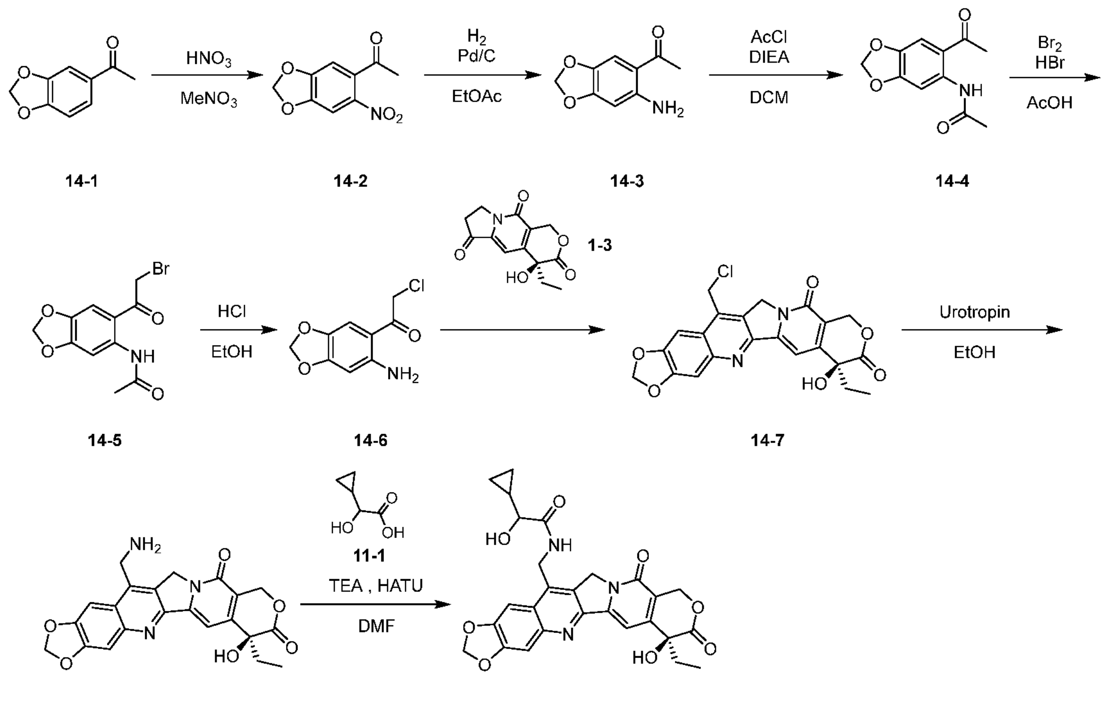


14-1 14-2 14-3 14-4

14-5 14-6 14-7

14-8 14

**Step 1: synthesis of 1-(6-nitrobenzo[*d*][1,3]dioxol-5-yl)ethanone (intermediate 14-2)**

**Intermediate 14-1** (10.0 g, 60.92 mmol) was dissolved in nitromethane (100 mL), nitric acid (35.43 g, 365.50 mmol, 65% purity) was slowly added thereto, and the reaction mixture was stirred at 25 °C for 2.5 h. After the reaction was completed, a saturated sodium bicarbonate solution was slowly added to the reaction mixture to adjust the pH to 7-8, and dichloromethane (100 mL) was then added thereto. The organic phase was washed with water (50 mL × 2), and the

washed organic phase was dried over an appropriate amount of anhydrous sodium sulfate. The organic phase was purified by preparative thin-layer chromatography (petroleum ether:ethyl acetate = 1:2) to give the title compound (5 g).
MS m/z (ESI): 210.0 $[M+H]^+$.

**Step 2: synthesis of 1-(6-aminobenzo[*d*][1,3]dioxol-5-yl)ethanone (intermediate 14-3)**

**Intermediate 14-2** (2.37 g, 11.33 mmol) was dissolved in anhydrous ethanol (25 mL), palladium on carbon (0.2 g, 10% purity) was added thereto, and the reaction mixture was stirred at 25 °C for 16 h under hydrogen atmosphere. After the reaction was completed, the reaction mixture was filtered, the filter cake was washed 2 times with ethyl acetate, and the filtrate was concentrated to dryness under reduced pressure to give the title compound (1.6 g).
MS m/z (ESI): 180.1 $[M+H]^+$.

**Step 3: synthesis of *N*-(6-acetylbenzo[*d*][1,3]dioxol-5-yl)acetamide (intermediate 14-4)**

**Intermediate 14-3** (1.0 g, 5.58 mmol) was dissolved in dichloromethane (10 mL), the reaction mixture was cooled to 0 °C, and *N*,*N*-diisopropylethylamine (DIEA) (1.08 g, 8.37 mmol) and acetyl chloride (569.55 mg, 7.26 mmol) were added thereto. The reaction mixture was stirred at 25 °C for 1.5 h. After the reaction was completed, the reaction mixture was concentrated to dryness under reduced pressure to give the title compound (1.23 g).
MS m/z (ESI):222.1 $[M+H]^+$.

**Step 4: synthesis of *N*-(6-(2-bromoacetyl)benzo[*d*][1,3]dioxol-5-yl)acetamide (intermediate 14-5)**

**Intermediate 14-4** (1.23 g, 5.00 mmol) was dissolved in acetic acid (12 mL), a solution of hydrogen bromide in acetic acid (1.84 g, 7.51 mmol, 33% purity) was added thereto, and then liquid bromine (959.69 mg, 6.01 mmol) was slowly added thereto. The reaction mixture was stirred at 25 °C for 1 h. After the reaction was completed, the reaction mixture was poured into ice water and stirred for 10 min. The mixture was filtered. The filter cake was washed 2 times with water and concentrated to dryness under reduced pressure. Ethyl acetate (2 mL) and petroleum ether (10 mL) were added to the residue. The reaction mixture was stirred at 25 °C for 0.5 h. The reaction mixture was filtered, and the filter cake was dried to give the title compound (500 mg).
MS m/z (ESI): 300.0 $[M+H]^+$.

**Step 5: synthesis of 1-(6-aminobenzo[*d*][1,3]dioxol-5-yl)-2-chloroethanone (intermediate 14-6)**

**Intermediate 14-5** (0.2 g, 666.43 μmol) was dissolved in anhydrous ethanol (1 mL) and concentrated hydrochloric acid (1 mL), and the reaction mixture was stirred at 60 °C for 16 h. After the reaction was completed, the reaction mixture was cooled to room temperature, ice water (10 mL) and saturated sodium bicarbonate (10 mL) were added slowly and sequentially, and then dichloromethane (50 mL) was added. The organic phase was washed with water (20 mL × 2), and the washed organic phase was dried over an appropriate amount of anhydrous sodium sulfate. The organic phase was purified by preparative thin-layer chromatography (petroleum ether:ethyl acetate = 6:1) to give the title compound (160 mg).
MS m/z (ESI): 214.0 $[M+H]^+$.

**Step 6: synthesis of (*S*)-14-(bromomethyl)-7-ethyl-7-hydroxy-10,13-dihydro-11*H*-[1,3]dioxolo [4,5-*g*]pyrano [3',4':6,7]indolizino[1,2-*b*]quinoline-8,11(7*H*)-dione (intermediate 14-7)**

**Intermediate 14-6** (50 mg, 234.06 μmol) and **intermediate 1-3** (61.62 mg, 234.06 μmol) were dissolved in toluene (1 mL), and pyridinium *p*-toluenesulfonate (5.88 mg, 23.41 μmol) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) and dried to give the title compound (60 mg).
MS m/z (ESI): 441.1 $[M+H]^+$.

**Step 7: synthesis of (*S*)-14-(aminomethyl)-7-ethyl-7-hydroxy-10,13-dihydro-11*H*-[1,3]dioxolo [4,5-*g*]pyrano [3',4':6,7]indolizino[1,2-*b*]quinoline-8,11(7*H*)-dione (intermediate 14-8)**

**Intermediate 14-7** (55.00 mg, 124.76 μmol) was dissolved in ethanol (1 mL), and urotropin (52.47 mg, 374.29 μmol) was added thereto. The reaction mixture was stirred at 80 °C for 1.5 h. After the reaction was completed, the reaction mixture was cooled to room temperature, concentrated to dryness under reduced pressure and purified by preparative

high performance liquid chromatography (YMC-Actus Triart C18 column, 5 μm, 25 mm diameter, 100 mm length; a mixture of water (containing 0.225% formic acid) and methanol with decreasing polarity was used as the eluent; methanol gradient: 0%-27%, elution time: 12 min) to give the title compound (10 mg).

MS m/z (ESI): 422.1 [M+H]$^+$.

**Step 8: synthesis of 2-cyclopropyl-*N*-(((*S*)-7-ethyl-7-hydroxy-8,11-dioxo-7,8,11,13-tetrahydro-10*H*-[1,3]dioxolo [4,5-g]pyrano[3',4':6,7]indolizino[1,2-*b*]quinolin-14-yl)methyl)-2-hydroxyacetamide (compound 14)**

**Intermediate 14-8** (10.00 mg, 20.17 μmol) and **intermediate 11-1** (23.42 mg, 201.71 μmol) were dissolved in anhydrous *N*,*N*-dimethylformamide (0.5 mL), and 2-(7-azabenzotriazole)-*N*,*N*,*N*,*N*-tetramethyluronium hexafluorophosphate (HATU) (11.50 mg, 30.26 μmol) and *N,N*-diisopropylethylamine (7.82 mg, 60.51 μmol) were added thereto. The reaction mixture was stirred at 25 °C for 1.5 h. After the reaction was completed, the reaction mixture was filtered and purified by preparative high performance liquid chromatography (YMC-Actus Triart C18 column, 5 μm, 30 mm diameter, 150 mm length; a mixture of water (containing 0.225% formic acid) and acetonitrile with decreasing polarity was used as the eluent; acetonitrile gradient: 4%-44%, elution time: 9 min) to give the title compound (3 mg).

MS m/z (ESI): 520.1 [M+H]$^+$.

**$^1$H NMR (400MHz, DMSO-*d*$_6$)** δ = 8.37 (t, *J* = 5.8 Hz, 1H), 7.62 (s, 1H), 7.28 (s, 1H), 7.00 (s, 1H), 6.25 (s, 1H), 6.05 (s, 2H), 5.27 (d, *J* = 5.0 Hz, 1H), 5.23 (s, 2H), 5.18 (s, 2H), 4.48 (d, *J* = 5.5 Hz, 2H), 1.66-1.55 (m, 2H), 0.76-0.40 (m, 1H), 0.63 (t, *J* = 7.3 Hz, 3H), 0.14-0.06 (m, 2H), 0.05-0.04 (m, 2H).

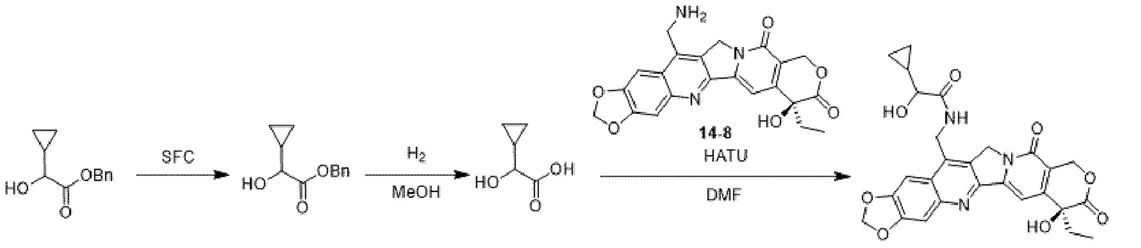

14-9 14-9-P1/P2 14-10-P1/P2 14-8

Compound 14-P1/P2

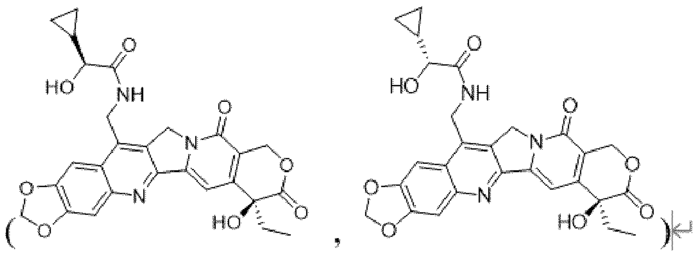

**Step 9: preparation of benzyl 2-cyclopropyl-2-hydroxyacetate (intermediate 14-9-P1/P2)**

Intermediate 14-9 was resolved to prepare isomers 14-9-P1 and 14-9-P2. **Intermediate 14-9** (1.3 g) was prepared by supercritical fluid chromatography (DAICEL CHIRALPAK AD column, 10 μm silica, 30 mm diameter, 250 mm length; ethanol (containing 0.1% ammonia water) was used as the eluent) to give **intermediate 14-9-P1** (600 mg) and **intermediate 14-9-P2** (600 mg).

The two isomers described above were further analyzed by the following chiral supercritical fluid chromatographic conditions.

| | |
|---|---|
| Column | Chiralpak AD-3 150*4.6mm I.D., 3um |

(continued)

| | | |
|---|---|---|
| Mobile phase | A: carbon dioxide | |
| | B: methanol (0.05% diethylamine) | |
| | Time (min) | B phase% |
| | 0 | 5 |
| | 4 | 40 |
| | 0.2 | 5 |
| | 1.8 | 5 |
| Flow rate | 2.5 mL/min | |
| Wavelength | PDA 220nm | |
| Column temperature | 35°C | |
| Column pressure | 1500 Psi | |
| Instrument model | Waters UPCC with PDA Detector | |

**Intermediate 14-9-P1:**

Under the chiral supercritical fluid chromatographic condition described above, the retention time was 2.990 min;

**$^1$H NMR (400MHz, METHANOL-*d*$_4$)** δ 7.43-7.29 (m, 5H), 5.29-5.16 (m, 2H), 3.67 (d, *J* = 7.6 Hz, 1H), 1.19-1.07 (m, 1H), 0.58-0.38 (m, 4H).

**Intermediate 14-9-P2:**

Under the chiral supercritical fluid chromatographic condition described above, the retention time was 2.661 min.

**$^1$H NMR (400MHz, METHANOL-*d*$_4$)** δ 7.46-7.28 (m, 5H), 5.30-5.16 (m, 2H), 3.67 (d, *J* = 7.6 Hz, 1H), 1.21-1.03 (m, 1H), 0.60-0.36 (m, 4H).

**Step 10: synthesis of 2-cyclopropyl-2-hydroxyacetic acid (intermediate 14-10-P1/P2)**

**Intermediate 14-9-P1** (500 mg) was added to methanol (15 mL) under hydrogen atmosphere, wet palladium on carbon (10 mg, 10%) was added to the reaction mixture, and the reaction mixture was stirred at 25 °C for 16 h under hydrogen atmosphere. After the reaction was completed, the reactant was filtered, and the filtrate was concentrated under reduced pressure to give **intermediate 14-10-P1** (273 mg).

**$^1$H NMR (400MHz, METHANOL-*d*$_4$)** δ 3.63 (d, *J* = 7.2 Hz, 1H), 1.21-1.09 (m, 1H), 0.61-0.40 (m, 4H).

**Intermediate 14-9-P2** (500 mg) was added to methanol (15 mL) under hydrogen atmosphere, wet palladium on carbon (10 mg, 10%) was added to the reaction mixture, and the reaction mixture was stirred at 25 °C for 16 h under hydrogen atmosphere. After the reaction was completed, the reactant was filtered, and the filtrate was concentrated under reduced pressure to give **intermediate 14-10-P2** (279 mg).

**$^1$H NMR (400MHz, METHANOL-*d*$_4$)** δ 3.63 (d, *J* = 7.2 Hz, 1H), 1.19-1.08 (m, 1H), 0.60-0.39 (m, 4H).

**Step 11: synthesis of 2-cyclopropyl-*N*-(((*S*)-7-ethyl-7-hydroxy-8,11-dioxo-7,8,11,13-tetrahydro-10*H*-[1,3]dioxolo [4,5-*g*]pyrano[3',4':6,7]indolizino[1,2-*b*]quinolin-14-yl)methyl)-2-hydroxyacetamide (compound 14-P1/P2)**

**Intermediate 14-8** (40.00 mg) and **intermediate 14-10-P1** (28.11 mg) were dissolved in anhydrous *N,N*-dimethylformamide (1 mL), and 2-(7-azabenzotriazole)-*N*,*N*,*N*,*N*-tetramethyluronium hexafluorophosphate (HATU) (46.02 mg) and *N*,*N*-diisopropylethylamine (31.28 mg) were added thereto. The reaction mixture was stirred at 25 °C for 1.5 h. After the reaction was completed, the reaction mixture was purified by preparative high performance liquid chromatography (Boston Green ODS C18 column, 5 μm silica, 30 mm diameter, 150 mm length; a mixture of water (containing 0.225% formic acid) and acetonitrile with decreasing polarity was used as the eluent (acetonitrile gradient: 16%-46%, elution time: 12 min)) to give **compound 14-P1** (22.00 mg).

MS m/z (ESI): 520.1 $[M+H]^+$.

**$^1$H NMR (400MHz, DMSO-*d*$_6$)** δ = 8.62 (t, *J* = 5.7 Hz, 1H), 7.86 (s, 1H), 7.52 (s, 1H), 7.25 (s, 1H), 6.51 (s, 1H), 6.29 (s, 2H), 5.47 (s, 2H), 5.43 (s, 2H), 4.73 (d, *J* = 5.9 Hz, 2H), 3.54 (d, *J* = 5.9 Hz, 1H), 1.93-1.78 (m, 2H), 1.06-0.96 (m, 1H), 0.87

(t, *J* = 7.3 Hz, 3H), 0.39-0.30 (m, 2H), 0.29-0.21 (m, 2H).

**Intermediate 14-8** (10.00 mg) and **intermediate 14-10-P2** (8.27 mg) were dissolved in anhydrous *N*,*N*-dimethylformamide (0.5 mL), and 2-(7-azabenzotriazole)-*N*,*N*,*N*,*N*-tetramethyluronium hexafluorophosphate (18.05 mg) and *N*,*N*-diisopropylethylamine (6.13 mg) were added thereto. The reaction mixture was stirred at 25 °C for 1.5 h. After the reaction was completed, the reaction mixture was directly purified by preparative high performance liquid chromatography (Boston Green ODS C18 column, 5 μm silica, 30 mm diameter, 150 mm length; a mixture of water (containing 0.225% formic acid) and acetonitrile with decreasing polarity was used as the eluent (acetonitrile gradient: 16%-46%, elution time: 12 min)) to give **compound 14-P2** (8.00 mg).

MS m/z (ESI): 520.1 $[M+H]^+$.

**$^1$H NMR (400MHz, DMSO-*d*$_6$)** δ = 8.63 (t, *J* = 5.9 Hz, 1H), 7.86 (s, 1H), 7.52 (s, 1H), 7.24 (s, 1H), 6.30 (s, 2H), 5.46 (s, 2H), 5.43 (s, 2H), 4.72 (d, *J* = 6.0 Hz, 2H), 3.55 (d, *J* = 6.0 Hz, 1H), 1.92-1.81 (m, 2H), 1.03-0.97 (m, 1H), 0.88 (t, *J* = 7.3 Hz, 3H), 0.38-0.30 (m, 2H), 0.28-0.22 (m, 2H).

The two isomers were separately further analyzed by the following chiral supercritical fluid chromatography analysis method.

| Column | Chiralcel OJ-3 100A 4.6mm I.D., 3μm |
|---|---|
| Mobile phase | A: carbon dioxide |
| | B: ethanol (0.05% diethylamine) |
| Flow rate | 2.8 mL/min |
| Wavelength | PDA 254nm |
| Column temperature | 35°C |
| Column pressure | 1500 Psi |
| Instrument model | Waters UPCC with PDA Detector and QDa Detector |

**Compound 14-P1:**

Under the chiral supercritical fluid chromatographic condition described above, the retention time was 3.673 min.

**Compound 14-P2:**

Under the chiral supercritical fluid chromatographic condition described above, the retention time was 3.735 min.

**Example 14-2. (*S*)-2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 14-S)**

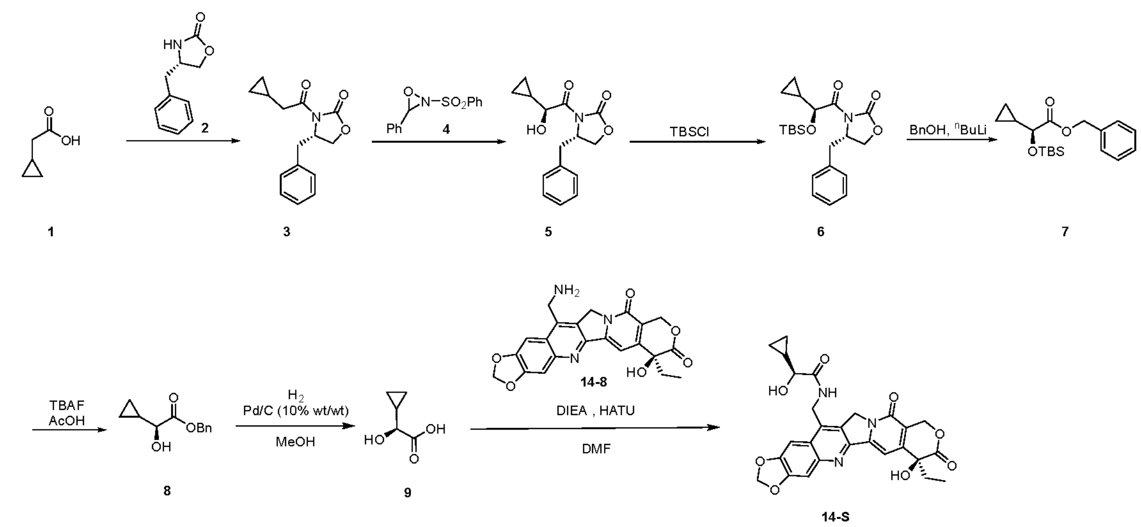

**Step 1: synthesis of (*S*)-4-benzyl-3-(2-cyclopropylacetyl)oxazolidin-2-one (intermediate 3)**

**Starting material 1** (150.0 g), 4-dimethylaminopyridine (160.15 g), and **starting material 2** (221.2 g) were weighed out, dissolved in 1500 mL of dichloromethane, and stirred at room temperature for 15 min. 1-Ethyl-(3-dimethylaminopropyl)carbodiimide hydrochloride (EDCI, 359.0 g) was weighed out, added to the reaction mixture in batches, and stirred at room temperature for about 5 h after the addition was completed. After the reaction was completed, dichloromethane (1500 mL) was added to the reaction mixture for dilution. The mixture was then sequentially washed with water (500 mL) 2 times, washed with 2 N HCl (500 mL) once, washed with a saturated sodium bicarbonate solution (500 mL) once, and washed with a saturated brine solution (500 mL) once. The organic phase was dried over anhydrous sodium sulfate and filtered. The filtrate was concentrated to dryness under reduced pressure to give the title compound (302 g).

**$^1$H NMR (400 MHz, $CDCl_3$)** δ 7.32-7.35 (m, 2H), 7.26-7.29 (m, 1H), 7.21-7.23 (m, 2H), 4.68-4.71 (m, 1H), 4.17-4.23 (m, 2H), 3.30-3.33 (dd, 1H), 2.91-2.95 (dd, 1H), 2.78-2.82 (m, 2H), 1.14-1.18 (m, 1H), 0.59-0.63 (m, 2H), 0.21-0.26 (m, 2H).

**Step 2: synthesis of (*S*)-4-benzyl-3-((*S*)-2-cyclopropyl-2-hydroxyacetyl)oxazolidin-2-one (intermediate 5)**

**Intermediate 3** (200 g) was weighed out, dissolved in 2000 mL of anhydrous tetrahydrofuran, and stirred at -78 °C for 15 min under nitrogen atmosphere. Subsequently, sodium bis(trimethylsilyl)amide (443.5 mL, 2 M in tetrahydrofuran) was added dropwise to the reaction mixture. After the dropwise addition was completed, the reaction mixture was stirred at -78 °C for 30 min. **Intermediate 4** (201.5 g) was completely dissolved in 700 mL of tetrahydrofuran and added dropwise and slowly to the reaction mixture. After the dropwise addition was completed, the mixture was stirred at -78 °C for 2 h. Subsequently, 220 mL of glacial acetic acid was added to the reaction mixture to quench the reaction. After the dropwise addition was completed, the mixture was gradually warmed to room temperature, 600 mL of 2 N HCl was added to the reaction mixture, and the mixture was stirred at room temperature (20-25 °C) for 10 h. Subsequently, the reaction mixture was concentrated under reduced pressure, ethyl acetate (1000 mL) and water (200 mL) were added to the residue, and the mixture was stirred for 20 min. The separated aqueous phase was extracted twice with ethyl acetate (500 mL × 2). The organic phases were combined, and sequentially washed twice with 400 mL of a saturated $NaHCO_3$ solution, twice with 400 mL of a saturated $Na_2S_2O_3$ solution and twice with saturated brine. The resulting organic phase was dried over anhydrous sodium sulfate and filtered. The filtrate was concentrated to dryness under reduced pressure, and the residue was purified by silica gel column chromatography (petroleum ether:ethyl acetate = 1/30) to give the title compound (128.7 g).

**$^1$H NMR (400 MHz, $CDCl_3$)** δ 7.37 (dd, J = 8.1, 6.8 Hz, 2H), 7.33-7.29 (m, 1H), 7.27-7.23 (m, 2H), 4.81 (dd, J = 7.9, 5.9 Hz, 1H), 4.72 (ddt, J = 10.0, 7.5, 2.9 Hz, 1H), 4.33 (t, J = 8.3Hz, 1H), 4.28 (dd, J = 9.1, 2.5 Hz, 1H), 3.48 (dd, J = 8.2, 3.9 Hz, 1H), 3.35 (dd, J = 13.5, 3.4 Hz, 1H), 2.88 (dd, J = 13.5, 9.4 Hz, 1H), 1.36-1.29 (m, 1H), 0.62-0.44 (m, 4H).

**Step 3: synthesis of (*S*)-4-benzyl-3-((*S*)-2-((*tert*-butyldimethylsilyl)oxy)-2-cyclopropylacetyl) oxazolidin-2-one (intermediate 6)**

**Intermediate 5** (128.7 g) was weighed out and dissolved in 1300 mL of dichloromethane. Imidazole (56.17 g) was added, and the mixture was stirred for 15 min under an ice bath. TBSCl (107.3 g) was then added to the reaction mixture in batches, and the mixture was stirred at room temperature for 3 h. To the reaction mixture was added 200 mL of 2 N HCl. The mixture was stirred for 20 min and separated. The organic phase was sequentially washed twice with 200 mL of a saturated $NaHCO_3$ solution and twice with saturated brine. The organic phase was dried over anhydrous sodium sulfate and filtered, the filtrate was concentrated to dryness under reduced pressure, and the residue was purified by silica gel column chromatography (petroleum ether:ethyl acetate = 80:1) to give the title compound (160 g).

**$^1$H NMR (400 MHz, $CDCl_3$)** δ 7.35-7.37 (m, 2H), 7.30-7.32 (m, 1H), 7.26-7.29 (m, 2H), 5.26-5.31 (m, 1H), 4.67-4.71 (m, 1H), 4.20-4.26 (m, 2H), 3.41-3.44 (dd, 1H), 2.72-2.76 (dd, 1H), 1.25-1.31 (m, 1H), 0.94 (s, 9H), 0.54-0.56 (m, 2H), 0.46-0.48 (m, 2H), 0.12 (s, 6H).

**Step 4: synthesis of benzyl (*S*)-2-((*tert*-butyldimethylsilyl)oxy)-2-cyclopropylacetate (intermediate 7)**

Benzyl alcohol (62.18 g) was weighed out, dissolved in 500 mL of tetrahydrofuran, and stirred at - 25 °C. *n*-Butyllithium (213.6 mL, 2.5 M in tetrahydrofuran) was measured out and added dropwise and slowly to the reaction mixture. After the dropwise addition was completed, the mixture was stirred at -25 °C for 1 h. **Intermediate 6** (160 g) was weighed out, dissolved in 320 mL of tetrahydrofuran, and added dropwise and slowly to the reaction mixture at -25°C. After the dropwise addition was completed, the mixture was stirred at -15 °C for 3 h. A saturated $NH_4Cl$ (200 mL) solution was added to the reaction mixture to quench the reaction. The reaction mixture was then concentrated under reduced pressure, and 400 mL of methyl *tert*-butyl ether and water (150 mL) were added to the reaction mixture. The mixture was stirred for 30

min and separated. The aqueous phase was extracted twice with methyl *tert*-butyl ether (200 mL × 2). The organic phases were combined, washed once with saturated brine (250 mL), dried over anhydrous sodium sulfate, and filtered. The filtrate was concentrated under reduced pressure, and the residue was purified by silica gel column chromatography (petroleum ether:ethyl acetate = 100:1) to give the title compound (125 g).

**$^1$H NMR (400 MHz, $CDCl_3$)** δ 7.28-7.40 (m, 5H), 5.17-5.26 (m, 2H), 3.86-3.89 (m, 1H), 1.21-1.46 (m, 1H), 0.90 (s, 9H), 0.45-0.51 (m, 4H), 0.05 (s, 6H).

**Step 5: synthesis of benzyl (*S*)-2-cyclopropyl-2-hydroxyacetate (intermediate 8)**

**Intermediate** 7 (125 g) was weighed out and dissolved in 1200 mL of tetrahydrofuran. Glacial acetic acid (35.1 g) was added, and the mixture was stirred at room temperature for 5 min. Tetrabutylammonium fluoride (TBAF, 585 mL, 1 M in tetrahydrofuran) was then added to the reaction mixture, and the reaction mixture was reacted at 45 °C for 4 h. The reaction mixture was concentrated under reduced pressure to remove tetrahydrofuran (600 mL). 300 mL of water and 400 mL of methyl *tert*-butyl ether were added to the residue. The mixture was stirred for 20 min and separated. The aqueous phase was extracted twice with methyl *tert*-butyl ether (200 mL). The organic phases were combined, and sequentially washed twice with 200 mL of a saturated $NaHCO_3$ solution and twice with saturated brine. The organic phase was dried over anhydrous sodium sulfate and filtered, the filtrate was concentrated to dryness under reduced pressure, and the residue was purified by silica gel column chromatography (petroleum ether:ethyl acetate = 60:1) to give the title compound (68.9 g).

**$^1$H NMR (400 MHz, $CDCl_3$)** δ 7.27-7.39 (m, 5H), 5.22-5.28 (m, 2H), 3.82 (d, 1H), 2.7 (brs, 1H), 1.11-1.15 (m, 1H), 0.41-0.56 (m, 4H).

**Intermediate 8** was further analyzed by the following chiral supercritical fluid chromatographic conditions.

| | | |
|---|---|---|
| Column | Chiralpak AD-3 150*4.6mm I.D., 3um | |
| Mobile phase | A: carbon dioxide | |
| | B: methanol (0.05% diethylamine) | |
| | Time (min) | B phase% |
| | 0 | 5 |
| | 4 | 40 |
| | 0.2 | 5 |
| | 1.8 | 5 |
| Flow rate | 2.5 mL/min | |
| Wavelength | PDA 220nm | |
| Column temperature | 35°C | |
| Column pressure | 1500 Psi | |
| Instrument model | Waters UPCC with PDA Detector | |

Under the chiral supercritical fluid chromatographic condition described above, the retention time of **intermediate 8** was 3.013 min, which was substantially consistent with the retention time (2.990 min) of **intermediate 14-9-P1** in Example 14-1 under the same chromatographic analysis condition. **Intermediate 8** was the same configuration as **intermediate 14-9-P1,** both being the same compound.

**Step 6: synthesis of (*S*)-2-cyclopropyl-2-hydroxyacetic acid (intermediate 9)**

**Intermediate 8** (5 g) was dissolved in methanol (80 mL), wet palladium on carbon (10% mass content, 0.7 g) was added to the reaction mixture, and the mixture was stirred at 25 °C for 16 h under hydrogen atmosphere. After the reaction was completed, the reaction mixture was filtered, and the filtrate was concentrated to dryness under reduced pressure to give the title compound (2.4 g).

**$^1$H NMR (400MHz, METHANOL-$d_4$)** δ = 3.63 (d, *J* = 7.3 Hz, 1H), 1.20-1.09 (m, 1H), 0.61-0.39 (m, 4H).

**Step 7: synthesis of (*S*)-2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (compound 14-S)**

**Intermediate 14-8** (90 mg) and **intermediate 9** (49.60 mg) were dissolved in anhydrous *N,N*-dimethylformamide (1 mL), and O-(7-azabenzotriazol-1-yl)-*N,N,N,N*-tetramethyluronium hexafluorophosphate (121.81 mg) and *N,N*-diisopropylethylamine (82.81 mg) were added thereto. The reaction mixture was stirred at 25 °C for 16 h. After the reaction was completed, the reaction mixture was purified by high performance liquid chromatography (column: Boston Green ODS 150 × 30 mm × 5 μm; mobile phase: [A: water (0.225% formic acid), B: acetonitrile]; B: 20%-50%, 12 min) to give the title compound (21 mg).

MS m/z (ESI): 520.0[M+H]$^+$.

**$^1$H NMR (400MHz, DMSO-$d_6$)** δ = 8.62 (t, *J* = 6.1 Hz, 1H), 7.87 (s, 1H), 7.52 (s, 1H), 7.24 (s, 1H), 6.48 (s, 1H), 6.30 (s, 2H), 5.48 (s, 2H), 5.43 (s, 2H), 4.73 (d, *J* =5.6 Hz, 2H), 3.54 (d, *J* = 5.5 Hz, 1H), 1.93-1.80 (m, 2H), 1.05-0.97 (m, 1H), 0.88 (t, *J* = 7.3 Hz, 3H), 0.39-0.30 (m, 2H), 0.30-0.22 (m, 2H). **Compound 14-S** was further analyzed by the following chiral supercritical fluid chromatographic conditions.

| | |
|---|---|
| Column | Chiralcel OJ-3 100A 4.6mm I.D., 3μm |
| Mobile phase | A: carbon dioxide |
| | B: ethanol (0.05% diethylamine) |
| Flow rate | 2.8 mL/min |
| Wavelength | PDA 254nm |
| Column temperature | 35°C |
| Column pressure | 1500 Psi |
| Instrument model | Waters UPCC with PDA Detector and QDa Detector |

Under the chiral supercritical fluid chromatographic condition described above, the retention time of **compound 14-S** was 3.654 min, which was substantially consistent with the retention time (3.673 min) of **compound 14-P1** prepared in Example 14-1 under the same chromatographic analysis condition. Thus, **compound 14-S** was determined to have the same configuration as **compound 14-P1** prepared in Example 14-1, both being the same compound.

**Example 15. (*S*)-*N*-((9-bromo-4-ethyl-4-hydroxy-3,14-dioxo-3,4,12,14-tetrahydro-1*H*-pyrano [3',4':6,7]indolizino [1,2-*b*][1,7]naphthyridin-11-yl)methyl)-2-hydroxyacetamide (Compound 15)**

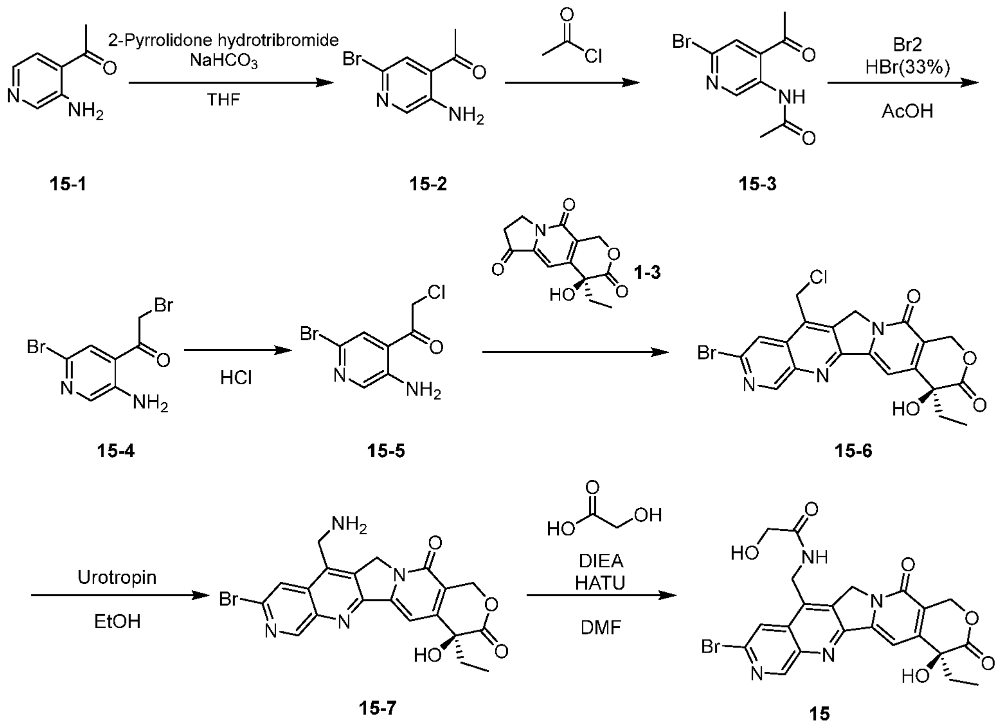


15-1 15-2 15-3 15-4 15-5 15-6 15-7 15

**Step 1: synthesis of 1-(5-amino-2-bromopyridin-4-yl)ethanone (intermediate 15-2)**

**Intermediate 15-1** (500 mg, 3.67 mmol) was dissolved in anhydrous tetrahydrofuran (90 mL), sodium bicarbonate (617.04 mg, 7.34 mmol) and 2-pyrrolidone hydrotribromide (1.64 g, 5.03 mmol) were added thereto, and the reaction mixture was stirred at 25 °C for 8 h. After the reaction was completed, the reaction mixture was filtered and purified by preparative thin-layer chromatography (silica, petroleum ether:ethyl acetate = 20:1) to give the title compound (300 mg).
MS m/z (ESI): 214.9 $[M+H]^+$.

**Step 2: synthesis of *N*-(4-acetyl-6-bromopyridin-3-yl)acetamide (intermediate 15-3)**

**Intermediate 15-2** (150 mg, 697.52 mmol) was dissolved in dichloromethane (2 mL), the reaction mixture was cooled to 0 °C, and *N*,*N*-diisopropylethylamine (180.30 mg, 1.40 mmol) and acetyl chloride (109.51 mg, 1.40 mmol) were added thereto. The reaction mixture was stirred at 25 °C for 3 h. After the reaction was completed, the reaction mixture was concentrated to dryness under reduced pressure and purified by preparative thin-layer chromatography (silica, petroleum ether:ethyl acetate = 3:1) to give the title compound (100 mg).
MS m/z (ESI):257.0 $[M+H]^+$.

**Step 3: synthesis of 1-(5-amino-2-bromopyridin-4-yl)-2-bromoethanone (intermediate 15-4)**

**Intermediate 15-3** (95.00 mg, 273.45 μmol) was dissolved in acetic acid (2 mL), a solution of hydrogen bromide in acetic acid (100.57 g, 410.18 μmol, 33% purity) was added thereto, and then liquid bromine (48.07 mg, 300.80 μmol) was slowly added thereto. The reaction mixture was stirred at 25 °C for 1 h. After the reaction was completed, the reaction mixture was concentrated to dryness under reduced pressure and purified by preparative thin-layer chromatography (silica, petroleum ether:ethyl acetate = 3:1) to give the title compound (45 mg).
MS m/z (ESI): 292.9 $[M+H]^+$.

**Step 4: synthesis of 1-(5-amino-2-bromopyridin-4-yl)-2-chloroethanone (intermediate 15-5)**

**Intermediate 15-4** (50.00 mg, 170.10 μmol) was dissolved in concentrated hydrochloric acid (1 mL), and the reaction mixture was stirred at 60 °C for 16 h. After the reaction was completed, the reaction mixture was cooled to room temperature, ice water (10 mL) and saturated sodium bicarbonate (10 mL) were added slowly and sequentially, and then dichloromethane (30 mL) was added. The organic phase was washed with water (20 mL × 2), and the washed organic phase was dried over an appropriate amount of anhydrous sodium sulfate. The organic phase was concentrated to dryness under reduced pressure to give the title compound (25 mg).

MS m/z (ESI): 248.9 $[M+H]^+$.

**Step 5: synthesis of (*S*)-9-bromo-11-(chloromethyl)-4-ethyl-4-hydroxy-1,12-dihydro-14*H*-pyrano[3',4':6,7]indolizino[1,2-*b*][1,7]naphthyridine-3,14(4*H*)-dione (intermediate 15-6)**

**Intermediate 15-5** (25 mg, 100.20 μmol) and **intermediate 1-3** (26.38 mg, 100.20 μmol) were dissolved in toluene (0.5 mL), and pyridinium *p*-toluenesulfonate (2.52 mg, 10.02 μmol) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) to give the title compound (30 mg).
MS m/z (ESI): 475.9 $[M+H]^+$.

**Step 6: synthesis of (*S*)-11-(aminomethyl)-9-bromo-4-ethyl-4-hydroxy-1,12-dihydro-14*H*-pyrano[3',4':6,7]indolizino[1,2-*b*][1,7]naphthyridine-3,14(4*H*)-dione (intermediate 15-7)**

**Intermediate 15-6** (30 mg, 62.93 μmol) was dissolved in ethanol (1 mL), and urotropin (17.64 mg, 125.86 μmol) was added thereto. The reaction mixture was stirred at 80 °C for 2 h. After the reaction was completed, the reaction mixture was cooled to room temperature, concentrated to dryness under reduced pressure and purified by preparative high performance liquid chromatography (YMC-Actus Triart C18 column, 5 μm, 25 mm diameter, 100 mm length; a mixture of water (containing 0.225% formic acid) and methanol with decreasing polarity was used as the eluent; methanol gradient: 0%-25%, elution time: 12 min) to give the title compound (4 mg).
MS m/z (ESI): 457.0 $[M+H]^+$.

**Step 7: synthesis of (*S*)-*N*-((9-bromo-4-ethyl-4-hydroxy-3,14-dioxo-3,4,12,14-tetrahydro-1*H*-pyrano[3',4':6,7]indolizino[1,2-*b*][1,7]naphthyridin-11-yl)methyl)-2-hydroxyacetamide (compound 15)**

**Intermediate 15-7** (4 mg, 8.75 μmol) and hydroxyacetic acid (3.33 mg, 43.74 μmol) were dissolved in anhydrous *N*,*N*-dimethylformamide (0.5 mL), and HATU (4.99 mg, 13.12 μmol) and *N,N*-diisopropylethylamine (3.39 mg, 26.24 μmol) were added thereto. The reaction mixture was stirred at 25 °C for 2 h. After the reaction was completed, the reaction mixture was filtered and purified by preparative high performance liquid chromatography (YMC-Actus Triart C18 column, 5 μm, 25 mm diameter, 100 mm length; a mixture of water (containing 0.05% formic acid) and acetonitrile with decreasing polarity was used as the eluent; acetonitrile gradient: 8%-28%, elution time: 12 min) to give the title compound (1.00 mg).
MS m/z (ESI): 515.0 $[M+H]^+$.
**$^1$H NMR (400MHz, DMSO-$d_6$)** δ = 9.38 (s, 1H), 8.88 (t, *J* = 6.2 Hz, 1H), 8.76 (s, 1H), 7.39 (s, 1H), 6.57 (s, 1H), 5.64-5.61 (m, 1H), 5.60 (s, 2H), 5.45 (s, 2H), 4.80 (d, *J* = 6.0 Hz, 2H), 3.83 (d, *J* = 5.7 Hz, 2H), 1.92-1.80 (m, 2H), 0.88 (t, *J* = 7.3 Hz, 3H).

**Example 16. (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-1-hydroxycyclopropane-1-carboxamide (Compound 16)**

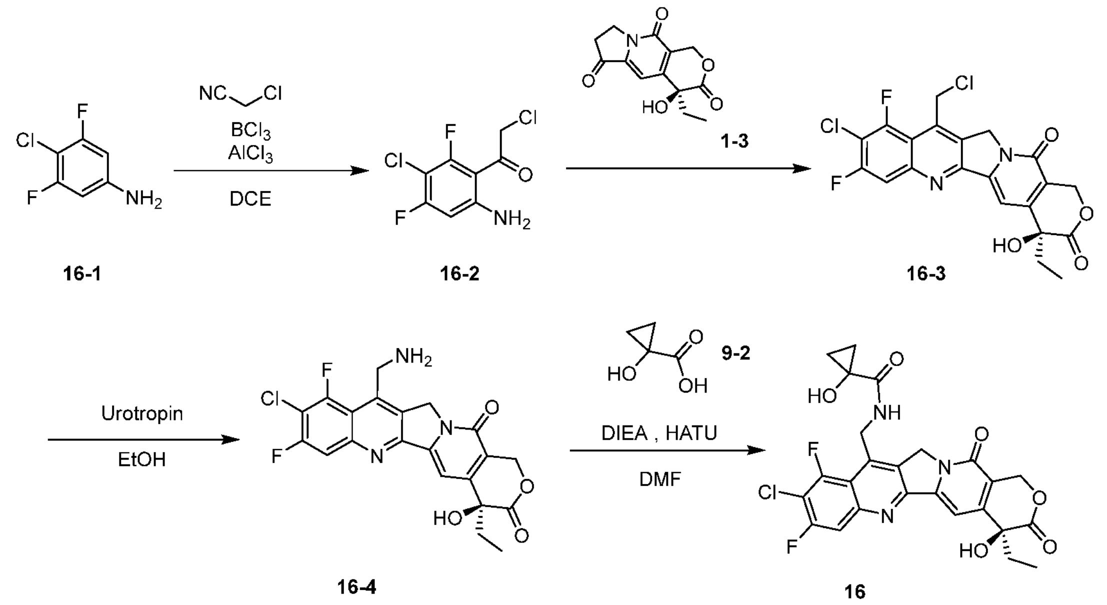


**Step 1: synthesis of 1-(6-amino-3-chloro-2,4-difluorophenyl)-2-chloroethane-1-one (intermediate 16-2)**

Boron trichloride (1 M, 6.11 mL) was dissolved in 1,2-dichloroethane (12 mL). The reaction mixture was cooled to 0 °C, and **reactant 16-1** (1 g, 6.11 mmol) and chloroacetonitrile (784.73 mg, 10.39 mmol) were added thereto. The reaction was stirred at 0 °C for 10 min, and aluminum trichloride (1.06 g, 7.95 mmol) was added thereto. Then the reaction mixture was warmed to 25 °C and stirred for 10 min under nitrogen atmosphere. The reaction mixture was stirred at 90 °C for 18 h under nitrogen atmosphere. The reaction was completed as detected by LC-MS. After the reaction mixture was cooled to room temperature, ice water (25 mL) and 5% hydrochloric acid (5 mL) were added sequentially and slowly and stirred at 25 °C for 30 min, and dichloromethane (50 mL) was added. The organic phase was washed with water (2 mL × 2), and the washed organic phase was dried over an appropriate amount of anhydrous sodium sulfate. The organic phase was concentrated to dryness under reduced pressure, and the crude product was purified by preparative thin-layer chromatography (silica, petroleum ether:ethyl acetate = 9:1) to give the title compound (340 mg).
MS m/z (ESI): 240.0 $[M+H]^+$.

**Step 2: synthesis of (*S*)-9-chloro-11-(chloromethyl)-4-ethyl-8,10-difluoro-4-hydroxy-1,12-dihydro-14*H*-pyrano [3',4':6,7]indolizino[1,2-*b*]quinoline-3,14(4*H*)-dione (intermediate 16-3)**

**Intermediate 16-2** (0.2 g, 833.22 μmol) and **intermediate 1-3** (219.34 mg, 833.22 μmol) were dissolved in toluene (4 mL), and pyridinium *p*-toluenesulfonate (20.94 mg, 83.32 μmol) was added thereto. The reaction mixture was stirred at 100 °C for 18 h. The reaction was completed as detected by LC-MS. After the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) to give a crude product of the title compound (190 mg).
MS m/z (ESI): 467.1 $[M+H]^+$.

**Step 3: synthesis of (*S*)-11-(aminomethyl)-9-chloro-4-ethyl-8,10-difluoro-4-hydroxy-1,12-dihydro-14*H*-pyrano [3',4':6,7]indolizino[1,2-*b*]quinoline-3,14(4*H*)-dione (16-4)**

**Intermediate 16-3** (50 mg, 107.01 μmol) was dissolved in ethanol (1 mL), and urotropin (45.00 mg, 321.03 μmol) was added thereto. The reaction mixture was stirred at 80 °C for 1.5 h. The reaction was completed as detected by LC-MS. After the reaction mixture was cooled to room temperature, the reaction mixture was concentrated to dryness under reduced pressure and purified by preparative high performance liquid chromatography (YMC-Actus Triart C18 column, 5 μm silica, 25 mm diameter, 100 mm length; a mixture of water (containing 0.225% formic acid) and acetonitrile with decreasing polarity was used as the eluent; acetonitrile gradient: 2%-32%, elution time: 12 min) to give the title compound (1.22 mg).
MS m/z (ESI): 448.0 $[M+H]^+$.
**$^1$H NMR (400MHz, DMSO-$d_6$)** δ = 8.14 (d, *J* = 9.8 Hz, 1H), 7.36 (s, 1H), 6.57 (s, 1H), 5.55 (s, 2H), 5.46 (s, 2H), 4.35 (d, *J* = 3.0 Hz, 2H), 1.94-1.83 (m, 2H), 0.88 (t, *J* = 7.3 Hz, 3H).

**Step 4: synthesis of (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-1-hydroxycyclopropane-1-carboxamide (compound 16)**

**Compound 16-4** (5.75 mg, 12.84 μmol) and **intermediate 9-2** (3.93 mg, 38.52 μmol) were dissolved in *N,N*-dimethylformamide (0.5 mL), and HATU (7.32 mg, 19.26 μmol) and diisopropylethylamine (4.98 mg, 38.52 μmol) were added thereto. The reaction mixture was stirred at 30 °C for 1 h. After the reaction was completed, the reaction mixture was filtered and purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 14%-34%, elution time: 12 min) to give the title compound (3 mg).

MS m/z (ESI): 532.1 $[M+H]^+$.

**$^1$H NMR (400MHz, DMSO-*d*$_6$)** δ = 8.46 (t, *J* = 5.9 Hz, 1H), 8.16 (d, *J* = 9.8 Hz, 1H), 7.36 (s, 1H), 6.56 (s, 1H), 6.33 (s, 1H), 5.53 (s, 2H), 5.45 (s, 2H), 4.93 (d, *J* = 3.6 Hz, 2H), 1.92-1.82 (m, 2H), 1.04-0.99 (m, 2H), 0.90-0.87 (m, 2H), 0.87-0.82 (m, 3H).

**Example 17. (*S*)-*N*-((8-chloro-4-ethyl-4-hydroxy-9-methyl-3,14-dioxo-3,4,12,14-tetrahydro-1H-pyrano[3',4':6,7]indolizino[1,2-*b*]quinolin-11-yl)methyl)-2-hydroxy-2-methylpropanamide (Compound 17)**

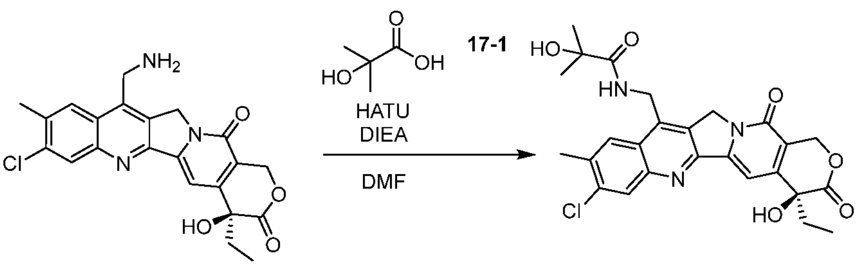


**6-4** **17**

**Intermediate 6-4** (5 mg, 11.74 μmol) and **intermediate 17-1** (2.44 mg, 23.48 μmol) were dissolved in anhydrous *N,N*-dimethylformamide (0.5 mL), and HATU (8.95 mg, 23.48 μmol) and *N,N*-diisopropylethylamine (1.19 mg, 11.74 μmol) were added thereto. The reaction mixture was stirred at 25 °C for 1 h. After the reaction was completed, the reaction mixture was filtered and purified by preparative high performance liquid chromatography (Waters Xbridge C18 column, 5 μm silica, 25 mm diameter, 100 mm length; a mixture of water (containing 0.05% formic acid) and acetonitrile with decreasing polarity was used as the eluent; acetonitrile gradient: 22%-42%, elution time: 12 min) to give the title compound (1 mg).

MS m/z (ESI): 512.1 $[M+H]^+$.

**$^1$H NMR (400MHz, Methanol-*d*$_4$)** δ = 8.32 (s, 1H), 8.22 (s, 1H), 7.67 (s, 1H), 5.62 (d, *J* = 16.4 Hz, 1H), 5.52 (s, 2H), 5.42 (d, *J* = 16.4 Hz, 1H), 5.01 (s, 2H), 2.65 (s, 3H), 2.05-1.94 (m, 2H), 1.38 (s, 6H), 1.03 (t, *J* = 7.5 Hz, 3H).

**Example 18. *N*-(((*S*)-8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxy-3-methylbutanamide (Compound 18)**

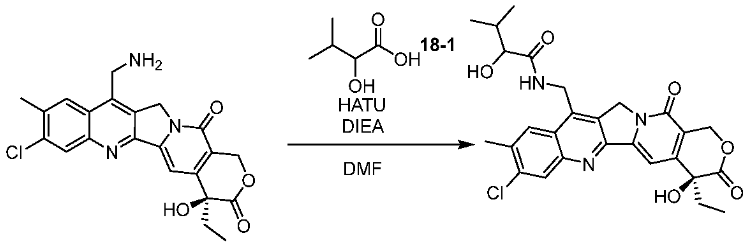


**6-4** **18**

**Intermediate 6-4** (5 mg, 11.74 μmol) and **intermediate 18-1** (2.77 mg, 23.48 μmol) were dissolved in anhydrous *N,N*-dimethylformamide (0.5 mL), and HATU (8.95 mg, 23.48 μmol) and *N,N*-diisopropylethylamine (1.19 mg, 11.74 μmol) were added thereto. The reaction mixture was stirred at 25 °C for 1 h. After the reaction was completed, the reaction

mixture was filtered and purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 25%-45%, elution time: 12 min) to give the title compound (1.20 mg).

MS m/z (ESI): 526.1 $[M+H]^+$.

**$^1$H NMR (400MHz, Methanol-*d*$_4$)** δ = 8.30 (s, 1H), 8.13 (s, 1H), 7.61 (s, 1H), 5.60 (d, *J* = 16.3 Hz, 1H), 5.56-5.45 (m, 2H), 5.42-5.37 (m, 1H), 5.00-4.90 (m, 2H), 3.91 (d, *J* = 3.3 Hz, 1H), 3.13 (d, *J* = 6.5 Hz, 1H), 2.62 (s, 3H), 2.01-1.94 (m, 2H), 1.05-1.00 (m, 6H), 0.77-0.70 (m, 3H).

**Example 19-1. 2-cyclopropyl-N-(((S)-7-ethyl-7-hydroxy-8,11-dioxo-7,8,11,13-tetrahydro-10H-[1,3]dioxolo[4,5-*g*]pyrano[3',4':6,7]indolizino[1,2-*b*]quinolin-14-yl-2,2-*d*$_2$)methyl)-2-hydroxyacetamide (Compound 19, Compound 19-P1/P2)**

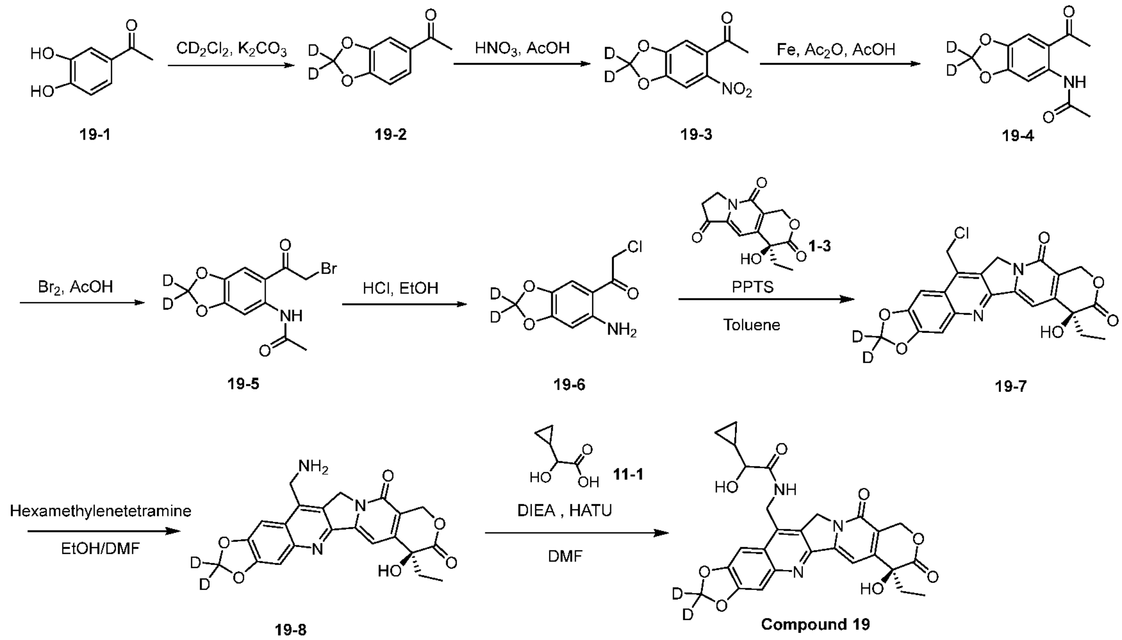


**Step 1: synthesis of 1-(benzo[*d*][1,3]dioxol-5-yl-2,2-*d*$_2$)ethane-1-one (intermediate 19-2)**

**Intermediate 19-1** (3 g) was dissolved in an anhydrous DMF solution (25 mL), deuterated dichloromethane (8.57 g) and potassium carbonate (8.18 g) were added, and after the addition was completed, the mixture was warmed to 90 °C and stirred for 16 h. The reaction mixture was then added to water (100 mL), and the mixture was extracted with ethyl acetate (200 mL × 2). The organic phases were combined, washed with saturated brine (100 mL) and dried over anhydrous sodium sulfate. After filtration, the filtrate was concentrated to dryness under reduced pressure, and the residue was purified by column chromatography (ethyl acetate/petroleum ether = 5:1) to give the title compound (2.4 g).

MS m/z (ESI): 167.1$[M+H]^+$.

**Step 2: synthesis of 1-(6-nitrobenzo[*d*][1,3]dioxol-5-yl-2,2-*d*$_2$)ethane-1-one (intermediate 19-3)**

**Intermediate 19-2** (2.4 g) was dissolved in anhydrous acetic acid (10 mL), concentrated nitric acid (32.50 g, 70% content) was added dropwise at 0 °C, and after the addition was completed, the mixture was stirred at 0 °C for 10 min. Then the mixture was warmed to room temperature and stirred for 1 h. After the reaction was completed, the reaction mixture was added dropwise to ice water (200 mL), and after filtration, the filter cake was dried to give the title compound (1.9 g).

MS m/z (ESI): 212.0 $[M+H]^+$.

**$^1$H NMR (400 MHz, DMSO-*d*$_6$)** δ 7.69 (s, 1H), 7.30 (s, 1H), 2.49 (s, 3H).

**Step 3: synthesis of *N*-(6-acetylbenzo[*d*][1,3]dioxol-5-yl-2,2-*d*$_2$)acetamide (intermediate 19-4)**

**Intermediate 19-3** (1.8 g) was dissolved in acetic acid (25 mL), acetic anhydride (1.84 g) and reduced iron

powder (4.76 g) were added, and the mixture was stirred at room temperature for 1 h. After the reaction was completed, the mixture was filtered, the filtrate was concentrated to dryness under reduced pressure, and the residue was purified by column chromatography (ethyl acetate/petroleum ether = 5:1) to give the title compound (1.5 g).

MS m/z (ESI): 224.1 $[M+H]^+$.

**Step 4: synthesis of *N*-(6-(2-bromoacetyl)benzo[*d*][1,3]dioxol-5-yl-2,2-*d*$_2$)acetamide (intermediate 19-5)**

A solution of HBr in acetic acid (2.39 g, 33% content) was added dropwise to a solution of **intermediate 19-4** (1.45 g) in anhydrous acetic acid (25 mL), and $Br_2$ (1.07 g) was added dropwise. After the dropwise addition was completed, the mixture was stirred at room temperature for 1 h. After the reaction was completed, the reaction mixture was concentrated to dryness under reduced pressure. The residue was added to water (50 mL), and the mixture was extracted with ethyl acetate (50 mL × 2). The organic phase was washed with saturated brine (50 mL), dried over anhydrous sodium sulfate, and filtered. The residue was purified by column chromatography (ethyl acetate/petroleum ether = 5:1) to give the title compound (1.3 g).

MS m/z (ESI): 302.1 $[M+H]^+$.

**Step 5: synthesis of 1-(6-aminobenzo[*d*][1,3]dioxol-5-yl-2,2-*d*$_2$)-2-chloroethane-1-one (intermediate 19-6)**

**Intermediate 19-5** (1.2 g) and concentrated hydrochloric acid (144.82 mg) were dissolved in ethanol (15 mL), and the reaction mixture was stirred at 60 °C for 16 h. After the reaction was completed, the reaction mixture was concentrated to dryness under reduced pressure. The residue was purified by high performance liquid chromatography (YMC-Actus Triart C18 column, 5 μm silica, 30 mm diameter, 150 mm length; a mixture of water (containing 0.05% $NH_4HCO_3$) and acetonitrile with decreasing polarity was used as the eluent (acetonitrile gradient: 40%-50%)) to give the title compound (577 mg).

MS m/z (ESI): 216.0 $[M+H]^+$.

**Step 6: synthesis of (*S*)-14-(chloromethyl)-7-ethyl-7-hydroxy-10,13-dihydro-11*H*-[1,3]dioxolo [4,5-*g*]pyrano [3',4':6,7]indolizino[1,2-*b*]quinoline-8,11(7*H*)-dione-2,2-*d*$_2$ (intermediate 19-7)**

**Intermediate 19-6** (100.0 mg) and **intermediate 1-3** (109.87 mg) were dissolved in toluene (1 mL) and acetic acid (1 mL), and pyridinium *p*-toluenesulfonate (5.24 mg) was added thereto. The reaction mixture was stirred at 100 °C for 16 h. After the reaction was completed, the reaction mixture was cooled to room temperature, and the reaction mixture was directly concentrated to dryness under reduced pressure. Ethanol (5 mL) was added, and the reaction mixture was stirred at 25 °C for 0.5 h. The reaction mixture was filtered, and the filter cake was washed with ethanol (5 mL × 2) to give the title compound (100.0 mg).

MS m/z (ESI): 443.0 $[M+H]^+$.

**Step 7: synthesis of (*S*)-14-(aminomethyl)-7-ethyl-7-hydroxy-10,13-dihydro-11*H*-[1,3]dioxolo [4,5-*g*]pyrano [3',4':6,7]indolizino[1,2-*b*]quinoline-8,11(7*H*)-dione-2,2-*d*$_2$ (intermediate 19-8)**

**Intermediate 19-7** (100.00 mg) was dissolved in anhydrous ethanol (1.5 mL) and anhydrous *N,N*-dimethylformamide (1.5 mL), and urotropin (94.97 mg) was added thereto. The reaction mixture was stirred at 50 °C for 6 h. After the reaction was completed, the reaction mixture was concentrated to dryness under reduced pressure, and the residue was purified by high performance liquid chromatography (column: Boston Green ODS 150 × 30 mm × 5 μm; mobile phase: [A: water (formic acid), B: acetonitrile]; B: 0%-30%, 12 min) to give the title compound (25.0 mg).

MS m/z (ESI): 424.0 $[M+H]^+$.

**Step 8: synthesis of 2-cyclopropyl-*N*-(((*S*)-7-ethyl-7-hydroxy-8,11-dioxo-7,8,11,13-tetrahydro-10*H*-[1,3]dioxolo [4,5-*g*]pyrano[3',4':6,7]indolizino[1,2-*b*]quinolin-14-yl-2,2-*d*$_2$)methyl)-2-hydroxyacetamide (compound 19)**

**Intermediate 19-8** (7 mg) and **intermediate 11-1** (5.76 mg) were dissolved in anhydrous *N,N*-dimethylformamide (0.5 mL), and 2-(7-azabenzotriazole)-*N,N,N,N*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was filtered and purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent (acetonitrile gradient: 20%-50%, elution time: 12 min)) to give the title compound (2.60 mg).

MS m/z (ESI): 522.1$[M+H]^+$.

**$^1$H NMR (400MHz, DMSO-$d_6$)** $\delta$ = 8.62 (t, *J* = 5.9 Hz, 1H), 7.84 (s, 1H), 7.51 (s, 1H), 7.24 (s, 1H), 6.49 (s, 1H), 5.48-5.41 (m, 5H), 4.72 (d, *J* = 5.5 Hz, 2H), 3.59-3.52 (m, 1H), 2.00-1.76 (m, 2H), 1.05-0.96 (m, 1H), 0.88 (t, *J* = 7.4 Hz, 3H), 0.37-0.30 (m, 2H), 0.29-0.19 (m, 2H).

**Step 9: synthesis of 2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$)methyl)-2-hydroxyacetamide (compound 19-P1/P2)**

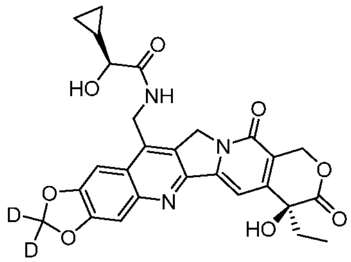

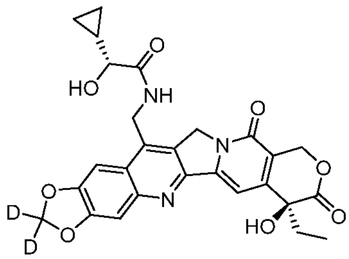

**Intermediate 19-8** (7 mg) and **intermediate 14-10-P1** (5.76 mg) were dissolved in anhydrous *N,N*-dimethylformamide (0.5 mL), and 2-(7-azabenzotriazole)-*N,N,N,N*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 15%-45%, elution time: 12 min) to give **compound 19-P1** (3.30 mg).
MS m/z (ESI): 522.1[M+H]$^+$.
**$^1$H NMR (400MHz, DMSO-$d_6$)** $\delta$ = 8.62 (t, *J* = 6.0 Hz, 1H), 7.86 (s, 1H), 7.52 (s, 1H), 7.25 (s, 1H), 6.51 (s, 1H), 5.54-5.51 (m, 1H), 5.47 (s, 2H), 5.43 (s, 2H), 4.72 (d, *J* = 6.0 Hz, 2H), 3.55-3.53 (m, 1H), 1.94-1.78 (m, 2H), 1.05-0.96 (m, 1H), 0.88 (t, *J* = 7.3 Hz, 3H), 0.40-0.30 (m, 2H), 0.29-0.19 (m, 2H).
**Intermediate 19-8** (7 mg) and **intermediate 14-10-P2** (5.76 mg) were dissolved in anhydrous *N,N*-dimethylformamide (0.5 mL), and 2-(7-azabenzotriazole)-*N,N,N,N*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent (acetonitrile gradient: 15%-45%, elution time: 12 min)) to give **compound 19-P2** (4.0 mg).
MS m/z (ESI): 522.1[M+H]$^+$.
**$^1$H NMR (400MHz, DMSO-$d_6$)** $\delta$ = 8.62 (t, *J* = 6.1 Hz, 1H), 7.86 (s, 1H), 7.52 (s, 1H), 7.25 (s, 1H), 6.50 (s, 1H), 5.54-5.51 (m, 1H), 5.46 (s, 2H), 5.43 (s, 2H), 4.72 (d, *J* = 5.8 Hz, 2H), 3.55-3.52 (m, 1H), 1.94-1.80 (m, 2H), 1.04-0.95 (m, 1H), 0.88 (t, *J* = 7.4 Hz, 3H), 0.39-0.30 (m, 2H), 0.29-0.21 (m, 2H).
The two isomers were separately further analyzed by the following chiral supercritical fluid chromatography analysis method.

| Column | Chiralcel OD-3 50A 4.6mm I.D., 3μm |
|---|---|
| Mobile phase | A: carbon dioxide |
| | B: ethanol (0.05% diethylamine) |
| Flow rate | 4 mL/min |
| Wavelength | PDA 254nm |
| Column temperature | 35°C |
| Column pressure | 1500 Psi |
| Instrument model | Waters UPCC with PDA Detector and QDa Detector |

**Compound 19-P1:**

Under the chiral high performance liquid chromatographic condition described above, the retention time was 2.877 min.

**Compound 19-P2:**

Under the chiral high performance liquid chromatographic condition described above, the retention time was 2.690 min.

**Confirmation of configuration of compound 19-P1 (X-ray single-crystal diffraction method)**

Single crystal culture method: 10 mg of a sample of compound 19-P1 was weighed out and placed in a 1.5 mL centrifuge tube, and 300 μL of pyridine was added. After complete dissolution by sonication, the tube was sealed with a sealing film on which three small holes were made with a needle for slow volatilization at 20-30 °C for 48 h to give a needle-like crystal.

The single crystal sample was subjected to X-ray analysis. The test results are shown in Table 1 and FIG. 1.

Table 1 Single crystal sample and crystal data of **compound 19-P1**

| Chemical formula | $C_{27}H_{23}D_2N_3O_8$, $H_2O$, $2(C_5H_5N)$ |
|---|---|
| Chemical formula weight | 697.73 |
| Crystal system | Monoclinic |
| Space group | P 1 21 1 |
| a, Å | a=13.7058(5) |
| b, Å | b= 6.7026(3) |
| c, Å | c= 18.6204(7) |
| α, deg | 90 |
| β, deg | 97.727(3) |
| γ, deg | 90 |
| Volume, $Å^3$ | 1695.02(12) |
| Z | 2 |
| $D_{calc}$, g $cm^{-3}$ | 1.367 |
| Temperature, K | 193 |
| Radiation (wavelength) | Cu Kα(1.54178) |
| Instrument | BRUKER D8 VENTURE |
| Ranges of h, k, and l | -15<=h<=17, -8<=k<=7, -23<=l<=23 |
| Range of θ | 3.254-79.721 |
| Program | SHELXL-2014 |
| Collected data | 25136 |
| Unique data | 6674 |
| $R(F_o)$ | 0.0571 |
| $R_W(F_o^2)$ | 0.1713 |
| Goodness of fit | 1.045 |
| Absolute configuration determination | 0.04(11) |

The chemical structure of **compound 19-P1** was determined by the X-ray crystal diffraction experiment described above to be:

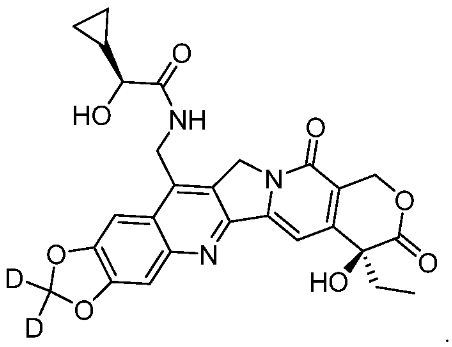

**Example 19-2. Synthesis of (*S*)-2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$) methyl)-2-hydroxyacetamide (Compound 19-S)**

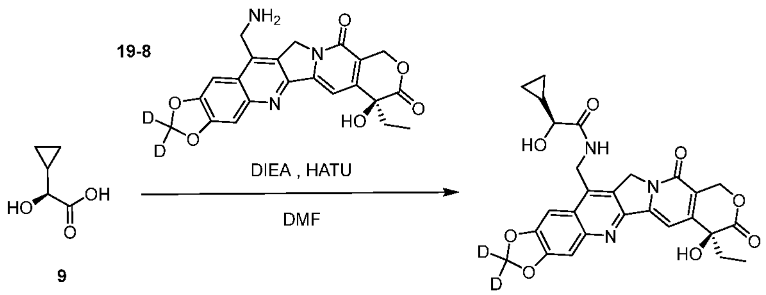


**Intermediate 19-8** (2.4 g) and **intermediate 9** (1645.5 mg) were dissolved in anhydrous *N,N*-dimethylformamide (25 mL), and O-(7-azabenzotriazol-1-yl)-*N,N,N,N*-tetramethyluronium hexafluorophosphate (3.24 g) and *N,N*-diisopropylethylamine (1465.11 mg) were added thereto. The reaction mixture was stirred at 25 °C for 3 h. After the reaction was completed, the reaction mixture was concentrated to dryness under reduced pressure. Ethyl acetate (100 mL) was added to the residue, and the mixture was stirred for 16 h. After filtration, methanol (50 mL) was added to the filter cake, and the mixture was stirred for 16 h. After filtration, the title compound (1.6 g) was obtained.

MS m/z (ESI): 522.1[M+H]$^+$.

**$^1$H NMR (400MHz, DMSO-$d_6$)** $\delta$ = 8.60 (t, *J* = 5.9 Hz, 1H), 7.84 (s, 1H), 7.50 (s, 1H), 7.23 (s, 1H), 6.48 (s, 1H), 5.49 (d, *J* = 5.1 Hz, 1H), 5.47-5.37 (m, 4H), 4.71 (d, *J* = 5.8 Hz, 2H), 3.54 (t, *J* = 5.6 Hz, 1H), 1.97-1.75 (m, 2H), 1.07-0.94 (m, 1H), 0.87 (t, *J* = 7.3 Hz, 3H), 0.40-0.29 (m, 2H), 0.29-0.20 (m, 2H).

**Compound 19-S** was further analyzed by the following chiral supercritical fluid chromatography analysis method.

| | |
|---|---|
| Column | Chiralcel OD-3 50A 4.6mm I.D., 3μm |
| Mobile phase | A: carbon dioxide |
| | B: ethanol (0.05% diethylamine) |
| Flow rate | 4 mL/min |
| Wavelength | PDA 254nm |
| Column temperature | 35°C |
| Column pressure | 1500 Psi |
| Instrument model | Waters UPCC with PDA Detector and QDa Detector |

Under the chiral supercritical fluid chromatographic condition described above, the retention time of **compound 19-S** prepared in this example was 2.853 min, which was substantially consistent with the retention time (2.877 min) of **compound 19-P1** prepared in Example 19-1 under the same chromatographic analysis condition. Thus, **compound 19-S**

**and compound 19-P1** were determined to have the same configuration, being the same compound.

**Example 20. (*S*)-*N*-((8-ethyl-8-hydroxy-9,12-dioxo-8,9,12,14-tetrahydro-11H-furo[3,2:*f*]pyrano [3',4':6,7]indolizino[1,2-*b*]quinolin-15-yl)methyl)-2-hydroxyacetamide (Compound 20)**

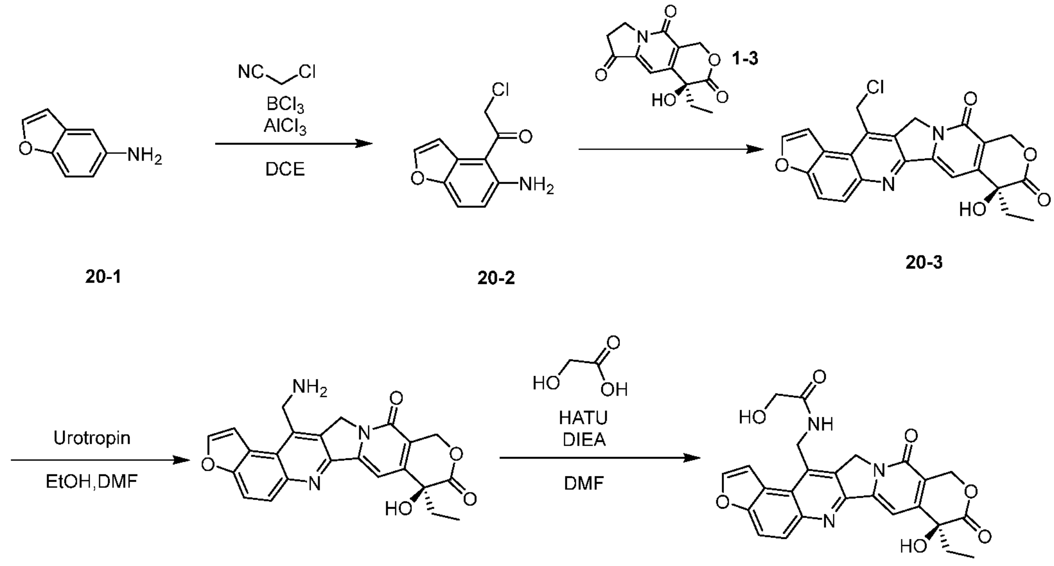


20-1 20-2 20-3

20-4 20

**Step 1: synthesis of 1-(5-aminobenzofuran-4-yl)-2-chloroethanone (intermediate 20-2)**

Boron trichloride (1 M, 9.61 mL) was dissolved in dichloroethane (14 mL). The reaction mixture was cooled to 0 °C, and **intermediate 20-1** (1.6 g, 12.02 mmol) and chloroacetonitrile (1.36 g, 18.03 mmol) were added thereto. The reaction was stirred at 0 °C for 10 min and aluminum trichloride (1.92 g, 14.42 mmol) was added thereto. The reaction mixture was warmed to 25 °C and stirred for 10 min under nitrogen atmosphere. The reaction mixture was stirred at 90 °C for 18 h under nitrogen atmosphere. After the reaction was completed, the reaction mixture was cooled to room temperature, ice water (50 mL) and 5% HCl (10 mL) were added sequentially and slowly and stirred at 25 °C for 30 min, and dichloromethane (60 mL) was added. The organic phase was washed with water (30 mL × 2), and the washed organic phase was dried over an appropriate amount of anhydrous sodium sulfate. The organic phase was purified by preparative thin-layer chromatography (petroleum ether:(ethyl acetate + ethanol = 3:1) = 9:1) to give the title compound (300 mg).

MS m/z (ESI): 210.0 $[M+H]^+$.

**$^1$H NMR (400MHz, chloroform-d)** δ = 7.72 (d, *J* = 2.1 Hz, 1H), 7.53 (d, *J* = 9.0 Hz, 1H), 6.89 (d, *J*= 1.4 Hz, 1H), 6.66 (d, *J* = 9.0 Hz, 1H), 4.78 (s, 2H)

**Step 2: synthesis of (*S*)-15-(chloromethyl)-8-ethyl-8-hydroxy-11,14-dihydro-12*H*-furo[3,2-*f*]pyrano[3',4':6,7]indolizino[1,2-*b*]quinoline-9,12(8*H*)-dione (intermediate 20-3)**

**Intermediate 20-2** (200 mg, 954.07 μmol) and **intermediate 1-3** (251.15 mg, 954.07 μmol) were dissolved in anhydrous toluene (4 mL), and pyridinium *p*-toluenesulfonate (23.98 mg, 95.41 μmol) was added thereto. The reaction mixture was stirred at 90 °C for 16 h under nitrogen atmosphere. After the reaction was completed, the reaction mixture was cooled to room temperature and filtered, and the filter cake was washed with ethanol (3 mL × 2) to give the title compound (300 mg).

MS m/z (ESI): 437.1 $[M+H]^+$.

**Step 3: synthesis of (*S*)-15-(aminomethyl)-8-ethyl-8-hydroxy-11,14-dihydro-12*H*-furo[3,2-*f*]pyrano[3',4':6,7]indolizino[1,2-*b*]quinoline-9,12(8*H*)-dione (intermediate 20-4)**

**Intermediate 20-3** (70 mg, 160.24 μmol) was dissolved in ethanol (0.5 mL) and anhydrous *N,N*-dimethylformamide (0.5 mL), and urotropin (89.85 mg, 640.96 μmol) was added thereto. The reaction mixture was stirred at 25 °C for 3 h. After the reaction was completed, the reaction mixture was cooled to room temperature, concentrated to dryness under reduced pressure and purified by preparative high performance liquid chromatography (YMC-Actus Triart C18 column, 5 μm, 25 mm diameter, 100 mm length; a mixture of water (containing 0.225% formic acid) and methanol with decreasing polarity was used as the eluent; methanol gradient: 5%-25%, elution time: 12 min) to give the title compound (17 mg).

MS m/z (ESI):418.1 [M+H]$^+$.

**Step 4: (*S*)-*N*-((8-ethyl-8-hydroxy-9,12-dioxo-8,9,12,14-tetrahydro-11*H*-furo[3,2-*f*]pyrano [3',4':6,7]indolizino [1,2-b]quinolin-15-yl)methyl)-2-hydroxyacetamide (compound 20)**

**Intermediate 20-4** (5.00 mg, 11.98 μmol) and hydroxyacetic acid (4.55 mg, 59.89 μmol) were dissolved in anhydrous *N,N*-dimethylformamide (0.5 mL), and HATU (6.83 mg, 17.97 μmol) and *N,N*-diisopropylethylamine (4.64 mg, 35.94 μmol) were added thereto. The reaction mixture was stirred at 25 °C for 1 h. After the reaction was completed, the reaction mixture was filtered and purified by preparative high performance liquid chromatography (YMC-Actus Triart C18 column, 5 μm, 25 mm diameter, 100 mm length; a mixture of water (containing 0.05% formic acid) and acetonitrile with decreasing polarity was used as the eluent; acetonitrile gradient: 15%-35%, elution time: 12 min) to give the title compound (3 mg).

MS m/z (ESI): 476.2 [M+H]$^+$.

**$^1$H NMR (400MHz, DMSO-*$d_6$*)** δ = 8.48-8.41 (m, 1H), 8.35 (d, *J* = 1.8 Hz, 1H), 8.22 (d, *J* = 8.0 Hz, 1H), 8.14 (d, *J* = 8.0 Hz, 1H), 7.76 (s, 1H), 7.35 (s, 1H), 6.54 (s, 1H), 5.58 (t, *J* = 5.6 Hz, 1H), 5.52 (s, 2H), 5.45 (s, 2H), 5.10 (d, *J* = 5.3 Hz, 2H), 3.88 (d, *J* = 5.6 Hz, 2H), 1.93-1.82 (m, 2H), 0.89 (t, *J* = 7.2 Hz, 3H).

**Example 21. (*S*)-*N*-((9-chloro-4-ethyl-8,10-difluoro-4-hydroxy-3,14-dioxo-3,4,12,14-tetrahydro-1*H*-pyrano [3',4':6,7]indolizino[1,2-*b*]quinolin-11-yl)methyl)-2-hydroxyacetamide (Compound 21)**

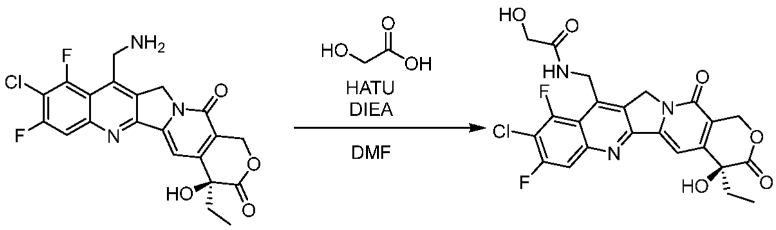


16-4 21

**Compound 16-4** (7 mg, 15.63 μmol) and hydroxyacetic acid (1.78 mg, 25.48 μmol) were dissolved in anhydrous *N,N*-dimethylformamide (0.5 mL), and HATU (11.89 mg, 31.26 μmol) and diisopropylethylamine (2.02 mg, 15.63 μmol) were added thereto. The reaction mixture was stirred at 25 °C for 1 h. After the reaction was completed, the reaction mixture was filtered and purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 20%-40%, elution time: 12 min) to give the title compound (1 mg).

MS m/z (ESI): 506.1 [M+H]$^+$.

**$^1$H NMR (400MHz, Methanol-$d_4$)** δ = 8.35 (t, *J*= 5.8 Hz, 1H), 8.15 (dd, *J*= 1.8, 9.8 Hz, 1H), 7.36 (s, 1H), 6.57 (s, 1H), 5.59-5.50 (m, 3H), 5.45 (s, 2H), 4.91 (d, *J* = 3.2 Hz, 2H), 3.84 (d, *J* = 5.7 Hz, 2H), 1.90-1.80 (m, 2H), 0.87 (t, *J* = 7.3 Hz, 3H).

**Example 22. (*S*)-*N*-((9-chloro-4-ethyl-8,10-difluoro-4-hydroxy-3,14-dioxo-3,4,12,14-tetrahydro-1*H*-pyrano [3',4':6,7]indolizino[1,2-*b*]quinolin-11-yl)methyl)-2-hydroxy-2-methylpropanamide (Compound 22)**

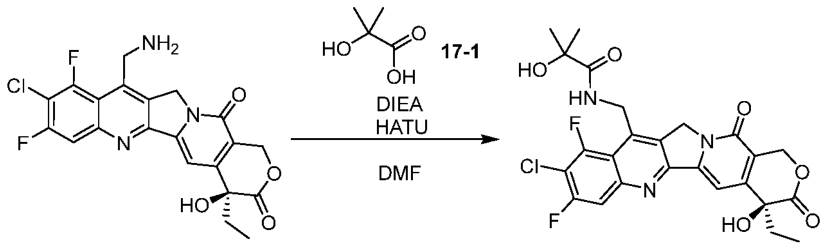


16-4 22

**Compound 16-4** (20 mg, 44.66 μmol) and **intermediate 17-1** (13.95 mg, 133.98 μmol) were dissolved in *N,N*-dimethylformamide (0.5 mL), and HATU (25.47 mg, 66.99 μmol) and *N,N*-diisopropylethylamine (17.32 mg, 133.98 μmol) were added thereto. The reaction mixture was stirred at 25 °C for 1 h. After the reaction was completed, the reaction mixture was filtered and purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 20%-40%, elution time: 12 min) to give the title compound (1.07 mg).

MS m/z (ESI): 534.2 $[M+H]^+$.

**$^1$H NMR (400MHz, DMSO-*d*$_6$)** δ = 8.30 (t, *J* = 5.9 Hz, 1H), 8.19-8.07 (m, 1H), 7.36 (s, 1H), 6.56 (s, 1H), 5.55-5.45 (m, 3H), 5.45 (s, 2H), 4.90 (d, *J* = 3.7 Hz, 2H), 1.90-1.82 (m, 2H), 1.24 (d, *J* = 4.3 Hz, 6H), 0.87 (t, *J* = 7.3 Hz, 3H).

**Example 23. *N*-(((*S*)-9-chloro-4-ethyl-8,10-difluoro-4-hydroxy-3,14-dioxo-3,4,12,14-tetrahydro-1*H*-pyrano [3',4':6,7]indolizino[1,2-*b*]quinolin-11-yl)methyl)-2-cyclopropyl-2-hydroxyacetamide (Compound 23)**

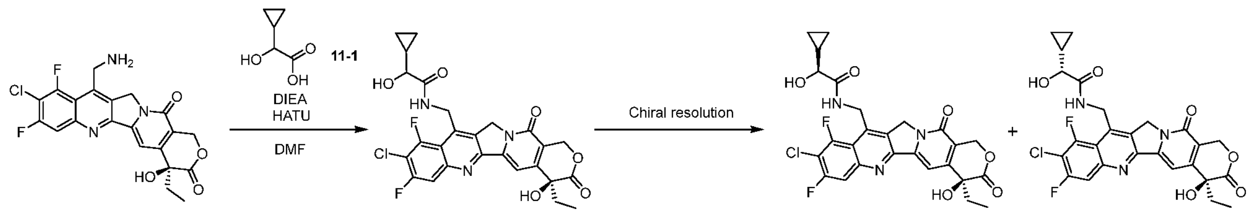


16-4 23

**Compound 16-4** (7.0 mg, 15.63 μmol) and **intermediate 11-1** (9.08 mg, 78.16 μmol) were dissolved in *N,N*-dimethylformamide (0.5 mL), and HATU (8.92 mg, 23.45 μmol) and diisopropylethylamine (6.06 mg, 46.89 μmol) were added thereto. The reaction mixture was stirred at 25 °C for 1 h. After the reaction was completed, the reaction mixture was filtered and purified by preparative high performance liquid chromatography (YMC-Actus Triart C18 column, 5 μm, 25 mm diameter, 100 mm length; a mixture of water (containing 0.225% formic acid) and acetonitrile with decreasing polarity was used as the eluent; acetonitrile gradient: 41%-61%, elution time: 12 min) to give the title compound (7 mg).

MS m/z (ESI): 546.2 $[M+H]^+$.

**Compound 23** (7 mg) was separated and purified by preparative supercritical fluid chromatography (column: DAICEL CHIRALCEL OD-H (250 mm × 30 mm, 5 μm); mobile phase: A: carbon dioxide; B: ethanol; B%: 50%; flow rate: 80 mL/min) to give **compound 23-1** (2.1 mg, RT: 5.106 min) and **compound 23-2** (2.09 mg, RT: 5.641 min).

**Compound 23-1:**

**$^1$H NMR (400MHz, DMSO-*d*$_6$)** δ = 8.32 (t, *J* = 5.5 Hz, 1H), 8.15 (d, *J* = 9.7 Hz, 1H), 7.36 (s, 1H), 6.57 (s, 1H), 5.53 (s, 2H), 5.47 (d, *J* = 5.1 Hz, 1H), 5.45 (s, 2H), 4.93-4.86 (m, 2H), 2.02-1.96 (m, 1H), 1.91-1.81 (m, 2H), 1.04-0.96 (m, 1H), 0.87 (t, *J* = 7.3 Hz, 3H), 0.37-0.31 (m, 2H), 0.29-0.23 (m, 2H)

MS m/z (ESI): 546.2 $[M+H]^+$.

**Compound 23-2:**

**$^1$H NMR (400MHz, DMSO-*d*$_6$)** δ = 8.37-8.29 (m, 1H), 8.15 (d, *J* = 9.9 Hz, 1H), 7.36 (s, 1H), 6.57 (s, 1H), 5.53 (s, 2H), 5.50-5.46 (m, 1H), 5.45 (s, 2H), 4.96-4.86 (m, 2H), 2.10-1.95 (m, 1H), 1.92-1.81 (m, 2H), 1.04-0.98 (m, 1H), 0.87 (t, *J* = 7.3 Hz, 3H), 0.40-0.31 (m, 2H), 0.30-0.25 (m, 2H) MS m/z (ESI): 546.2 $[M+H]^+$.

**Example 25. (*R*)-*N*-(((*S*)-9-chloro-4-ethyl-8,10-difluoro-4-hydroxy-3,14-dioxo-3,4,12,14-tetrahydro-1*H*-pyrano [3',4':6,7]indolizino[1,2-*b*]quinolin-11-yl)methyl)-2-h**ydroxypropanamide **(Compound 25)**

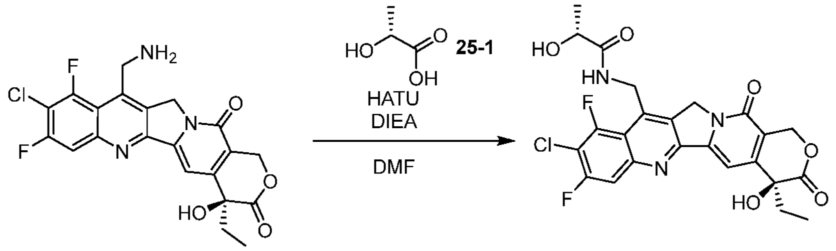


**16-4** **25**

**Compound 16-4** (6 mg, 13.40 μmol) and **intermediate 25-1** (2.41 mg, 26.80 μmol) were dissolved in anhydrous *N,N*-dimethylformamide (0.5 mL), and HATU (10.09 mg, 26.80 μmol) and diisopropylethylamine (1.73 mg, 13.49 μmol) were added thereto. The reaction mixture was stirred at 25 °C for 1 h. After the reaction was completed, the reaction mixture was filtered and purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 20%-40%, elution time: 12 min) to give the title compound (1.80 mg).
MS m/z (ESI): 520.1[M+H]$^+$.
**$^1$H NMR (400MHz, DMSO-*d*$_6$)** δ = 8.34 (t, *J* = 5.7 Hz, 1H), 8.15 (d, *J* = 9.9 Hz, 1H), 7.36 (s, 1H), 6.57 (s, 1H), 5.59 (d, *J* = 5.0 Hz, 1H), 5.51 (s, 2H), 5.45 (s, 2H), 4.90 (s, 2H), 4.13-3.89 (m, 1H), 1.95-1.77 (m, 2H), 1.21 (d, *J* =6.8 Hz, 3H), 0.87 (t, *J* = 7.3 Hz, 3H).

**Example 26. (*S*)-*N*-(((*S*)-9-chloro-4-ethyl-8,10-difluoro-4-hydroxy-3,14-dioxo-3,4,12,14-tetrahydro-1*H*-pyrano [3',4':6,7]indolizino[1,2-*b*]quinolin-11-yl)methyl)-2-hydroxypropanamide (Compound 26)**

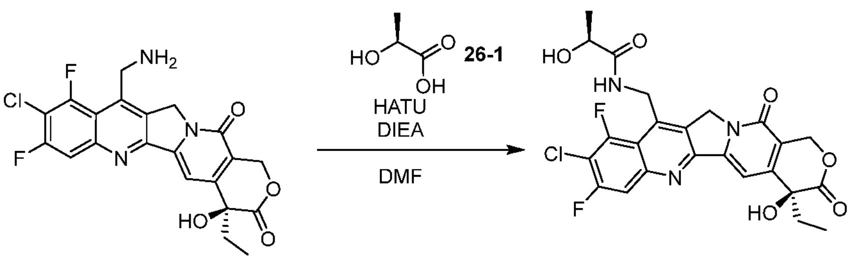


**16-4** **26**

**Compound 16-4** (6 mg, 13.40 μmol) and **intermediate 26-1** (2.41 mg, 26.80 μmol) were dissolved in anhydrous *N,N*-dimethylformamide (0.5 mL), and HATU (10.09 mg, 26.80 μmol) and *N,N*-diisopropylethylamine (1.73 mg, 13.49 μmol) were added thereto. The reaction mixture was stirred at 25 °C for 1 h. After the reaction was completed, the reaction mixture was filtered and purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 20%-40%, elution time: 12 min) to give the title compound (1.60 mg).
MS m/z (ESI): 520.1 [M+H]$^+$.
**$^1$H NMR (400MHz, DMSO-*d*$_6$)** δ = 8.34 (t, *J* = 5.7 Hz, 1H), 8.23-8.03 (m, 1H), 7.36 (s, 1H), 6.57 (s, 1H), 5.59 (d, *J* = 5.0 Hz, 1H), 5.51 (s, 2H), 5.45 (s, 2H), 4.90 (s, 2H), 4.07-3.96 (m, 1H), 1.93-1.81 (m, 2H), 1.21 (d, *J* =6.8 Hz, 3H), 0.87 (t, *J* = 7.3 Hz, 3H).

**Example 27. (*S*)-*N*-((4-chloro-8-ethyl-8-hydroxy-9,12-dioxo-8,9,12,14-tetrahydro-11*H*-furo[3,2-*f*]pyrano[3',4':6,7] indolizino[1,2-*b*]quinolin-15-yl)methyl)-2-hydroxyacetamide (Compound 27)**

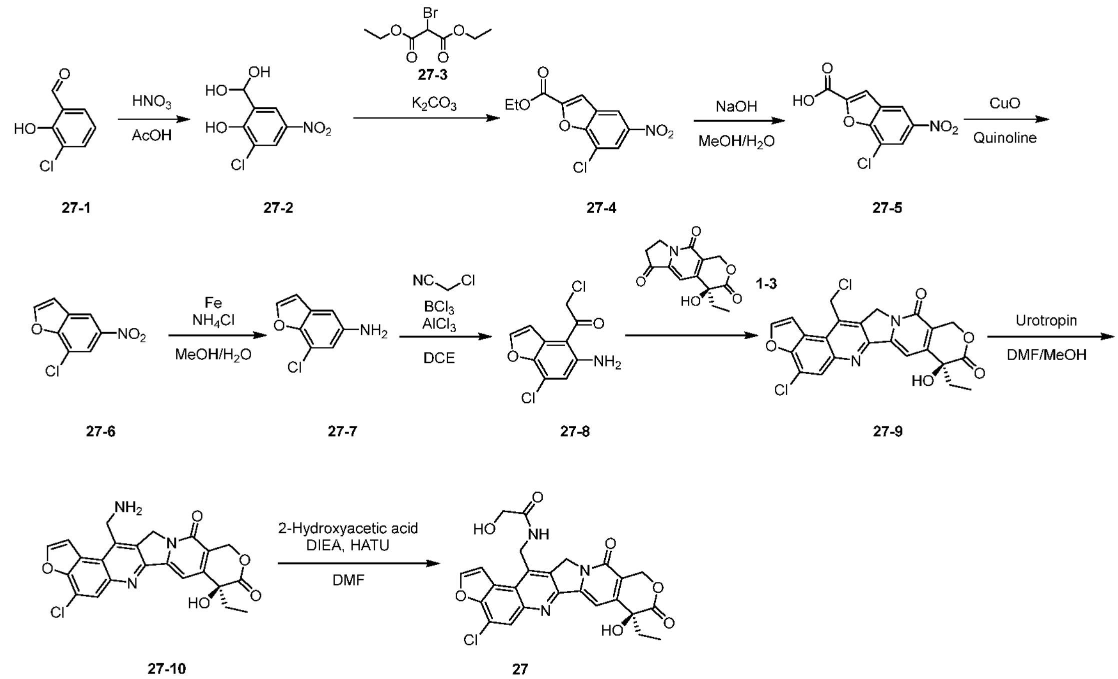

27-1 27-2 27-4 27-5

27-6 27-7 27-8 27-9

27-10 27

**Step 1: synthesis of (3-chloro-2-hydroxy-5-nitrophenyl)methylene glycol (intermediate 27-2)**

**Intermediate 27-1** (6 g) was dissolved in acetic acid (25 mL), the reaction mixture was cooled to 0 °C, and nitric acid (9.64 g) was slowly added thereto. The reaction mixture was stirred at 25 °C for 4 h. After the reaction was completed, the reaction mixture was slowly added dropwise into ice water, and the mixture was extracted with ethyl acetate (60 mL) three times. The organic phase was dried over anhydrous sodium sulfate, filtered, and concentrated to dryness under reduced pressure to give the title compound (5.88 g).

**$^1$H NMR (400MHz, METHANOL-*d4*)** δ = 8.16 (d, *J* =1.4, 2.4 Hz, 1H), 8.07-8.01 (m, 1H), 5.68 (s, 1H)

**Step 2: synthesis of ethyl 7-chloro-5-nitrobenzofuran-2-carboxylate (intermediate 27-4)**

**Intermediate 27-2** (5.88 g), **intermediate 27-3** (7.69 g), and potassium carbonate (7.41 g) were dissolved in acetone (60 mL), and the reaction mixture was stirred at 70 °C for 6 h. After the reaction was completed, water (50 mL) was added thereto, and the mixture was extracted with ethyl acetate (50 mL) three times. The organic phase was dried over anhydrous sodium sulfate, filtered, and concentrated to dryness under reduced pressure to give the title compound (4.5 g).

**$^1$H NMR (400MHz , chloroform-*d*)** δ = 8.55 (d, *J* = 2.1 Hz, 1H), 8.39 (d, *J* = 2.1 Hz, 1H), 7.68 (s, 1H), 4.49 (q, *J* = 7.1 Hz, 2H), 1.46 (t, *J* = 7.1 Hz, 3H)

**Step 3: synthesis of 7-chloro-5-nitrobenzofuran-2-carboxylic acid (intermediate 27-5)**

**Intermediate 27-4** (4.5 g) was dissolved in methanol (50 mL), an aqueous sodium hydroxide solution (2 g in 25 mL $H_2O$) was slowly added dropwise thereto, and the reaction mixture was stirred at 25 °C for 3 h. After the reaction was completed, water (120 mL) was added thereto, and the mixture was extracted with ethyl acetate (50 mL) twice. The aqueous phase was adjusted to pH 1 with diluted hydrochloric acid, and the mixture was extracted with ethyl acetate (50 mL) three times. The organic phase was dried over anhydrous sodium sulfate, filtered, and concentrated to dryness under reduced pressure to give the title compound (4.0 g).

**$^1$H NMR (400MHz , chloroform-*d*)** δ = 8.45 (d, *J* = 2.1 Hz, 1H), 8.25 (d, *J* = 2.1 Hz, 1H), 7.58 (s, 1H)

**Step 4: synthesis of 7-chloro-5-nitrobenzofuran (intermediate 27-6)**

**Intermediate 27-5** (4 g) and copper oxide (1.05 g) were dissolved in quinoline (28 mL), nitrogen was introduced into the reaction mixture, and the mixture was stirred at 200 °C for 0.5 h. After the reaction was completed, the mixture was cooled to 0 °C, diluted hydrochloric acid (80 mL) was slowly added dropwise thereto, water (30 mL) was then added, and the mixture was extracted with ethyl acetate (30 mL) three times. The organic phase was dried over anhydrous sodium sulfate, filtered, and concentrated to dryness under reduced pressure to give the title compound (2.4 g).

**$^1$H NMR (400MHz , chloroform-*d*)** $\delta$ = 8.49 (d, *J* = 2.1 Hz, 1H), 8.31 (d, *J* = 2.1 Hz, 1H), 7.88 (d, *J* = 2.3 Hz, 1H), 7.02 (d, *J* = 2.3 Hz, 1H)

**Step 5: synthesis of 7-chlorobenzofuran-5-amine (intermediate 27-7)**

**Intermediate 27-6** (2.4 g) and iron powder (1.55 g) were dissolved in methanol (5 mL), an aqueous ammonium chloride solution (148.91 mg, 5 mL) was added dropwise thereto, nitrogen was introduced into the reaction mixture, and the mixture was stirred at 80 °C for 0.5 h. After the reaction was completed, the mixture was cooled to 25 °C, water (10 mL) was added thereto, and the mixture was extracted with ethyl acetate (10 mL) three times. The organic phase was dried over anhydrous sodium sulfate, filtered, and concentrated to dryness under reduced pressure to give the title compound (1.2 g).
MS m/z (ESI): 167.8[M+H]$^+$.

**Step 6: synthesis of 1-(5-amino-7-chlorobenzofuran-4-yl)-2-chloroethan-1-one (intermediate 27-8)**

Boron trichloride (671.17 mg) was dissolved in 1,2-dichloroethane (8 mL). The reaction mixture was cooled to 0 °C, and **intermediate 27-7** (1.2 g) and chloroacetonitrile (702.75 mg) were added thereto. The reaction was stirred at 0 °C for 10 min, and aluminum trichloride (1.24 g) was added thereto. Then the reaction mixture was warmed to 25 °C and stirred for 10 min under nitrogen atmosphere. The reaction mixture was stirred at 90 °C for 18 h under nitrogen atmosphere. After the reaction was completed and the reaction mixture was cooled to room temperature, ice water (5 mL) and 5% HCl (1 mL) were added sequentially and slowly and stirred at 25 °C for 30 min, and dichloromethane (4 mL) was added. The organic phase was washed with water (2 mL $\times$ 2), and the washed organic phase was dried over an appropriate amount of anhydrous sodium sulfate. After the organic phase was filtered, the organic phase was concentrated to dryness under reduced pressure, and the residue was purified by preparative thin-layer chromatography (silica, petroleum ether:(a 3/1 mixed solvent of ethyl acetate and ethanol) = 9:1) to give the title compound (500 mg).
MS m/z (ESI): 243.8 [M+H]$^+$.

**Step 7: synthesis of (*S*)-4-chloro-15-(chloromethyl)-8-ethyl-8-hydroxy-11,14-dihydro-12*H*-furo[3,2-*f*]pyrano [3',4':6,7]indolizino[1,2-*b*]quinoline-9,12(8*H*)-dione (intermediate 27-9)**

**Intermediate 27-8** (450 mg) and **intermediate 1-3** (480.35 mg) were dissolved in toluene (5 mL), and pyridinium *p*-toluenesulfonate (23.17 mg) was added thereto. The reaction mixture was stirred at 90 °C for 18 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with petroleum ether (2 mL $\times$ 2) and dried to give the title compound (500 mg).
MS m/z (ESI): 471.0 [M+H]$^+$.

**Step 8: synthesis of (*S*)-15-(aminomethyl)-4-chloro-8-ethyl-8-hydroxy-11,14-dihydro-12*H*-furo[3,2-*f*]pyrano [3',4':6,7]indolizino[1,2-*b*]quinoline-9,12(8*H*)-dione (intermediate 27-10)**

**Intermediate 27-9** (450 mg) was dissolved in a mixed solution of methanol (1 mL) and *N,N*-dimethylformamide (1 mL), and urotropin (267.71 mg) was added thereto. The reaction mixture was stirred at 50 °C for 4 h. After the reaction was completed, the reaction mixture was cooled to room temperature. Concentrated hydrochloric acid (2 mL) was added, and the mixture was stirred and then concentrated to dryness under reduced pressure. The residue was purified by preparative high performance liquid chromatography (Waters Xbridge C18 column, 5 $\mu$m, 25 mm diameter, 100 mm length; a mixture of water (containing 0.225% formic acid) and acetonitrile with decreasing polarity was used as the eluent; acetonitrile gradient: 13%-43%, elution time: 12 min) to give the title compound (60.0 mg).
MS m/z (ESI): 451.9 [M+H]$^+$.

**Step 9: synthesis of (*S*)-*N*-((4-chloro-8-ethyl-8-hydroxy-9,12-dioxo-8,9,12,14-tetrahydro-11*H*-furo[3,2-*f*]pyrano [3',4':6,7]indolizino[1,2-*b*]quinolin-15-yl)methyl)-2-hydroxyacetamide (compound 27)**

**Intermediate 27-10** (10 mg) and 2-hydroxyacetic acid (5.05 mg) were dissolved in anhydrous *N,N*-dimethylformamide (0.5 mL), HATU (16.83 mg) and diisopropylethylamine (2.86 mg) were added thereto, and the reaction mixture was stirred at 25 °C for 1 h. After the reaction was completed, the reaction mixture was filtered. The residue was purified by preparative high performance liquid chromatography (Waters Xbridge C18 column, 5 $\mu$m, 25 mm diameter, 100 mm length; a mixture of water (containing 0.05% formic acid) and acetonitrile with decreasing polarity was used as the eluent; acetonitrile gradient: 28%-48%, elution time: 12 min) to give the title compound (6.0 mg).
MS m/z (ESI):510.1 [M+H]$^+$.

**$^1$H NMR (400MHz, DMSO-$d_6$)** δ = 8.46 (s, 2H), 8.36-8.20 (m, 1H), 7.85 (s, 1H), 7.35 (s, 1H), 6.54 (s, 1H), 5.58 (t, *J* = 5.6 Hz, 1H), 5.49 (d, *J* = 2.9 Hz, 2H), 5.45 (s, 2H), 5.07 (s, 2H), 3.88 (d, *J* = 5.6 Hz, 2H), 2.02-1.75 (m, 2H), 0.89 (t, *J* = 7.3 Hz, 3H).

**Example 28. *N*-(((*S*)-4-chloro-8-ethyl-8-hydroxy-9,12-dioxo-8,9,12,14-tetrahydro-11*H*-furo[3,2-*f*]pyrano[3',4':6,7] indolizino[1,2-*b*]quinolin-15-yl)methyl)-2-cyclopropyl-2-hydroxyacetamide (Compound 28)**

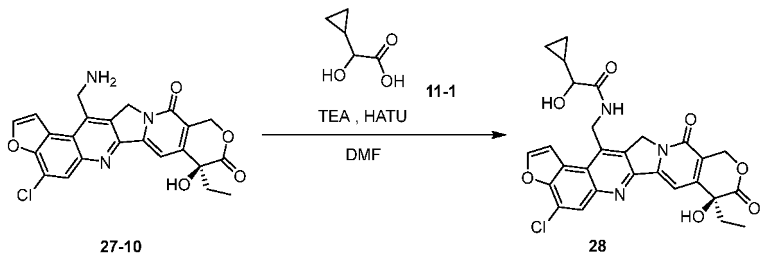


27-10 28

**Intermediate 27-10** (10 mg) and **intermediate 11-1** (8.34 mg) were dissolved in anhydrous *N,N*-dimethylformamide (0.5 mL), HATU (13.65 mg) and diisopropylethylamine (3.10 mg) were added thereto, and the reaction mixture was stirred at 25 °C for 1 h. After the reaction was completed, the reaction mixture was filtered, and the filtrate was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent; acetonitrile gradient: 34%-54%, elution time: 12 min) to give the title compound (6.2 mg).

MS m/z (ESI): 550.1[M+H]$^+$.

**$^1$H NMR (400MHz, DMSO-$d_6$)** δ = 8.48-8.43 (m, 2H), 8.29 (s, 1H), 7.84 (s, 1H), 7.35 (s, 1H), 6.56 (s, 1H), 5.51 (s, 2H), 5.45 (s, 2H), 5.15-4.98 (m, 2H), 3.57 (d, *J* = 6.0 Hz, 1H), 1.95-1.80 (m, 2H), 1.10-1.00 (m, 1H), 0.89 (t, *J* =7.3 Hz, 3H), 0.41-0.32 (m, 2H), 0.32-0.24 (m, 2H).

**Example 29. 2-cyclopropyl-*N*-(((*S*)-7-ethyl-7-hydroxy-15-nitro-8,11-dioxo-7,8,11,13-tetrahydro-10*H*-[1,3]dioxolo [4,5-*g*]pyrano[3',4':6,7]indolizino[1,2-*b*]quinolin-14-yl)methyl)-2-hydroxyacetamide (Compound 29)**

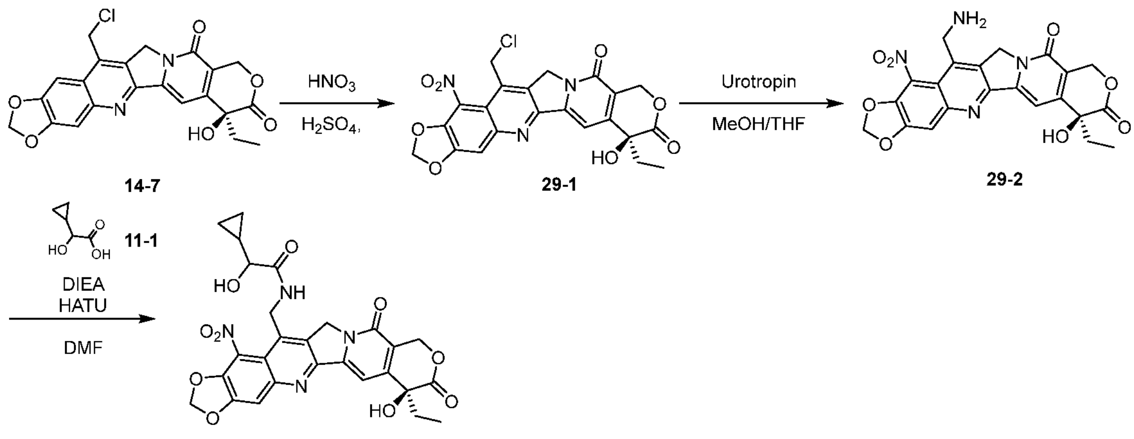


14-7 29-1 29-2

29

**Step 1: synthesis of (*S*)-14-(chloromethyl)-7-ethyl-7-hydroxy-15-nitro-10,13-dihydro-11*H*-[1,3]dioxolo[4,5-*g*] pyrano[3',4':6,7]indolizino[1,2-*b*]quinoline-8,11(7*H*)-dione (intermediate 29-1)**

**Intermediate 14-7** (500.0 mg) was dissolved in sulfuric acid (15 mL), and the reaction mixture was cooled to 0 °C. Nitric acid (357.35 g, 70% purity) was then added slowly thereto. The reaction mixture was stirred at 25 °C for 1.5 h. After the reaction was completed, ice water (10 mL) and dichloromethane (30 mL) were added slowly and sequentially. The organic phase was washed with 40 mL of water (20 mL × 2), and the washed organic phase was dried over an appropriate

amount of anhydrous sodium sulfate. After filtration, the filtrate was concentrated to dryness under reduced pressure to give a crude product of the title compound (280.0 mg).

MS m/z (ESI): 486.0 [M+H]$^+$.

**Step 2: synthesis of (*S*)-14-(aminomethyl)-7-ethyl-7-hydroxy-15-nitro-10,13-dihydro-11*H*-[1,3]dioxolo[4,5-*g*]pyrano[3',4':6,7]indolizino[1,2-*b*]quinoline-8,11(7*H*)-dione (intermediate 29-2)**

**Intermediate 29-1** (270.0 mg) was dissolved in methanol (2 mL) and tetrahydrofuran (2 mL), and urotropin (233.73 mg) was added thereto. The reaction mixture was stirred at 60 °C for 16 h. After the reaction was completed, the reaction mixture was cooled to room temperature, concentrated to dryness under reduced pressure, and the residue was purified by preparative high performance liquid chromatography (YMC-Pack CN C18 column, 5 μm silica, 30 mm diameter, 150 mm length; a mixture of water (containing 0.225% FA) and methanol with decreasing polarity was used as the eluent; methanol gradient: 9%-29%, elution time: 12 min) to give the title compound (15.0 mg).

MS m/z (ESI): 467.1 [M+H]$^+$.

**Step 3: synthesis of 2-cyclopropyl-*N*-(((*S*)-7-ethyl-7-hydroxy-15-nitro-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (compound 29)**

**Intermediate 29-2** (12.00 mg) and **intermediate 11-1** (14.94 mg) were dissolved in anhydrous *N,N*-dimethylformamide (1 mL), HATU (14.67 mg) and *N,N*-diisopropylethylamine (9.98 mg) were added thereto, and the reaction mixture was stirred at 25 °C for 1.5 h. After the reaction was completed, the reaction mixture was filtered, and the filtrate was concentrated to dryness under reduced pressure. The residue was purified by preparative high performance liquid chromatography (Boston Prime C18 column, 5 μm silica, 30 mm diameter, 150 mm length; a mixture of water (containing 0.05% formic acid) and acetonitrile with decreasing polarity was used as the eluent; acetonitrile gradient: 15%-45%, elution time: 12 min) to give the title compound (5.20 mg).

MS m/z (ESI): 565.2 [M+H]$^+$.

**$^1$H NMR (400MHz, DMSO-$d_6$)** δ = 8.25 (t, *J* = 5.3 Hz, 1H), 7.81 (s, 1H), 7.28 (s, 1H), 6.52 (d, *J* = 2.6 Hz, 3H), 5.55 (d, *J* = 4.9 Hz, 1H), 5.43 (s, 2H), 5.36 (s, 2H), 4.48 (d, *J* = 5.1 Hz, 2H), 3.53 (t, *J* = 5.7 Hz, 1H), 1.91-1.83 (m, 2H), 1.10-0.98 (s, 1H), 0.87 (t, *J* = 7.3 Hz, 3H), 0.42-0.28 (m, 4H).

**Example 30. *N*-(((*S*)-15-chloro-7-ethyl-7-hydroxy-8,11-dioxo-7,8,11,13-tetrahydro-10*H*-[1,3]dioxolo [4,5-*g*]pyrano[3',4':6,7]indolizino[1,2-*b*]quinolin-14-yl)methyl)-2-cyclopropyl-2-hydroxyacetamide (Compound 30)**

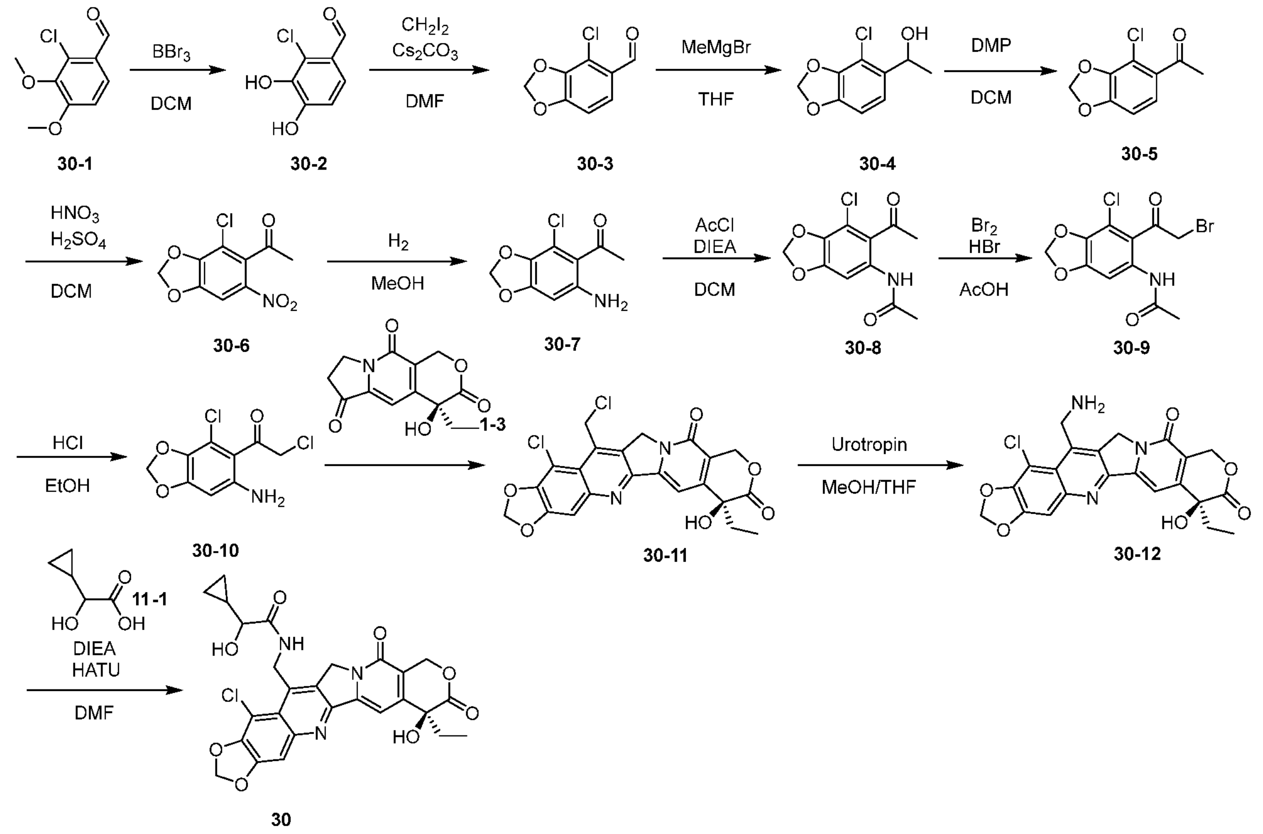


30-1, 30-2, 30-3, 30-4, 30-5, 30-6, 30-7, 30-8, 30-9, 1-3, 30-10, 30-11, 30-12, 11-1, 30

**Step 1: synthesis of 2-chloro-3,4-dihydroxybenzaldehyde (intermediate 30-2)**

**Intermediate 30-1** (20.0 g) was dissolved in anhydrous dichloromethane (100 mL), the reaction mixture was cooled to 0 °C, boron tribromide (87.41 g) was slowly added thereto, and the reaction mixture was stirred at 25 °C for 4 h. After the reaction was completed, the reaction mixture was slowly poured into ice water, ethyl acetate (200 mL) was then added, the organic phase was washed with water (100 mL × 2), and the washed organic phase was dried over an appropriate amount of anhydrous sodium sulfate. After filtration, the filtrate was concentrated to dryness under reduced pressure, and the residue was stirred and washed by ethyl acetate:petroleum ether = 1:4 solution and filtered to give the title compound (14 g).

MS m/z (ESI): 173.1 $[M+H]^+$.

**Step 2: synthesis of 4-chlorobenzo[*d*][1,3]dioxole-5-carbaldehyde (intermediate 30-3)**

**Intermediate 30-2** (10.0 g) was dissolved in anhydrous *N,N*-dimethylformamide (100 mL). Cesium carbonate (28.32 g) and diiodomethane (23.28 g) were added thereto. The reaction mixture was stirred at 100 °C for 1 h. After the reaction was completed, the reaction mixture was cooled to room temperature, water (100 mL) and ethyl acetate (150 mL) were added slowly and sequentially, the organic phase was washed with water (50 mL × 2), and the washed organic phase was dried over an appropriate amount of anhydrous sodium sulfate. After filtration, the filtrate was concentrated to dryness under reduced pressure to give the title compound (5.2 g).

MS m/z (ESI):185.4 $[M+H]^+$.

**Step 3: synthesis of 1-(4-chlorobenzo[*d*][1,3]dioxol-5-yl)ethan-1-ol (intermediate 30-4)**

**Intermediate 30-3** (5.0 g) was dissolved in anhydrous tetrahydrofuran (100 mL), the reaction mixture was cooled to -78 °C, and methylmagnesium bromide (4.85 g, 3 M) was added slowly thereto. The reaction mixture was stirred at 25 °C for 4 h under nitrogen atmosphere. After the reaction was completed, water (50 mL) and ethyl acetate (100 mL) were added slowly and sequentially, the organic phase was washed with water (50 mL × 2), and the washed organic phase was dried over an appropriate amount of anhydrous sodium sulfate. After filtration, the filtrate was concentrated to dryness under reduced pressure, and the residue was purified by preparative column chromatography (petroleum ether:ethyl acetate = 2:1) to give the title compound (5.3 g).

**$^1$H NMR (400MHz, chloroform-*d*)** δ = 6.99 (d, *J* = 8.3 Hz, 1H), 6.69 (d, *J* = 8.1 Hz, 1H), 5.97 (s, 2H), 5.13 (q, *J* = 6.4 Hz, 1H), 1.41 (d, *J* = 6.4 Hz, 3H)

**Step 4: synthesis of 1-(4-chlorobenzo[*d*][1,3]dioxol-5-yl)ethan-1-one (intermediate 30-5)**

**Intermediate 30-4** (5.2 g) was dissolved in anhydrous dichloromethane (100 mL), the reaction mixture was cooled to 0 °C, and Dess-Martin periodinane (DMP) (16.49 g) was slowly added thereto. The reaction mixture was stirred at 25 °C for 2 h under nitrogen atmosphere. After the reaction was completed, water (50 mL) and ethyl acetate (100 mL) were added slowly and sequentially, the organic phase was washed with water (50 mL × 2), and the washed organic phase was dried over an appropriate amount of anhydrous sodium sulfate. After filtration, the filtrate was concentrated to dryness under reduced pressure, and the residue was purified by preparative column chromatography (petroleum ether:ethyl acetate = 3:1) to give the title compound (3.0 g).

MS m/z (ESI): 199.2 $[M+H]^+$.

**Step 5: synthesis of 1-(4-chloro-6-nitrobenzo[*d*][1,3]dioxol-5-yl)ethan-1-one (intermediate 30-6)**

**Intermediate 30-5** (2.50 g) was dissolved in anhydrous dichloromethane (20 mL), and concentrated sulfuric acid (1.23 g) and nitric acid (5.67 g) were slowly added thereto. The reaction mixture was stirred at 25 °C for 3 h. After the reaction was completed, the reaction mixture was slowly poured into ice water, ethyl acetate (150 mL) was then added, the organic phase was washed with water (50 mL × 2), and the washed organic phase was dried over an appropriate amount of anhydrous sodium sulfate. After filtration, the filtrate was concentrated to dryness under reduced pressure, and the residue was purified by preparative column chromatography (petroleum ether:ethyl acetate = 2:1) to give the title compound (1.8 g).

MS m/z (ESI): 244.1 $[M+H]^+$.

**Step 6: synthesis of 1-(6-amino-4-chlorobenzo[*d*][1,3]dioxol-5-yl)ethan-1-one (intermediate 30-7)**

**Intermediate 30-6** (0.80 g) was dissolved in anhydrous methanol (6 mL), and Raney Ni (400.0 mg) was added thereto. The reaction mixture was stirred at 25 °C for 16 h under hydrogen atmosphere. After the reaction was completed, the reaction mixture was filtered, and the filtrate was concentrated to dryness under reduced pressure to give the title compound (510.0 mg).

MS m/z (ESI): 214.2 $[M+H]^+$.

**Step 7: synthesis of *N*-(6-acetyl-7-chlorobenzo[*d*][1,3]dioxol-5-yl)acetamide (intermediate 30-8)**

**Intermediate 30-7** (260.0 mg) was dissolved in anhydrous dichloromethane (5 mL), the reaction mixture was cooled to 0 °C, and *N,N*-diisopropylethylamine (235.95 mg) and chloroacetyl (143.32 mg) were added thereto. The reaction mixture was stirred at 25 °C for 1.5 h. After the reaction was completed, the reaction mixture was concentrated to dryness under reduced pressure, and the residue was purified by preparative chromatography (petroleum ether:ethyl acetate = 5:1) to give the title compound (140.0 mg).

MS m/z (ESI): 256.0 $[M+H]^+$.

**Step 8: synthesis of *N*-(6-(2-bromoacetyl)-7-chlorobenzo[*d*][1,3]dioxol-5-yl)acetamide (intermediate 30-9)**

**Intermediate 30-8** (110.00 mg) was dissolved in acetic acid (2 mL), a solution of hydrogen bromide in acetic acid (158.24 mg, 33% content) was added thereto, and then liquid bromine (72.20 mg) was slowly added thereto. The reaction mixture was stirred at 25 °C for 1 h. After the reaction was completed, the reaction mixture was slowly poured into ice water, ethyl acetate (30 mL) was then added, the organic phase was washed with water (20 mL × 2), and the washed organic phase was dried over an appropriate amount of anhydrous sodium sulfate. The organic phase was concentrated to dryness under reduced pressure to give the title compound (110.0 mg).

MS m/z (ESI): 334.0 $[M+H]^+$.

**Step 9: synthesis of 1-(6-amino-4-chlorobenzo[*d*][1,3]dioxol-5-yl)-2-chloroethan-1-one (intermediate 30-10)**

**Intermediate 30-9** (110.00 mg) was dissolved in anhydrous ethanol (1 mL) and concentrated hydrochloric acid (1 mL), and the reaction mixture was stirred at 60 °C for 16 h. After the reaction was completed, the reaction mixture was cooled to room temperature, ice water (10 mL) and saturated sodium bicarbonate (10 mL) were added slowly and sequentially, and then dichloromethane (30 mL) was added. The organic phase was washed with water (20 mL × 2), and

the washed organic phase was dried over an appropriate amount of anhydrous sodium sulfate. After filtration, the filtrate was concentrated to dryness under reduced pressure, and the residue was purified by preparative thin-layer chromatography (petroleum ether:ethyl acetate = 3:1) to give the title compound (75 mg).

MS m/z (ESI): 248.0 [M+H]+.

**Step 10: synthesis of (*S*)-15-chloro-14-(chloromethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (intermediate 30-11)**

**Intermediate 30-10** (75.00 mg) and **intermediate 1-3** (83.57 mg) were dissolved in toluene (3 mL), and pyridinium p-toluenesulfonate (PPTS) (11.4 mg) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) and dried to give the title compound (75.0 mg).

MS m/z (ESI): 475.1 [M+H]+.

**Step 11: synthesis of (*S*)-14-(aminomethyl)-15-chloro-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (intermediate 30-12)**

**Intermediate 30-11** (70.00 mg) was dissolved in anhydrous methanol (2 mL) and anhydrous tetrahydrofuran (1 mL), and urotropin (61.94 mg) was added thereto. The reaction mixture was stirred at 80 °C for 2 h. After the reaction was completed, the reaction mixture was cooled to room temperature, concentrated to dryness under reduced pressure, and the residue was purified by preparative high performance liquid chromatography (Boston Prime C18 column, 5 μm silica, 30 mm diameter, 150 mm length; a mixture of water (containing 0.225% formic acid) and acetonitrile with decreasing polarity was used as the eluent; acetonitrile gradient: 5%-25%, elution time: 12 min) to give the title compound (16.0 mg).

MS m/z (ESI): 456.1 [M+H]+.

**Step 12: synthesis of *N*-(((*S*)-15-chloro-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-cyclopropyl-2-hydroxyacetamide (compound 30)**

**Intermediate 30-12** (5.00 mg) and **intermediate 11-1** (6.37 mg) were dissolved in anhydrous *N,N*-dimethylformamide (0.5 mL), HATU (6.26 mg) and *N,N*-diisopropylethylamine (4.25 mg) were added thereto, and the reaction mixture was stirred at 25 °C for 1 h. After the reaction was completed, the reaction mixture was filtered, and the filtrate was concentrated to dryness under reduced pressure. The residue was purified by preparative high performance liquid chromatography (Boston Prime C18 column, 5 μm silica, 30 mm diameter, 150 mm length; a mixture of water (containing 0.05% formic acid) and acetonitrile with decreasing polarity was used as the eluent; acetonitrile gradient: 31%-51%, elution time: 12 min) to give the title compound (2.30 mg).

MS m/z (ESI): 554.2 [M+H]+.

**$^1$H NMR (400MHz, DMSO-$d_6$)** δ = 7.78 (t, *J* = 5.4 Hz, 1H), 7.35 (s, 1H), 7.01 (s, 1H), 6.30-6.25 (m, 1H), 6.16 (d, *J* = 1.5 Hz, 2H), 5.27-5.21 (m, 2H), 5.19 (s, 2H), 4.93-4.78 (m, 2H), 3.29 (dd, *J* = 2.4, 6.1 Hz, 1H), 1.68-1.56 (m, 2H), 0.81-0.72 (m, 1H), 0.63 (t, *J* = 7.3 Hz, 3H), 0.14-0.06 (m, 2H), 0.05-0.03 (m, 2H).

**Example 31. (*S*)-*N*-((15-chloro-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 31)**

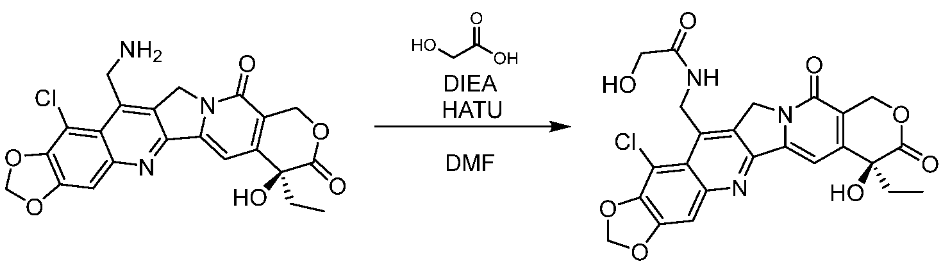


30-12

31

**Intermediate 30-12** (5 mg) and hydroxyacetic acid (4.17 mg) were dissolved in *N,N*-dimethylformamide (0.5 mL), HATU (6.26 mg) and *N,N*-diisopropylethylamine (4.25 mg) were added thereto, and the reaction mixture was stirred

at 25 °C for 1 h. After the reaction was completed, the reaction mixture was filtered, and the filtrate was concentrated to dryness under reduced pressure. The residue was purified by preparative high performance liquid chromatography (Boston Prime C18 column, 5 μm silica, 30 mm diameter, 150 mm length; a mixture of water (containing 0.225% formic acid) and acetonitrile with decreasing polarity was used as the eluent; acetonitrile gradient: 20%-40%, elution time: 12 min) to give the title compound (2.50 mg).

MS m/z (ESI): 514.2 $[M+H]^+$.

**$^1$H NMR (400MHz, DMSO-$d_6$)** δ = 8.03 (t, *J* = 5.8 Hz, 1H), 7.60 (s, 1H), 7.25 (s, 1H), 6.54-6.48 (m, 1H), 6.41 (s, 2H), 5.48 (s, 2H), 5.43 (s, 2H), 5.12 (d, *J* = 6.0 Hz, 2H), 3.82 (s, 2H), 1.93-1.78 (m, 2H), 0.87 (t, *J* = 7.4 Hz, 3H)

**Example 33. (*S*)-*N*-((4-chloro-8-ethyl-8-hydroxy-9,12-dioxo-2,3,8,9,12,14-hexahydro-1H,11H-cyclopenta[*f*]pyrano[3',4':6,7]indolizino[1,2-*b*]quinolin-15-yl)methyl)-2-hydroxyacetamide (Compound 33)**

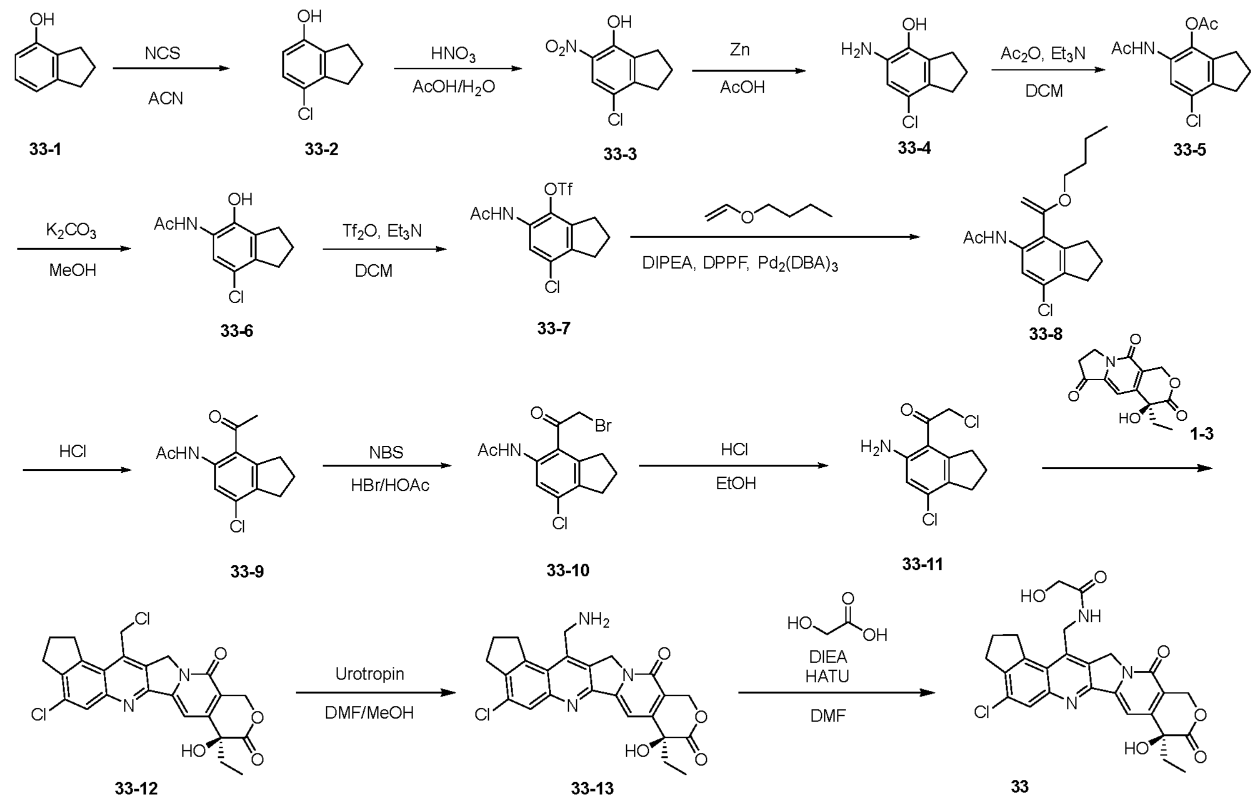


**Step 1: synthesis of 7-chloro-2,3-dihydro-1*H*-inden-4-ol (intermediate 33-2)**

**Intermediate 33-1** (500 mg) was dissolved in anhydrous acetonitrile (5 mL), and NCS (547 mg) was added thereto. After the addition was completed, the mixture was stirred at 25 °C for 2 h. After the reaction was completed, water (10 mL) was added to the reaction mixture. The mixture was extracted with ethyl acetate (10 mL × 3 times). The organic layer was washed with saturated brine (30 mL) and dried over anhydrous sodium sulfate. After the organic phase was filtered, the organic phase was concentrated under reduced pressure to remove the solvent, and the residue was purified by silica gel column chromatography (petroleum ether:ethyl acetate = 100: 1 to 10: 1) to give the title compound (600 mg).

MS m/z (ESI): 169.0 $[M+H]^+$.

**Step 2: synthesis of 7-chloro-5-nitro-2,3-dihydro-1*H*-inden-4-ol (intermediate 33-3)**

**Intermediate 33-2** (10 g) was dissolved in AcOH (50 mL) and $H_2O$ (10 mL), and $HNO_3$ (8.62 g, 65% mass fraction) was added thereto at 0 °C. The reaction mixture was stirred at 0 °C for 2 h. After the reaction was completed, the reaction mixture was added slowly to ice water (200 mL). After filtration, the mixture was concentrated under reduced pressure to remove the solvent to give the title compound (10 g).

MS m/z (ESI): 214.0 $[M+H]^+$.

**Step 3: synthesis of 5-amino-7-chloro-2,3-dihydro-1*H*-inden-4-ol (intermediate 33-4)**

**Intermediate 33-3** (5 g) was dissolved in anhydrous DCM (50 mL), and AcOH (14.04 g) and Zn (7.61 g) were added thereto. The reaction mixture was stirred at 25 °C for 12 h. After the reaction was completed, water (200 mL) and ethyl acetate (200 mL) were added sequentially. The organic phase was washed with a saturated aqueous sodium bicarbonate solution (50 mL × 2), and the washed organic phase was dried over an appropriate amount of anhydrous sodium sulfate. The organic phase was concentrated under reduced pressure to remove the solvent to give the title compound (4 g).
MS m/z (ESI): 184.0 $[M+H]^+$.

**Step 4: synthesis of 5-acetamido-7-chloro-2,3-dihydro-1*H*-inden-4-ylacetate (intermediate 33-5)**

**Intermediate 33-4** (4 g) was dissolved in anhydrous dichloromethane (40 mL), and acetic anhydride ($Ac_2O$) (6.67 g) and triethylamine (TEA) (6.61 g) were added thereto, The reaction mixture was stirred at 25 °C for 12 h. After the reaction was completed, water (200 mL) and ethyl acetate (200 mL) were added sequentially. The organic phase was washed with a saturated aqueous NaCl solution (50 mL × 2), and the washed organic phase was dried over an appropriate amount of anhydrous sodium sulfate. The organic phase was concentrated under reduced pressure to remove the solvent, and the residue was purified by silica gel column chromatography (petroleum ether:ethyl acetate = 20:1 to 5:1) to give the title compound (4 g).
MS m/z (ESI): 268.0 $[M+H]^+$.

**Step 5: synthesis of *N*-(7-chloro-4-hydroxy-2,3-dihydro-1*H*-inden-5-yl)acetamide (intermediate 33-6)**

**Intermediate 33-5** (4 g) was dissolved in anhydrous methanol (20 mL), and $K_2CO_3$ (6.19 g) was added thereto. The reaction mixture was stirred at 25 °C for 12 h. After the reaction was completed, water (200 mL) and ethyl acetate (200 mL) were added sequentially. The organic phase was washed with a saturated aqueous NaCl solution (50 mL × 2), and the washed organic phase was dried over an appropriate amount of anhydrous sodium sulfate. The organic phase was concentrated under reduced pressure to remove the solvent, and the residue was purified by silica gel column chromatography (petroleum ether:ethyl acetate = 20:1 to 1:1) to give the title compound (2.8 g).
MS m/z (ESI): 226.0 $[M+H]^+$.

**Step 6: synthesis of 5-acetamido-7-chloro-2,3-dihydro-1*H*-inden-4-yltrifluoromethanesulfonate (intermediate 33-7)**

**Intermediate 33-6** (500 mg) was dissolved in dichloromethane (5 mL), and trifluoromethanesulfonic anhydride ($Tf_2O$) (749 mg) and triethylamine (TEA) (672 mg) were added thereto, The reaction mixture was stirred at 25 °C for 12 h. After the reaction was completed, water (50 mL) and ethyl acetate (50 mL) were added sequentially. The organic phase was washed with a saturated aqueous NaCl solution (50 mL × 2), and the washed organic phase was dried over an appropriate amount of anhydrous sodium sulfate. The organic phase was concentrated under reduced pressure to remove the solvent, and the residue was purified by silica gel column chromatography (petroleum ether:ethyl acetate = 20:1 to 1:1) to give the title compound (580 mg).
MS m/z (ESI): 358.0 $[M+H]^+$.

**Step 7: synthesis of *N*-(4-(1-butoxyvinyl)-7-chloro-2,3-dihydro-1*H*-inden-5-yl)acetamide (intermediate 33-8)**

**Intermediate 33-7** (430 mg) and vinyl *n*-butyl ether (360.60 mg) were dissolved in dioxane (20 mL). Diisopropylethylamine (DIEA) (466 mg), 1,1'-bis(diphenylphosphino)ferrocene (DPPF) (66 mg), and tris(dibenzylideneacetone) dipalladium ($Pd_2(dba)_3$) (110 mg) were added, and the reaction mixture was stirred for reaction at 80 °C for 16 h under nitrogen atmosphere. After the reaction was completed, the reaction mixture was diluted with water (50 mL). The mixture was extracted with ethyl acetate (50 mL × 3 times). The organic phases were combined and dried over anhydrous sodium sulfate. After the organic phase was filtered, the organic phase was concentrated under reduced pressure to remove the solvent, and the residue was purified by silica gel column chromatography (petroleum ether:ethyl acetate = 20:1 to 1:1) to give the title compound (300 mg).
MS m/z (ESI): 308.0 $[M+H]^+$.

**Step 8: synthesis of *N*-(4-acetyl-7-chloro-2,3-dihydro-1*H*-inden-5-yl)acetamide (intermediate 33-9)**

**Intermediate 33-8** (200 mg) was dissolved in dioxane (5 mL), 1 N HCl (5 mL) was added, and the mixture was

stirred for reaction at 25 °C for 2 h. After the reaction was completed, the organic phase was concentrated under reduced pressure to remove the solvent to give the title compound (150 mg). MS m/z (ESI): 252.0 $[M+H]^+$.

**Step 9: synthesis of *N*-(4-(2-bromoacetyl)-7-chloro-2,3-dihydro-1*H*-inden-5-yl)acetamide (intermediate 33-10)**

**Intermediate 33-9** (300 mg) was dissolved in HBr/AcOH (4 mL, 33% mass fraction), NBS (318.20 mg) was added, and the reaction mixture was stirred at 25 °C for reaction for 2 h. After the reaction was completed, the reaction mixture was concentrated under reduced pressure to remove the solvent to give the title compound (390 mg).
MS m/z (ESI): 330.0 $[M+H]^+$.

**Step 10: synthesis of 1-(5-amino-7-chloro-2,3-dihydro-1*H*-inden-4-yl)-2-chloroethane-1-one (intermediate 33-11)**

**Intermediate 33-10** (300 mg) was dissolved in anhydrous ethanol, HCl (12 M, 8.00 mL) was added, and the reaction mixture was stirred at 80 °C for reaction for 2 h. After the reaction was completed, the reaction mixture was concentrated under reduced pressure to remove the solvent, and the residue was purified by preparative high performance liquid chromatography (column: Gemini NX C18 5 μm × 10 × 150 mm; mobile phase: A: water (0.225% formic acid v/v), B: acetonitrile; B%: 30%-70%) to give the title compound (64 mg).
MS m/z (ESI): 244.0 $[M+H]^+$.

**Step 11: synthesis of (*S*)-4-chloro-15-(chloromethyl)-8-ethyl-1,2,3,8,11,14-hexahydro-9*H*,12*H*-cyclopenta[*f*]pyrano[3',4':6,7]indolizino[1,2-*b*]quinoline-9,12-dione (intermediate 33-12)**

**Intermediate 33-11** (45.00 mg) and **intermediate 1-3** (48.53 mg) were dissolved in toluene (1 mL), and pyridinium *p*-toluenesulfonate (4.63 mg) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) and dried to give the title compound (80.0 mg).
MS m/z (ESI): 471.1 $[M+H]^+$.

**Step 12: synthesis of (*S*)-15-(aminomethyl)-4-chloro-8-ethyl-8-hydroxy-1,2,3,8,11,14-hexahydro-9*H*,12*H*-cyclopenta[*f*]pyrano[3',4':6,7]indolizino[1,2-*b*]quinoline-9,12-dione (intermediate 33-13)**

**Intermediate 33-12** (40.00 mg) was dissolved in anhydrous methanol (1 mL) and anhydrous *N,N*-dimethylformamide (0.5 mL), and urotropin (35.69 mg) was added thereto. The reaction mixture was stirred at 50 °C for 16 h. After the reaction was completed, the reaction mixture was cooled to room temperature, concentrated to dryness under reduced pressure, and the residue was purified by preparative high performance liquid chromatography (Boston Prime C18 column, 5 μm silica, 30 mm diameter, 150 mm length; a mixture of water (containing 0.225% FA) and acetonitrile with decreasing polarity was used as the eluent; acetonitrile gradient: 35%-55%, elution time: 12 min) to give the title compound (15.0 mg).
MS m/z (ESI): 452.1 $[M+H]^+$.

**Step 13: synthesis of (*S*)-*N*-((4-chloro-8-ethyl-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-hydroxyacetamide (compound 33)**

**Intermediate 33-13** (5.00 mg) and hydroxyacetic acid (4.21 mg, 55.30 μmol) were dissolved in anhydrous *N,N*-dimethylformamide (0.5 mL), HATU (6.31 mg) and *N,N*-diisopropylethylamine (4.29 mg) were added thereto, and the reaction mixture was stirred at 25 °C for 1 h. After the reaction was completed, the reaction mixture was filtered and purified by preparative high performance liquid chromatography (Boston Green ODS C18 column, 5 μm silica, 30 mm diameter, 150 mm length; a mixture of water (containing 0.05% FA) and acetonitrile with decreasing polarity was used as the eluent; acetonitrile gradient: 32%-52%, elution time: 12 min) to give the title compound (2.0 mg).
MS m/z (ESI): 510.3 $[M+H]^+$.
**$^1$H NMR (400MHz, DMSO-$d_6$)** δ = 8.36-8.30 (m, 1H), 8.14 (s, 1H), 7.31 (s, 1H), 6.55 (s, 1H), 5.44 (s, 2H), 5.38 (s, 2H), 4.96 (d, *J* = 5.3 Hz, 2H), 3.88 (s, 2H), 3.74-3.66 (m, 2H), 3.14 (t, *J* = 7.6 Hz, 2H), 2.27-2.19 (m, 2H), 1.92-1.82 (m, 2H), 0.88 (t, *J* = 7.3 Hz, 3H).

**Example 34. *N*-(((*S*)-4-chloro-8-ethyl-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-cyclopropyl-2-hydroxyacetamide (Compound 34)**

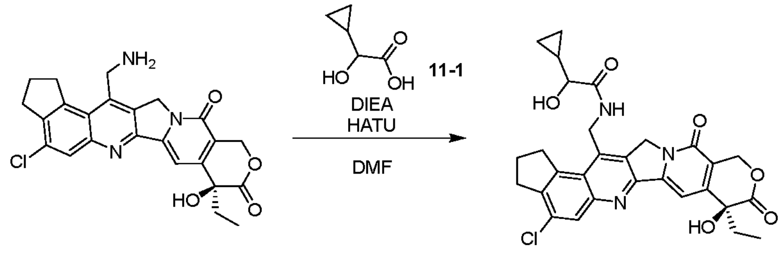


33-13 34

**Intermediate 33-13** (5.00 mg) and **intermediate 11-1** (6.42 mg) were dissolved in anhydrous *N,N*-dimethylformamide (0.5 mL), HATU (6.31 mg) and diisopropylethylamine (4.29 mg) were added thereto, and the reaction mixture was stirred at 25 °C for 1 h. After the reaction was completed, the reaction mixture was filtered and purified by preparative high performance liquid chromatography (Boston Prime C18 column, 5 μm silica, 30 mm diameter, 150 mm length; a mixture of water (containing 0.05% FA) and acetonitrile with decreasing polarity was used as the eluent; acetonitrile gradient: 35%-55%, elution time: 12 min) to give the title compound (2.0 mg).

MS m/z (ESI): 550.3 $[M+H]^+$.

**$^1$H NMR (400MHz, DMSO-*d*$_6$)** δ = 8.36-8.31 (m, 1H), 8.14 (s, 1H), 7.31 (s, 1H), 6.54 (s, 1H), 5.47-5.41 (m, 3H), 5.38 (s, 2H), 4.96-4.90 (m, 2H), 3.69 (t, *J* = 7.1 Hz, 2H), 3.56 (t, *J* = 5.8 Hz, 1H), 3.14 (t, *J* = 7.4 Hz, 2H), 2.27-2.18 (m, 2H), 1.93-1.81 (m, 2H), 1.09-1.03 (m, 1H), 0.88 (t, *J* = 7.2 Hz, 3H), 0.41-0.26 (m, 4H).

**Example 35. 2-cyclopropyl-*N*-(((*S*)-7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 35)**

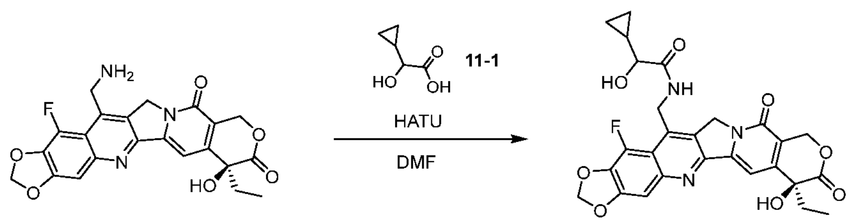


12-11 Compound 35

**Intermediate 12-11** (6 mg) and **intermediate 11-1** (7.93 mg) were dissolved in *N,N*-dimethylformamide (0.5 mL), and 2-(7-azabenzotriazole)-*N,N,N,N*-tetramethyluronium hexafluorophosphate (7.79 mg) and *N,N*-dimethyldiisopropylamine (5.29 mg) were added thereto. The reaction mixture was stirred at 25 °C for 1 h. After the reaction was completed, the reaction mixture was concentrated to dryness under reduced pressure. The residue was purified by preparative high performance liquid chromatography (Boston Prime C18 column, 5 μm silica, 30 mm diameter, 150 mm length; a mixture of water (containing 0.225% formic acid) and acetonitrile with decreasing polarity was used as the eluent (acetonitrile gradient: 15%-45%, elution time: 12 min)) to give the title compound (6.50 mg).

MS m/z (ESI): 538.1 $[M+H]^+$.

**$^1$H NMR (400MHz, DMSO-*d*$_6$)** δ = 8.21 (t, *J* = 6.0 Hz, 1H), 7.51 (s, 1H), 7.26 (s, 1H), 6.53 (s, 1H), 6.40 (s, 2H), 5.47 (s, 2H), 5.43 (s, 2H), 4.84 (d, *J* = 3.8 Hz, 2H), 3.53 (d, *J* = 6.3 Hz, 1H), 1.90-1.81 (m, 2H), 1.01 (d, *J* = 5.5 Hz, 1H), 0.89-0.85 (m, 3H), 0.33 (d, *J* = 6.0 Hz, 2H), 0.30-0.25 (m, 2H).

**Synthesis of Compounds 35-P1 and 35-P2**

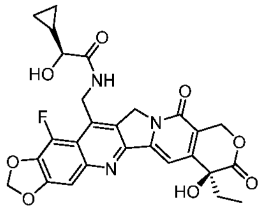 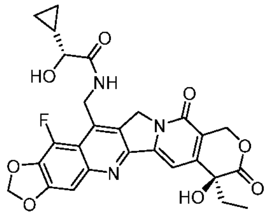

**Intermediate 12-11** (6 mg) and **intermediate 14-10-P1** (7.93 mg) were dissolved in *N,N*-dimethylformamide (1 mL), and 2-(7-azabenzotriazole)-N,N,N,N-tetramethyluronium hexafluorophosphate (7.79 mg) and *N,N*-dimethyldiisopropylamine (5.29 mg) were added thereto. The reaction mixture was stirred at 25 °C for 1 h. After the reaction was completed, the reaction mixture was concentrated to dryness under reduced pressure, and the residue was purified by preparative high performance liquid chromatography (Boston Green ODS C18 column, 5 μm silica, 30 mm diameter, 150 mm length; a mixture of water (containing 0.225% formic acid) and acetonitrile with decreasing polarity was used as the eluent; acetonitrile gradient: 17%-47%, elution time: 12 min) to give compound 35-P1 (2.87 mg).

MS m/z (ESI): 538.1 $[M+H]^+$.

**$^1$H NMR (400MHz, DMSO-$d_6$)** δ = 8.19 (t, *J* = 5.9 Hz, 1H), 7.51 (s, 1H), 7.26 (s, 1H), 6.51 (s, 1H), 6.40 (s, 2H), 5.47 (s, 2H), 5.43 (s, 2H), 4.84 (d, *J* = 4.6 Hz, 2H), 3.53 (d, *J* = 6.4 Hz, 1H), 1.91-1.80 (m, 2H), 1.06-0.97 (m, 1H), 0.87 (t, *J* = 7.3 Hz, 3H), 0.38-0.31 (m, 2H), 0.31-0.24 (m, 2H). **Intermediate 12-11** (6 mg) and **intermediate 14-10-P2** (4.76 mg) were dissolved in *N,N*-dimethylformamide (1 mL), and 2-(7-azabenzotriazole)-*N,N,N,N*-tetramethyluronium hexafluorophosphate (10.38 mg) and *N,N*-dimethyldiisopropylamine (1.76 mg) were added thereto. The reaction mixture was stirred at 25 °C for 1 h. After the reaction was completed, the reaction mixture was concentrated to dryness under reduced pressure, and the residue was purified by preparative high performance liquid chromatography (Boston Green ODS C18 column, 5 μm silica, 30 mm diameter, 150 mm length; a mixture of water (containing 0.225% formic acid) and acetonitrile with decreasing polarity was used as the eluent; acetonitrile gradient: 17%-47%, elution time: 12 min) to give compound 35-P2 (2.01 mg).

MS m/z (ESI): 538.1 $[M+H]^+$.

**$^1$H NMR (400MHz, DMSO-$d_6$)** δ = 8.26-8.16 (m, 1H), 7.51 (s, 1H), 7.26 (s, 1H), 6.51 (s, 1H), 6.40 (s, 2H), 5.46 (s, 2H), 5.43 (s, 2H), 4.87-4.82 (m, 2H), 3.53 (d, *J* = 6.2 Hz, 1H), 1.94-1.80 (m, 2H), 1.05-0.96 (m, 1H), 0.87 (t, *J* = 7.3 Hz, 3H), 0.39-0.30 (m, 2H), 0.31-0.20 (m, 2H).

The two isomers were separately further analyzed by the following chiral supercritical fluid chromatography analysis method.

| Column | Chiralcel OJ-3 100A 4.6mm I.D., 3μm |
|---|---|
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector |

**Compound 35-P1:**

Under the chiral high performance liquid chromatographic condition described above, the retention time was 3.519 min.

**Compound 35-P2:**

Under the chiral high performance liquid chromatographic condition described above, the retention time was 3.573 min.

**Example 36. (*S*)-*N*-((7-ethyl-15-fluoro-7-hydroxy-8,11-dioxo-7,8,11,13-tetrahydro-10*H*-[1,3]dioxolo[4,5-*g*]pyrano [3',4':6,7]indolizino[1,2-*b*]quinolin-14-yl)methyl)-2-hydroxyacetamide (Compound 36)**

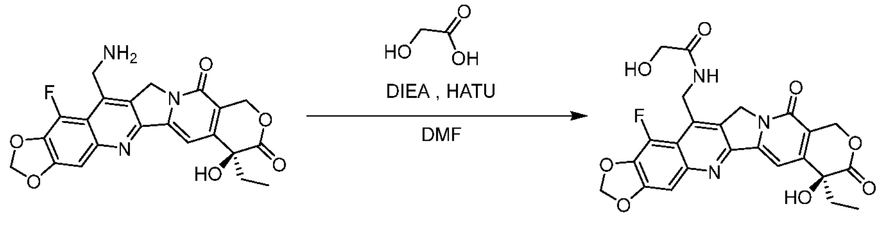


12-11 Compound 36

**Intermediate 12-11** (6 mg) and hydroxyacetic acid (3.21 mg) were dissolved in anhydrous *N,N*-dimethylformamide (0.5 mL), and 2-(7-azabenzotriazole)-*N,N,N,N*-tetramethyluronium hexafluorophosphate (10.38 mg) and diisopropylethylamine (1.76 mg) were added thereto. The reaction mixture was stirred at 25 °C for 1 h. After the reaction was completed, the reaction mixture was concentrated to dryness under reduced pressure. The residue was purified by preparative high performance liquid chromatography (Waters Xbridge C18 column, 5 μm, 25 mm diameter, 100 mm length; a mixture of water (containing 0.05% formic acid) and acetonitrile with decreasing polarity was used as the eluent (acetonitrile gradient: 18%-48%, elution time: 12 min)) to give the title compound (2.40 mg).

MS m/z (ESI): 498.1[M+H]$^+$.

**$^1$H NMR (400MHz, DMSO-*d6*)** δ = 8.20 (t, *J* = 5.9 Hz, 1H), 7.51 (s, 1H), 7.26 (s, 1H), 6.39 (s, 2H), 5.46 (s, 2H), 5.43 (s, 2H), 4.85 (d, *J* = 4.3 Hz, 2H), 3.83 (s, 2H), 1.92-1.80 (m, 2H), 0.87 (t, *J* = 7.3 Hz, 3H).

**Example 37: Synthesis of *N*-((12*S*)-12-benzyl-4-cyclopropyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-3,8,11,14,17-pentaoxo-5-oxy-2,7,10,13,16-pentaazaoctadecan-18-yl)-6-(2,5-dioxo-2,5-dihydro-1*H*-pyrrol-1-yl)hexanamide (Compound L1-14) and isomers L1-14-P1 and L1-14-P2**

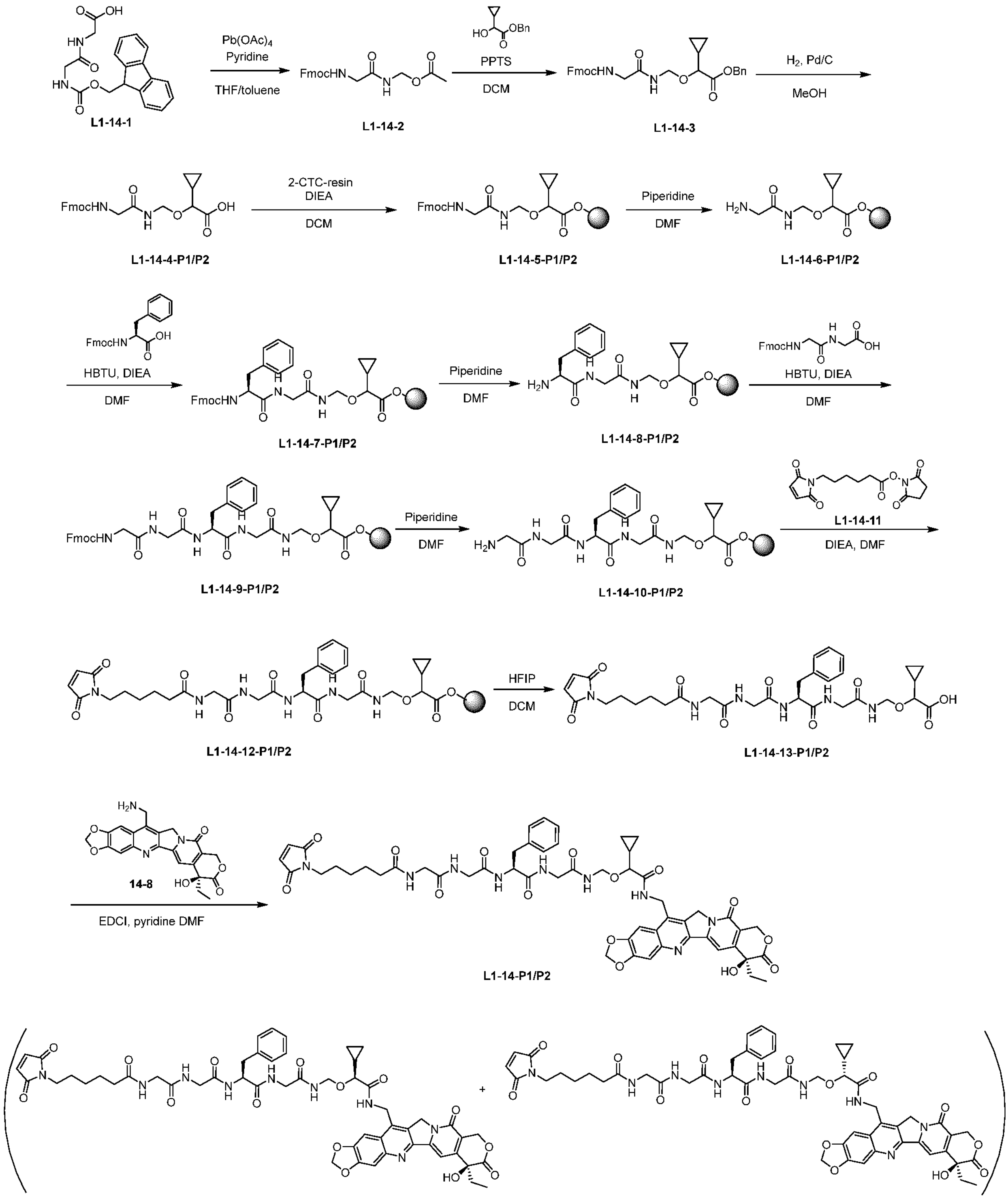


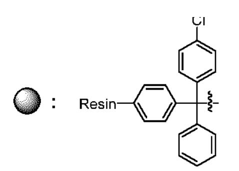


Fmoc:

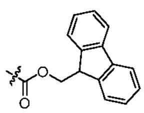

**Step 1: synthesis of intermediate L1-14-2**

**Starting material L1-14-1** (25 g), lead acetate (43.79 g), and pyridine (6.98 g) were dissolved in a mixed solvent

of tetrahydrofuran (600 mL) and toluene (200 mL), and the mixture was heated to 85 °C under nitrogen atmosphere and stirred for reaction for 18 h. After the reaction was completed, the reaction mixture was cooled to room temperature, filtered, the filtrate was concentrated to dryness under reduced pressure, and the residue was purified by column chromatography (column: ISCO®; 330 g SepaFlash® Silica Flash Column, mobile phase gradient: 0-75% ethyl acetate/petroleum ether, flow rate: 100 mL/min) to give the title compound (18 g).

**$^1$H NMR (400MHz, METHANOL-*d*$_4$)** $\delta$ = 7.82 (d, *J* = 7.5 Hz, 2H), 7.69 (d, *J* = 7.3 Hz, 2H), 7.44-7.38 (m, 2H), 7.36-7.31 (m, 2H), 5.22 (s, 2H), 4.39 (d, *J* = 6.8 Hz, 2H), 4.28-4.22 (m, 1H), 3.81 (s, 2H), 2.03 (s, 3H).

MS m/z (ESI): 391.1 $[M+Na]^+$.

**Step 2: synthesis of intermediate L1-14-3**

**Intermediate L1-14-2** (5 g), benzyl 2-cyclopropyl-2-hydroxyacetate (8.40 g), and pyridinium *p*-toluenesulfonate (PPTS, 341.09 mg) were dissolved in dichloromethane (150 mL), and the reaction mixture was heated to 65 °C under nitrogen atmosphere and stirred for reaction for 48 h. After the reaction was completed, the reaction mixture was cooled to room temperature, filtered, the filtrate was concentrated to dryness under reduced pressure, and the residue was purified by column chromatography (column: ISCO®; 120 g SepaFlash® Silica Flash Column, mobile phase gradient: 0-45% ethyl acetate/petroleum ether, flow rate: 80 mL/min) and then further purified by high performance liquid chromatography (column: Boston Prime C18 150 × 30 mm × 5 μm; mobile phase: [A: water (0.225% formic acid), B: acetonitrile]; B%: 42-82%, 13min) to give the title compound (1.4 g).

MS m/z (ESI): 537.2 $[M+Na]^+$.

**Step 3: synthesis of intermediates L1-14-4-P1 and L1-14-4-P2**

**Intermediate L1-14-3** (1.4 g) was dissolved in a mixed solvent of methanol (15 mL) and water (15 mL), wet palladium on carbon (10% mass content, 0.15 g) was added, and the reaction mixture was stirred at 25 °C for reaction for 16 h under hydrogen atmosphere. After the reaction was completed, the reaction mixture was filtered, the filtrate was concentrated to dryness under reduced pressure, and the residue was purified by high performance liquid chromatography (column: Boston Prime C18 150 × 30 mm × 5 μm; mobile phase: [A: water (0.225% formic acid), B: acetonitrile]; B%: 24% - 64%, 13 min) and then further purified by preparative supercritical fluid chromatography (column: DAICEL CHIRALPAK IC column, 10 μm silica, 30 mm diameter, 250 mm length; isopropanol (containing 0.1% ammonia water) was used as the eluent) to give **intermediate L1-14-4-P1** (130 mg) and **intermediate L1-14-4-P2** (130 mg).

The two isomers were separately further analyzed by the following chiral high performance liquid chromatography analysis method.

The chiral high performance liquid chromatographic condition was as follows:

| | |
|---|---|
| Column | Chiralpak IC-3 100A, 4.6mm I.D., 3um |
| Mobile phase | A: carbon dioxide |
| | B: isopropanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

**Intermediate L1-14-4-P1:**

Under the chiral high performance liquid chromatographic condition described above, the retention time was 4.471 min.

MS m/z (ESI): 447.5 $[M+Na]^+$.

**Intermediate L1-14-4-P2:**

Under the chiral high performance liquid chromatographic condition described above, the retention time was 5.692 min.

MS m/z (ESI): 447.3 [M+Na]+.

**Step 4: synthesis of intermediates L1-14-5-P1 and L1-14-5-P2**

2-Chlorotrityl chloride resin (2-CTC-resin) (specification: about 1.19 mmol/g) (257 mg) was added to dichloromethane (3 mL), and **intermediate L1-14-4-P1** (130 mg) and diisopropylethylamine (59.37 mg) were added. The reaction mixture was shaken on a shaker at 25 °C for reaction for 16 h under nitrogen atmosphere. After the reaction was completed, the resin was sequentially washed with methanol (10 mL) and dichloromethane (10 mL). The procedure was repeated 3 times. After filtration, the filter cake was dried to give **intermediate L1-14-5-P1** (330 mg).

**Intermediate L1-14-5-P2** (350 mg) was prepared according to the method described above using **intermediate L1-14-4-P2** (130 mg) as the starting material.

**Step 5: synthesis of intermediates L1-14-6-P1 and L1-14-6-P2**

**Intermediate L1-14-5-P1** (330 mg) was dissolved in *N,N*-dimethylformamide (5 mL), and piperidine (1.08 g) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-6-P1** (220 mg).

**Intermediate L1-14-6-P2** (220 mg) was prepared according to the method described above using **intermediate L1-14-5-P2** (350 mg) as the starting material.

**Step 6: synthesis of intermediates L1-14-7-P1 and L1-14-7-P2**

**Intermediate L1-14-6-P1** (220 mg) and (((9H-fluoren-9-yl)methoxy)carbonyl)-*L*-phenylalanine (230.17 mg) were dissolved in *N,N*-dimethylformamide (5 mL), and benzotriazole-*N,N,N,N*-tetramethyluronium hexafluorophosphate (HBTU) (225.31 mg) and diisopropylethylamine (99.96 mg) were added to the reaction mixture. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-7-P1** (377 mg).

**Intermediate L1-14-7-P2** (361 mg) was prepared according to the method described above using **intermediate L1-14-6-P2** (220 mg) as the starting material.

**Step 7: synthesis of intermediates L1-14-8-P1 and L1-14-8-P2**

**Intermediate L1-14-7-P1** (377 mg) was dissolved in *N,N*-dimethylformamide (5 mL), and piperidine (37.22 mg) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-8-P1** (270 mg).

**Intermediate L1-14-8-P2** (260 mg) was prepared according to the method described above using **intermediate L1-14-7-P2** (361 mg) as the starting material.

**Step 8: synthesis of intermediates L1-14-9-P1 and L1-14-9-P2**

**Intermediate L1-14-8-P1** (270 mg) was dissolved in *N,N*-dimethylformamide (5 mL), and *N*-((*9H*-fluoren-9-ylmethoxy)carbonyl)glycylglycine (209.58 mg), benzotriazole-*N,N,N,N*-tetramethyluronium hexafluorophosphate (224.29 mg), and diisopropylethylamine (99.51 mg) were sequentially added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **intermediate L1-14-9-P1** (403 mg).

**Intermediate L1-14-9-P2** (420 mg) was prepared according to the method described above using **intermediate L1-14-8-P2** (260 mg) as the starting material.

**Step 9: synthesis of intermediates L1-14-10-P1 and L1-14-10-P2**

**Intermediate L1-14-9-P1** (403 mg) was dissolved in *N,N*-dimethylformamide (5 mL), and piperidine (1.08 g) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-10-P1** (300 mg).

**Intermediate L1-14-10-P2** (320 mg) was prepared according to the method described above using **intermediate L1-14-9-P2** (420 mg) as the starting material.

**Step 10: synthesis of intermediates L1-14-12-P1 and L1-14-12-P2**

**Intermediate L1-14-10-P1** (300 mg) was dissolved in *N,N*-dimethylformamide (5 mL), and **compound L1-14-11** (182.13 mg) and diisopropylethylamine (99.41 mg) were sequentially added. The reaction mixture was shaken on a shaker at 25 °C for 16 h. After the reaction was completed, the resin was sequentially washed with methanol (10 mL) and dichloromethane (10 mL). The procedure was repeated 3 times. After filtration, the filter cake was dried to give **L1-14-12-P1** (374 mg). **Intermediate L1-14-12-P2** (387 mg) was prepared according to the method described above using **intermediate L1-14-10-P2** (320 mg) and **intermediate L1-14-11** (194.27 mg) as the starting materials.

**Step 11: synthesis of intermediates L1-14-13-P1 and L1-14-13-P2**

**Intermediate L1-14-12-P1** (374 mg) was added to a mixed solvent of dichloromethane (8 mL) and hexafluoroisopropanol (HFIP, 2 mL), and the reaction mixture was shaken on a shaker at 25 °C for 0.5 h. After the reaction was completed, the reaction mixture was filtered to remove the resin, the filtrate was concentrated to dryness under reduced pressure, and the residue was purified by high performance liquid chromatography (column: Boston Prime C18 150 × 30 mm × 5 μm; mobile phase: [A: water (0.225% formic acid), B: acetonitrile]; B: 15%-35%, 9 min) to give **intermediate L1-14-13-P1** (74 mg).

**$^1$H NMR (400MHz, METHANOL-$d_4$)** δ = 7.33-7.20 (m, 5H), 6.81 (s, 2H), 4.77-4.70 (m, 2H), 4.60-4.52 (m, 1H), 3.96-3.70 (m, 6H), 3.61 (d, *J* = 7.6 Hz, 1H), 3.55-3.47 (m, 2H), 3.27-3.23 (m, 1H), 3.05-2.98 (m, 1H), 2.30 (t, *J* = 7.4 Hz, 2H), 1.72-1.55 (m, 4H), 1.38-1.29 (m, 2H), 1.16-1.07 (m, 1H), 0.60-0.47 (m, 4H).

MS m/z (ESI): 679.7 $[M+Na]^+$.

**Intermediate L1-14-13-P2** (86 mg) was prepared according to the method described above using **intermediate L1-14-12-P2** (387 mg) as the starting material.

**$^1$H NMR (400MHz, METHANOL-$d_4$)** δ = 7.40-7.21 (m, 5H), 6.82 (s, 2H), 4.81-4.67 (m, 2H), 4.60-4.50 (m, 1H), 3.97-3.70 (m, 6H), 3.66-3.57 (m, 1H), 3.56-3.47 (m, 2H), 3.27-3.22 (m, 1H), 3.08-2.97 (m, 1H), 2.35-2.26 (m, 2H), 1.75-1.55 (m, 4H), 1.41-1.32 (m, 2H), 1.16-1.06 (m, 1H), 0.60-0.45 (m, 4H).

MS m/z (ESI): 679.5 $[M+Na]^+$.

**Step 12: synthesis of Compounds L1-14-P1 and L1-14-P2**

**Intermediate L1-14-13-P1** (31.17 mg), **intermediate 14-8** (20 mg), 1-(3-dimethylaminopropyl)-3-ethylcarbodiimide hydrochloride (EDCI) (18.20 mg), pyridine (11.26 mg), and 1-hydroxybenzotriazole (12.83 mg) were dissolved in *N,N*-dimethylformamide (1 mL), and the reaction mixture was stirred at 25 °C for reaction for 2 h under nitrogen atmosphere. After the reaction was completed, the reaction mixture was purified by high performance liquid chromatography (column: Boston Green ODS 150 × 30 mm × 5 μm; mobile phase: [A: water (0.225% formic acid), B: acetonitrile]; B: 26%-46%, 12 min) to give **compound L1-14-P1** (12.3 mg).

**$^1$H NMR** (400MHz, **DMSO-$d_6$**) δ = 8.65 (t, *J* = 5.9 Hz, 1H), 8.58 (t, *J* = 6.6 Hz, 1H), 8.27 (t, *J* = 5.6 Hz, 1H), 8.17-8.02 (m, 2H), 7.99 (t, *J* = 5.6 Hz, 1H), 7.79 (s, 1H), 7.52 (s, 1H), 7.28-7.19 (m, 5H), 7.19-7.14 (m, 1H), 6.98 (s, 2H), 6.49 (s, 1H), 6.29 (d, *J* = 3.7 Hz, 2H), 5.48-5.37 (m, 4H), 4.85-4.70 (m, 2H), 4.70-4.65 (m, 1H), 4.52-4.45 (m, 1H), 4.43-4.37 (m, 1H), 3.79-3.54 (m, 7H), 3.49 (d, *J* = 7.1 Hz, 1H), 3.08-3.01 (m, 1H), 2.85-2.74 (m, 1H), 2.14-2.06 (m, 2H), 1.94-1.80 (m, 2H), 1.52-1.42 (m, 4H), 1.27-1.13 (m, 2H), 1.01-0.92 (m, 1H), 0.88 (t, *J* = 7.3 Hz, 3H), 0.41-0.28 (m, 4H).

MS m/z (ESI): 1060.3 $[M+H]^+$.

**Compound L1-14-P2** (11.4 mg) was prepared according to the method described above using **intermediate L1-14-13-P2** (31.17 mg) and **intermediate 14-8** (20 mg) as the starting materials.

**$^1$H NMR (400MHz, DMSO-$d_6$)** δ = 8.72-8.52 (m, 2H), 8.33-8.25 (m, 1H), 8.17-7.94 (m, 3H), 7.80 (s, 1H), 7.51 (s, 1H), 7.32-7.19 (m, 5H), 7.18-7.12 (m, 1H), 6.99 (s, 2H), 6.49 (s, 1H), 6.35-6.25 (m, 2H), 5.48-5.36 (m, 4H), 4.84-4.59 (m, 3H), 4.54-4.45 (m, 1H), 4.44-4.35 (m, 1H), 3.81-3.54 (m, 7H), 3.49 (d, *J* = 6.7 Hz, 1H), 3.08-3.01 (m, 1H), 2.87-2.73 (m, 1H), 2.14-2.05 (m, 2H), 1.95-1.77 (m, 2H), 1.53-1.39 (m, 4H), 1.25-1.13 (m, 2H), 1.03-0.82 (m, 4H), 0.43-0.25 (m, 4H)

MS m/z (ESI): 1060.3 $[M+H]^+$.

The two isomers were separately further analyzed by the following chiral high performance liquid chromatography method.

The chiral high performance liquid chromatographic condition was as follows:

| | |
|---|---|
| Column | Chiralcel OJ-3 100A, 4.6mm I.D., 3um |

(continued)

| | |
|---|---|
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral high performance liquid chromatographic condition described above, the retention time of **compound L1-14-P1** was 3.735 min.

Under the chiral high performance liquid chromatographic condition described above, the retention time of **compound L1-14-P2** was 3.901 min.

**Example 38: *N*-((12*S*)-12-benzyl-4-cyclopropyl-1-((*S*)-7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)-3,8,11,14,17-pentaoxo-5-oxy-2,7,10,13,16-pentaazaoctadecan-18-yl)-6-(2,5-dioxo-2,5-dihydro-1*H*-pyrrol-1-yl)hexanamide (Compound L1-35-P1)**

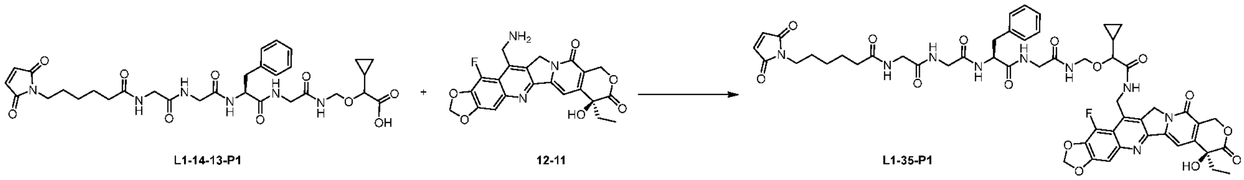

L1-14-13-P1 + 12-11 → L1-35-P1

**Intermediate L1-14-13-P1** (8 mg), **intermediate 12-11** (5.89 mg), 1-(3-dimethylaminopropyl)-3-ethylcarbodiimide hydrochloride (4.67 mg), pyridine (2.89 mg), and 1-hydroxybenzotriazole (3.29 mg) were dissolved in *N,N*-dimethylformamide (1 mL), and the reaction mixture was stirred at 25 °C for reaction for 2 h under nitrogen atmosphere. After the reaction was completed, the reaction mixture was purified by high performance liquid chromatography (column: Boston Prime C18 150 × 30 mm × 5 μm; mobile phase: [A: water (0.225% formic acid), B: acetonitrile]; B: 23%-45%, 10 min) to give **L1-35-P1** (2.3 mg).

**$^1$H NMR (400MHz, DMSO-$d_6$)** δ = 8.59 (t, *J* = 6.1 Hz, 1H), 8.36-8.31 (m, 1H), 8.28 (t, *J* = 5.6 Hz, 1H), 8.14-8.02 (m, 2H), 8.00 (t, *J* = 5.8 Hz, 1H), 7.49 (s, 1H), 7.27-7.16 (m, 6H), 6.99 (s, 2H), 6.52 (s, 1H), 6.38 (d, *J* = 2.0 Hz, 2H), 5.49-5.41 (m, 4H), 4.90-4.82 (m, 2H), 4.69-4.62 (m, 1H), 4.53-4.43 (m, 2H), 3.79-3.54 (m, 7H), 3.47 (d, *J* = 7.0 Hz, 1H), 3.06-3.00 (m, 1H), 2.80-2.74 (m, 1H), 2.10 (t, *J* = 7.3 Hz, 2H), 1.91-1.79 (m, 2H), 1.51-1.41 (m, 4H), 1.22-1.14 (m, 2H), 1.02-0.94 (m, 1H), 0.87 (t, *J* = 7.2 Hz, 3H), 0.40-0.27 (m, 4H).

MS m/z (ESI): 1078.2 [M+H]$^+$.

Further analysis was performed by the following supercritical fluid chromatography.

The supercritical fluid chromatographic condition was as follows:

| Column | Chiralcel OJ-3 100A, 4.6mm I.D., 3um | |
|---|---|---|
| Mobile phase | A: carbon dioxide | |
| | B: ethanol (0.05% diethylamine) | |
| | Time (min) | B phase% |
| | 0 | 5 |
| | 4 | 40 |
| | 2.5 | 40 |
| | 1.5 | 5 |

(continued)

| | |
|---|---|
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the supercritical fluid chromatographic condition described above, the retention time of **compound L1-35-P1** was 3.732 min.

**Example 39-1: Synthesis of *N*-((1*S*)-12-benzyl-4-cyclopropyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$)-3,8,11,14,17-pentaoxo-5-oxy-2,7,10,13,16-pentaazaoctadecan-18-yl)-6-(2,5-dioxo-2,5-dihydro-1*H*-pyrrol-1-yl)hexanamide (Compound L1-19-P1), isomer L1-19-P2, and racemate L1-19**

**Intermediate L1-14-13-P1** (7.75 mg), **intermediate 19-8** (5.0 mg), 1-(3-dimethylaminopropyl)-3-ethylcarbodiimide hydrochloride (4.53 mg), pyridine (2.80 mg), and 1-hydroxybenzotriazole (3.19 mg) were dissolved in *N,N*-dimethylformamide (1 mL), and the reaction mixture was stirred at 25 °C for reaction for 2 h under nitrogen atmosphere. After the reaction was completed, the reaction mixture was purified by high performance liquid chromatography (column: Boston Green ODS 150 × 30 mm × 5 μm; mobile phase: [A: water (0.225% formic acid), B: acetonitrile]; B: 22%-52%, 12min) to give **L1-19-P1** (6.0 mg).

MS m/z (ESI): 1062.3 $[M+H]^+$.

**$^1$H NMR (400MHz, DMSO-$d_6$)** δ = 8.74-8.69 (m, 1H), 8.59 (t, *J* = 6.7 Hz, 1H), 8.32-8.26 (t, *J* = 5.6 Hz, 1H), 8.11 (d, *J* = 7.8 Hz, 1H), 8.06 (t, *J* = 5.3 Hz, 1H), 7.99-7.94 (m, 1H), 7.80 (s, 1H), 7.52 (s, 1H), 7.27-7.20 (m, 5H), 7.19-7.11 (m, 1H), 6.99 (s, 2H), 6.50 (s, 1H), 5.49-5.41 (m, 4H), 4.84-4.72 (m, 2H), 4.71-4.63 (m, 1H), 4.53-4.48 (m, 1H), 4.44-4.36 (m, 1H), 3.77-3.56 (m, 6H), 3.49 (d, *J* = 7.0 Hz, 1H), 3.06-3.02 (m, 1H), 2.83-2.74 (m, 1H), 2.10 *(t, J*= 7.6 Hz, 2H), 1.91-1.82 (m, 2H), 1.50-1.42 (m, 4H), 1.22-1.13 (m, 2H), 1.02-0.90 (s, 1H), 0.88 (t, *J*= 7.3 Hz, 3H), 0.38-0.29 (m, 4H).

**Compound L1-19-P2** (19.0 mg) was prepared according to the method described above using **intermediate L1-14-13-P2** (30.0 mg) and **intermediate 19-8** (25.2 mg) as the starting materials. MS m/z (ESI): 1062.3 $[M+H]^+$.

**$^1$H NMR (400MHz, DMSO-$d_6$)** δ = 8.73-8.63 (m, 1H), 8.58 ( t, *J* = 6.4 Hz, 1H), 8.30 (t, *J* = 5.7 Hz, 1H), 8.13 (d, *J* = 8.1 Hz, 1H), 8.07 (t, *J* = 5.8 Hz, 1H), 8.01 (t, *J* = 5.4 Hz, 1H), 7.81 (s, 1H), 7.52 (s, 1H), 7.28-7.19 (m, 5H), 7.19-7.12 (m, 1H), 7.00 (s, 2H), 6.50 (s, 1H), 5.48-5.39 (m, 4H), 4.84-4.71 (m, 2H), 4.68-4.55 (m, 1H), 4.53-4.43 (m, 1H), 4.42-4.35 (m, 1H), 3.77-3.54 (m, 6H), 3.49 (d, *J* =7.0 Hz, 1H), 3.08-3.02 (m, 1H), 2.85-2.76 (m, 1H), 2.09 (t, *J* = 7.5 Hz, 2H), 1.93-1.79 (m, 2H), 1.52-1.39 (m, 4H), 1.25-1.12 (m, 2H), 1.02-0.94 (m, 1H), 0.88 (t, *J* = 7.3 Hz, 3H), 0.44-0.24 (m, 4H).

The two isomers were separately further analyzed by the following chiral high performance liquid chromatography analysis method.

The chiral high performance liquid chromatographic condition was as follows:

| | |
|---|---|
| Column | Chiralcel OJ-3 100A, 4.6mm I.D., 3um |
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic condition described above, the retention time of **L1-19-P1** was 3.775 min.

Under the chiral supercritical fluid chromatographic condition described above, the retention time of **L1-19-P2** was 3.973 min.

**Synthesis of racemate compound L1-19**

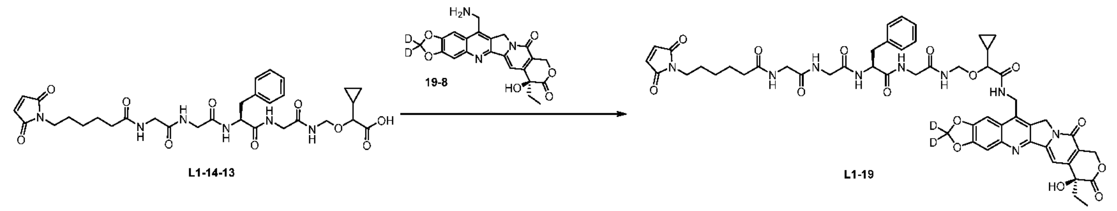


**Intermediate L1-14-13** (30.00 mg, prepared with reference to the synthesis method of **L1-14-13-P1** using benzyl 2-cyclopropyl-2-hydroxyacetate (racemate) as the starting material), **intermediate 19-8** (25.15 mg), 1-(3-dimethylaminopropyl)-3-ethylcarbodiimide hydrochloride (17.52 mg), pyridine (10.84 mg), and 1-hydroxybenzotriazole (12.35 mg) were dissolved in *N*,*N*-dimethylformamide (1 mL), and the reaction mixture was stirred at 25 °C for reaction for 2 h under nitrogen atmosphere. After the reaction was completed, the reaction mixture was purified by high performance liquid chromatography (column: Boston Green ODS 150 × 30 mm × 5 μm; mobile phase: [A: water (formic acid), B: acetonitrile]; B: 33%-33%, 14 min) to give racemate compound L1-19 (15.4 mg).

**$^1$H NMR (400MHz, DMSO-$d_6$)** δ = 8.76-8.66 (m, 1H), 8.59 (t, *J* = 6.8 Hz, 1H), 8.36-8.27 (m, 1H), 8.12 (d, *J*= 6.6 Hz, 1H), 8.09-8.05 (m, 1H), 8.04-8.00 (m, 1H), 7.81 (s, 1H), 7.52 (s, 1H), 7.27-7.19 (m, 5H), 7.17-7.12 (m, 1H), 7.00 (s, 2H), 6.50 (s, 1H), 5.49-5.42 (m, 4H), 4.84-4.71 (m, 2H), 4.71-4.63 (m, 1H), 4.53-4.42 (m, 1H), 4.44-4.35 (m, 1H), 3.83-3.54 (m, 6H), 3.49 (d, *J* = 7.1 Hz, 1H), 3.08-3.02 (m, 1H), 2.86-2.73 (m, 1H), 2.10 (t, *J* = 7.3 Hz, 2H), 1.93-1.79 (m, 2H), 1.49-1.37 (m, 4H), 1.25-1.13 (m, 2H), 1.01-0.92 (m, 1H), 0.88 (t, *J* = 7.2 Hz, 3H), 0.43-0.25 (m, 4H).

MS m/z (ESI): 1062.4 [M+H]$^+$.

**Example 39-2: *N*-((4*S*,12*S*)-12-benzyl-4-cyclopropyl-1-((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$)-3,8,11,14,17-pentaoxo-5-oxy-2,7,10,13,16-pentaazaoctadecan-18-yl)-6-(2,5-dioxo-2,5-dihydro-1*H*-pyrrol-1-yl)hexanamide (Compound L1-19-S)**

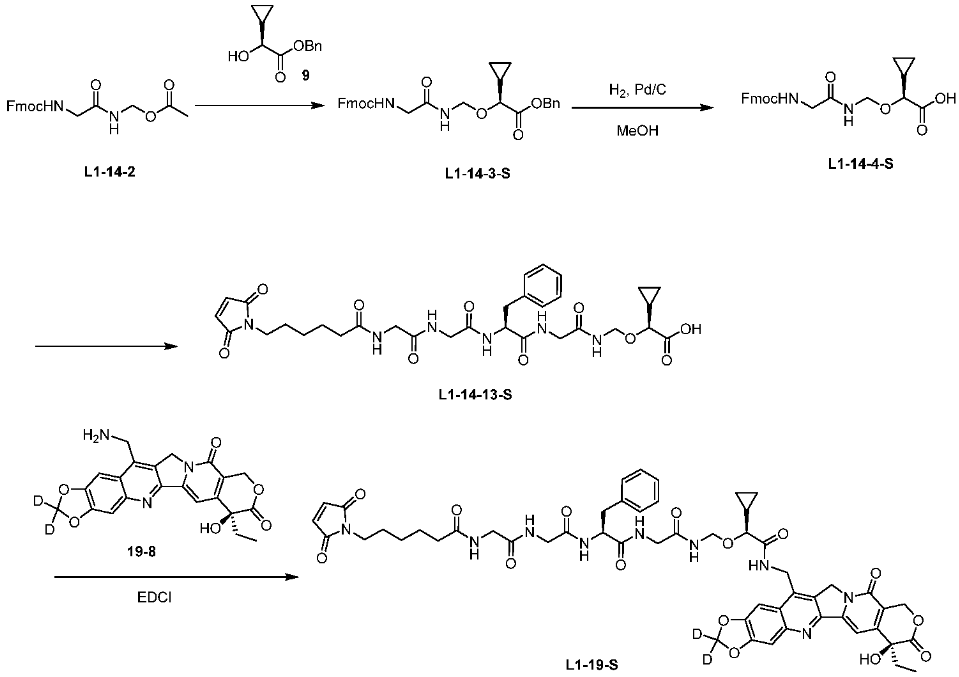

**Step 1: synthesis of L1-14-3-S**

**Intermediate L1-14-2** (28 g) and **intermediate 9** (23.5 g, prepared from Example 14-2) were dissolved in dichloromethane (25 mL), silver trifluoromethanesulfonate (139.50 mg) was added to the reaction mixture, and the reaction mixture was stirred at 25 °C for reaction for 108 h under nitrogen atmosphere. After the reaction was completed, the reaction mixture was filtered, the filtrate was concentrated to dryness under reduced pressure, and the residue was purified by a flash silica gel column (mobile phase gradient: tetrahydrofuran/dichloromethanol: 0-7%, flow rate: 70 mL/min) to give the title compound (10.3 g).

MS m/z (ESI): 537.3 $[M+Na]^+$.

**Step 2: synthesis of L1-14-4-S**

**Intermediate L1-14-3-S** (10 g) was dissolved in tetrahydrofuran (100 mL), wet palladium on carbon (10% mass content, 1 g) was added, and the reaction mixture was stirred at 0 °C for reaction for 16 h under hydrogen atmosphere. After the reaction was completed, the reaction mixture was filtered, and the filtrate was concentrated to dryness under reduced pressure. Petroleum ether/ethyl acetate (10 mL/0.5 mL) was added to the residue, and the mixture was stirred for 2 h and filtered to give the title compound (6 g).

MS m/z (ESI): 447.2 $[M+Na]^+$.

**L1-14-4-S** was further analyzed by the following chiral high performance liquid chromatography analysis method.

The chiral high performance liquid chromatographic condition was as follows:

| Column | Chiralpak IC-3 100A, 4.6mm I.D., 3um |
|---|---|
| Mobile phase | A: carbon dioxide |
| | B: isopropanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic condition described above, the retention time of **L1-14-4-S** was 4.385 min, which was substantially consistent with the retention time (4.471 min) of **compound L1-14-4-P1** under the same chromatographic analysis condition. Thus, **L1-14-4-S** and **L1-14-4-P1** have the same configuration, being the same compound.

**Step 3: synthesis of L1-19-S**

**Intermediate L1-14-13-S** (600 mg, prepared with reference to the synthesis method of **L1-14-13-P1** using **L1-14-4-S** as the starting material), **intermediate 19-8** (502.93 mg), 1-(3-dimethylaminopropyl)-3-ethylcarbodiimide hydrochloride (EDCI) (350.31 mg), pyridine (216.82 mg), and 1-hydroxybenzotriazole (246.91 mg) were dissolved in *N,N*-dimethylformamide (2 mL), and the reaction mixture was stirred at 25 °C for reaction for 2 h under nitrogen atmosphere. After the reaction was completed, the reaction mixture was purified by high performance liquid chromatography (column: Boston Green ODS 150 × 30 mm × 5 μm; mobile phase: [A: water (0.05% formic acid), B: acetonitrile]; B: 24%-54%, 12 min) to give the title compound (286 mg).

**$^1$H NMR (400MHz, DMSO-$d_6$)** δ = 8.72-8.63 (m, 1H), 8.62-8.52 (m, 1H), 8.34-8.26 (m, 1H), 8.16-7.94 (m, 3H), 7.78 (s, 1H), 7.51 (s, 1H), 7.26-7.13(m, 6H), 6.99 (s, 1H), 6.56-6.42 (m, 1H), 5.52-5.40 (m, 4H), 4.85-4.60 (m, 3H), 4.59-4.46 (m, 1H), 4.44-4.38 (m, 1H), 3.79-3.53 (m, 6H), 3.52-3.44 (m, 2H), 3.08-3.02 (m, 1H), 2.85-2.75 (m, 1H), 2.09 (t, *J*=7.8 Hz, 2H), 1.95-1.75 (m, 2H), 1.58-1.38 (m, 4H), 1.26-1.09 (m, 2H), 1.04-0.96 (m, 1H), 0.88 (t, *J* = 7.1 Hz, 3H), 0.42-0.26 (m, 4H).

MS m/z (ESI): 1062.5 $[M+H]^+$.

**L1-19-S** was further analyzed by the following chiral high performance liquid chromatography method.

The chiral high performance liquid chromatographic condition was as follows:

| Column | Chiralcel OJ-3 100A, 4.6mm I.D., 3um |
|---|---|
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic condition described above, the retention time of **L1-19-S** was 3.748 min, which was substantially consistent with the retention time (3.775 min) of **compound L1-19-P1** prepared in Example 39-1 under the same chromatographic analysis condition. Thus, **L1-19-S** and **L1-19-P1** were determined to have the same configuration, belonging to the same compound.

For the synthesis of compounds LI-0 and L1-00, reference was made to WO2019195665A1 and WO2020063676A1, respectively.

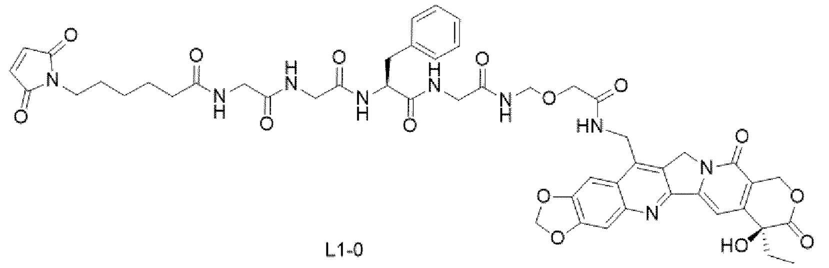

L1-0

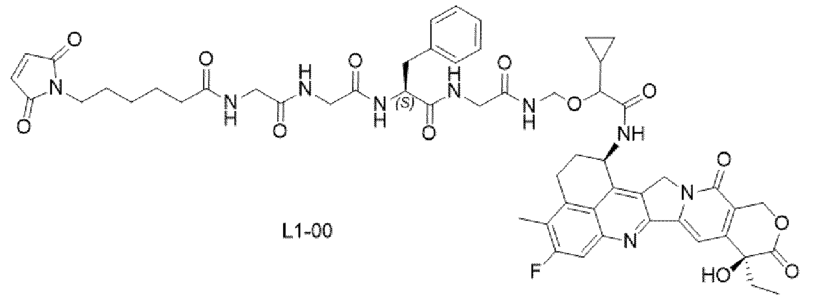

L1-00

**Example 40: Preparation of antibody-drug conjugate**

40.1 Antibody:

40.1.1 Construction and production of anti-human HER2 monoclonal antibody

The sequence of the anti-human HER2 monoclonal antibody is shown in Table 2 below. The nucleic acid sequences encoding VHs and VLs of the antibodies were recombined into expression vector pTT5 carrying a signal peptide and heavy chain constant region/light chain constant region sequences to give recombinant plasmids expressing VH-CH1-Fc/VL-CL. After verification by sequencing, the plasmid was extracted, and a host cell was transfected. After culturing, an antibody-secreting cell culture supernatant was obtained.

Table 2 Sequence information of anti-human HER2 antibody and CDR analysis thereof (according to Kabat numbering scheme)

| Antibody | Sequence No. | Sequence information |
|---|---|---|
| trastuzumab-VH | SEQ ID NO:1 | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSS |
| trastuzumab-VL | SEQ ID NO:2 | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIK |
| pertuzumab-VH | SEQ ID NO:3 | EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSS |
| pertuzumab-VL | SEQ ID NO:4 | DIQMTQSPSSLSASVGDRVTITCKASQDVSIGVAWYQQKPGKAPKLLIYSASYRYTGVPSRFSGSGSGTDFTLTISSLQPEDFATYYCQQYYIYPYTFGQGTKVEIK |
| human CH1-Fc | SEQ ID NO:5 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| human CL | SEQ ID NO:6 | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

| Antibody | CDR1 | CDR2 | CDR3 |
|---|---|---|---|
| trastuzumab-VH | DTYIH (SEQ ID NO:7) | RIYPTNGYTRYADSVKG (SEQ ID NO:8) | WGGDGFYAMDY (SEQ ID NO:9) |
| trastuzumab-VL | RASQDVNTAVA (SEQ ID NO:10) | SASFLYS (SEQ ID NO:11) | QQHYTTPPT (SEQ ID NO:12) |
| pertuzumab-VH | DYTMD (SEQ ID NO:13) | DVNPNSGGSIYNQRFKG (SEQ ID NO:14) | NLGPSFYFDY (SEQ ID NO:15) |
| pertuzumab-VL | KASQDVSIGVA (SEQ ID NO:16) | SASYRYT (SEQ ID NO:17) | QQYYIYPYT (SEQ ID NO:18) |

40.1.2 Screening of anti-human p95HER2 monoclonal antibody

Anti-human p95HER2 monoclonal antibodies were produced by immunization of mice. The immunogen was hu p95HER2.ECD-Fc protein. Spleen lymphocytes of mice with high antibody titer and titer approaching to a plateau in serum were selected to prepare hybridoma cells. Positive hybridoma clones were obtained by screening through ELISA, FACS and other conventional methods in the field. The antibodies were further prepared by a serum-free cell culture method, and the antibodies were purified and sequenced. The murine anti-human p95HER2 antibody was obtained by sequencing.

By comparing the IMGT database (http://imgt.cines.fr) for germline genes from heavy and light chain variable regions of human antibodies with the Molecular Operating Environment (MOE) software, germline genes, with high homology with murine antibodies, from heavy chain and light chain variable regions were respectively selected as templates, and CDRs of murine antibodies were respectively grafted into corresponding human templates to form a variable region sequence of FR1-CDR1-FR2-CDR2-FR3-CDR3-FR4. Based on this, back mutation was performed as needed to maintain the original affinity and/or hotspot mutation was performed to eliminate the risk of molecular modifications. The final optimized humanized antibodies and corresponding sequences are shown in Table 3.

Table 3 Sequence information of optimized humanized antibody and CDR analysis thereof (according to Kabat numbering scheme)

| Antibody | Sequence No. | Sequence information |
|---|---|---|
| P95-Hab01-VH | SEQ ID NO:19 | EVTLRESGPALVKPTQTLTLTCTFSGFSLYTSGMGVSWIRQPPGKALEWLANIYWDNDKRYNPSLKSRLTISKDTSGNQVVLTMTNMDPVDTATYYCARSFLYYGNSHWYFDVWGQGTTVTVSS |
| P95-Hab01-VL | SEQ ID NO:20 | EIVLTQSPATLSLSPGERATLSCSASSSVSYMYWYQQKPGQAPRLLIYRTSNLASGIPARFSGSGSGTDFTLTISSLEPEDFAVYYCQQYHSYPPTFGQGTKLEIK |
| P95-Hab02-VH | SEQ ID NO:21 | EVTLKESGPALVKPTQTLTLTCTFSGFSLSTFGLGVGWIRQPPGKALEWLAHIWWDTAKNFNPVLKSRLTVSKDTSKNQVVLTMTNMDPVDTATYYCVRIGNYGSPYFDYWGQGTTVTVSS |
| P95-Hab02-VL | SEQ ID NO:22 | LIQMTQSPSSLSASVGDRVTITCLASQSIGTYLAWYQQKPGKAPKLLIYAATSLTDGVPSRFSGSGSGTDFTLTISSLQPEDFATYYCQQVHSSPYSFGGGTKVEIK |

| Antibody | CDR1 | CDR2 | CDR3 |
|---|---|---|---|
| P95-Hab01-VH | TSGMGVS (SEQ ID NO:23) | NIYWDNDKRYNPSLKS (SEQ ID NO:24) | SFLYYGNSHWYFDV (SEQ ID NO:25) |
| P95-Hab01-VL | SASSSVSYMY (SEQ ID NO:26) | RTSNLAS (SEQ ID NO:27) | QQYHSYPPT (SEQ ID NO:28) |
| P95-Hab02-VH | TFGLGVG (SEQ ID NO:29) | HIWWDTAKNFNPVLKS (SEQ ID NO:30) | IGNYGSPYFDY (SEQ ID NO:31) |
| P95-Hab02-VL | LASQSIGTYLA (SEQ ID NO:32) | AATSLTD (SEQ ID NO:33) | QQVHSSPYS (SEQ ID NO:34) |

40.1.3 Construction and production of p95HER2/HER2 bispecific antibody

The antibody in the DVD-Ig form is composed of VH1-linker-VH2-CH1-Fc and VL1-linker-VL2-CL, wherein VH1 and VL1 target HER2, VH2 and VL2 target p95HER2, and the linker may be omitted. See Table 4 for the specific sequence of the bispecific antibody. According to the designed structure, bispecific antibody light and heavy chain expression plasmids were separately constructed on a pTT5 vector and were expressed in HEK293 cells.

Table 4 Sequence information of p95HER2/HER2 bispecific antibody

| Antibody | Sequence No. | Sequence information |
|---|---|---|
| BsAb02-P heavy chain full-length sequence | SEQ ID NO:35 | EVQLVESGGGLVQPGGSLRLSCAASGFTFTDYTMDWVRQAPGKGLEWVA DVNPNSGGSIYNQRFKGRFTLSVDRSKNTLYLQMNSLRAEDTAVYYCARN LGPSFYFDYWGQGTLVTVSSGGGGSGGGGSGGGGSEVTLKESGPALVKPT QTLTLTCTFSGFSLSTFGLGVGWIRQPPGKALEWLAHIWWDTAKNFNPVL KSRLTVSKDTSKNQVVLTMTNMDPVDTATYYCVRIGNYGSPYFDYWGQG TTVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDKSR WQQGNVFSCSVMHEALHNHYTQKSLSLSPGK |
| BsAb02-P light chain full-length sequence | SEQ ID NO:36 | DIQMTQSPSSLSASVGDRVTITCKASQDVSIGVAWYQQKPGKAPKLLIYSA SYRYTGVPSRFSGSGSGTDFTLTISSLQPEDFATYYCQQYYIYPYTFGQGTK VEIKGGGGSGGGGSGGGGSLIQMTQSPSSLSASVGDRVTITCLASQSIGTYL AWYQQKPGKAPKLLIYAATSLTDGVPSRFSGSGSGTDFTLTISSLQPEDFAT YYCQQVHSSPYSFGGGTKVEIKRTVAAPSVFIFPPSDEQLKSGTASVVCLLN NFYPREAKVQWKVDNALQSGNSQESVTEQDSKDSTYSLSSTLTLSKADYE KHKVYACEVTHQGLSSPVTKSFNRGEC |

40.1.4 Construction and production of anti-human CDH6 antibody

The sequences of the anti-human CDH6 monoclonal antibodies CDH6-Ab and CDH6-Ab-1 are shown in Table 5 below (antibody sequences from US20200171163A1).

Table 5 Sequence information of anti-human CDH6 antibodies CDH6-Ab and CDH6-Ab-1 and CDR analysis thereof (according to Kabat numbering scheme)

| Antibody | Sequence No. | Sequence information |
|---|---|---|
| CDH6-Ab-VH | SEQ ID NO:37 | EVQLVQSGAEVKKPGASVKVSCKASGYTFTRNFMHWVRQAPGQGL EWMGWIYPGDGETEYAQKFQGRVTITADTSTSTAYMELSSLRSEDTA VYYCARGVYGGFAGGYFDFWGQGTLVTVSS |
| CDH6-Ab-VL | SEQ ID NO:38 | DIQMTQSPSSLSASVGDRVTITCKASQNIYKNLAWYQQKPGKAPKLL IYDANTLQTGVPSRFSGSGSGSDFTLTISSLQPEDFATYFCQQYYSGW AFGQGTKVEIK |
| CDH6-Ab-1-VH | SEQ ID NO:46 | QVQLLESGGGLVQPGGSLRLSCAASGFTFSSHGMHWVRQAPGKGLE WVSVISGSGSNTGYADSVKGRFTISRDNSKNTLYLQMNSLRAEDTAV YYCARQWGSYAFDSWGQGTLVTVSS |
| CDH6-Ab-1-VL | SEQ ID NO:47 | DIQMTQSPSSLSASVGDRVTITCRASQSISSYLNWYQQKPGKAPKLLI YAVSTLQSGVPSRFSGSGSGTDFTLTISSLQPEDFATYYCQQSGTFPPT TFGQGTKVEIK |
| human CH1-Fc-1 | SEQ ID NO:39 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPELLGGPSVFLFPPKPKDTLMISRTPEVTCV VVDVSHEDPEVKFNWYVDGVEVHNAKTKPREEQYNSTYRVVSVLT VLHQDWLNGKEYKCKVSNKALPAPIEKTISKAKGQPREPQVYTLPPS REEMTKNQVSLTCLVKGFYPSDIAVEWESNGQPENNYKTTPPVLDSD GSFFLYSKLTVDKSRWQQGNVFSCSVMHEALHNHYTQKSLSLSPGK |
| human CL | SEQ ID NO:6 | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |

| Antibody | CDR1 | CDR2 | CDR3 |
|---|---|---|---|
| CDH6-Ab-VH | RNFMH (SEQ ID NO:40) | WIYPGDGETEYAQKFQG (SEQ ID NO:41) | GVYGGFAGGYFDF (SEQ ID NO:42) |
| CDH6-Ab-VL | KASQNIYKNLA (SEQ ID NO:43) | DANTLQT (SEQ ID NO:44) | QQYYSGWA (SEQ ID NO:45) |
| CDH6-Ab-1-VH | SHGMH (SEQ ID NO:48) | VISGSGSNTGYADSVKG (SEQ ID NO:49) | QWGSYAFDS (SEQ ID NO:50) |
| CDH6-Ab-1-VL | RASQSISSYLN (SEQ ID NO:51) | AVSTLQS (SEQ ID NO:52) | QQSGTFPPTT (SEQ ID NO:53) |

CDH6-Ab-2 and CDH6-Ab-3 were produced by immunization of mice. The immunogens were human CDH6-hFc protein and HEK293T cells overexpressing human CDH6. Spleen lymphocytes of mice with high antibody titer in serum were selected to prepare hybridoma cells. Positive hybridoma clones were obtained by screening through ELISA, FACS and other conventional methods in the field. The antibodies were further prepared by a serum-free cell culture method, and the antibodies were purified and sequenced to give a murine anti-human CDH6 antibody and a variable region sequence thereof. By comparing the IMGT database (http://imgt.cines.fr) for germline genes from heavy and light chain variable regions of human antibodies with the Molecular Operating Environment (MOE) software, germline genes, with high homology with murine antibodies, from heavy chain and light chain variable regions were respectively selected as templates, and CDRs of murine antibodies were respectively grafted into corresponding human templates to form a variable region sequence of FR1-CDR1-FR2-CDR2-FR3-CDR3-FR4. Based on this, back mutation was performed as needed to maintain the original affinity and/or hotspot mutation was performed to eliminate the risk of molecular modifications. The VH and the VL corresponding to the finally optimized humanized antibodies CDH6-Ab-2 and CDH6-Ab-3 and the CDR sequences divided according to the Kabat form are shown in Table 6.

Table 6 Sequence information of anti-human CDH6 antibodies CDH6-Ab-2 and CDH6-Ab-3 and CDR analysis thereof (according to Kabat numbering scheme)

| Antibody | Sequence No. | Sequence information |
|---|---|---|
| CDH6-Ab-2-VH | SEQ ID NO:54 | EVQLVQSGAEVKKPGASVKVSCKASGYTFTDNYINWVRQAPGQRLEWMGWIYPGTGNSYYNEKFKGRVTITVDTSASTAYMELSSLRSEDTAVYLCASSYGDSFDYWGQGTTVTVSS |
| CDH6-Ab-2-VL | SEQ ID NO:55 | DIVMTQSPDSLAVSLGERATINCRASQDIRNYLNWYQQKPGQTPKLLISYTSRLHSGVPDRFSGSGSGTDFTLTISSLQAEDVAVYYCQQYSQLPWTFGGGTKVEIK |
| CDH6-Ab-3-VB | SEQ ID NO:56 | EVQLVQSGAEVKKPGASVKVSCKASGYTFTDYYMAWVRQAPGQGLEWMGYIFPNSGGTDYSQKFKGRVTLTVDKSTSTVYMELSSLRSEDTAVYYCARELGADYGDYYAMDHWGQGTTVTVSS |
| CDH6-Ab-3-VL | SEQ ID NO:57 | DIQMTQSPSSLSASVGDRVTITCRASENIDSYLAWYQQKPGKSPKLLVYSATLLADGVPSRFSGSGSGTDYTLTISSLQPEDFATYYCQHYYRTPPTFGQGTKLEIK |
| human CH1-Fc-1 | SEQ ID NO:5 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| human CL | SEQ ID NO:6 | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

| Antibody | CDR1 | CDR2 | CDR3 |
|---|---|---|---|
| CDH6-Ab-2-VH | DNYIN (SEQ ID NO:58) | WIYPGTGNSYYNEKFKG (SEQ ID NO:59) | SYGDSFDY (SEQ ID NO:60) |
| CDH6-Ab-2-VL | RASQDIRNYLN (SEQ ID NO:61) | YTSRLHS (SEQ ID NO:62) | QQYSQLPWT (SEQ ID NO:63) |
| CDH6-Ab-3-VB | DYYMA (SEQ ID NO:64) | YIFPNSGGTDYSQKFKG (SEQ ID NO:65) | ELGADYGDYYAMDH (SEQ ID NO:66) |
| CDH6-Ab-3-VL | RASENIDSYLA (SEQ ID NO:67) | SATLLAD (SEQ ID NO:68) | QHYYRTPPT (SEQ ID NO:69) |

The nucleic acid sequences encoding VHs and VLs of the antibodies were recombined into expression vector pTT5 carrying a signal peptide (MGWSWILLFLLSVTAGVHS, SEQ ID NO: 70) and heavy chain constant region/light chain constant region sequences to give recombinant plasmids expressing VH-CH1-Fc/VL-CL. The plasmid and the transfection reagent PEI (Polysciences, Cat. No. 24765-1) were added to OPTI-MEM (Gibco, Cat. No. 11058021). The mixture was well mixed and let stand for 15 min. Expi293F cells (manufacturer: Thermofisher, Cat. No. A14527) were added, and the system was incubated on a shaker at 37 °C in 5% $CO_2$ at 120 rpm. On day 2 of the transfection, OPM-293 ProFeed (Shanghai OPM Biosciences Co., Ltd., Cat. No. F081918-001) and 6 g/L glucose (manufacturer: Sigma, Cat. No. G7528) were added. On day 6 of the transfection, an antibody-secreting cell culture supernatant was obtained.

40.1.5 Construction and production of anti-human LIV-1 monoclonal antibody

The sequence of the anti-human LIV-1 monoclonal antibody LIV1-Ab-1 is shown in Table 7 below (antibody sequence from US20200165335A). The nucleic acid sequences encoding VHs and VLs of the antibodies were recombined into expression vector pTT5 carrying a signal peptide and heavy chain constant region (sequence)/light chain constant region sequences to give recombinant plasmids expressing LIV1-Ab-1. After verification by sequencing, the plasmid was extracted. The plasmid and the transfection reagent PEI (Polysciences, Cat. No. 24765-1) were added to OPTI-MEM (Gibco, Cat. No. 11058021). The mixture was well mixed and let stand for 15 min. Expi293 cells (manufacturer: Thermofisher, Cat. No. A14527) were added, and the system was incubated on a shaker at 37 °C in 5% $CO_2$ at 120 rpm. On day 2 of the transfection, OPM-293 ProFeed (Shanghai OPM Biosciences Co., Ltd., Cat. No. F081918-001) and 6 g/L glucose (manufacturer: Sigma, Cat. No. G7528) were added. On day 6 of the transfection, the cell supernatant was collected.

Table 7 Sequence information of anti-human LIV-1 antibody and CDR analysis thereof (according to Kabat numbering scheme)

| Sequence name | Sequence No. | Sequence information | |
|---|---|---|---|
| LIV1-Ab-1-VH | SEQ ID NO:71 | QVQLVQSGAEVKKPGASVKVSCKASGLTIEDYYMHWVRQAPGQGL<br>EWMGWIDPENGDTEYGPKFQGRVTMTRDTSINTAYMELSRLRSDDT<br>AVYYCAVHNAHYGTWFAYWGQGTLVTVSS | |
| LIV1-Ab-1-VL | SEQ ID NO:72 | DVVMTQSPLSLPVTLGQPASISCRSSQSLLHSSGNTYLEWYQQRPGQ<br>SPRPLIYKISTRFSGVPDRFSGSGSGTDFTLKISRVEAEDVGVYYCFQG<br>SHVPYTFGGGTKVEIKR | |
| human CH1-Fc-2 | SEQ ID NO:73 | ASTKGPSVFPLAPSSKSTSGGTAALGCLVKDYFPEPVTVSWNSGALTS<br>GVHTFPAVLQSSGLYSLSSVVTVPSSSLGTQTYICNVNHKPSNTKVDK<br>KVEPKSCDKTHTCPPCPAPELLGGPSVFLFPPKPKDTLMISRTPEVTC<br>VVVDVSHEDPEVKFNWYVDGVEVHNAKTKPREEQYNSTYRVVSVL<br>TVLHQDWLNGKEYKCKVSNKALPAPIEKTISKAKGQPREPQVYTLPP<br>SRDELTKNQVSLTCLVKGFYPSDIAVEWESNGQPENNYKTTPPVLDS<br>DGSFFLYSKLTVDKSRWQQGNVFSCSVMHEALHNHYTQKSLSLSPG<br>K | |
| human CL | SEQ ID NO:6 | RTVAAPSVFIFPPSDEQLKSGTASVVCLLNNFYPREAKVQWKVDNAL<br>QSGNSQESVTEQDSKDSTYSLSSTLTLSKADYEKHKVYACEVTHQGL<br>SSPVTKSFNRGEC | |
| Sequence name | CDR1 | CDR2 | CDR3 |
| LIV1-Ab-1-VH | DYYMH (SEQ ID NO:74) | WIDPENGDTEYGPKFQG (SEQ ID NO:75) | HNAHYGTWFAY (SEQ ID NO:76) |
| LIV1-Ab-1-VL | RSSQSLLHSSG<br>NTYLE<br>(SEQ ID NO:77) | KISTRFS (SEQ ID NO:78) | FQGSHVPYT (SEQ ID NO:79) |

40.1.6 Construction and production of anti-human ROR1 monoclonal antibody

The variable region sequence of the anti-human ROR1 monoclonal antibody ROR1-Ab-1 was from patent WO2020198531A2, the variable region sequence of ROR1-Ab-2 was from patent CN113521300A, and the variable region sequence of ROR1-Ab-3 was from patent WO2020074724A1 (see Table 8). The nucleic acid sequence encoding VHs and VLs of the antibodies were recombined into a pTT59 expression vector carrying CH and CL of human IgG1 to give recombinant plasmids expressing ROR1-Ab-1, ROR1-Ab-2, and ROR1-Ab-3, respectively.

The plasmid and the transfection reagent PEI (Polysciences, 24765-1) were added to OPTI-MEM (Gibco, Cat. No. 11058021). The mixture was well mixed and let stand for 15 min. Expi293 cells (Thermofisher, A14527) were added, and the system was incubated on a shaker at 37 °C in 5% $CO_2$ at 120 rpm. On day 2 of the transfection, OPM-293 ProFeed (Shanghai OPM Biosciences Co., Ltd., F081918-001) and 6 g/L glucose (Sigma, G7528) were added. On day 6 of the transfection, the cell supernatant was collected.

Table 8 Sequence information of anti-human ROR1 antibody and CDR analysis thereof (according to Kabat numbering scheme)

| Sequence name | Sequence No. | Amino acid sequence |
| --- | --- | --- |
| ROR1-Ab-1-VH | (SEQ ID NO:80) | QVQLQESGPGLVKPSQTLSLTCTVSGYAFTAYNIHWVRQA PGQGLEWMGSFDPYDGGSSYNQKFKDRLTISKDTSKNQV VLTMTNMDPVDTATYYCARGWYYFDYWGHGTLVTVSS |
| ROR1-Ab-1-VL | (SEQ ID NO:81) | DIVMTQTPLSLPVTPGEPASISCRASKSISKYLAWYQQKPG QAPRLLIYSGSTLQSGIPPRFSGSGYGTDFTLTINNIESEDA AYYFCQQHDESPYTFGEGTKVEIK |
| ROR1-Ab-2-VH | (SEQ ID NO:82) | EVQLLESGGGLVQPGGSLRLSCAASGFTFSNYAMSWVRQ APGKGLEWVSSISHNSGSTYYADSVKGRFTISRDNSKNTL YLQMNSLRAEDTAVYYCAKFISARKSLGRSYSNGMDVW GQGTLVTVSS |
| ROR1-Ab-2-VL | (SEQ ID NO:83) | QSVLTQPPSASGTPGQRVTISCTGSSSNIGSNDVTWYQQLP GTAPKLLIYADSKRPSGVPDRFSGSKSGTSASLAISGLRSE DEADYYCGTWDYSLSGYVFGGGTKLTVL |
| ROR1-Ab-3-VH | (SEQ ID NO:84) | QVQLRESGPGLVKPSETLSLTCTVSGFDISSYYMSWVRQP PGKGLEWIGAIGISGNAYYASWAKSRVTISRDTSKNQFSLK LSSVTAADTAVYYCARDHPTYGMDLWGPGTLVTVSS |
| ROR1-Ab-3-VL | (SEQ ID NO:85) | SYELTQPPSVSVAPGKTARITCEGNNIGSKAVHWYQQKPG QAPVLVIYDDDERPSGIPERFSGSNSGNTATLTISRVEAGDE ADYYCQVWDSSAYVFGGGTKLTVL |
| human CH1-Fc | SEQ ID NO:5 | ASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKKVEPKSCDKTHTCPPCPAPELLGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVYTLPPSREE MTKNQVSLTCLVKGFYPSDIAVEWESNGQPENNYKTTPP VLDSDGSFFLYSKLTVDKSRWQQGNVFSCSVMHEALHNH YTQKSLSLSPGK |
| human CL | SEQ ID NO:6 | RTVAAPSVFIFPPSDEQLKSGTASVVCLLNNFYPREAKVQ WKVDNALQSGNSQESVTEQDSKDSTYSLSSTLTLSKADY EKHKVYACEVTHQGLSSPVTKSFNRGEC |

(continued)

| Sequence name | CDR1 | CDR2 | CDR3 |
|---|---|---|---|
| ROR1-Ab-1-VH | AYNIH (SEQ ID NO:86) | SFDPYDGGSSYNQKFKD (SEQ ID NO:87) | GWYYFDY (SEQ ID NO:88) |
| ROR1-Ab-1-VL | RASKSISKYLA (SEQ ID NO:89) | SGSTLQS (SEQ ID NO:90) | QQHDESPYT (SEQ ID NO:91) |
| ROR1-Ab-2-VH | NYAMS (SEQ ID NO:92) | SISHNSGSTYYADSVKG (SEQ ID NO:93) | FISARKSLGRSYSNGMDV (SEQ ID NO:94) |
| ROR1-Ab-2-VL | TGSSSNIGSNDV T (SEQ ID NO:95) | ADSKRPS (SEQ ID NO:96) | GTWDYSLSGYV (SEQ ID NO:97) |
| **Sequence name** | **Sequence No.** | **Amino acid sequence** | |
| ROR1-Ab-3-VH | SYYMS (SEQ ID NO:98) | AIGISGNAYYASWAKS (SEQ ID NO:99) | DHPTYGMDL (SEQ ID NO:100) |
| ROR1-Ab-3-VL | EGNNIGSKAVH (SEQ ID NO:101) | DDDERPS (SEQ ID NO:102) | QVWDSSAYV (SEQ ID NO:103) |

40.2 Antibody purification: The antibody described above was purified from the cell culture supernatant using ProteinA affinity chromatography. The Protein A affinity column was washed with 3-5 column volumes of 6 M guanidine hydrochloride, then with 3-5 column volumes of pure water. The column was equilibrated with 3-5 column volumes of, for example, 1× PBS (pH 7.4) buffer system as an equilibration buffer. The cell supernatant was loaded on the column at a low flow rate for binding, with the flow rate controlled to allow for a retention time of about 1 min or longer. After the binding was completed, the chromatographic column was washed with 3-5 column volumes of 1× PBS (pH 7.4) until the UV absorbance fell back to baseline. The sample was eluted with a 0.1 M acetic acid-sodium acetate (pH 3.0-3.5) buffer, the elution peaks were collected by UV monitoring, and the eluted product was rapidly adjusted to pH 5-6 with 1 M Tris-HCl (pH 8.0) and temporarily stored. For the eluted product, solution exchange may be performed by methods well known to those skilled in the art, such as ultrafiltration concentration using an ultrafiltration tube and exchange of the solution to a desired buffer system, or exchange with a desired buffer system by molecular exclusion (e.g., G-25 desalination), or removal of polymer components from the eluted product using a high-resolution molecular exclusion column such as Superdex 200 to improve sample purity. The protein which meets the purity requirement after purification was dialyzed, buffer exchanged and subjected to subsequent conjugation and detection.

40.3 Conjugation: The antibody described above was added into an ultrafiltration tube Amicon-Ultra-30kD, concentrated and buffer exchanged into a buffer of 50 mM phosphate, 150 mM NaCl, and 1 mM EDTA at pH 6.5. 7-8 times of a 10 mM tris(2-carboxyethyl)phosphine solution (TCEP) was added into the antibody solution, and the mixed solution was placed on a thermostatic metal shaker for reducing the antibody at 25 °C for 2-3 h. 15-20 times (dissolved in DMSO) of the corresponding drug-linker compound (prepared in Examples 37-39 or prepared with reference to the methods of Examples 37-39) was added to the reaction system, and the reaction mixture was conjugated at 25 °C for 2-16 h. The reaction product was concentrated and buffer exchanged by ultrafiltration into a phosphate (PBS) buffer to remove the unreacted free small molecule toxin. The ADC product was analyzed for purity and DAR value using SEC and LC-MS methods.

40.4 SEC purity analysis: The protein sample to be detected was analyzed using an SEC-HPLC method, the molecular size uniformity of the recombinant protein was characterized, and the purity of the recombinant protein was determined. The HPLC used in this method was Agilent 1260, the column was TSKgel G3000SWXL (purchased from Tosoh Bioscience), the mobile phase was 200 mM phosphate buffer, pH 7.0/isopropanol (v/v 9:1), the detection temperature was 25 °C, the flow rate was 0.5 mL/min, the detection wavelength was 280 nm, the loading amount of the target protein was 50 μg, and the analysis time was 40 min.

40.5 DAR value determination: The DAR value of the ADC molecule was determined using an ultrahigh performance liquid chromatography-mass spectrometry (UHPLC-MS) method. Firstly, the ADC molecule to be detected was treated with PNGase F to remove N glycosylation modification, then treated with dithiothreitol (DTT), incubated at 37 °C for 1 h for reduction into light and heavy chains, and then analyzed using Thermo Vanquish UHPLC-Q Exactive Plus mass spectrum system. 2 μg of the protein was injected into a Waters ACQUITY Protein BEH molecular exclusion chromatographic column, the mobile phase was an aqueous solution containing 0.1% formic acid, 0.05% TFA, and 25% acetonitrile, the flow rate was 0.2 mL/min, the analysis time was 30 min, the mass spectrometer was Thermo Q Exactive Plus, and the main parameters of the mass spectrum were spray voltage of 3.8 kV, capillary heating temperature of 300 °C, sheath gas flow rate of 35 arb, parent ion scanning range of 800-3000, and the like. Finally, deconvolution processing was performed through Respect algorithm using mass spectrum data analysis software Biopharma Finder 4.1, and the molecular weight information of mass spectrum peaks of the light chain and the heavy chain and the mass spectrum response signals of each component were separately calculated so as to calculate the DAR value of the ADC sample to be detected.

An isotype control antibody Ab-ISO (anti-FITC-hIgG1 antibody) was prepared by the same method described above, and was conjugated with a Linker + Payload compound to give the isotype control of the corresponding ADC.

By the same method described above, antibodies ROR1-Ab-1, ROR1-Ab-2, and ROR1-Ab-3 were separately conjugated with compound L1-0 to prepare ADC-L1-0-8, ADC-L1-0-9, and ADC-L1-0-10, and the DAR values were 6.6, 5.5, and 7.5, respectively; antibody LIV1-Ab-1 was conjugated with compound L1-0 to give ADC-L1-0-7, and the DAR value was 6.7; antibodies trastuzumab and pertuzumab were separately conjugated with a compound L1-00 to give ADC-L1-00-2 and ADC-L1-00-11, and the DAR values were 6.8 and 7.3, respectively; antibodies trastuzumab and pertuzumab were separately conjugated with compound L1-0 to give ADC-L1-0-2 and ADC-L1-0-11, and the DAR values were both 7.3; antibodies CDH6-Ab, CDH6-Ab-2, and CDH6-Ab-3 were separately conjugated with compound L1-0 to give ADC-L1-0-3, ADC-L1-0-5, and ADC-L1-0-6, and the DAR values were 7.4, 7.4, and 7.2, respectively.

Table 9 Antibody-drug conjugate and DAR value and SEC purity thereof

| Conjugate No. | ADC structure | DAR | SEC purity (monomer%) |
|---|---|---|---|
| ADC-L1-14-P2-1 | 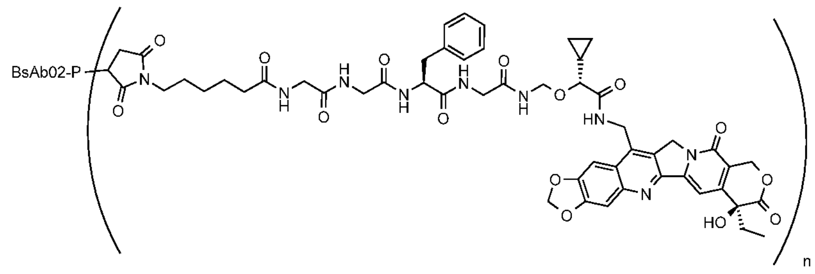 | 8.0 | 95.7 |
| ADC-L2-14-P2-1 | 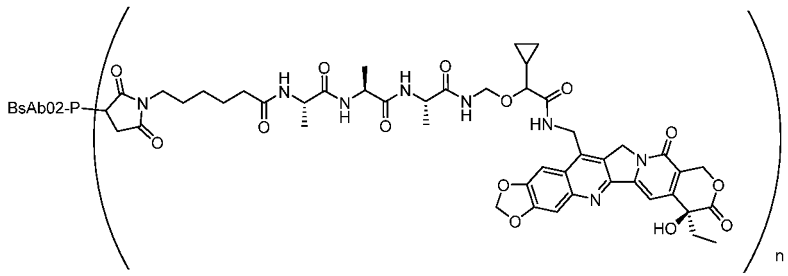 | 8.4 | 97.0 |
| ADC-L1-21-1 | 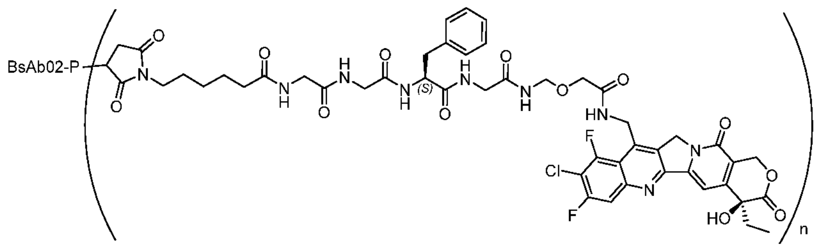 | 7.6 | 94.3 |
| ADC-L2-21-1 | 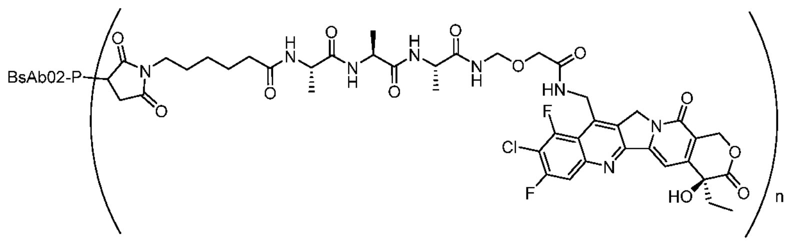 | 6.7 | 92.3 |

(continued)

| Conjugate No. | ADC structure | DAR | SEC purity (monomer%) |
|---|---|---|---|
| ADC-L2-31-2 | 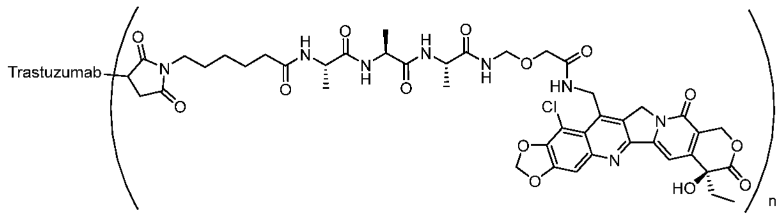 | 7.4 | 91.4 |
| ADC-L1-14-P1-2 | 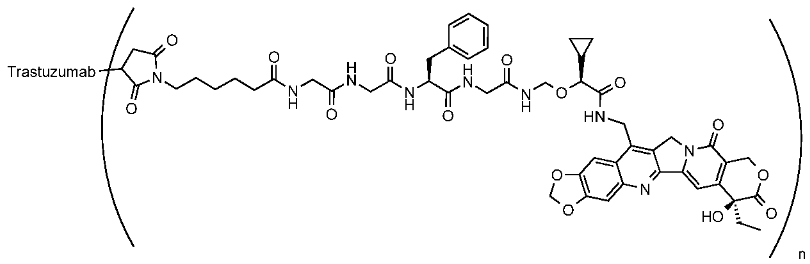 | 7.0 | 90.1 |
| ADC-L1-14-P2-2 | 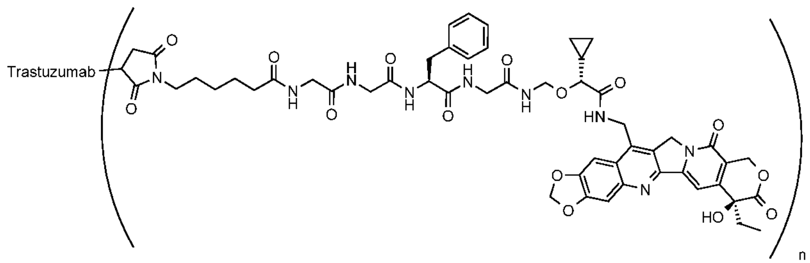 | 7.4 | 83.7 |
| ADC-L1-14-P1-3 | 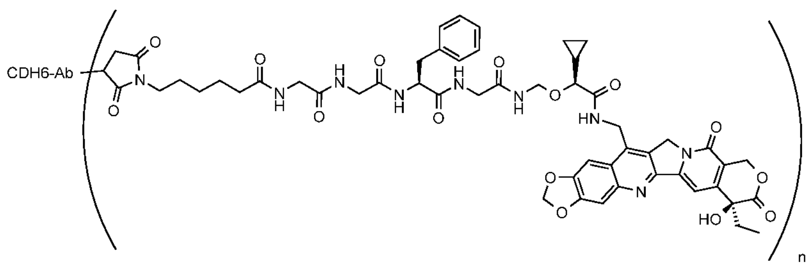 | 8.5 | 93.1 |

(continued)

| Conjugate No. | ADC structure | DAR | SEC purity (monomer%) |
|---|---|---|---|
| ADC-L1-35-P1-3 | 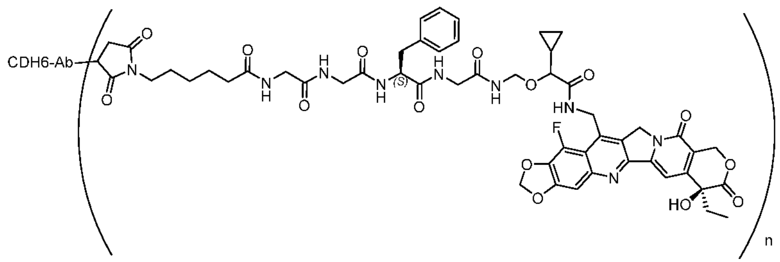 | 8.0 | 93.1 |
| ADC-L1-19-P1-3 | 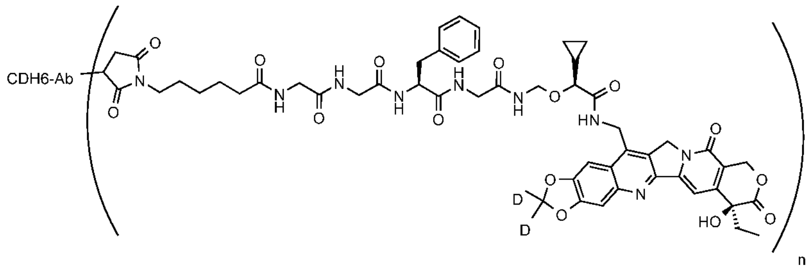 | 7.2 | 93.7 |
| ADC-DS | 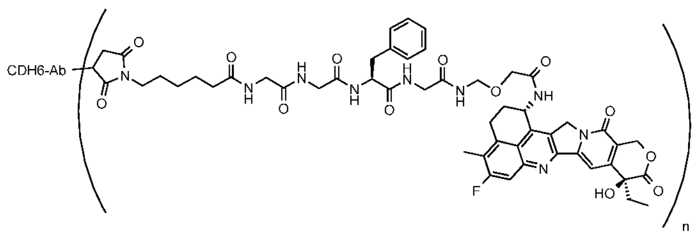 | 7.5 | 92.83 |
| ADC-L1-19-P1-4 | 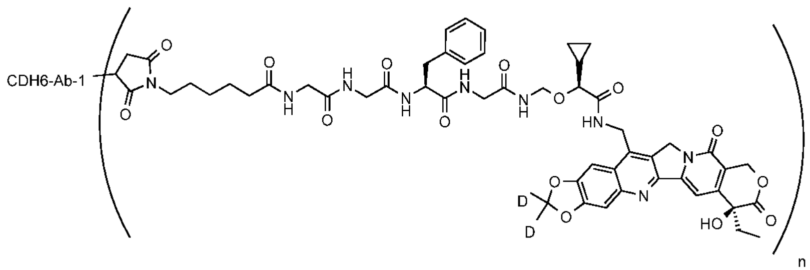 | 6.9 | 95.25 |

(continued)

| Conjugate No. | ADC structure | DAR | SEC purity (monomer%) |
|---|---|---|---|
| ADC-L1-19-P1-5 | 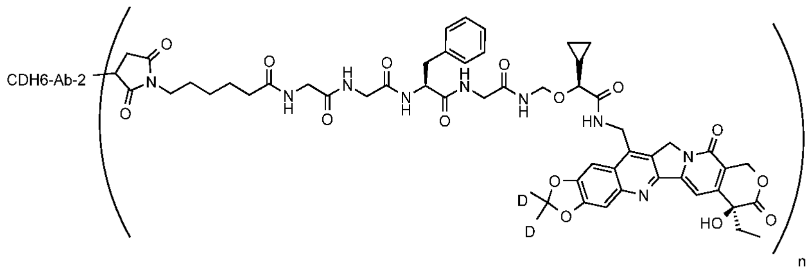 | 8.2 | 96.03 |
| ADC-L1-14-P1-5 | 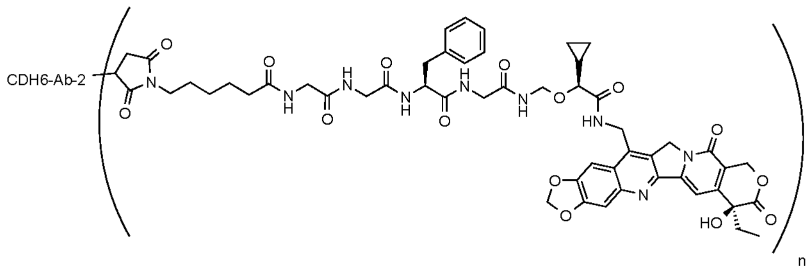 | 7.8 | 97.05 |
| ADC-L1-19-P1-6 | 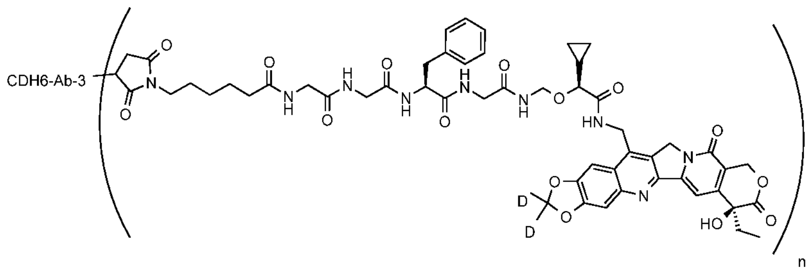 | 8.0 | 92.86 |
| ADC-L1-14-P1-6 | 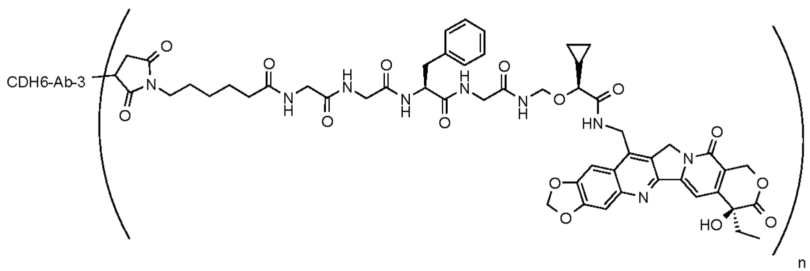 | 7.6 | 92.89 |

(continued)

| Conjugate No. | ADC structure | DAR | SEC purity (monomer%) |
|---|---|---|---|
| ADC-L1-19-P1-2 | 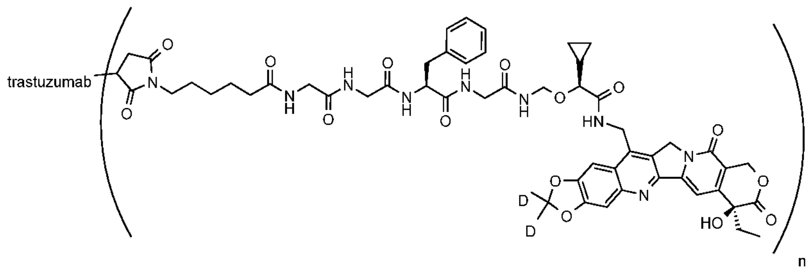 | 7.6 | 83.33 |
| ADC-L1-19-2 | 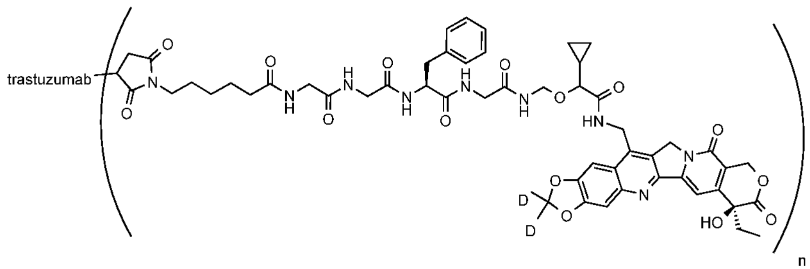 | 7.0 | 88.3 |
| ADC-L1-19-3 | 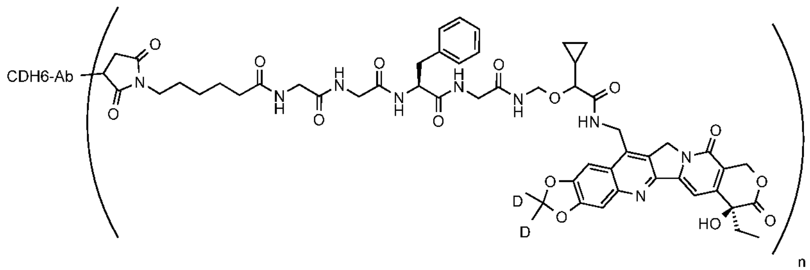 | 7.1 | 94.09 |
| ADC-L1-19 - 4 | 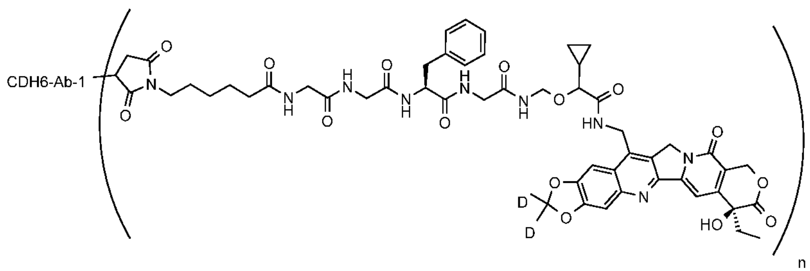 | 6.3 | 94.25 |

(continued)

| Conjugate No. | ADC structure | DAR | SEC purity (monomer%) |
|---|---|---|---|
| ADC-L1-19 - 5 | 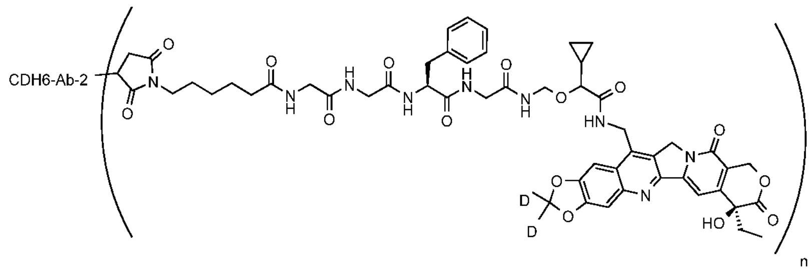 | 6.8 | 93.95 |
| ADC-L1-19 - 6 | 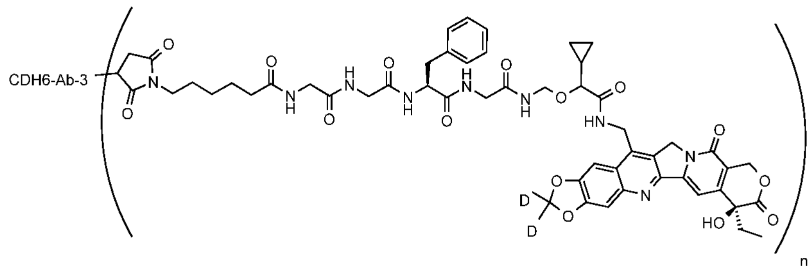 | 7.4 | 91.92 |
| ADC-L1-19-P1-7 | 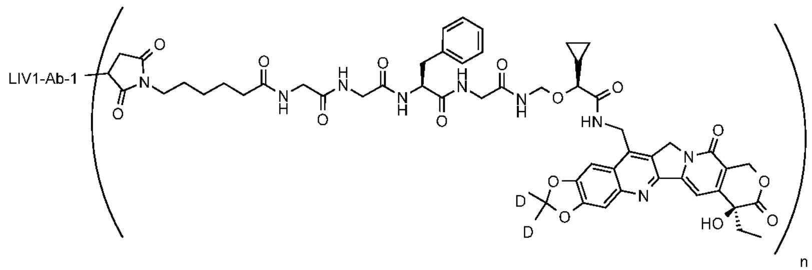 | 8.3 | 95.2 |
| ADC-L1-19 - 7 | 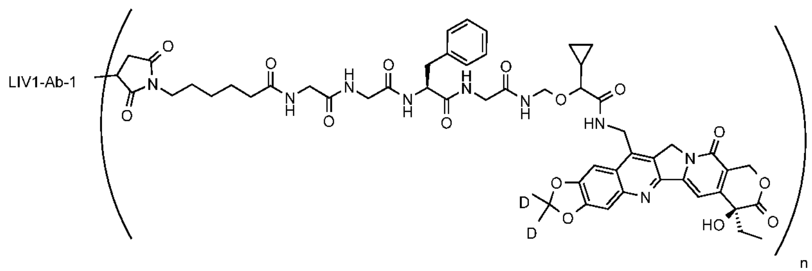 | 6.5 | 90.1 |

(continued)

| Conjugate No. | ADC structure | DAR | SEC purity (monomer%) |
|---|---|---|---|
| ADC-L1-19-P1-8 | 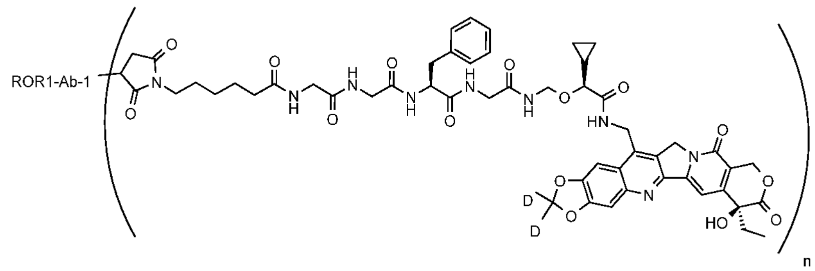 | 7.7 | 93.7 |
| ADC-L1-19 - 8 | 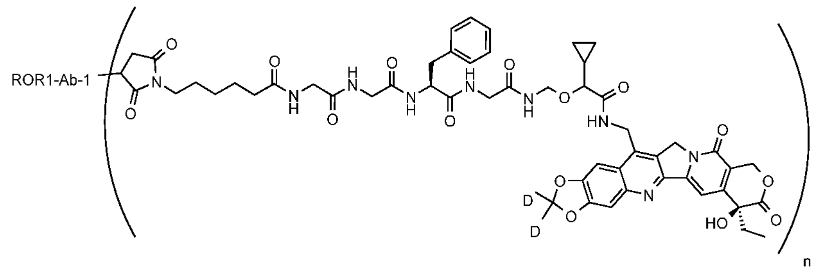 | 7.1 | 91.38 |
| ADC-L1-19-P1-9 | 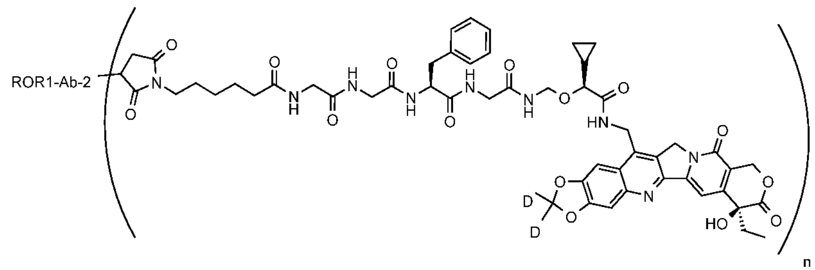 | 7.7 | 98.96 |
| ADC-L1-19 - 9 | 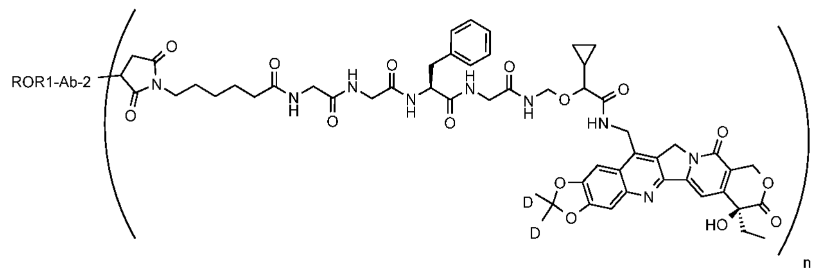 | 7.3 | 94.62 |

(continued)

| Conjugate No. | ADC structure | DAR | SEC purity (monomer%) |
|---|---|---|---|
| ADC-L1-19-P1-10 | 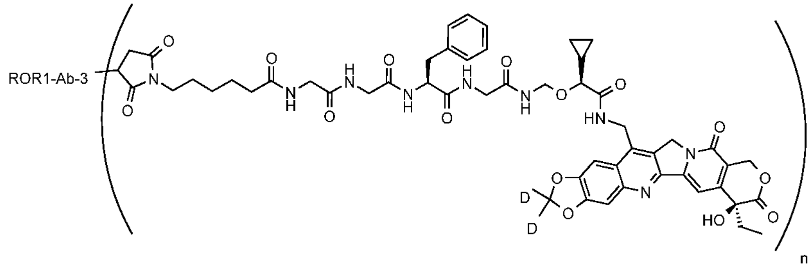 | 8.0 | 93.9 |
| ADC-L1-19 - 10 | 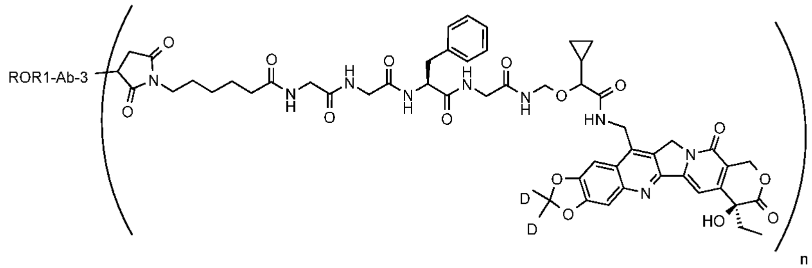 | 7.2 | 88.1 |
| ADC-L1-19-P1-11 | 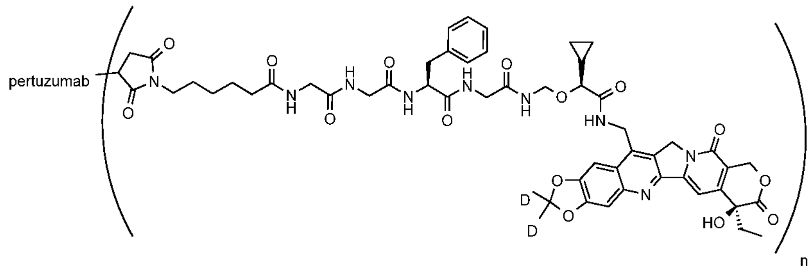 | 7.3 | 90.71 |
| ADC-L1-19 - 11 | 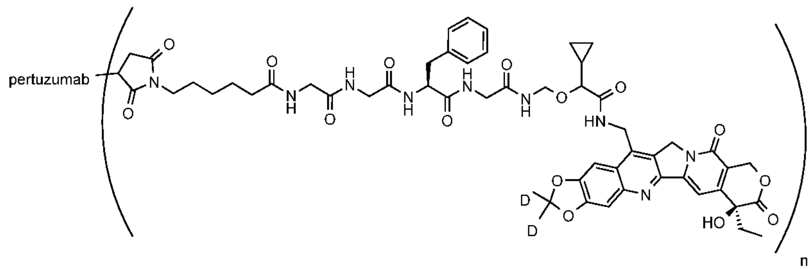 | 7.0 | 90.04 |

**Example 41: Biological Activity and Related Properties Test**

The compounds in the following test examples were prepared according to the methods in the above examples disclosed herein.

**Test Example 1. Test for Anti-Tumor Cell Proliferation Activity of Compound of Formula (D-H)**

**Cell and material:** The human colorectal cancer cell line HCT116 was purchased from KYinno, the human breast cancer cell line SKBR3 was purchased from ATCC, the human ovarian cancer cell line OVCAR3 was purchased from ATCC, bovine serum (Gibco#10099-141C), McCoy's 5a medium (Gibco#16600-082), 1640 medium (Gibco#A10491-01), penicillin-streptomycin (Gibco#15140-122), and 0.25% Trypsin-EDTA (Gibco#25200-056) were purchased from Gibco (U.S.), bovine insulin (Solarbio#I8040) was purchased from Solarbio, 96 well plates (Greiner Bio-one#655098) was purchased from Corning (U.S.), and Cell-Titer Glo reagent (Promega#G7568) was purchased from Promega (U.S.).

**Cell culture:** HCT116 cells and SKBR3 cells were cultured with a McCoy's 5a medium containing 10% fetal bovine serum + 1% penicillin-streptomycin at 37 °C with 5% $CO_2$, and OVCAR3 cells were cultured with a 1640 medium containing 20% fetal bovine serum + 2 μg/mL bovine insulin + 1% penicillin-streptomycin at 37 °C with 5% $CO_2$. Cells at logarithmic growth phase were available for the experiment.

**Cell proliferation activity test:** The Cell-Titer Glo reagent was used to test the compound for inhibitory activity on proliferation of the three cell strains HCT116, SKBR3, and OVCAR3. HCT116 cells (1500 per well), SKBR3 cells (3000 per well), and OVCAR3 cells (5000 per well) were seeded on 96-well plates and incubated at 37 °C with 5% $CO_2$ for 24 h. After a solution of the compound to be detected was added (the compound was dissolved in DMSO so that the compound concentration was 1 mM, then the compound was diluted to 3 μM in DMSO at 9 concentrations in a 3-fold dilution, and 10 μL of the prepared compound solution was transferred to the 96-well plate so that the final concentration was 0-300 nM), the mixture was incubated at 37 °C with 5% $CO_2$. HCT116 cells were cultured for 3 days, and SKBR3 cells and OVCAR3 cells were cultured for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:** %Inhibition was calculated, and compound $IC_{50}$ was fitted.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the compound of the present disclosure exhibited strong proliferation inhibitory activity on HCT116 cells, SKBR3 cells, and OVCAR3 cells. The corresponding anti-cell proliferation activity of the compound of the present disclosure is shown in Table 10.

**Table 10** Anti-tumor cell proliferation activity of compound of formula (D-H)

| Compound | HCT116 Antiproliferative activity $IC_{50}$ (nM) | SKBR3 Antiproliferative activity $IC_{50}$ (nM) | OVCAR3 Antiproliferative activity $IC_{50}$ (nM) |
|---|---|---|---|
| Compound 1 | N/A | 8.5 | N/A |
| Compound 3 | N/A | 3.6 | N/A |
| Compound 5 | N/A | 3.3 | N/A |
| Compound 6 | N/A | 4.8 | 2.1 |
| Compound 7 | ++ | N/A | N/A |
| Compound 9 | +++ | 2.1 | N/A |
| Compound 10 | +++ | 2.3 | N/A |
| Compound 11 | N/A | 3.2 | N/A |

(continued)

| Compound | HCT116 Antiproliferative activity $IC_{50}$ (nM) | SKBR3 Antiproliferative activity $IC_{50}$ (nM) | OVCAR3 Antiproliferative activity $IC_{50}$ (nM) |
|---|---|---|---|
| Compound 13 | +++ | N/A | 0.7 |
| Compound 14 | +++ | 2.2 | 0.9 |
| Compound 14-P1 | +++ | 2.1 | 0.6 |
| Compound 14-P2 | ++ | 3.5 | 1.0 |
| Compound 15 | ++ | N/A | N/A |
| Compound 16 | +++ | 2.3 | N/A |
| Compound 17 | +++ | 2.7 | N/A |
| Compound 18 | ++ | 5.1 | N/A |
| Compound 19 | +++ | 2.9 | 1.0 |
| Compound 19-P1 | +++ | 1.9 | 0.6 |
| Compound 19-P2 | +++ | 2.9 | 1.0 |
| Compound 20 | ++ | 10.5 | N/A |
| Compound 21 | +++ | 4.0 | 1.7 |
| Compound 22 | +++ | 3.5 | 1.4 |
| Compound 23 | +++ | 5.3 | 3.9 |
| Compound 25 | +++ | 3.7 | 2.0 |
| Compound 26 | +++ | 4.2 | 1.6 |
| Compound 27 | ++ | N/A | N/A |
| Compound 28 | +++ | N/A | N/A |
| Compound 29 | ++ | N/A | N/A |
| Compound 30 | +++ | N/A | 0.4 |
| Compound 31 | +++ | N/A | 0.2 |
| Compound 33 | +++ | N/A | 0.8 |
| Compound 34 | +++ | N/A | 0.6 |
| Compound 35 | +++ | 3.5 | 1.2 |
| Compound 35-P1 | +++ | 2.1 | 0.5 |
| Compound 35-P2 | N/A | 3.6 | 1.8 |

In the above table, the symbols used to indicate the binding activity represent the following meanings: "+++" indicates that the compound to be detected has an $IC_{50}$ range of < 10 nM for proliferation inhibitory activity on cells.

"++" indicates that the compound to be detected has an $IC_{50}$ range of 10-100 nM for proliferation inhibitory activity on cells.

"N/A" represents untested.

**Test Example 2. Biacore Assay of Anti-p95HER2/HER2 Bispecific Antibody BsAb02-P**

Biacore 8K (GE) instrument was used in the experiment. The affinity of the antibody to be detected BsAb02-P for human p95HER2 (hu p95HER2.ECD-Fc, SEQ ID NO: 104, the extracellular domain of human p95HER2 are in italics, the Fc label is underlined: *MPIWKFPDEEGACQPCPINCTHSCVDLDDKGCPAEQRASPLT*EPKSSDKTHTCPPCPAPELLG GPSVFLFPPKPKDTLMISRTPEVTCVVVDVSHEDPEVKFNWYVDGVEVHNAKTKPREEQY NSTYRVVSVLTVLHQD WLNGKEYKCKVSNKALPAPIEKTISKAKGQPREPQVYTLPPSREE MTKNQVSLTCLVKGFYPSDIAVEWESNGQPEN NYKTTPPVLDSDGSFFLYSKLTVDKSRWQ QGNVFSCSVMHEALHNHYTQKSLSLSPGK, and the protein was self-expressed and prepared) and HER2 (purchased from Acro, HE2-H5225) was determined using multi-cycle kinetics.

The experimental running buffer was 1× HBS-EP+ buffer (10 mM HEPES, 150 mM NaCl, 3 mM EDTA, 0.05% surfactant P20) (Cat. # BR-1006-69, GE). The flow cell temperature was set at 25 °C, and the sample chamber temperature was set at 16 °C. Both were pretreated with the running buffer. A Protein A biosensor chip (Cat. # 29127556, GE) was allowed to affinity capture a certain amount of the antibody to be detected, and then a certain concentration of human p95HER2 antigen or human HER2 antigen was allowed to flow over the surface of the chip. The reaction signals were detected in real time using a Biacore 8K instrument (GE) to obtain an association and dissociation curve. After each cycle of dissociation was completed, the antigen-antibody complex was washed thoroughly and regenerated with a pH 1.5 glycine-hydrochloric acid regeneration solution (Cat. # BR-1003-54, GE). Specifically, the association process was determined by injecting different concentrations of human p95HER2 and human HER2 antigens in the solution for 240 s at a flow rate of 30 μL/min, which were 1:1 diluted from 50 nM with a series of concentration gradients being set. The dissociation time was up to 900 s, and finally the regeneration of the chip surface was completed by washing with a 10 mM glycine-hydrochloric acid solution (pH 1.5) at a flow rate of 30 μL/min for 30 s.

The data obtained from the experiment were fitted with the (1:1) Langmuir model using GE Biacore 8K Evaluation version 2.0 software to give the association rate (Ka), dissociation rate (Kd), and affinity value (KD), specifically as shown in Tables 11-12. The experimental results show that the p95HER2/HER2 bispecific antibody of the present disclosure can bind to human p95HER2 and HER2 with high affinity.

Table 11 Reaction affinity of p95HER2/HER2 bispecific antibody for human p95HER2 protein

| Antibody | Ka (1/Ms) | Kd (1/s) | KD (M) |
|---|---|---|---|
| BsAb02-P | 6.23E+04 | 6.03E-05 | 9.68E-10 |

Table 12 Reaction affinity of p95HER2/HER2 bispecific antibody for human HER2 protein

| Antibody | Ka (1/Ms) | Kd (1/s) | KD (M) |
|---|---|---|---|
| BsAb02-P | 2.07E+05 | 6.11E-05 | 2.95E-10 |

**Test Example 3-1. Binding Activity of Anti-HER2 ADC and Antibody with SKBR3 Tumor Cell by Flow Cytometry Assay (FACS)**

SKBR3 cells (from ATCC) were collected, counted, and plated in a 96-well plate (Corning, 3795) at $2 \times 10^5$ cells/well. The serially diluted sample to be detected was added and incubated at 4 °C for 1 h. After the mixture was washed twice with ice PBS, Alexa Fluor-647 goat anti-human Fc secondary antibody (JacksonImmuno, 109-605-098) was added, and the mixture was incubated at 4 °C for 1 h. After the mixture was washed twice with ice PBS, the cells were resuspended, and the mean fluorescence intensity (MFI) was analyzed using a flow cytometer (BD FACSCanto™ II). Data were analyzed by four-parameter curve fitting using Graphpad Prism software to give $EC_{50}$ values. The results are shown in Table 13.

Table 13. Assay results for binding reaction of anti-HER2 ADC and antibody with SKBR3 tumor cell by FACS

| Conjugate/Antibody | $EC_{50}$ (nM) |
|---|---|
| ADC-L1-19-2 | 3.34 |
| ADC-L1-19-P1-2 | 3.26 |
| Trastuzumab | 3.38 |
| ADC-L1-19-11 | 4.75 |
| ADC-L1-19-P1-11 | 4.51 |
| Pertuzumab | 3.73 |

**Test Example 3-2. Binding Activity Test of Anti-ROR1 ADC and Antibody with Stably Transfected Cell Strain MCF7-hROR1 Clone 2G2**

**Construction of stably transfected cell strain**

A nucleotide sequence encoding the amino acid sequence of human ROR1 (SEQ ID NO: 105) was cloned into a pLVX lentiviral vector, and viral particles were prepared in HEK293T cells. After lentivirus infection, the MCF7 cell line

(purchased from Chinese Academy of Science), was selectively cultured in a DMEM (Gibco, Cat. No. 11995073) medium containing 5 μg/mL puromycin (Gibco, Cat. No. A1113803) and 10% (w/w) fetal bovine serum (ExCell Bio, Cat. No. FND500) for 1 week. Positive cell populations with different expression levels were sorted on a flow cytometer FACS Aria III (purchased from BD Biosciences) using ROR1-Ab-1 and goat anti-mouse IgG (H+L) antibody (Jackson, Cat. No. 115605006) and cultured in 96-well plates at 37 °C with 5% (v/v) $CO_2$. After about 2 weeks, a portion of the cells were selected for expansion, and positive cell populations with better growth, higher fluorescence intensity, and better homogeneity were selected, further expanded and cryopreserved in liquid nitrogen. Identification of monoclonal stably transfected cell strains is shown in Table 14.

Table 14. Assay results for MCF7 cell line stably transfected with human ROR1 protein by FACS

| No. | Stably transfected positive cell population | Mean fluorescence intensity of cells | |
|---|---|---|---|
| | | IgG subtype control | ROR1-Ab-1 |
| 1 | MCF7-hROR1 clone 2G2 | 54.7 | 26497 |

Amino acid sequence of full-length human ROR1 (SEQ ID NO: 105):

QETELSVSAELVPTSSWNISSELNKDSYLTLDEPMNNITTSLGQTAELHCKVSGNPPPTIRWF
KNDAPVVQEPRRLSFRSTIYGSRLRIRNLDTTDTGYFQCVATNGKEVVSSTGVLFVKFGPPP
TASPGYSDEYEEDGFCQPYRGIACARFIGNRTVYMESLHMQGEIENQITAAFTMIGTSSHLS
DKCSQFAIPSLCHYAFPYCDETSSVPKPRDLCRDECEILENVLCQTEYIFARSNPMILMRLKL
PNCEDLPQPESPEAANCIRIGIPMADPINKNHKCYNSTGVDYRGTVSVTKSGRQCQPWNSQ
YPHTHTFTALRFPELNGGHSYCRNPGNQKEAPWCFTLDENFKSDLCDIPACDSKDSKEKNK
MEILY

**Assay on binding activity of anti-ROR1 ADC and antibody with stably transfected cell strain MCF7-hROR1 clone 2G2 by flow cytometry assay (FACS)**

Stably transfected cell strain MCF7-hROR1 clone 2G2 constructed in the steps described above was expanded to a logarithmic growth phase in a T-75 cell culture flask, and the medium supernatant was discarded after centrifugation. The cell pellet was washed twice with PBS. The ADC and the antibody to be detected were added, serially 5-fold diluted from 100 nM to give 8 concentration points, and incubated at 4 °C for 1 h. After the mixture was washed twice with PBS, a secondary antibody was added: Alexa Fluor® 647 AffiniPure Goat Anti-Human IgG (H+L) (purchased from Jackson Immuno, Cat. No: 109-605-088), and the mixture was incubated at 4 °C for 1 h. After the mixture was washed twice with PBS, the cells were resuspended, and detected and analyzed by FACS (FACS CantoTM, purchased from BD Biosciences). Data were analyzed by four-parameter curve fitting using Graphpad Prism software to give $EC_{50}$ values. The results are shown in Table 15.

Table 15 Assay on binding activity of anti-ROR1 ADC and antibody with MCF7-hROR1 clone 2G2 cell by FACS

| Conjugate/Antibody | $EC_{50}$ (nM) |
|---|---|
| ADC-L1-19-8 | 0.68 |
| ADC-L1-19-P1-8 | 0.34 |
| ADC-L1-0-8 | 0.75 |
| ROR1-Ab-1 | 0.99 |
| ADC-L1-19-9 | 0.42 |
| ADC-L1-19-P1-9 | 0.50 |
| ADC-L1-0-9 | 0.43 |
| ROR1-Ab-2 | 0.98 |
| ADC-L1-19-10 | 0.98 |
| ADC-L1-19-P1-10 | 0.57 |
| ADC-L1-0-10 | 1.25 |

(continued)

| Conjugate/Antibody | $EC_{50}$ (nM) |
|---|---|
| ROR1-Ab-3 | 0.91 |

**Test Example 3-3. Binding Activity of Anti-LIV-1 ADC and Antibody with OVCAR3 Tumor Cell by Flow Cytometry Assay (FACS)**

OVCAR3 cells (from ATCC) belongs to the LIV-1 highly expressed tumor model. OVCAR3 cells were collected, counted, and plated in a 96-well plate (Corning, 3795) at $2 \times 10^5$ cells/well. The serially diluted sample to be detected was added and incubated at 4 °C for 1 h. After the mixture was washed twice with ice PBS, Alexa Fluor-647 Goat anti-human Fc secondary antibody (JacksonImmuno, 109-605-098) was added, and the mixture was incubated at 4 °C for 1 h. After the mixture was washed twice with ice PBS, the cells were resuspended, and the mean fluorescence intensity (MFI) was analyzed using a flow cytometer (BD FACSCanto™ II). Data were analyzed by four-parameter curve fitting using Graphpad Prism software to give $EC_{50}$ values. The results are shown in Table 16.

Table 16. Assay results for binding reaction of anti-LIV-1 ADC and antibody with OVCAR3 tumor cell by FACS

| Conjugate/Antibody | $EC_{50}$ (nM) |
|---|---|
| ADC-L1-19-7 | 0.83 |
| ADC-L1-19-P1-7 | 0.67 |
| ADC-L1-0-7 | 1.45 |
| LIV1-Ab-1 | 0.53 |

**Test Example 4-1. Test 1 for Anti-HER2-ADC Tumor Cell Proliferation Activity**

**Cell and material:** The human breast cancer cell line SKBR3 was purchased from ATCC, the human breast cancer cell line SKBR3-p95HER2 was constructed by Simcere Pharmaceutical (see patent application CN202210094806.6 for the construction method), the human ovarian cancer cell line SKOV3 was purchased from ATCC, bovine serum, McCoy's 5a medium, penicillin-streptomycin, and 0.25% Trypsin-EDTA were purchased from Gibco (U.S., catalog number same as that of Test Example 1), 96-well plates were purchased from Corning (U.S., catalog number same as that of Test Example 1), and Cell-Titer Glo reagent was purchased from Promega (U.S., catalog number same as that of Test Example 1).

**Cell culture:** SKBR3 cells, SKBR3-p95HER2 cells, and SKOV3 cells were cultured with a McCoy's 5a medium containing 10% fetal bovine serum + 1% penicillin-streptomycin at 37 °C with 5% $CO_2$. Cells at logarithmic growth phase were available for the experiment.

**Cell proliferation activity test:** The Cell-Titer Glo reagent was used to test ADC for inhibitory activity on proliferation of the three cell strains SKBR3, SKBR3-p95HER2, and SKOV3. SKBR3 cells (3000 per well), SKBR3-p95HER2 cells (3000 per well), and SKOV3 cells (600 per well) were seeded on 96-well plates and incubated at 37 °C with 5% $CO_2$ for 24 h. After the ADC solution to be detected was added (the ADC was diluted with the medium for the corresponding cells described above, the ADC concentration was adjusted to 20 nM or 200 nM, then the medium was continuously and serially diluted 3-fold to give a total of 8 concentrations, and 10 μL of the prepared ADC solution was transferred to the 96-well plate so that the initial final concentration actually was 2 nM or 20 nM), the mixture was incubated at 37 °C with 5% $CO_2$. SKBR3 cells, SKBR3-p95HER2 cells, and SKOV3 cells were cultured for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:** %Inhibition was calculated, and compound $IC_{50}$ was fitted.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:** Under the condition of the experiment, the ADC of the present disclosure exhibited strong proliferation inhibitory activity on SKBR3 cells, SKBR3-p95HER2 cells, and SKOV3 cells. The details are shown in Table 17.

Table 17 Anti-tumor cell proliferation activity of ADC

| Conjugate No. | SKBR3/p95 Antiproliferative activity ($IC_{50}$, nM) | SKBR3 Antiproliferative activity ($IC_{50}$, nM) | SKOV3 Antiproliferative activity ($IC_{50}$, nM) |
|---|---|---|---|
| ADC-L1-14-P2-1 | 0.05187 | N/A | N/A |
| ADC-L2-14-P2-1 | 0.03691 | N/A | N/A |
| ADC-L1-21-1 | 0.1428 | N/A | N/A |
| ADC-L2-21-1 | 0.1694 | N/A | N/A |
| ADC-L2-31-2 | N/A | 0.04567 | 0.2052 |
| ADC-L1-14-P1-2 | N/A | 0.03227 | 0.1249 |
| ADC-L1-14-P2-2 | N/A | 0.03108 | 0.116 |
| "N/A" represents untested. | | | |

**Test Example 4-2. Test 2 for Anti-HER2-ADC Tumor Cell Proliferation Activity**

**Cell and material:** The human breast ductal carcinoma cell line T47D (moderate-expression cell line) was purchased from ATCC, the human breast cancer cell line SKBR3 was purchased from ATCC, McCoy's 5a medium (Gibco#16600-082), 1640 medium (Gibco#A10491-01), penicillin-streptomycin (Gibco#15140-122), and 0.25% Trypsin-EDTA (Gibco#25200-056) were purchased from Gibco (U.S.), 96 well plates (Greiner Bio-one#655098) was purchased from Corning (U.S.), and Cell-Titer Glo reagent (Promega#G7568) was purchased from Promega (U.S.).

**Cell culture:** SKBR3 cells were cultured with a McCoy's 5a medium containing 10% fetal bovine serum + 1% penicillin-streptomycin, and T47D cells were cultured with a 1640 medium containing 10% fetal bovine serum + 1% penicillin-streptomycin. The two cell strains were both cultured at 37 °C with 5% $CO_2$. Cells at logarithmic growth phase were available for the experiment.

**Cell proliferation activity test:** The Cell-Titer Glo reagent was used to test ADC for inhibitory activity on proliferation of both SKBR3 and T47D cell strains. SKBR3 and T47D cells were digested and pipetted in cell culture flasks, and resuspended using the corresponding fresh medium. The cell density was adjusted. T47D cells were seeded on a 96-well plate at 2000 cells/90 μL/well and incubated at 37 °C with 5% $CO_2$ overnight. The ADC concentration was diluted to 1000 nM with a complete medium and subjected to 3-fold gradient dilution to give a total of 8 concentration gradients, and then 10 μL of an ADC diluted solution was transferred to the 96-well plate, i.e., the initial final concentration of the ADC was 100 nM. The 96-well plate was incubated at 37 °C with 5% $CO_2$ for 7 days. The Cell-Titer Glo reagent was added and the cell activity was measured.

SKBR3 cells were seeded on a 96-well plate at 5000 cells/90 μL/well and incubated at 37 °C with 5% $CO_2$ overnight. The ADC concentration was diluted to 1000 nM with a complete medium and subjected to 5-fold gradient dilution to give a total of 9 concentration gradients, and then 10 μL of an ADC diluted solution was transferred to the 96-well plate, i.e., the initial final concentration of the ADC was 100 nM. The 96-well plate was incubated at 37 °C with 5% $CO_2$ for 3 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%Inhibition = 1 - 100\% \times (Signal - Bottom) / (Top - Bottom).$$

Signal referred to the signal value of the experimental group, Bottom referred to the average signal value of the negative control group, and Top referred to the average signal value of the positive control group.

**Experimental results:**

Under the condition of the experiment, the anti-HER2-ADC of the present disclosure exhibited strong proliferation inhibitory activity on human breast ductal carcinoma cell line T47D and human breast cancer cell line SKBR3, **see** Table 18.

Table 18 Anti-tumor cell proliferation activity of ADC

| Conjugate No. | T47D | | SKBR3 | |
|---|---|---|---|---|
| | $IC_{50}$ (nM) | Maximum inhibition rate (%) | $IC_{50}$ (nM) | Maximum inhibition rate (%) |
| ADC-L 1-19-11 | 5.329 | 89.4 | 0.0005041 | 64.5 |
| ADC-L1-19-P1-11 | 1.671 | 88.9 | 0.01225 | 55.4 |
| ADC-L 1-00-11 | 12.16 | 90.8 | 1.413 | 35.4 |
| ADC-L1-19-2 | 6.93 | 90.6 | 0.0832 | 54.0 |
| ADC-L1-19-P1-2 | 3.552 | 92.5 | 0.0852 | 54.3 |
| ADC-L1-00-2 | 13.97 | 90.5 | 0.4424 | 41.7 |

**Test Example 5. Cell binding Activity of Anti-CDH6 Antibody with Corresponding ADC**

OVCAR3 cells were collected, counted, and plated in a 96-well plate (Corning, 3795) at $2 \times 10^5$ cells/well. The serially diluted sample to be detected was added and incubated at 4 °C for 1 h. After the mixture was washed twice with ice PBS, Alexa Fluor-647 Goat anti-human Fc secondary antibody (JacksonImmuno, 109-605-098) was added, and the mixture was incubated at 4 °C for 1 h. After the mixture was washed twice with ice PBS, the cells were resuspended, and the mean fluorescence intensity (MFI) was analyzed using a flow cytometer (BD FACSCanto™ II). Data were analyzed by four-parameter curve fitting using Graphpad Prism software to give $EC_{50}$ values. The results are shown in Table 19.

Table 19 Binding activity of CDH6 antibody and CDH6-ADC with OVCAR3 cell

| Conjugate/Antibody | $EC_{50}$ (nM) |
|---|---|
| ADC-L1-14-P1-3 | 0.49 |
| ADC-L1-19-3 | 0.34 |
| ADC-L1-19-P1-3 | 0.36 |
| CDH6-Ab | 0.71 |
| ADC-L1-19-5 | 0.51 |
| ADC-L1-19-P1-5 | 0.53 |
| CDH6-Ab-2 | 0.63 |
| ADC-L1-19-6 | 0.85 |
| ADC-L1-19-P1-6 | 0.80 |
| CDH6-Ab-3 | 0.78 |

**Test Example 6-1. Test for Anti-Tumor Cell Proliferation Activity of Anti-CDH6-ADC**

**Cell and material:** The human ovarian cancer cell line OVCAR3 (CDH6 high-expression cell strain) was purchased from ATCC, the human ovarian teratoma cell line PA-1 was purchased from ATCC, bovine serum, 1640 medium (Gibco#A10491-01), MEM medium (Gibco#11095-080), MEM NEAA (Gibco#11140-050), sodium pyruvate (Gibco#11360-070), penicillin-streptomycin, and 0.25% Trypsin-EDTA (Gibco#25200-056) were purchased from Gibco,

bovine insulin was purchased from Solarbio, 96 well plates were purchased from Corning (U.S.), and Cell-Titer Glo reagent was purchased from Promega (U.S.).

**Cell culture:** OVCAR3 cells were cultured with a 1640 medium containing 20% fetal bovine serum + 2 μg/mL bovine insulin + 1% penicillin-streptomycin at 37 °C with 5% $CO_2$, and PA-1 cells were cultured with an MEM medium containing 10% fetal bovine serum + 1% MEM NEAA + 1% sodium pyruvate + 1% penicillin-streptomycin at 37 °C with 5% $CO_2$. Cells at logarithmic growth phase were available for the experiment.

**Cell proliferation activity test:** The Cell-Titer Glo reagent was used to test ADC for inhibitory activity on proliferation of both OVCAR3 and PA-1 cell strains. OVCAR3 and PA-1 cells were digested and pipetted in cell culture flasks, and resuspended using the corresponding fresh medium. The cell density was adjusted. OVCAR3 cells (5000 per well) and PA-1 (800 per well) were seeded on 96-well plates and incubated at 37 °C with 5% $CO_2$ for 24 h. After the ADC solution to be detected was added (the ADC was diluted with the medium for the corresponding cells described above, the ADC concentration was adjusted to 100 nM, then the medium was continuously and serially diluted 3-fold to give a total of 8 concentrations, and 10 μL of the prepared ADC solution was transferred to the 96-well plate so that the final concentration was 0-10 nM), the mixture was incubated at 37 °C with 5% $CO_2$. OVCAR3 cells and PA-1 cells were cultured for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:** %Inhibition was calculated, and compound $IC_{50}$ was fitted.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:** Under the condition of the experiment, the anti-CDH6-ADC of the present disclosure exhibited strong proliferation inhibitory activity on both human ovarian cancer cell line OVCAR3 and human ovarian teratoma cell line PA-1 cells. The details are shown in Table 20.

Table 20 Anti-tumor cell proliferation activity of ADC

| Conjugate No. | OVCAR3 | PA-1 |
|---|---|---|
| | $IC_{50}$ (nM) | $IC_{50}$ (nM) |
| ADC-L1-14-P1-3 | 0.012 | 0.063 |
| ADC-L1-35-P1-3 | 0.018 | 0.043 |
| ADC-L1-19-P1-3 | 0.015 | 0.018 |
| ADC-L1-19-3 | 0.015 | 0.0094 |
| ADC-L1-14-P1-6 | 0.031 | 0.070 |
| ADC-L1-19-P1-6 | 0.015 | 0.017 |
| ADC-L1-19-6 | 0.022 | 0.023 |
| ADC-L1-14-P1-5 | 0.028 | 0.030 |
| ADC-L1-19-P1-5 | 0.019 | 0.018 |
| ADC-L1-19-5 | 0.021 | 0.017 |

### Test Example 6-2. Test for Anti-Tumor Cell Proliferation Activity of Anti-LIV-1-ADC

**Cell and material:** The human ovarian cancer cell line OVCAR3 and the human non-small cell lung cancer cell line H838 were purchased from ATCC, 1640 medium (Gibco#A10491-01), penicillin-streptomycin (Gibco#15140-122), and 0.25% Trypsin-EDTA (Gibco#25200-056) were purchased from Gibco (U.S.), bovine insulin (Solarbio#I8040) was purchased from Solarbio, 96 well plates (Greiner Bio-one#655098) was purchased from Corning (U.S.), and Cell-Titer Glo reagent (Promega#G7568) was purchased from Promega (U.S.).

**Cell culture:** OVCAR3 cells were cultured with a 1640 medium containing 20% fetal bovine serum + 2 μg/mL bovine insulin + 1% penicillin-streptomycin at 37 °C with 5% $CO_2$, and H838 cells were cultured with a 1640 medium

containing 10% fetal bovine serum + 1% penicillin-streptomycin at 37 °C with 5% $CO_2$. Cells at logarithmic growth phase were available for the experiment.

**Cell proliferation activity test:** The Cell-Titer Glo reagent was used to test ADC for inhibitory activity on proliferation of OVCAR3 and H838 cell strains. OVCAR3 or H838 cells were digested and pipetted in cell culture flasks, and resuspended using the corresponding fresh medium. The cell density was adjusted. OVCAR3 cells were seeded on a 96-well plate at 1500 cells/90 μL/well and H838 cells were seeded on a 96-well plate at 450 cells/90 μL/well, and they were incubated at 37 °C with 5% $CO_2$ overnight. The ADC concentration was diluted to 5000 nM with a complete medium and subjected to 3-fold gradient dilution to give a total of 8 concentration gradients, and then 10 μL of an ADC diluted solution was transferred to the 96-well plate, i.e., the initial final concentration of the ADC was 500 nM. The 96-well plate was incubated at 37 °C with 5% $CO_2$ for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the anti-LIV-1-ADC of the present disclosure exhibited strong proliferation inhibitory activity on human ovarian cancer cell line OVCAR3 and human non-small cell lung cancer cell line H838. The details are shown in Table 21.

Table 21 Anti-tumor cell proliferation activity of ADC

| Conjugate No. | H838 | | OVCAR3 | |
|---|---|---|---|---|
| | $IC_{50}$ (nM) | Maximum inhibition rate (%) | $IC_{50}$ (nM) | Maximum inhibition rate (%) |
| ADC-L1-19-7 | 21.8 | 97.5 | 5.126 | 90.7 |
| ADC-L1-19-P1-7 | 17.3 | 96.6 | 3.195 | 91.6 |
| ADC-L1-0-7 | 127.8 | 90.9 | 10.5 | 85.3 |

**Test Example 6-3. Test for Anti-Tumor Cell Proliferation Activity of Anti-ROR1-ADC**

**Cell and material:** The human breast cancer cell line hROR1-MCF7 was constructed by Simcere Zaiming, DMEM medium (Gibco#11995-065), penicillin-streptomycin (Gibco#15140-122), and 0.25% Trypsin-EDTA (Gibco#25200-056) were purchased from Gibco (U.S.), 96 well plates (Greiner Bio-one#655098) was purchased from Corning (U.S.), and Cell-Titer Glo reagent (Promega#G7568) was purchased from Promega (U.S.).

**Cell culture:** hROR1-MCF7 cells were cultured with a DMEM medium containing 10% fetal bovine serum + 1% penicillin-streptomycin at 37 °C with 5% $CO_2$. Cells at logarithmic growth phase were available for the experiment.

**Cell proliferation activity test:** The Cell-Titer Glo reagent was used to test ADC for inhibitory activity on proliferation of hROR1-MCF7 cell strains. hROR1-MCF7 cells were digested and pipetted in cell culture flasks, and resuspended using the corresponding fresh medium. The cell density was adjusted. hROR1-MCF7 cells were seeded on a 96-well plate at 1700 cells/90 μL/well and incubated at 37 °C with 5% $CO_2$ overnight. The ADC concentration was diluted to 500 nM with a complete medium and subjected to 4-fold gradient dilution to give a total of 9 concentration gradients, and then 10 μL of an ADC diluted solution was transferred to the 96-well plate, i.e., the initial final concentration of the ADC was 50 nM. The 96-well plate was incubated at 37 °C with 5% $CO_2$ for 5 days. The Cell-Titer Glo reagent was added and the cell activity was measured.

A negative control group and a positive control group were respectively used as Bottom and Top. The negative

control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%Inhibition = 1 - 100\% \times (Signal - Bottom) / (Top - Bottom).$$

Signal referred to the signal value of the experimental group, Bottom referred to the average signal value of the negative control group, and Top referred to the average signal value of the positive control group.

**Experimental results:**

Under the condition of the experiment, the anti-ROR1-ADC of the present disclosure exhibited strong proliferation inhibitory activity on human ovarian cancer cell line hROR1-MCF7. The details are shown in Table 22.

Table 22 Anti-tumor cell proliferation activity of ADC

| Conjugate No. | hROR1-MCF7 | |
|---|---|---|
| | $IC_{50}$ (nM) | Maximum inhibition rate (%) |
| ADC-L1-19-8 | 0.18 | 39.88 |
| ADC-L1-19-P1-8 | 0.10 | 38.61 |
| ADC-L1-19-9 | 0.31 | 42.34 |
| ADC-L1-19-P1-9 | 0.22 | 44.73 |
| ADC-L1-19-10 | 0.04 | 37.45 |
| ADC-L1-19-P1-10 | 0.03 | 44.77 |

**Test Example 7. Evaluation of Efficacy of OVCAR3 Subcutaneous Tumor Model-1**

**Experimental reagent:** Human ovarian cancer OVCAR3 cells were purchased from ATCC, RPMI-1640 medium was purchased from Gibco (Cat. No. A104910), fetal bovine serum was purchased from Excell (Cat. No. FND500), penicillin-streptomycin was purchased from Gibco (Cat. No. 15140122), bovine insulin was purchased from Yeasen (Cat. No. 40107ES60), 0.25% trypsin-EDTA was purchased from Gibco (Cat. No. 25200-072), D-PBS (phosphate-buffered saline without calcium and magnesium ions) was purchased from Hyclone (Cat. No. SH30256.01), and Matrigel was purchased from Corning (Cat. No. 356237).

**Procedures:**

Animal information: Balb/c nude mice, female, 5-6 weeks, weight about 14-20 g, purchased from Beijing Vital River Biotechnology Co., Ltd. The mice were bred in SPF-rated environment with air exhaust for each cage individually. All animals had free access to standard certified commercial laboratory diets and water.

Cell culture: The human ovarian cancer OVCAR3 cell strain was cultured *in vitro* under the conditions of RPMI-1640 added with 20% fetal bovine serum, 1% penicillin-streptomycin, and 10 μg/mL bovine insulin, and an incubator at 37 °C with 5% $CO_2$. The cells were digested with a 0.25% trypsin-EDTA digestive solution once a week for passaging as per conventional practice. When the cell saturation was 80%-90% and a required amount was achieved, the cells were collected and counted.

Cell inoculation: 0.1 mL of OVCAR3 cell suspension (containing $1 \times 10^7$ cells, RPMI-1640: Matrigel, volume ratio 1:1) was inoculated subcutaneously into the axilla of each mouse. On day 26 after inoculation of the cells, the mice were randomly grouped and administrated based on tumor volume. The day when random grouping was performed was day 0.

**Tumor measurement and experimental indices:**

Tumor diameters were measured twice weekly using a vernier caliper. The tumor volume was calculated using the following formula: $V = 0.5a \times b^2$, where a and b represent the long diameter and short diameter of the tumor, respectively. Mouse body weight was measured twice weekly.

The anti-tumor therapeutic effect of the test drug was evaluated by tumor growth inhibition rate TGI (%). TGI (%) = [(1 - (average tumor volume at the end of administration in a treatment group - average tumor volume at the start of administration of the treatment group)) / (average tumor volume at the end of treatment of the solvent control group - average tumor volume at the start of treatment of the solvent control group)] $\times$ 100%.

**Experimental results:**

In the mouse subcutaneous xenograft tumor OVCAR3 model, ADC-L1-14-P1-5 has a significant inhibitory effect on tumor growth after single intravenous administration at a dose of 3 mg/kg ($P < 0.0001$). The results are shown in Table 23 and FIG. 2.

Table 23 OVCAR3 subcutaneous tumor model tumor volume

| Test drug | Dose (mg/kg) | Administration regimen | Frequency of administration | Mean tumor volume ($mm^3$) | | | | | | | | | TGI (%) |
|---|---|---|---|---|---|---|---|---|---|---|---|---|---|
| | | | | Day0 | Day4 | Day7 | Day11 | Day14 | Dayl8 | Day22 | Day25 | Day28 | Day28 |
| Solvent control group | / | Intravenous injection (IV) | Single | 154.3 | 247.1 | 297.8 | 335.8 | 420.3 | 568.8 | 598.1 | 734.3 | 873.7 | / |
| ADC-DS | 3 | | | 154.4 | 211.3 | 194.1 | 159.5 | 131.5 | 95.5 | 73.6 | 66.0 | 77. 8 | 110.6 |
| ADC-L1-14-P1-5 | 3 | | | 151.4 | 207.0 | 183.5 | 143.4 | 113.3 | 71.3 | 64.1 | 69.7 | 66.9 | 111.7 |

In the mouse subcutaneous xenograft tumor OVCAR3 model, ADC-L1-19-P1-5 has a significant inhibitory effect on tumor growth after single intravenous administration at a dose of 3 mg/kg ($P < 0.0001$). The results are shown in Table 24 and FIG. 3.

Table 24 OVCAR3 subcutaneous tumor model tumor volume

| Test drug | Dose (mg/kg) | Administration regimen | Frequency of administration | Mean tumor volume ($mm^3$) | | | | | | | | TGI (%) |
|---|---|---|---|---|---|---|---|---|---|---|---|---|
| | | | | Day0 | Day5 | Day8 | Day12 | Dayl6 | Dayl9 | Day22 | Day27 | Day27 |
| Solvent control group | / | Intravenous injection (IV) | Single | 151.7 | 223.8 | 246.0 | 299.3 | 370.5 | 409.4 | 462.8 | 506.9 | / |
| ADC-DS | 3 | | | 145.0 | 165.9 | 153.4 | 91.4 | 56.2 | 41.2 | 28.9 | 23.4 | 134.2 |
| ADC-L1-19-P1-5 | 3 | | | 153.4 | 165.0 | 149.3 | 97.3 | 68.7 | 56.6 | 49.4 | 27.3 | 135.5 |

**Test Example 8. ADC Plasma Stability Test**

ADC molecules (final concentration: 100 μg/mL) were separately incubated with human plasma (Oribiotech, PB021-C) and monkey plasma (Shinuoda Bio, SND-X0107) in an incubator at 37 °C. The day of incubation was defined as day 0. Samples were taken on days 7, 14 and 28 and tested for the free small molecule.

300 μL of an internal standard working solution (acetonitrile preparation) was added to 20 μL of the sample, and the mixture was vortexed for mixing for 5 min and centrifuged for 5 min (14000 rpm). 4 μL of the supernatant was analyzed by LC-MS/MS (API 6500+). The results are shown in Table 25. The results show that the ADC molecule tested was stable in both human and monkey plasma.

Table 25 Plasma stability of CDH6-ADC

| Type of plasma | Incubation time (day) | Free small molecule% | |
|---|---|---|---|
| | | ADC-DS | ADC-L1-19-P1-5 |
| Human plasma | 0 | 0.59 | N/A |
| | 7 | 0.72 | 0.32 |
| | 14 | 1.21 | 0.54 |
| | 28 | 1.90 | 0.99 |
| Monkey plasma | 0 | 0.64 | N/A |
| | 7 | 2.28 | 0.34 |
| | 14 | 4.03 | 0.75 |
| | 28 | 9.66 | 1.53 |
| N/A means that no free small molecules were detected and the free small molecule% can not be calculated. | | | |

**Test Example 9. Evaluation of Efficacy of OVCAR3 Subcutaneous Tumor Model-2 Experimental reagent:**

Human ovarian cancer OVCAR3 cells: ATCC
RPMI-1640 medium: Gbico; Cat No.: A104910
Fetal bovine serum: Excell, FND500
Bovine insulin: Yeasen, 40107ES60
0.25% trypsin-EDTA: Gibco, Cat No.: 25200-072
D-PBS (phosphate-buffered saline without calcium and magnesium ions): Hyclone, Cat. No.: SH30256.01
Matrigel: Corning, Cat. No.: 356237

**Procedures:**

Animal information: Balb/c nude mice, female, 5-6 weeks, weight about 14-20 g, purchased from Beijing Vital River Biotechnology Co., Ltd. The mice were bred in SPF-rated environment with air exhaust for each cage individually. All animals had free access to standard certified commercial laboratory diets and water.

Cell culture: The human ovarian cancer OVCAR3 cell strain was cultured *in vitro* under the conditions of RPMI-1640 (cell culture medium) added with 20% fetal bovine serum, 1% Pen Strep, and 10 μg/mL bovine insulin, and an incubator at 37 °C with 5% CO2. The cells were digested with a 0.25% trypsin-EDTA digestive solution once a week for passaging as per conventional practice. When the cell saturation was 80%-90% and a required amount was achieved, the cells were collected and counted.

Cell inoculation: 0.1 mL/(containing $1 \times 10^7$) OVCAR3 cell suspension (RPMI-1640: Matrigel, volume ratio 1:1) was inoculated subcutaneously into the axilla of each mouse. On day 23 after inoculation of the cells, the mice were randomly grouped and administrated based on tumor volume. The day when random grouping was performed was day 0.

Administration: ADC-L1-19-P1-7 and isotype control ADC-L1-19-P1-ISO were both administered at a dose of 3 mg/kg by intraperitoneal administration (Q4D). Each group had 6 mice.

**Tumor measurement and experimental indices:**

Tumor diameters were measured twice weekly using a vernier caliper. The tumor volume was calculated using the following formula: $V = 0.5a \times b^2$, where a and b represent the long diameter and short diameter of the tumor, respectively. Mouse body weight was measured twice weekly.

The anti-tumor therapeutic effect of the compound was evaluated by tumor growth inhibition rate TGI (%). TGI (%) = [(1 - (average tumor volume at the end of administration in a treatment group - average tumor volume at the start of administration of the treatment group)) / (average tumor volume at the end of treatment of the solvent control group - average tumor volume at the start of treatment of the solvent control group)] × 100%.

**Experimental results:**

See Table 26, FIG. 4 and FIG. 5.

Table 26 OVCAR3 subcutaneous tumor model tumor volume

| Test drug | Dose (mg/kg) | Administration regimen | Frequency of administration | Mean tumor volume ($mm^3$) | | | | | | | | TGI (%) |
|---|---|---|---|---|---|---|---|---|---|---|---|---|
| | | | | Day 0 | Day 4 | Day 7 | Day 11 | Day 14 | Day 26 | Day 33 | Day 40 | |
| Solvent control group | / | Intraperitoneal injection | Once every four days | 129 | 175 | 229 | 287 | 319 | 597 | 778 | 912 | / |
| ADC-L1-19-P1-ISO | 3 | | | 124 | 171 | 174 | 183 | 182 | 155 | 175 | 248 | 84.2 |
| ADC-L1-19-P1-7 | 3 | | | 126 | 161 | 140 | 108 | 79 | *55* | 63 | 65 | 107.93 |

**Experimental conclusion:**

In the mouse subcutaneous xenograft tumor OVCAR3 model, the compound ADC-L1-19-P1-7 of the present disclosure and the isotype control ADC-L1-19-P1-ISO have a significant inhibitory effect on tumor growth after intraperitoneal administration once every four days at 3 mg/kg ($P < 0.0001$), but the isotype control ADC-L1-19-P1-ISO inhibits the tumor significantly weaker than ADC-L1-19-P1-7 ($P < 0.001$). This example did not show any effect on mouse body weight at the doses tried, nor did it cause any death of the mice, which can be tolerated by the mice.

**Test Example 10. Evaluation of Efficacy of NCI-H838 Subcutaneous Tumor Model Experimental reagent:**

Human lung cancer NCI-H838 cells: Cobioer
RPMI-1640 medium: Gbico; Cat No.: 61870-036
Fetal bovine serum: Gibco; Cat No.:10099-141C
0.25% trypsin-EDTA: Gibco, Cat No.: 25200-072
D-PBS (phosphate-buffered saline without calcium and magnesium ions): Hyclone, Cat. No.: SH30256.01
Matrigel: Corning, Cat. No.: 356237

**Procedures:**

Animal information: B-NDG mice, female, 5-6 weeks, weight about 14-20 g, purchased from Biocytogen. The mice were bred in SPF-rated environment with air exhaust for each cage individually. All animals had free access to standard certified commercial laboratory diets and water. Cell culture: The human lung cancer NCI-H838 cell strain was cultured *in vitro* under the conditions of RPMI-1640 (cell culture medium) added with 10% fetal bovine serum and 1% Pen Strep, and an incubator at 37 °C with 5% CO2. The cells were digested with a 0.25% trypsin-EDTA digestive solution twice a week for passaging as per conventional practice. When the cell saturation was 80%-90% and a required amount was

achieved, the cells were collected and counted.

Cell inoculation: 0.1 mL/(containing 1 × $10^7$) NCI-H838 cell suspension (RPMI-1640: Matrigel, volume ratio 1:1) was inoculated subcutaneously into the axilla of each mouse. On day 23 after inoculation of the cells, the mice were randomly grouped and administrated based on tumor volume. The day when random grouping was performed was day 0.

Administration: ADC-L1-19-P1-7 and isotype control ADC-L1-19-P1-ISO were both administered at a dose of 3 mg/kg by intraperitoneal administration (Q4D). Each group had 6 mice.

**Tumor measurement and experimental indices:**

Tumor diameters were measured twice weekly using a vernier caliper. The tumor volume was calculated using the following formula: $V = 0.5a \times b^2$, where a and b represent the long diameter and short diameter of the tumor, respectively. Mouse body weight was measured twice weekly. The anti-tumor therapeutic effect of the compound was evaluated by tumor growth inhibition rate TGI (%). TGI (%) = [(1 - (average tumor volume at the end of administration in a treatment group - average tumor volume at the start of administration of the treatment group)) / (average tumor volume at the end of treatment of the solvent control group - average tumor volume at the start of treatment of the solvent control group)] × 100%.

**Experimental results:**

See Table 27, FIG. 6 and FIG. 7.

Table 27 H838 subcutaneous tumor model tumor volume

| Test drug | Dose (mg/kg) | Administration regimen | Frequency of administration | Mean tumor volume ($mm^3$) | | | | | | | TGI (%) |
|---|---|---|---|---|---|---|---|---|---|---|---|
| | | | | Day 0 | Day 3 | Day 8 | Day 11 | Day 14 | Day 17 | Day 21 | |
| Solvent control group | / | Intraperitoneal injection | Once every four days | 99 | 137 | 322 | 449 | 758 | 959 | 1444 | / |
| ADC-L1-19-P1-ISO | 3 | | | 98 | 126 | 202 | 240 | 315 | 461 | 707 | 54.8 |
| ADC-L1-19-P1-7 | 3 | | | 99 | 138 | 149 | 163 | 163 | 200 | 290 | 85.8 |

**Experimental conclusion:**

In the mouse subcutaneous xenograft tumor NCI-H838 model, the compound ADC-L1-19-P1-7 of the present disclosure and the isotype control ADC-L1-19-P1-ISO have a significant inhibitory effect on tumor growth after intraperitoneal administration once every four days at 3 mg/kg ($P < 0.0001$), but the isotype control ADC-L1-19-P1-ISO inhibits the tumor significantly weaker than ADC-L1-19-P1-7 ($P < 0.001$). This example did not show any effect on mouse body weight at the doses tried, nor did it cause any death of the mice, which can be tolerated by the mice.

**Test Example 11. Bystander Effect Test of Anti-LIV-1-ADC**

**Cell and material:** The human ovarian cancer cell line OVCAR3 was purchased from ATCC, the human non-small cell lung cancer cell line NCI-H838-hLIV1-KO was self-constructed, bovine serum (Gibco#10099-141C), 1640 medium (Gibco#A10491-01), penicillin-streptomycin (Gibco#15140-122), and 0.25% Trypsin-EDTA (Gibco#25200-056) were purchased from Gibco (U.S.), bovine insulin (Solarbio#I8040) was purchased from Solarbio, 96 well plates (Greiner Bio-one#655098) was purchased from Corning (U.S.), and Cell-Titer Glo reagent (Promega#G7568) was purchased from Promega (U.S.).

Construction method of NCI-H838-hLIV1-KO: Six kinds of sgRNA were designed for the human LIV1 gene, then the sgRNA was cloned into a pLVX lentiviral vector, and viral particles were prepared in HEK293T (purchased from Chinese Academy of Science) cells. After lentivirus infection, the NCI-H838 (purchased from ATCC) cell line was

selectively cultured in a RPMI 1640 medium containing 1.5 μg/mL puromycin (purchased from Gibco, Cat. No. A1113802) and 10% (w/w) fetal bovine serum for 2 weeks to give the H838-LIV1 KO pool cell strain. Labeled with human anti-LIV1 antibody (Ladiratuzumab, self-produced) and goat anti-human IgG (H+L) antibody (Jackson, Cat. No. 109605088), H838-LIV1 KO monoclonal cells were sorted on a flow cytometer FACSAriaII (purchased from BD Biosciences) into a 96-well plate and incubated at 37 °C with 5% (v/v) $CO_2$. After about 2 weeks, a portion of the monoclonal wells were selected for expansion. The expanded clones were screened by flow cytometry. Monoclonal cell lines with better growth and low fluorescence intensity were selected for further expansion and cryopreserved in liquid nitrogen. **Cell culture:** OVCAR3 cells were cultured with a 1640 medium containing 20% fetal bovine serum + 2 μg/mL bovine insulin + 1% penicillin-streptomycin, and NCI-H838-hLIV1-KO cells were cultured with a 1640 medium containing 10% fetal bovine serum + 1% penicillin-streptomycin. The two cell strains were both cultured at 37 °C with 5% $CO_2$. Cells at logarithmic growth phase were available for the experiment.

**Bystander effect detection:** The supernatant after incubation of the ADC to be detected with LIV-1 positive cells OVCAR3 was transferred to LIV-1 negative cells NCI-H838-hLIV1-KO for further incubation, and the Cell-Titer Glo reagent was used for detecting the influence of the supernatant on the cell proliferation activity of NCI-H838-hLIV1-KO, so that the bystander effect of the ADC was reflected. OVCAR3 cells were digested and pipetted in cell culture flasks, and resuspended using the corresponding fresh medium. The cell density was adjusted. The cells were seeded on a 96-well plate at 10000 cells/180 μL/well and incubated at 37 °C with 5% $CO_2$ overnight. The ADC concentration was diluted to 5000 nM with a complete medium and subjected to 5-fold gradient dilution to give a total of 8 concentration gradients, and then 20 μL of an ADC diluted solution was transferred to the 96-well plate, i.e., the initial concentration of the ADC was 500 nM. The 96-well plate was incubated at 37 °C with 5% $CO_2$ for 5 days. 150 μL of the supernatant in the OVCAR3 cell culture plate was pipetted and transferred to the NCI-H838-hLIV1-KO cell plate (450 cells/50 μL/well) seeded one day in advance, and incubated at 37 °C with 5% $CO_2$ for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the supernatant after incubation of the ADC to be detected with LIV-1 positive cell OVCAR3 was further incubated with LIV-1 negative cell NCI-H838-hLIV1-KO, and the killing thereof on negative cells was detected by adding a Cell-Titer Glo reagent. The results are shown in Table 28 and show that the anti-LIV1-ADC of the present disclosure has a good bystander effect.

Table 28 Bystander effect of anti-LIV1-ADC

| Conjugate No. | $IC_{50}$ (NCI-H838-hLIV1-KO negative cell, nM) | Maximum inhibition rate (%) |
|---|---|---|
| ADC-L 1-19-7 | 13.7 | 95.3 |
| ADC-L1-19-P1-7 | 4.351 | 97.0 |
| ADC-L1-0-7 | 48.64 | 88.0 |

**Test Example 12. Bystander Effect Test of Anti-ROR1-ADC**

**Cell and material:** The human breast cancer cell line hROR1-MCF7 was constructed by Simcere Zaiming, the human breast cancer cell line MCF7 was purchased from ATCC, bovine serum (Gibco#10099-141C), DMEM medium (Gibco#11995-065), penicillin-streptomycin (Gibco#15140-122), and 0.25% Trypsin-EDTA (Gibco#25200-056) were purchased from Gibco (U.S.), 96 well plates (Greiner Bio-one#655098) was purchased from Corning (U.S.), and Cell-Titer Glo reagent (Promega#G7568) was purchased from Promega (U.S.).

**Cell culture:** hROR1-MCF7 cells and MCF7 cells were cultured with a DMEM medium containing 10% fetal bovine serum + 1% penicillin-streptomycin at 37 °C with 5% $CO_2$. Cells at logarithmic growth phase were available for the experiment.

**Bystander effect detection:** The supernatant after incubation of the ADC to be detected with ROR1 positive cells hROR1-MCF7 was transferred to ROR1 negative cells MCF7 for further incubation, and the Cell-Titer Glo reagent was used for detecting the influence of the supernatant on the cell proliferation activity of MCF7, so that the bystander effect of the ADC was reflected. hROR1-MCF7 cells were digested and pipetted in cell culture flasks, and resuspended using the corresponding fresh medium. The cell density was adjusted. The cells were seeded on a 96-well plate at 20000 cells/180 μL/well and incubated at 37 °C with 5% $CO_2$ overnight. The ADC concentration was diluted to 5000 nM with a complete medium and subjected to 3-fold gradient dilution to give a total of 8 concentration gradients, and then 20 μL of an ADC diluted solution was transferred to the 96-well plate, i.e., the initial concentration of the ADC was 500 nM. The 96-well plate was incubated at 37 °C with 5% $CO_2$ for 5 days. 150 μL of the supernatant in the hROR1-MCF7 cell culture plate was pipetted and transferred to the MCF7 cell plate (1500 cells/50 μL/well) seeded one day in advance, and incubated at 37 °C with 5% $CO_2$ for 5 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the supernatant after incubation of the ADC to be detected with ROR1 positive cell hROR1-MCF7 was further incubated with ROR1 negative cell MCF7, and the killing thereof on negative cells was detected by adding a Cell-Titer Glo reagent. The results are shown in Table 29 and show that the anti-ROR1-ADC of the present disclosure has a good bystander effect.

Table 29 Bystander effect of anti-ROR1-ADC

| Conjugate No. | $IC_{50}$ (negative cell, nM) | Maximum inhibition rate (%) |
|---|---|---|
| ADC-L 1-19-8 | 0.03 | 56.83 |
| ADC-L1-19-P1-8 | 0.05 | 53.62 |
| ADC-L1-19-9 | 0.10 | 57.76 |
| ADC-L1-19-P1-9 | 0.07 | 53.70 |
| ADC-L1-19-10 | 0.02 | 61.37 |
| ADC-L1-19-P1-10 | 0.03 | 57.86 |

**Test Example 13. Bystander Effect Test of Anti-HER2-ADC**

**Cell and material:** The human breast cancer cell line SKBR3 was purchased from ATCC, the human small cell lung cancer cell line NCI-H2171 was purchased from ATCC, bovine serum (Gibco#10099-141C), McCoy's 5a medium (Gibco#16600-082), 1640 medium (Gibco#A10491-01), penicillin-streptomycin (Gibco#15140-122), and 0.25% Trypsin-EDTA (Gibco#25200-056) were purchased from Gibco (U.S.), 96 well plates (Greiner Bio-one#655098) was purchased from Corning (U.S.), and Cell-Titer Glo reagent (Promega#G7568) was purchased from Promega (U.S.).

**Cell culture:** SKBR3 cells were cultured with a McCoy's 5a medium containing 10% fetal bovine serum + 1% penicillin-streptomycin, and NCI-H2171 cells were cultured with a 1640 medium containing 10% fetal bovine serum + 1%

penicillin-streptomycin. The two cell strains were both cultured at 37 °C with 5% $CO_2$. Cells at logarithmic growth phase were available for the experiment.

**Bystander effect detection:** The supernatant after incubation of the ADC with HER2 positive cells SKBR3 was transferred to HER2 negative cells NCI-H2171 for further incubation, and the Cell-Titer Glo reagent was used for detecting the influence of the supernatant on the cell proliferation activity of NCI-H2171, so that the bystander effect of the ADC was reflected. SKBR3 cells were digested and pipetted in cell culture flasks, and resuspended using the corresponding fresh medium. The cell density was adjusted. The cells were seeded on a 96-well plate at 10000 cells/180 μL/well and incubated at 37 °C with 5% $CO_2$ overnight. The ADC concentration was diluted to 5000 nM with a complete medium and subjected to 3-fold gradient dilution to give a total of 8 concentration gradients, and then 20 μL of an ADC diluted solution was transferred to the 96-well plate, i.e., the initial concentration of the ADC was 500 nM. The 96-well plate was incubated at 37 °C with 5% $CO_2$ for 3 days. 150 μL of the supernatant in the SKBR3 cell culture plate was pipetted and transferred to the NCI-H2171 cell plate (6000 cells/50 μL/well) seeded one day in advance, and incubated at 37 °C with 5% $CO_2$ for 5 days. The Cell-Titer Glo reagent was added and the cell activity was measured. A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the supernatant after incubation of the ADC to be detected with HER2 positive cell SKBR3 was further incubated with HER2 negative cell NCI-H2171, and the killing thereof on negative cells was detected by adding a Cell-Titer Glo reagent. The results are shown in Table 30 and show that the anti-HER2-ADC of the present disclosure has a good bystander effect.

Table 30 Bystander effect of anti-HER2-ADC

| Conjugate No. | $IC_{50}$ (negative cell, nM) | Maximum inhibition rate (%) |
|---|---|---|
| ADC-L1-0-11 | 1.90 | 89.17 |
| ADC-L1-19-11 | 0.16 | 81.43 |
| ADC-L1-19-P1-11 | 0.12 | 84.55 |
| ADC-L1-0-2 | 3.42 | 99.14 |
| ADC-L1-19-2 | 0.15 | 81.33 |
| ADC-L1-19-P1-2 | 0.17 | 86.51 |

**Test Example 14. Bystander Effect Test of Anti-CDH6-ADC**

**Cell and material:** The human ovarian cancer cell line OVCAR3 was purchased from ATCC, the human ovarian cancer cell line SKOV3 was purchased from ATCC, bovine serum (Gibco#10099-141C), 1640 medium (Gibco#A10491-01), McCoy's 5a medium (Gibco#16600-082), penicillin-streptomycin (Gibco#15140-122), and 0.25% Trypsin-EDTA (Gibco#25200-056) were purchased from Gibco (U.S.), bovine insulin (Solarbio#I8040) was purchased from Solarbio, 96 well plates (Greiner Bio-one#655098) was purchased from Corning (U.S.), and Cell-Titer Glo reagent (Promega#G7568) was purchased from Promega (U.S.).

**Cell culture:** OVCAR3 cells were cultured with a 1640 medium containing 20% fetal bovine serum + 2 μg/mL bovine insulin + 1% penicillin-streptomycin, and SKOV3 cells were cultured with a McCoy's 5a medium containing 10% fetal bovine serum + 1% penicillin-streptomycin. The two cell strains were both cultured at 37 °C with 5% $CO_2$. Cells at

logarithmic growth phase were available for the experiment.

**Bystander effect detection:** The supernatant after incubation of the ADC with CDH6 positive cells OVCAR3 was transferred to CDH6 negative cells SKOV3 for further incubation, and the Cell-Titer Glo reagent was used for detecting the influence of the supernatant on the cell proliferation activity of SKOV3, so that the bystander effect of the ADC was reflected. OVCAR3 cells were digested and pipetted in cell culture flasks, and resuspended using the corresponding fresh medium. The cell density was adjusted. The cells were seeded on a 96-well plate at 20000 cells/180 μL/well and incubated at 37 °C with 5% $CO_2$ overnight. The ADC concentration was diluted to 4500 nM with a complete medium and subjected to 3-fold gradient dilution to give a total of 8 concentration gradients, and then 20 μL of an ADC diluted solution was transferred to the 96-well plate, i.e., the initial concentration of the ADC was 450 nM. The 96-well plate was incubated at 37 °C with 5% $CO_2$ for 5 days. 150 μL of the supernatant in the OVCAR3 cell culture plate was pipetted and transferred to the SKOV3 cell plate (700 cells/50 μL/well) seeded one day in advance, and incubated at 37 °C with 5% $CO_2$ for 7 days. The Cell-Titer Glo reagent was added and the cell activity was measured.

A negative control group and a positive control group were respectively used as Bottom and Top. The negative control group was not added with cells, only added with the culture medium in the same volume, and other operations were consistent with those of the experimental group. The positive control group was not added with the test drug, and other operations were consistent with those of the experimental group.

**Data analysis:**

%Inhibition was calculated and fitted to give compound $IC_{50}$.

$$\%\text{Inhibition} = 1 - 100\% \times (\text{Signal} - \text{Bottom}) / (\text{Top} - \text{Bottom}).$$

Signal referred to the signal value of the experimental group, Bottom referred to the average signal value of the negative control group, and Top referred to the average signal value of the positive control group.

**Experimental results:**

Under the condition of the experiment, the supernatant after incubation of the ADC to be detected with CDH6 positive cell OVCAR3 was further incubated with CDH6 negative cell SKOV3, and the killing thereof on negative cells was detected by adding a Cell-Titer Glo reagent. The results are shown in Table 31 and show that the anti-CDH6-ADC of the present disclosure has a good bystander effect.

Table 31 Bystander effect of anti-CDH6-ADC

| Conjugate No. | $IC_{50}$ (negative cell, nM) | Maximum inhibition rate (%) |
|---|---|---|
| ADC-L1-0-3 | 4.70 | 99.76 |
| ADC-L1-19-3 | 1.39 | 102.7 |
| ADC-L1-19-P1-3 | 1.37 | 105.4 |
| ADC-L1-0-6 | 3.76 | 100.9 |
| ADC-L1-19-6 | 2.21 | 104 |
| ADC-L1-19-P1-6 | 1.80 | 104.9 |
| ADC-L1-0-5 | 12.74 | 100.9 |
| ADC-L1-19-5 | 1.01 | 103.1 |

**Claims**

**1.** A ligand-drug conjugate having a structural general formula of $Pc\text{-}(L\text{-}D)_n$ or a pharmaceutically acceptable salt thereof, wherein

Pc is a ligand unit;
L is a linker unit;
D is a drug unit of the following formula (D-I):

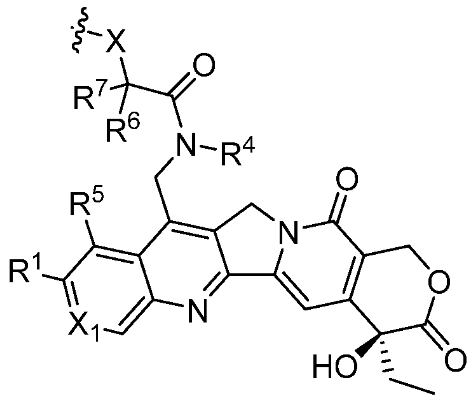

(D-I)

wherein

X is selected from NH or O;

$R^1$ is selected from halogen, CN, $C_1$-$C_6$ alkyl, $C_3$-$C_6$ cycloalkyl, or $C_2$-$C_6$ alkynyl, wherein the $C_1$-$C_6$ alkyl, $C_3$-$C_6$ cycloalkyl, or $C_2$-$C_6$ alkynyl is optionally substituted with one or more $R^{a1}$;

$X_1$ is selected from $CR^2$ or N;

$R^2$ is selected from H, halogen, and CN, or $R^1$ and $R^2$, together with the atom linked thereto, form 5-6 membered heterocyclyl, wherein the 5-6 membered heterocyclyl is optionally substituted with one or more $R^{a2}$;

$R^4$ is selected from H, $C_1$-$C_3$ alkyl, $C_3$-$C_6$ cycloalkyl, or 4-7 membered heterocyclyl, wherein the $C_1$-$C_3$ alkyl, $C_3$-$C_6$ cycloalkyl, or 4-7 membered heterocyclyl is optionally substituted with one or more $R^{a4}$;

$R^5$ is selected from H, halogen, CN, $NH_2$, or $NO_2$, or $R^1$ and $R^5$, together with the atom linked thereto, form 5-6 membered heterocyclyl, 5-6 membered heteroaryl, or $C_5$-$C_7$ cycloalkenyl, wherein the 5-6 membered heterocyclyl, 5-6 membered heteroaryl, or $C_5$-$C_7$ cycloalkenyl is optionally substituted with one or more $R^{a5}$;

$R^6$ is selected from H or $C_1$-$C_3$ alkyl;

$R^7$ is selected from H, $C_1$-$C_3$ alkyl, or $C_3$-$C_6$ cycloalkyl, or $R^6$ and $R^7$, together with the C atom linked thereto, form $C_3$-$C_6$ cycloalkyl, wherein the $C_3$-$C_6$ cycloalkyl is optionally substituted with one or more $R^{a7}$;

each $R^{a1}$, $R^{a2}$, $R^{a4}$, $R^{a5}$, $R^{a7}$ is independently selected from D, halogen, CN, =O, OH, $NH_2$, $C_1$-$C_3$ alkyl, $C_3$-$C_6$ cycloalkyl, or 4-7 membered heterocyclyl, wherein the OH, $NH_2$, $C_1$-$C_3$ alkyl, $C_3$-$C_6$ cycloalkyl, or 4-7 membered heterocyclyl is optionally substituted with one or more $R^b$;

each $R^b$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$;

provided that: i) when $R^1$ is selected from methyl and $R^2$ is selected from F, $R^6$ and $R^7$, together with the C atom linked thereto, form cyclopropyl; ii) when X is selected from NH, $R^5$ is not selected from H; iii) the compound of formula (D-I) does not include

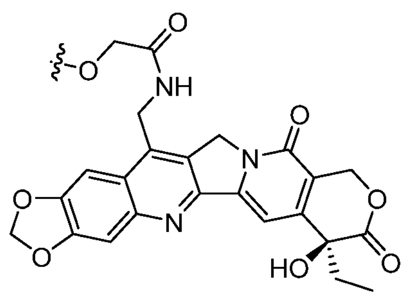

;

moreover, n is a real number of 1-16.

2. The ligand-drug conjugate or the pharmaceutically acceptable salt thereof according to claim 1, wherein $R^1$ is selected from halogen, $C_1$-$C_3$ alkyl, $C_3$-$C_6$ cycloalkyl, or $C_2$-$C_3$ alkynyl.

3. The ligand-drug conjugate or the pharmaceutically acceptable salt thereof according to claim 1 or 2, wherein $R^2$ is selected from H, halogen, and CN, or $R^1$ and $R^2$, together with the atom linked thereto, form 5-6 membered heterocyclyl, wherein the 5-6 membered heterocyclyl comprises 1 or 2 oxygen atoms as a ring atom, and the 5-6 membered heterocyclyl is optionally substituted with one or more D atoms.

4. The ligand-drug conjugate or the pharmaceutically acceptable salt thereof according to claim 1 or 2, wherein $R^2$ is selected from H, F, or Cl, or $R^1$ and $R^2$, together with the atom linked thereto, form

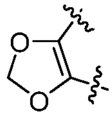, 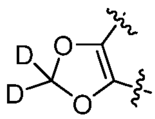, or 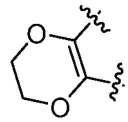.

5. The ligand-drug conjugate or the pharmaceutically acceptable salt thereof according to any one of claims 1 to 4, wherein $R^5$ is selected from H, Cl, F, $NH_2$, or $NO_2$, or $R^1$ and $R^5$, together with the atom linked thereto, form

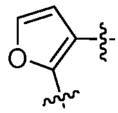 or 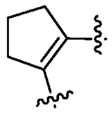.

6. The ligand-drug conjugate or the pharmaceutically acceptable salt thereof according to any one of claims 1 to 5, wherein $R^4$ is selected from H or $C_1$-$C_3$ alkyl.

7. The ligand-drug conjugate or the pharmaceutically acceptable salt thereof according to any one of claims 1 to 6, wherein $R^7$ is selected from H, $C_1$-$C_3$ alkyl, or $C_3$-$C_6$ cycloalkyl optionally substituted with one or more D, or $R^6$ and $R^7$, together with the C atom linked thereto, form $C_3$-$C_6$ cycloalkyl.

8. The ligand-drug conjugate or the pharmaceutically acceptable salt thereof according to any one of claims 1 to 7, wherein structural unit

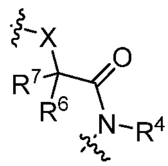

is selected from

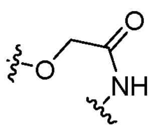, 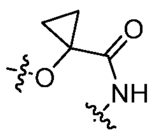,

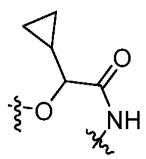, 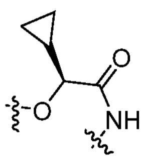, 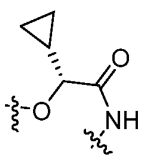, 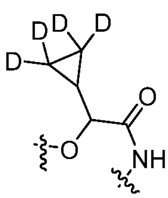, 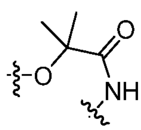, 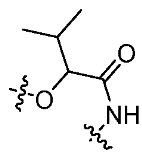, 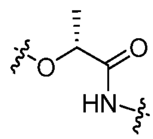,

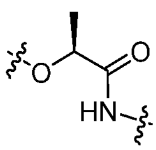, or 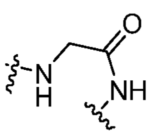.

9. The ligand-drug conjugate or the pharmaceutically acceptable salt thereof according to any one of claims 1 to 8, wherein the drug unit of formula (D-I) is selected from the drug unit of formula (D-Ia):

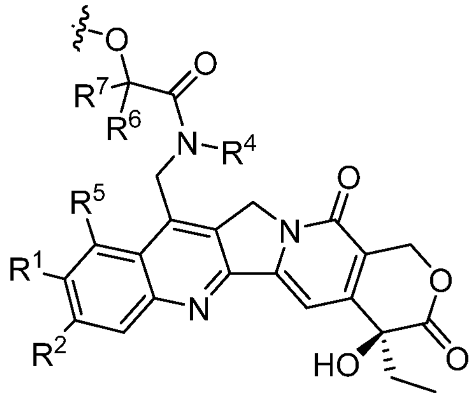

(D-Ia)

wherein $R^1$, $R^2$, $R^4$, $R^5$, $R^6$, and $R^7$ are as defined in any one of claims 1 to 8.

**10.** The ligand-drug conjugate or the pharmaceutically acceptable salt thereof according to any one of claims 1 to 9, wherein the compound of formula (D-I) is selected from the following compounds:

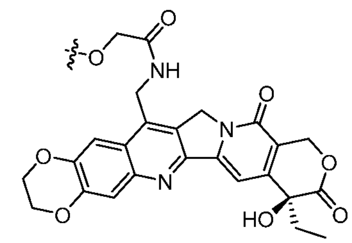

1'

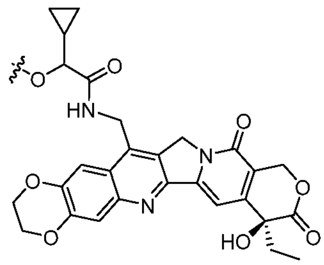

2'

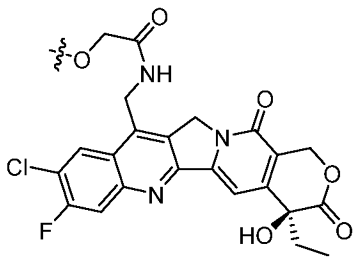

3'

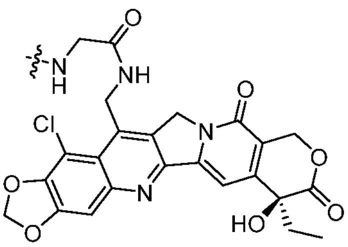

4'

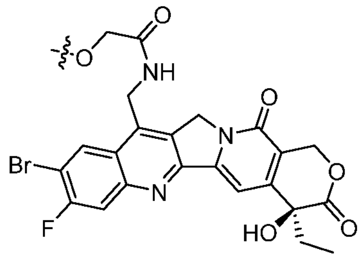

5'

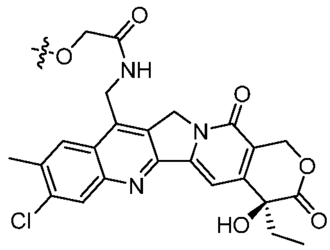

6'

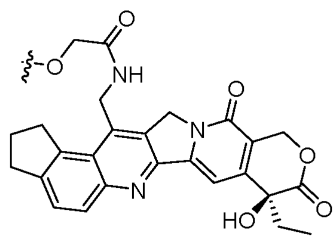

7'

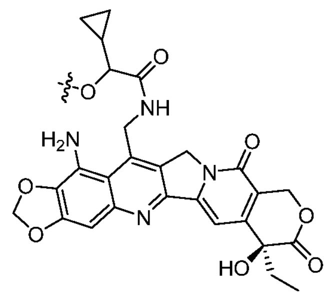

8'

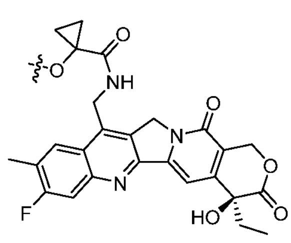

9'

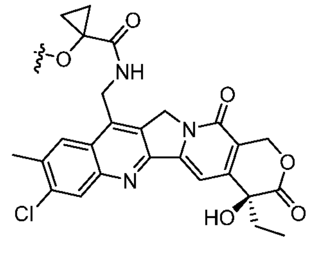

10'

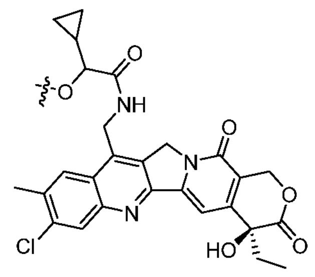

11'

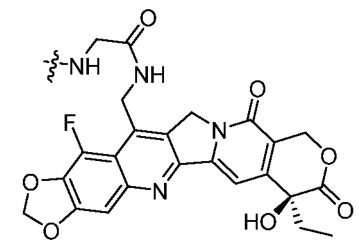

12'

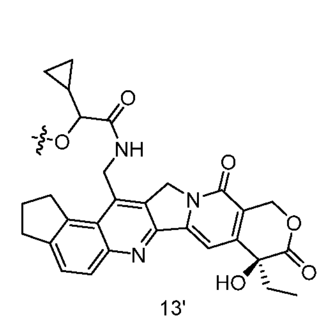

13'

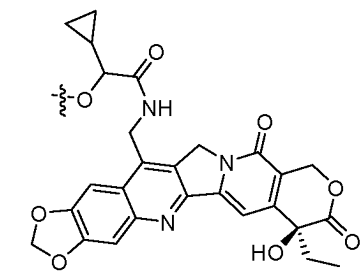

14'

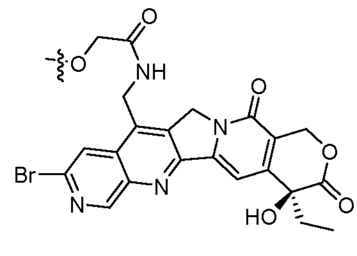

15'

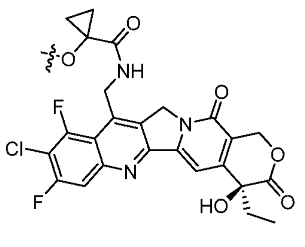

16'

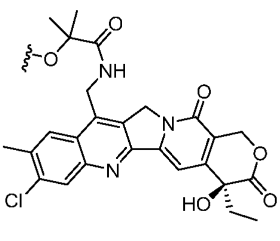

17'

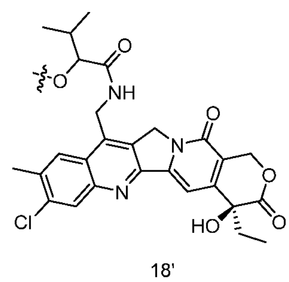

18'

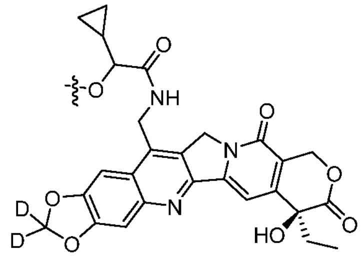

19'

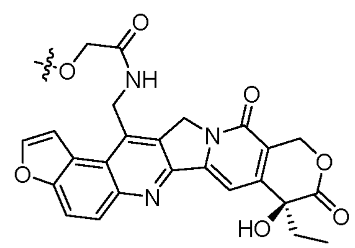

20'

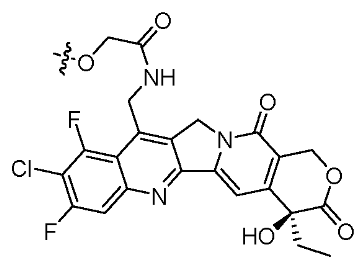

21'

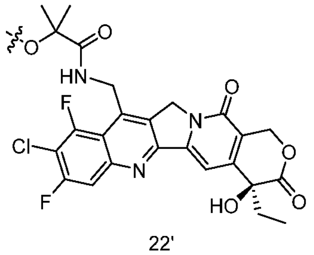

22'

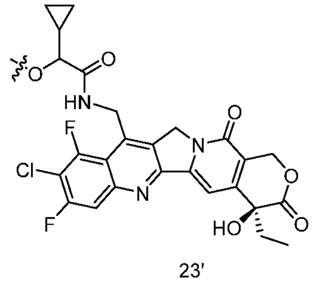

23'

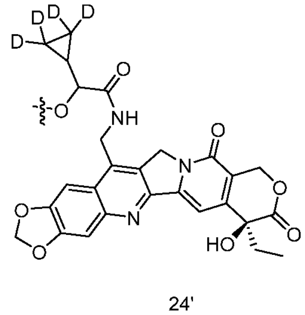

24'

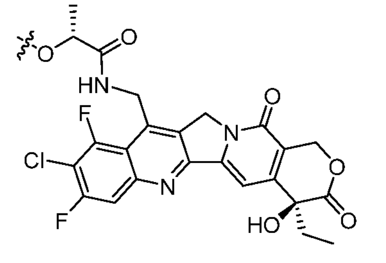

25'

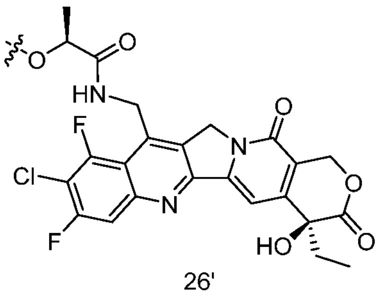

26'

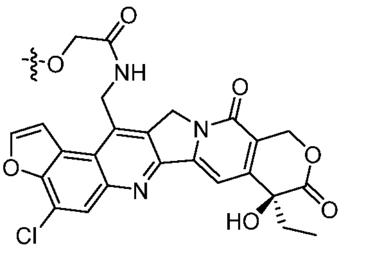

27'

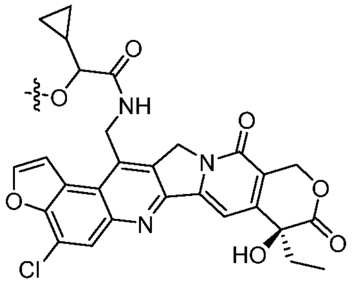

28'

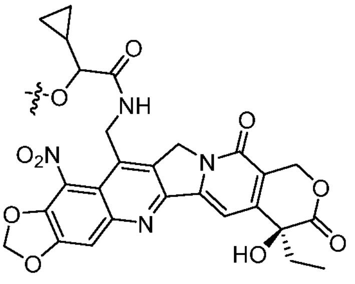

29'

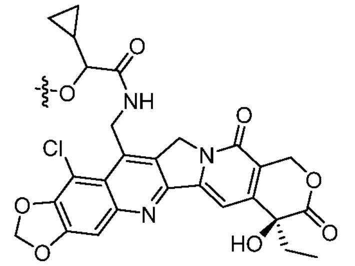

30'

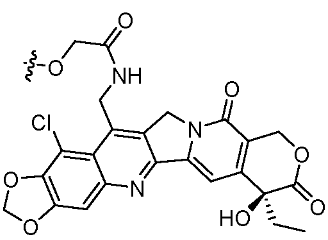

31'

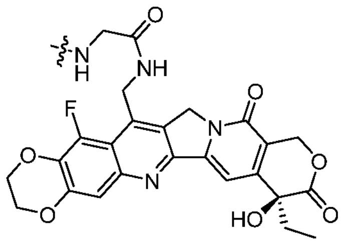

32'

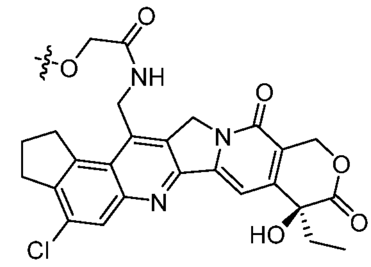

33'

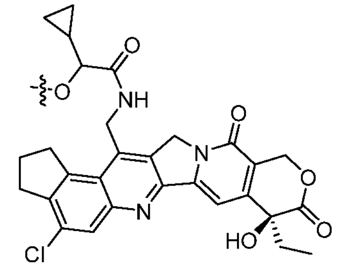

34'

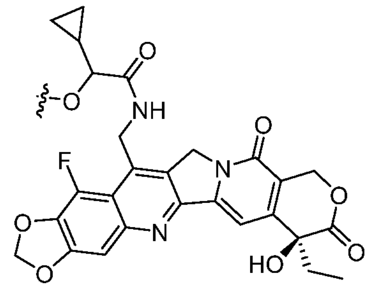

35'

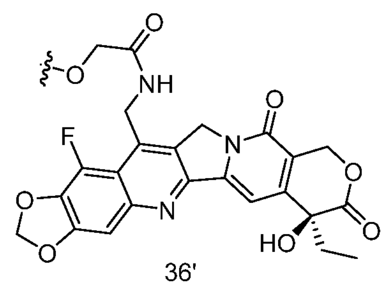

36'

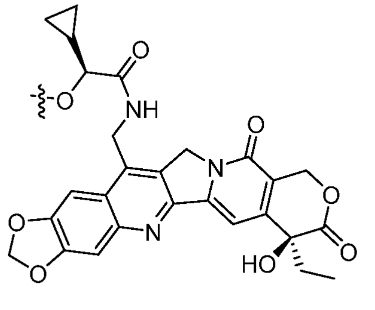

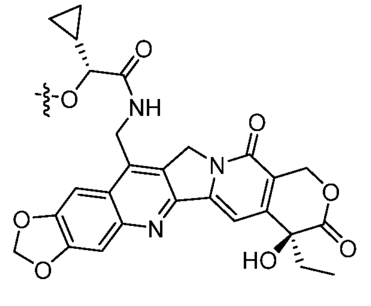

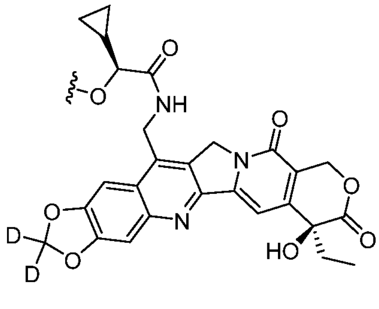

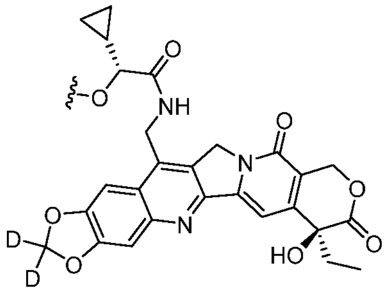
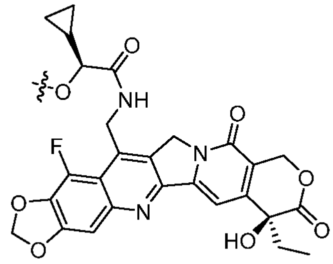
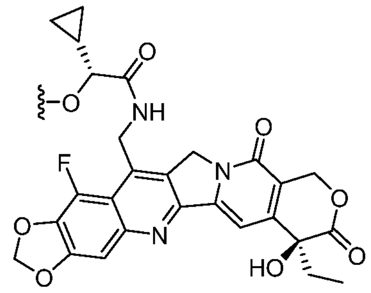

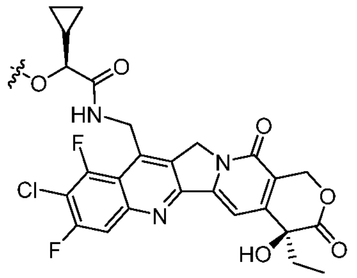
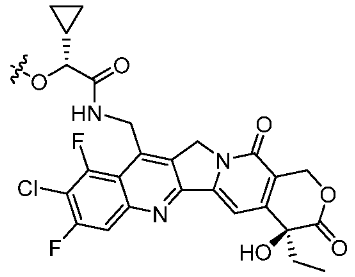

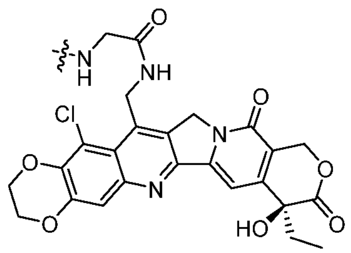
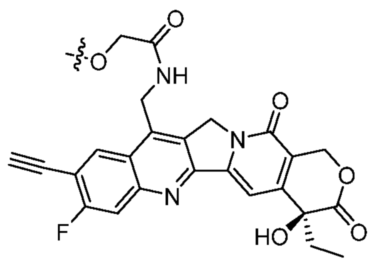
or
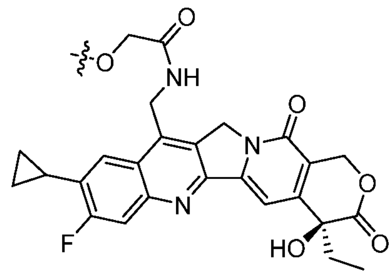
.

**11.** The ligand-drug conjugate or the pharmaceutically acceptable salt thereof according to any one of claims 1 to 10, wherein linker unit L is selected from

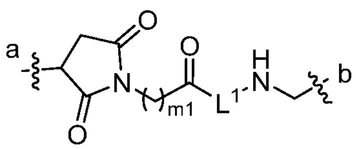
or
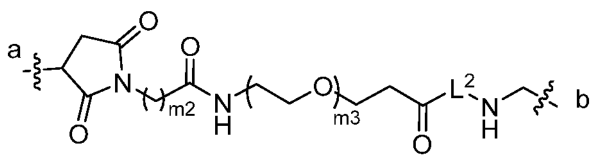
,

the end a of which is covalently linked to ligand unit Pc, and the end b is covalently linked to drug unit D, wherein m1 and m2 are each independently selected from integers of 2-8, m3 is selected from integers of 1-16, and $L^1$ and $L^2$ are each independently selected from peptide residues consisting of 1 to 8 amino acids, wherein the peptide residue is further optionally substituted with one or more substituents of halogen, CN, =O, $C_1$-$C_6$ alkyl, OH, O($C_1$-$C_6$ alkyl), $NH_2$, NH($C_1$-$C_6$ alkyl), N($C_1$-$C_6$ alkyl)$_2$, $C_3$-$C_6$ cycloalkyl, and 4-7 membered heterocyclyl.

**12.** The ligand-drug conjugate or the pharmaceutically acceptable salt thereof according to claim 11, wherein $L^1$ and $L^2$ are each independently selected from peptide residues consisting of 2, 3, or 4 amino acids, wherein the peptide residue is further optionally substituted with one or more substituents of halogen, CN, =O, $C_1$-$C_6$ alkyl, OH, O($C_1$-$C_6$ alkyl), $NH_2$, NH($C_1$-$C_6$ alkyl), N($C_1$-$C_6$ alkyl)$_2$, $C_3$-$C_6$ cycloalkyl, and 4-7 membered heterocyclyl.

**13.** The ligand-drug conjugate or the pharmaceutically acceptable salt thereof according to claim 11 or 12, wherein $L^1$ is a Gly-Gly-Phe-Gly tetrapeptide residue or an Ala-Ala-Ala tripeptide residue, and $L^2$ is a Gly-Gly-Phe-Gly tetrapeptide residue or a Val-Lys dipeptide residue.

**14.** The ligand-drug conjugate or the pharmaceutically acceptable salt thereof according to any one of claims 11 to 13, wherein m1 is selected from 5, m2 is selected from 2, and m3 is selected from 8.

**15.** The ligand-drug conjugate or the pharmaceutically acceptable salt thereof according to any one of claims 11 to 14, wherein linker unit L is selected from

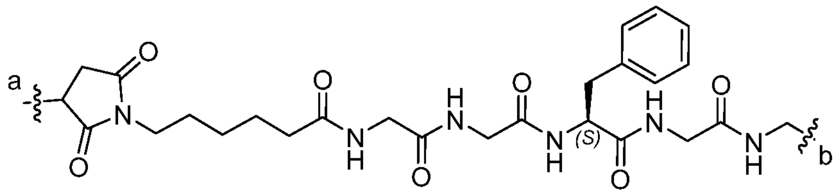

**L1**

,

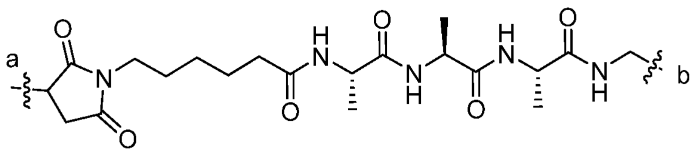

**L2**

,

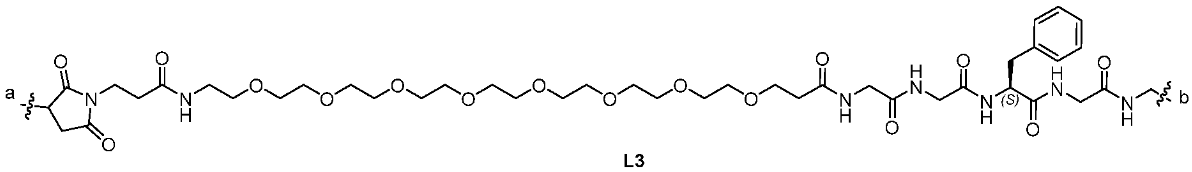

**L3**

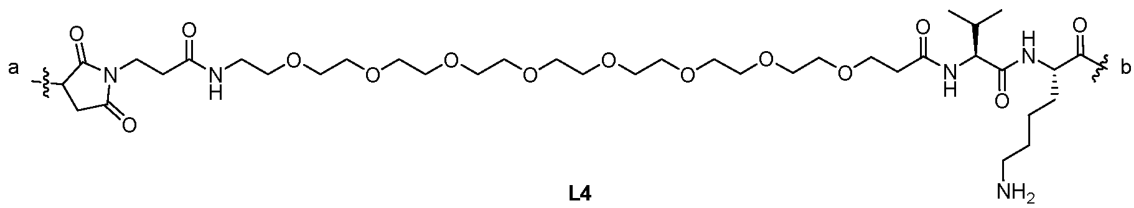

or **L4** ,

the end a of which is covalently linked to ligand unit Pc, and the end b is covalently linked to drug unit D.

**16.** The ligand-drug conjugate or the pharmaceutically acceptable salt thereof according to any one of claims 1 to 15, wherein the ligand-drug conjugate or the pharmaceutically acceptable salt thereof is selected from the following ligand-drug conjugates or pharmaceutically acceptable salts thereof:

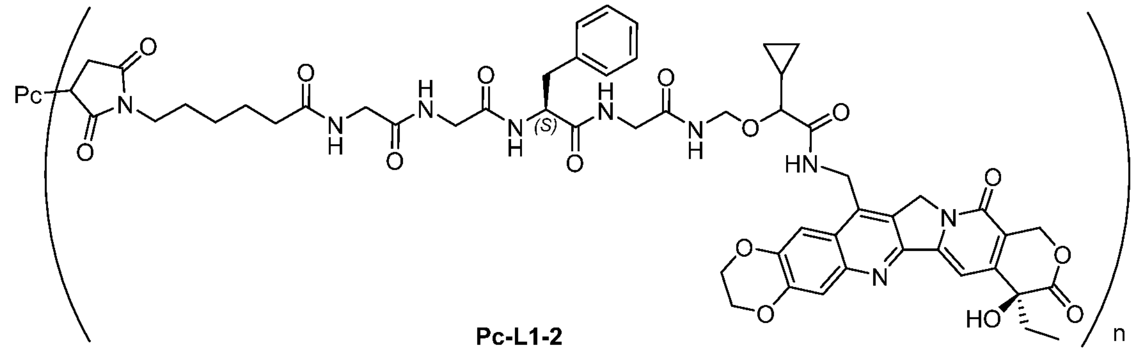

Pc-L1-2

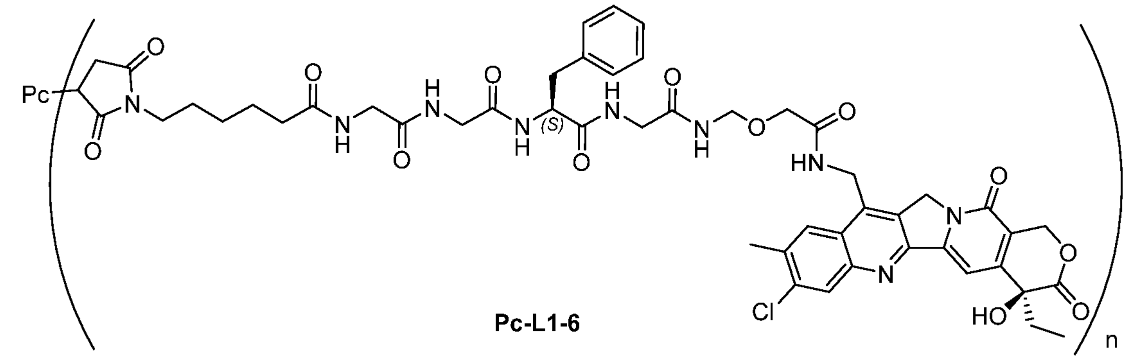

Pc-L1-6

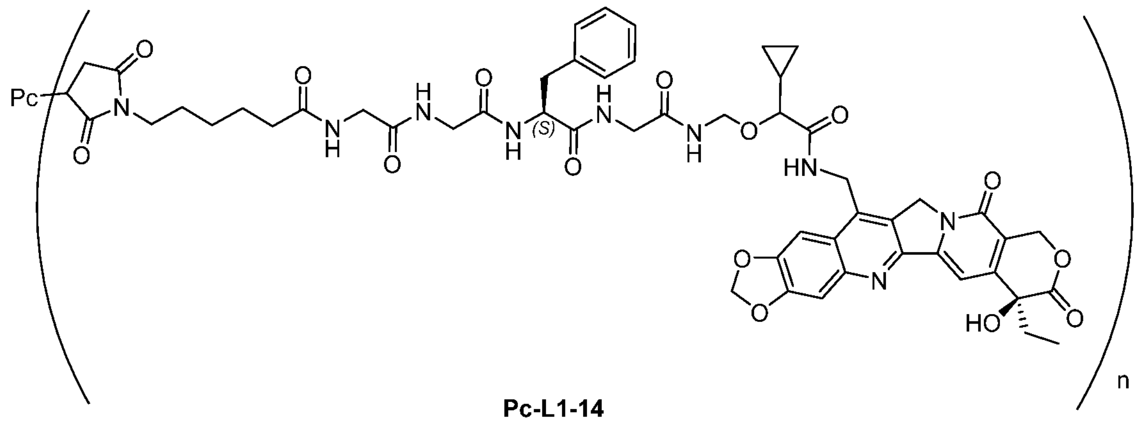

Pc-L1-14

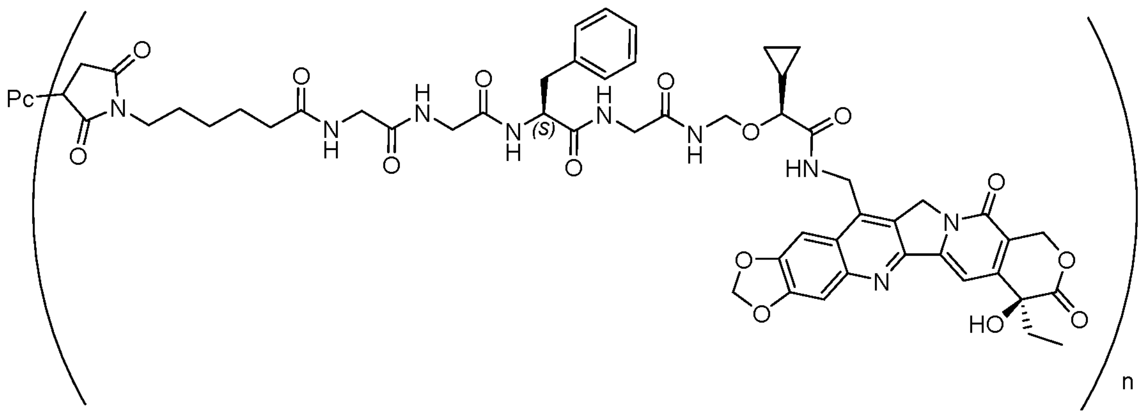

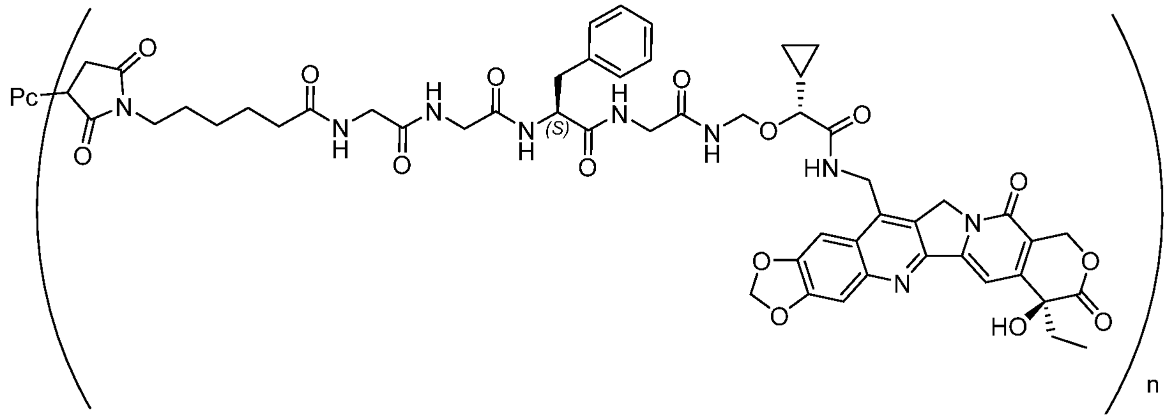

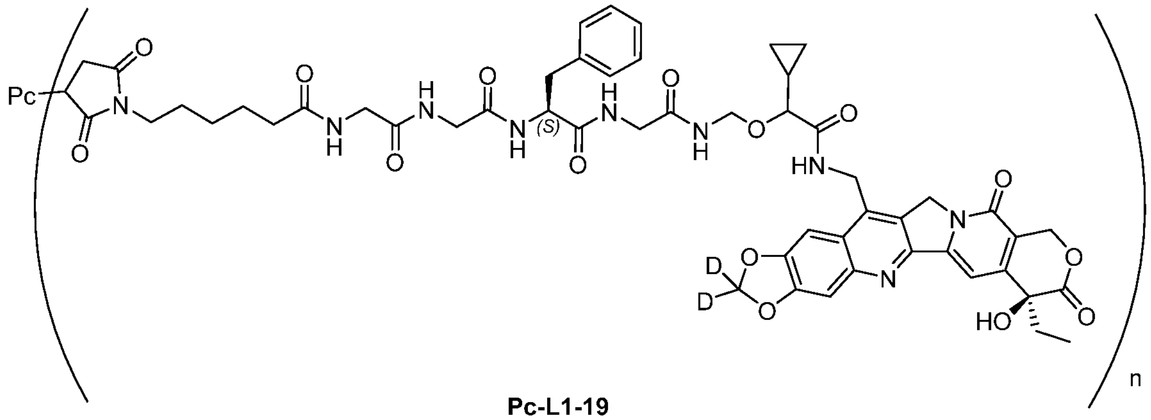

**Pc-L1-19**

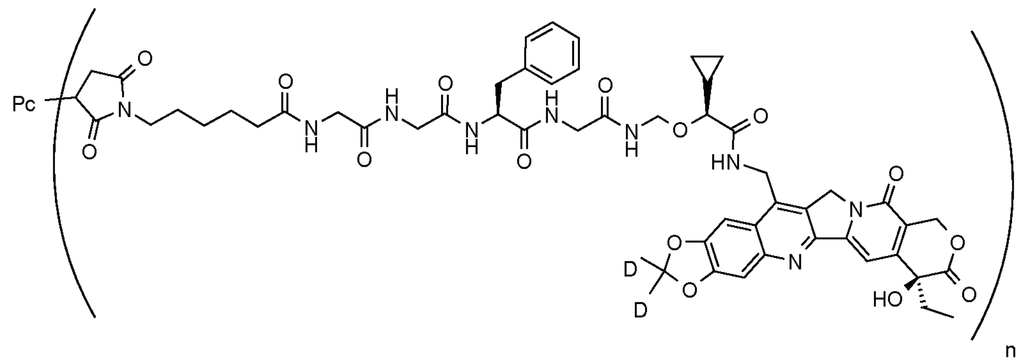

**Pc-L1-19-P1**

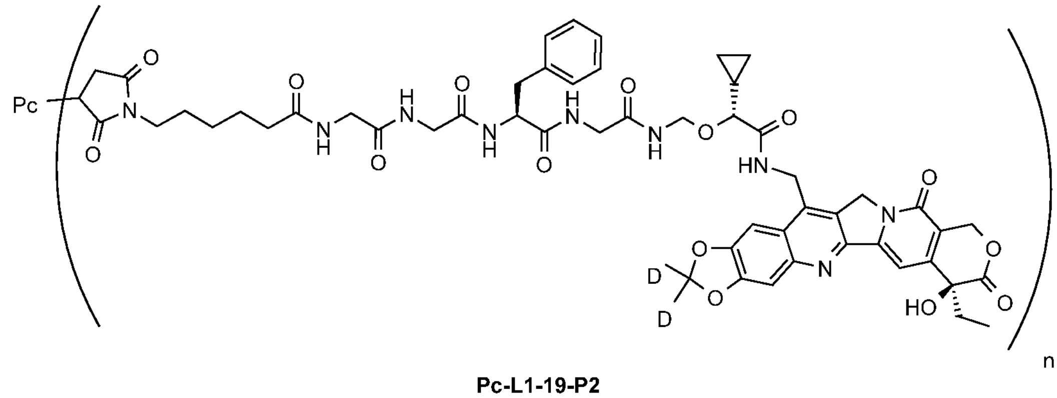

**Pc-L1-19-P2**

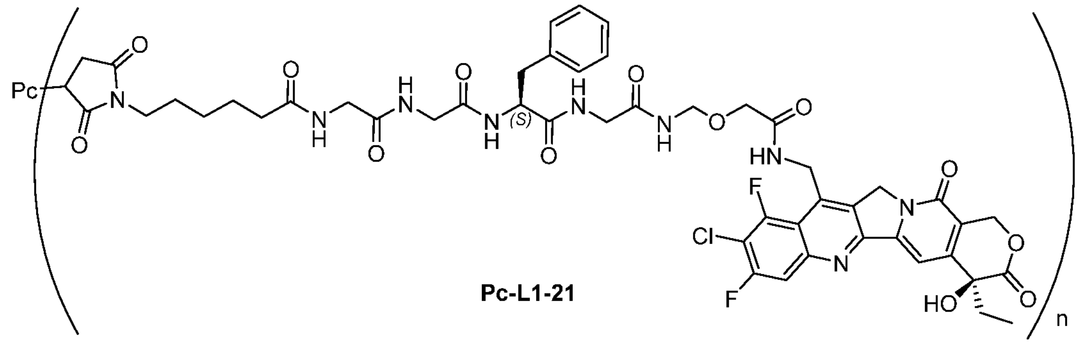

Pc-L1-21

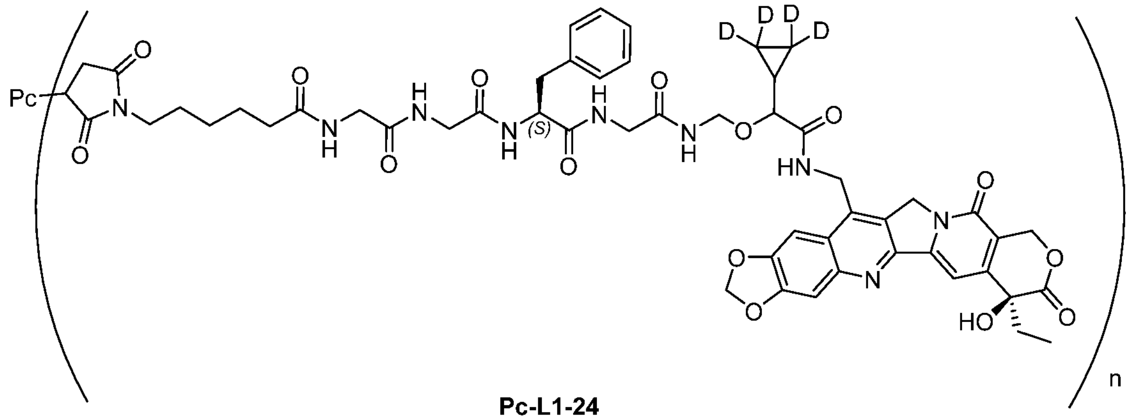

Pc-L1-24

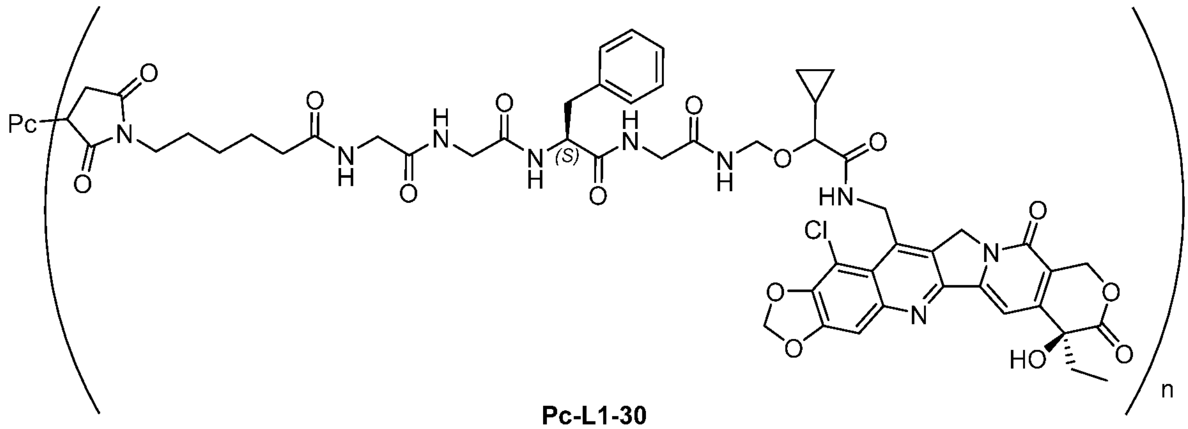

Pc-L1-30

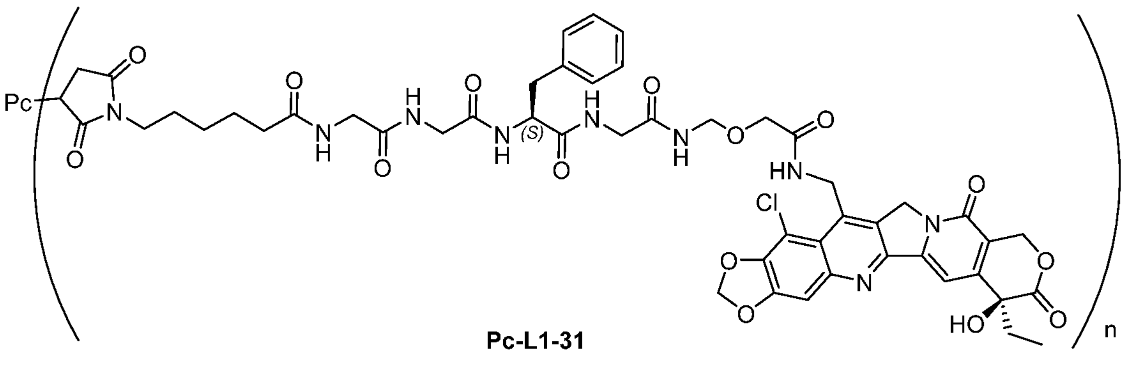

Pc-L1-31

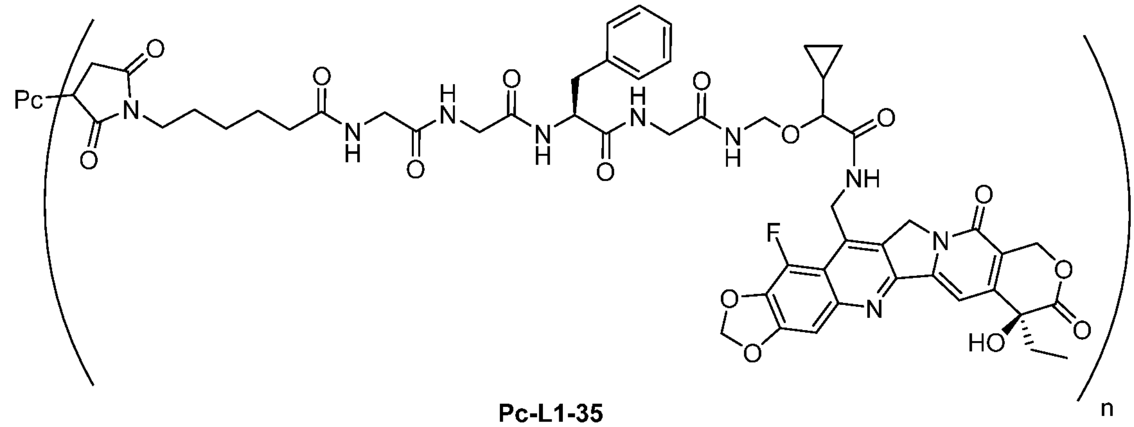

Pc-L1-35

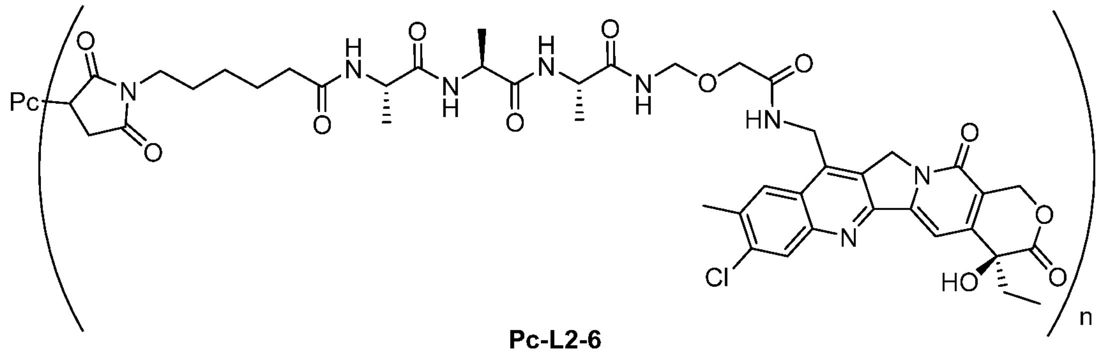

Pc-L2-6

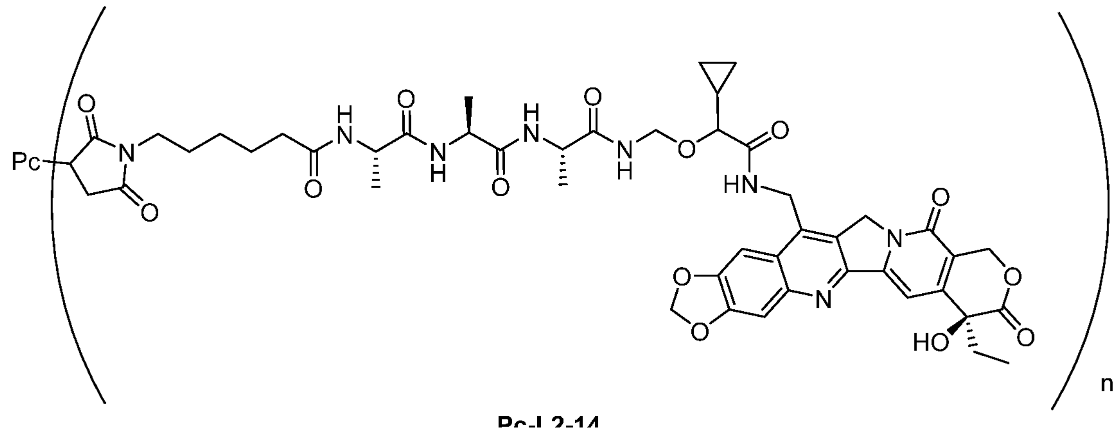

Pc-L2-14

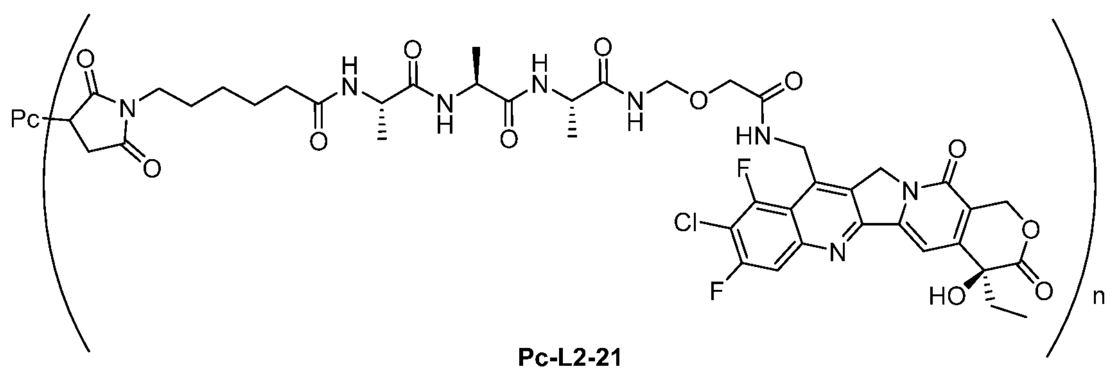

Pc-L2-21

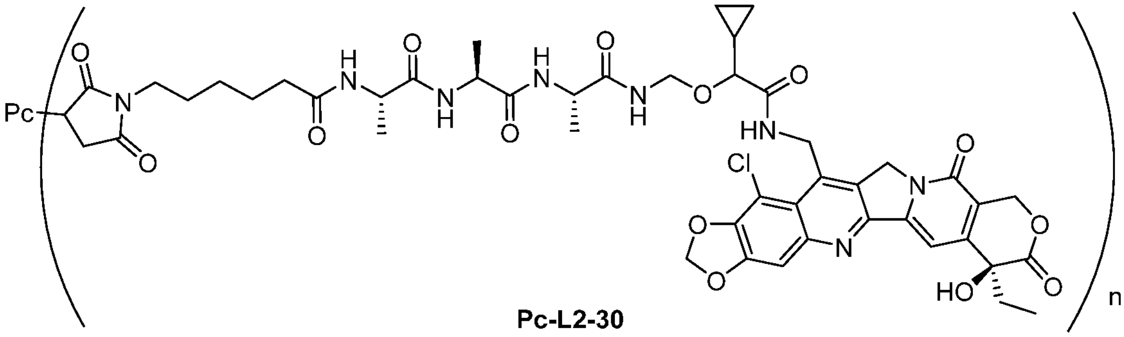

Pc-L2-30

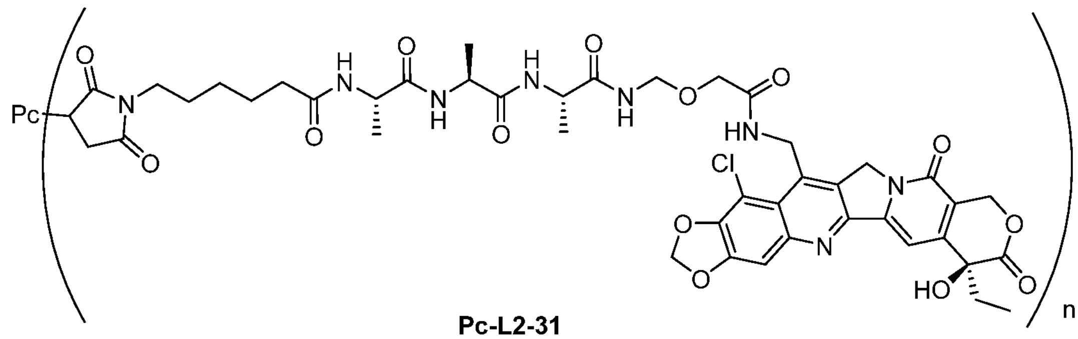

Pc-L2-31

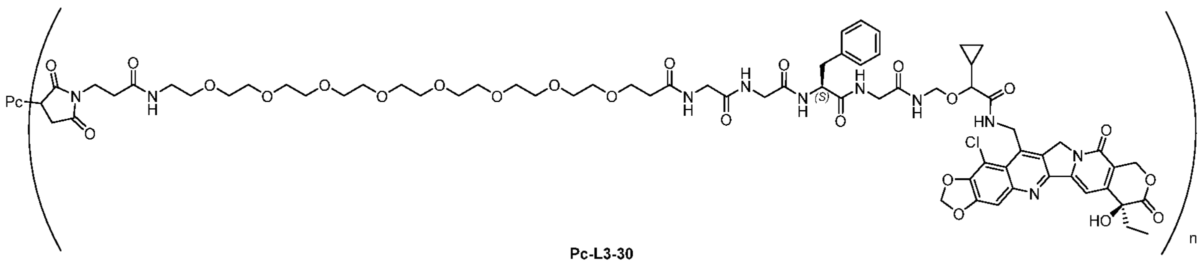

Pc-L3-30

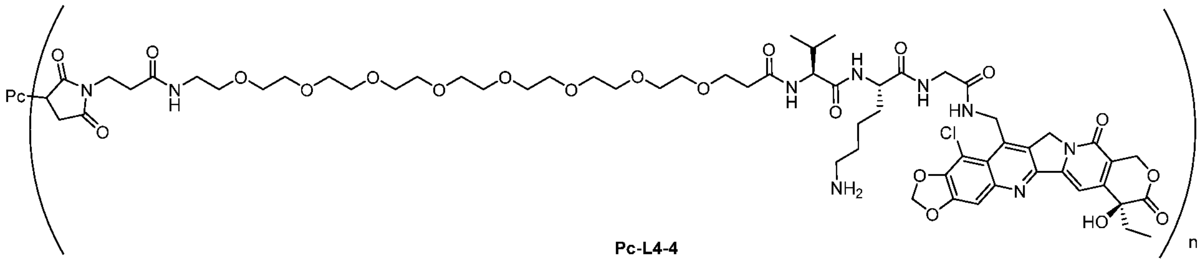

Pc-L4-4

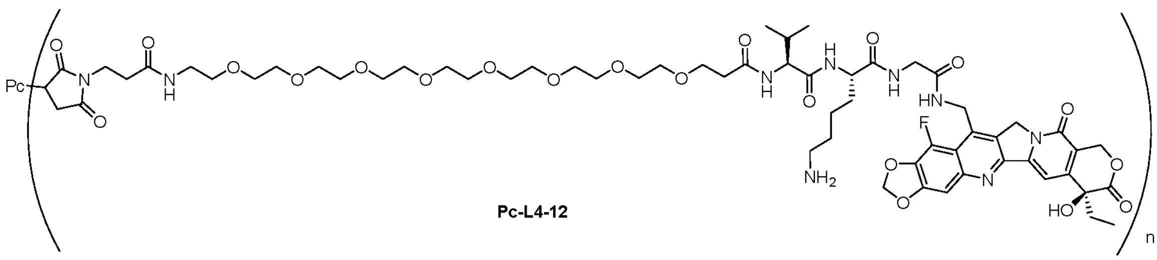

Pc-L4-12

or

Pc and n are as defined in claim 1.

**17.** The ligand-drug conjugate or the pharmaceutically acceptable salt thereof according to any one of claims 1 to 16, wherein ligand unit Pc is selected from a polypeptide, an antibody, or an antigen-binding fragment thereof.

**18.** The ligand-drug conjugate or the pharmaceutically acceptable salt thereof according to any one of claims 1 to 17, wherein ligand unit Pc can specifically bind to one or more antigens selected from the group consisting of: HER2, p95HER2, HER3, CD3, CD16, ROR1, DLL3, CDH6, CD70, CD5, CD20, BCMA, EGFR, VEGF, and LIV-1.

**19.** The ligand-drug conjugate or the pharmaceutically acceptable salt thereof according to claim 17 or 18, wherein Pc is an antibody or an antigen-binding fragment thereof that specifically binds to HER2, p95HER2, CDH6, ROR1, or LIV-1; the antibody or the antigen-binding fragment thereof comprises a heavy chain variable region (VH) or/and a light chain variable region (VL), wherein optionally: (1) the heavy chain variable region comprises HCDR1, HCDR2, and HCDR3 contained in the VH set forth in SEQ ID NO: 1, 3, 19, 21, 37, 46, 54, 56, 71, 80, 82, or 84; or/and the light chain variable region comprises LCDR1, LCDR2, and LCDR3 contained in the VL set forth in SEQ ID NO: 2, 4, 20, 22, 38, 47, 55, 57, 72, 81, 83, or 85; or (2) the heavy chain variable region or/and the light chain variable region comprises an amino acid sequence having at least 80% identity or having at most 3 insertion, deletion, or substitution mutations compared with each CDR of the HCDR1-3 or/and the LCDR1-3 in group (1).

**20.** The ligand-drug conjugate or the pharmaceutically acceptable salt according to claim 19, wherein the antibody or the antigen-binding fragment thereof comprises a heavy chain variable region (VH) or/and a light chain variable region (VL), wherein the heavy chain variable region comprises HCDR1, HCDR2, and HCDR3, or/and the light chain variable region comprises LCDR1, LCDR2, and LCDR3, wherein the HCDR1-3 or/and the LCDR1-3 are selected from the following;

(1) the HCDR1-3 are SEQ ID NOs: 7-9; or/and the LCDR1-3 are SEQ ID NOs: 10-12;
(2) the HCDR1-3 are SEQ ID NOs: 13-15; or/and the LCDR1-3 are SEQ ID NOs: 16-18;
(3) the HCDR1-3 are SEQ ID NOs: 23-25; or/and the LCDR1-3 are SEQ ID NOs: 26-28;
(4) the HCDR1-3 are SEQ ID NOs: 29-31; or/and the LCDR1-3 are SEQ ID NOs: 32-34;
(5) the HCDR1-3 are SEQ ID NOs: 40-42; or/and the LCDR1-3 are SEQ ID NOs: 43-45;
(6) the HCDR1-3 are SEQ ID NOs: 48-50; or/and the LCDR1-3 are SEQ ID NOs: 51-53;
(7) the HCDR1-3 are SEQ ID NOs: 58-60; or/and the LCDR1-3 are SEQ ID NOs: 61-63;
(8) the HCDR1-3 are SEQ ID NOs: 64-66; or/and the LCDR1-3 are SEQ ID NOs: 67-69;
(9) the HCDR1-3 are SEQ ID NOs: 74-76; or/and the LCDR1-3 are SEQ ID NOs: 77-79;
(10) the HCDR1-3 are SEQ ID NOs: 86-88; or/and the LCDR1-3 are SEQ ID NOs: 89-91;
(11) the HCDR1-3 are SEQ ID NOs: 92-94; or/and the LCDR1-3 are SEQ ID NOs: 95-97;
(12) the HCDR1-3 are SEQ ID NOs: 98-100; or/and the LCDR1-3 are SEQ ID NOs: 101-103; or
(13) the HCDR1-3 or/and the LCDR1-3 have an amino acid sequence having at least 80% identity or having at most 3 insertion, deletion, or substitution mutations compared with each CDR in the HCDR1-3 or/and the LCDR1-3 of any one of groups (1) - (12).

**21.** The ligand-drug conjugate or the pharmaceutically acceptable salt thereof according to claim 17 or 18, wherein the antibody or the antigen-binding fragment thereof comprises a heavy chain variable region (VH) or/and a light chain variable region (VL), wherein the heavy chain variable region comprises the amino acid sequence set forth in SEQ ID NO: 1, 3, 19, 21, 37, 46, 54, 56, 71, 80, 82, or 84, or/and the light chain variable region comprises the amino acid sequence set forth in SEQ ID NO: 2, 4, 20, 22, 38, 47, 55, 57, 72, 81, 83, or 85; alternatively, the heavy chain variable region and the light chain variable region comprises an amino acid sequence having at least 80% identity compared with any one of the heavy chain variable regions and the light chain variable regions described above, respectively.

**22.** The ligand-drug conjugate or the pharmaceutically acceptable salt according to any one of claims 17-21, wherein the antibody or the antigen-binding fragment comprises a heavy chain constant region sequence and/or a light chain constant region sequence; optionally, the heavy chain constant region and/or the light chain constant region is selected from an intact constant region sequence or a fragment thereof, and the constant region fragment comprises CH1, hinge region, CH2, CH3, or Fc; optionally, the heavy chain constant region is selected from a human or murine IgG1, IgG2, IgG3, or IgG4 constant region, and the light chain constant region is selected from a human or murine kappa constant region or lamda constant region; optionally, the antibody or the antigen-binding fragment comprises an intact heavy chain and light chain, the heavy chain consists of the VH and the heavy chain constant region, the heavy chain constant region has the amino acid sequence set forth in SEQ ID NO: 5, 39, or 73, the light chain consists of the VL and the light chain constant region, and the light chain constant region has the amino acid sequence set forth in SEQ ID NO: 6.

**23.** The ligand-drug conjugate or the pharmaceutically acceptable salt thereof according to any one of claims 17-22, wherein the antibody is selected from trastuzumab, pertuzumab, or rituximab.

**24.** A drug-linker compound having a structural general formula of L'-D or a pharmaceutically acceptable salt thereof, wherein

drug unit D is as defined in any one of claims 1-10; linker unit L' is selected from

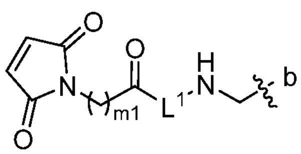

or

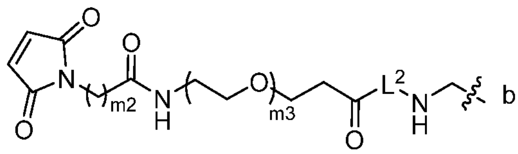

,

the end b of which is covalently linked to drug unit D, and $L^1$, $L^2$, m1, m2, and m3 are as defined in any one of claims 11-15.

**25.** The drug-linker compound or the pharmaceutically acceptable salt thereof according to claim 24, wherein L' is selected from

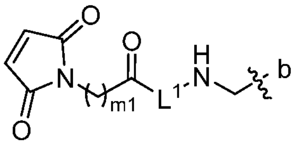

,

the end b of which is covalently linked to drug unit D, m1 is selected from 5, and $L^1$ is selected from Gly-Gly-Phe-Gly tetrapeptide residue or Ala-Ala-Ala tripeptide residue.

**26.** The drug-linker compound or the pharmaceutically acceptable salt thereof according to claim 24, wherein L' is selected from

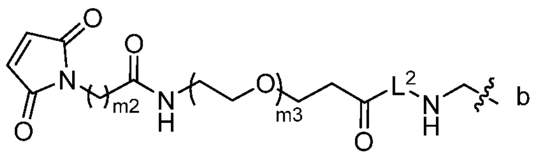

,

the end b of which is covalently linked to drug unit D, m2 is selected from 2, m3 is selected from 8, and $L^2$ is selected from Gly-Gly-Phe-Gly tetrapeptide residue or Val-Lys dipeptide residue.

**27.** The drug-linker compound or the pharmaceutically acceptable salt thereof according to any one of claims 24 to 26, wherein L' is selected from the following chemical structures:

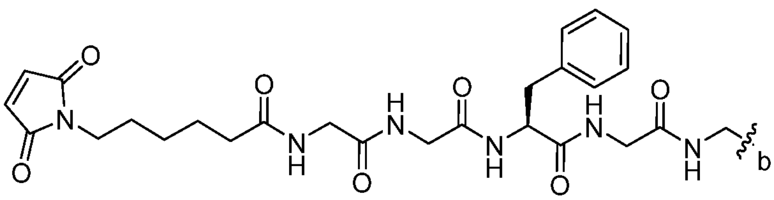

**L'1**

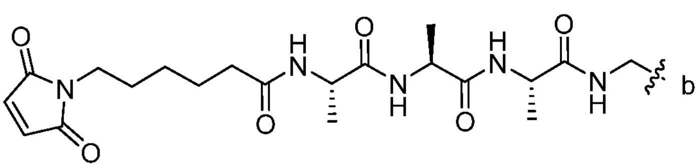

**L'2**

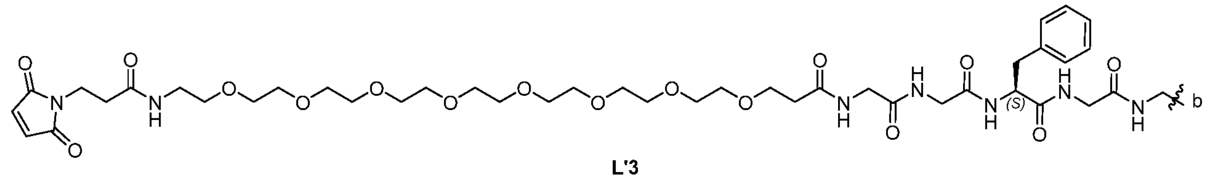

L'3

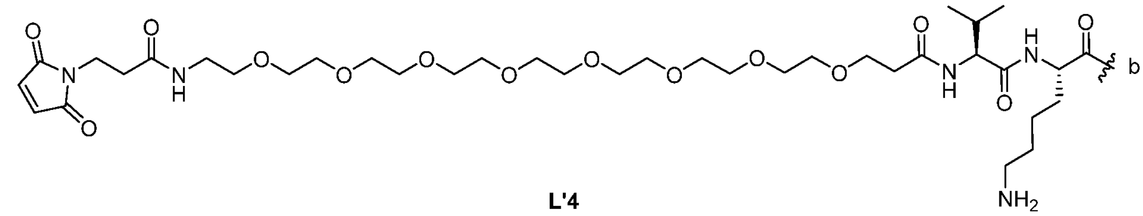

or

L'4

,

the end b of which is covalently linked to drug unit D.

**28.** The drug-linker compound or the pharmaceutically acceptable salt thereof according to any one of claims 24 to 27 selected from the following compounds or pharmaceutically acceptable salts thereof:

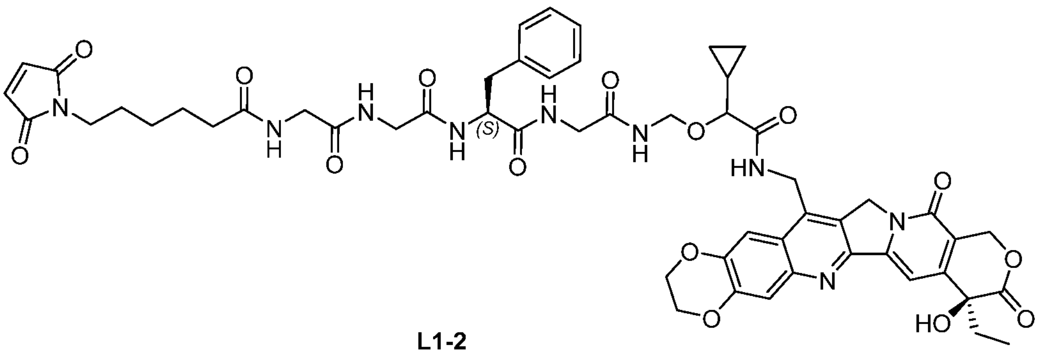

L1-2

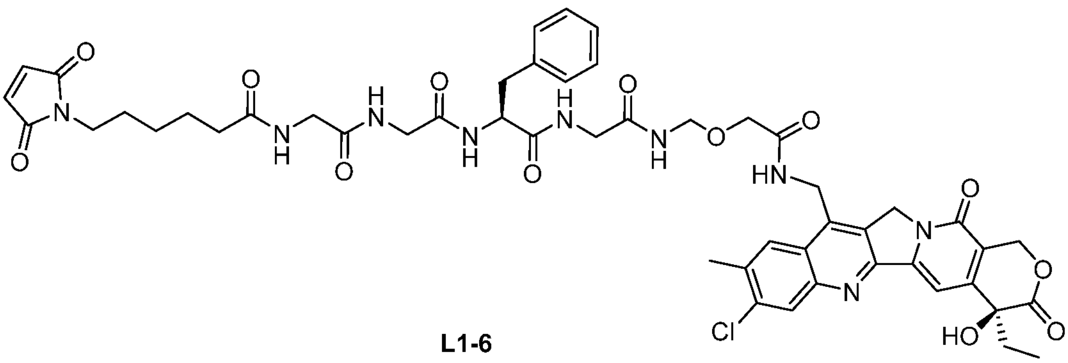

L1-6

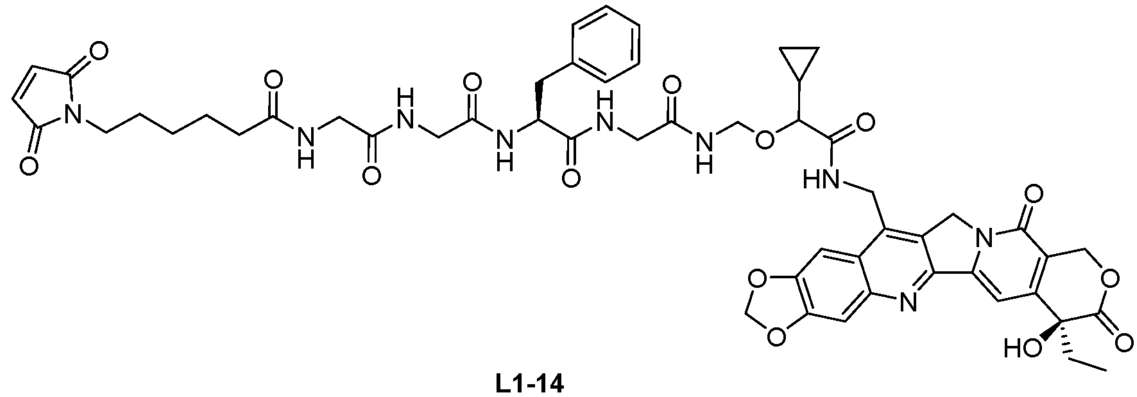

**L1-14**

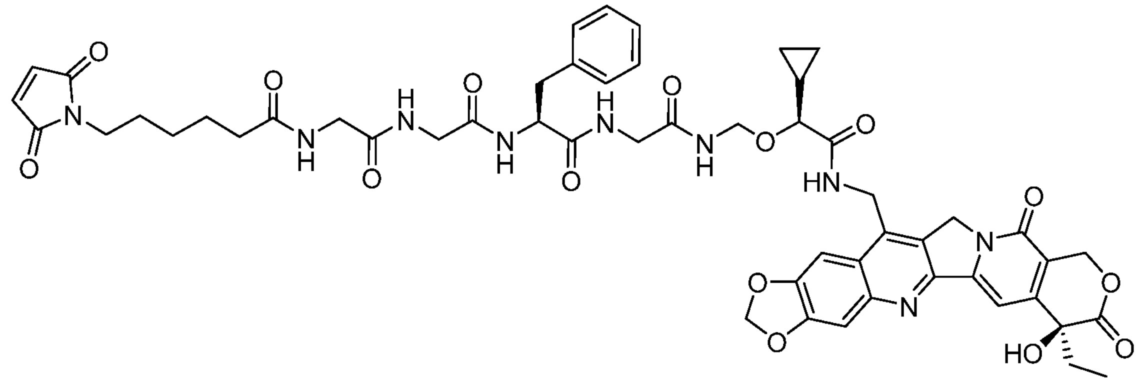

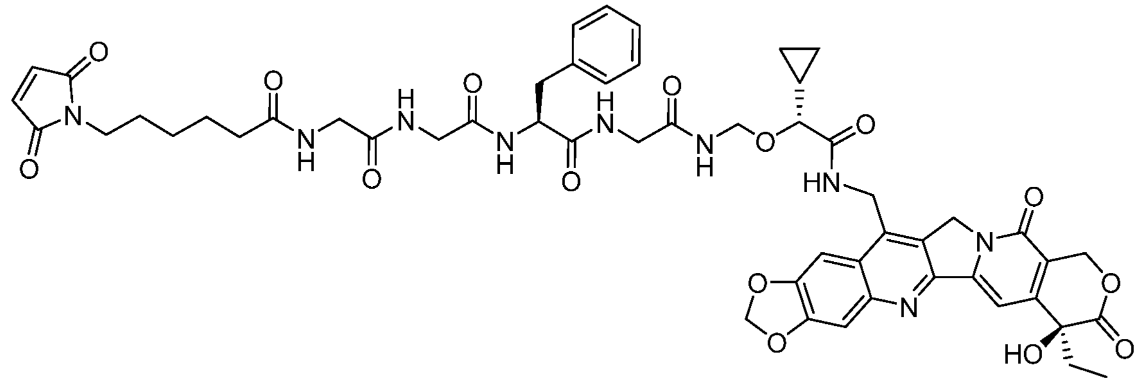

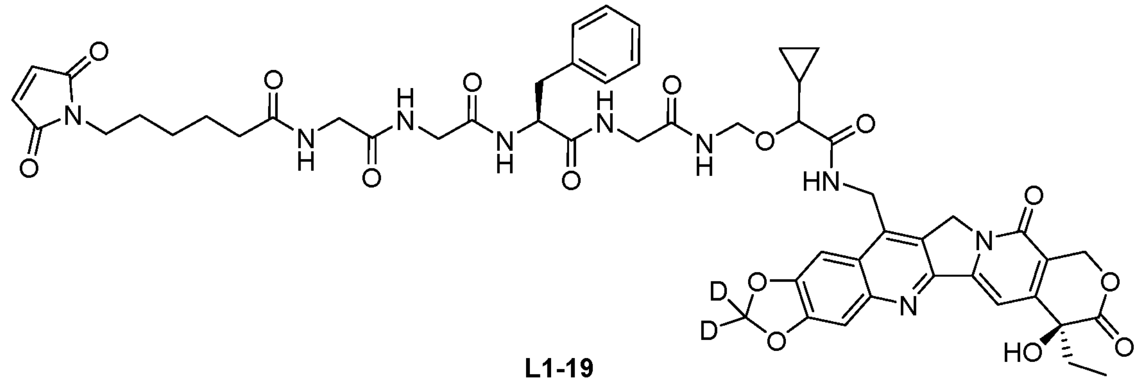

**L1-19**

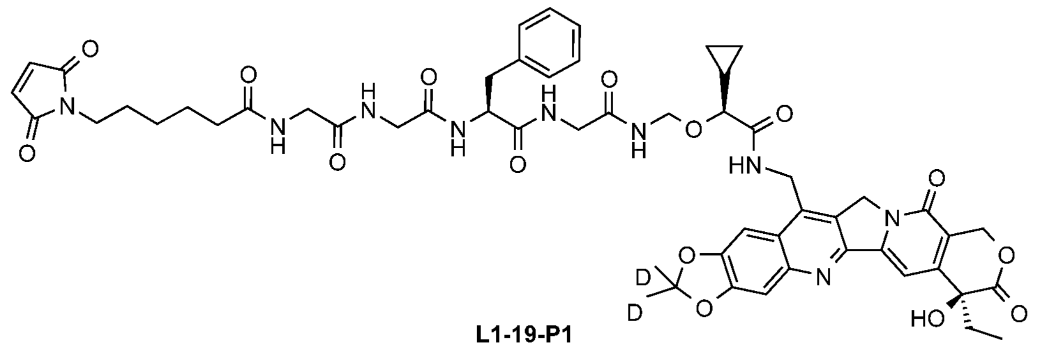

L1-19-P1

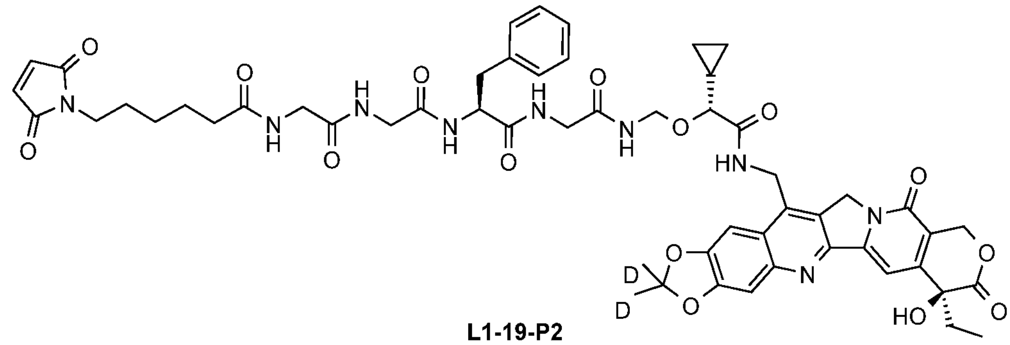

L1-19-P2

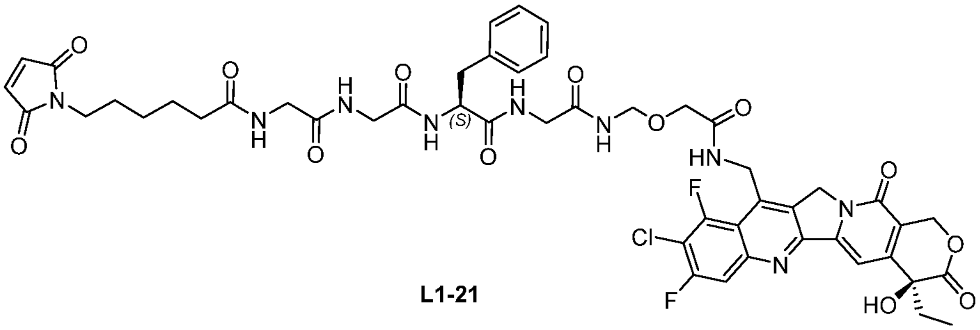

L1-21

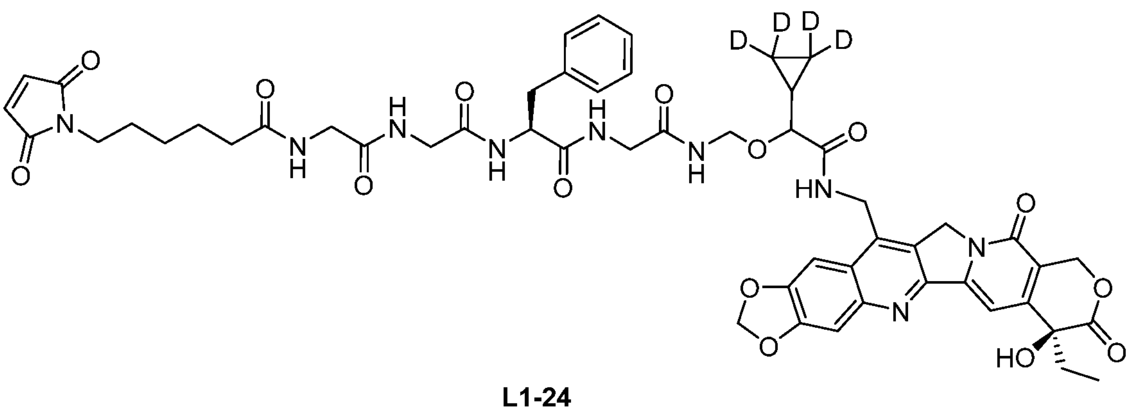

L1-24

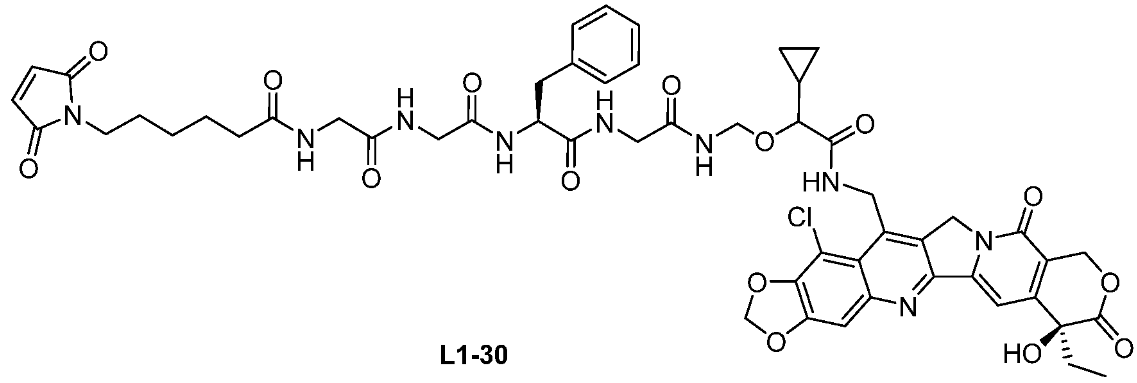

**L1-30**

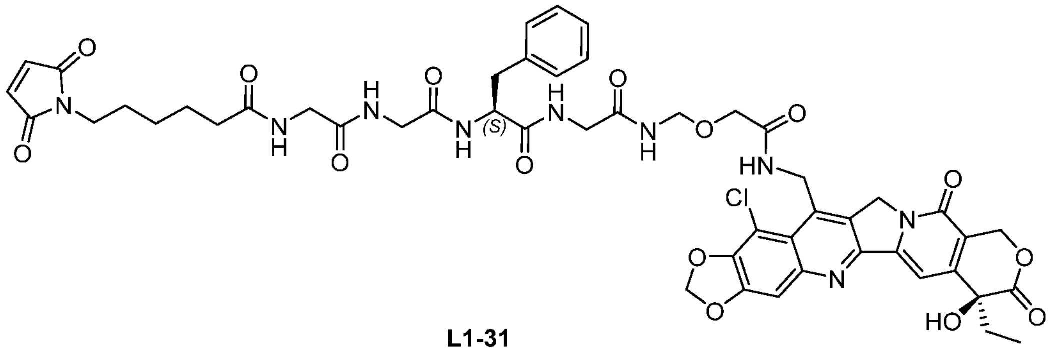

**L1-31**

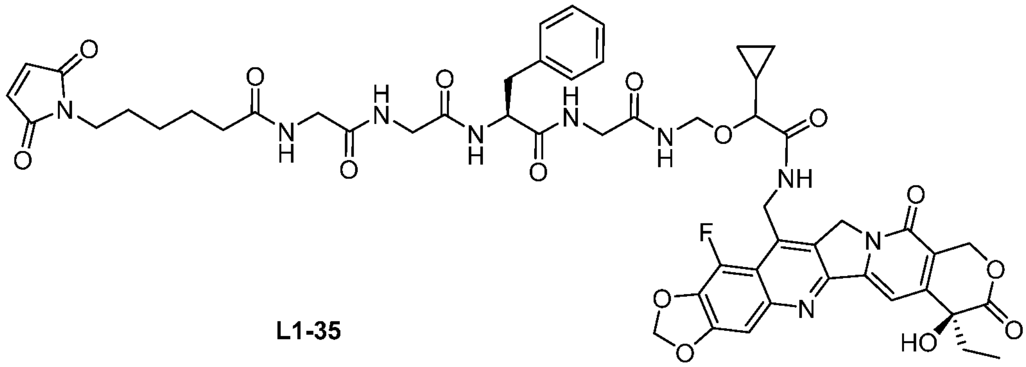

**L1-35**

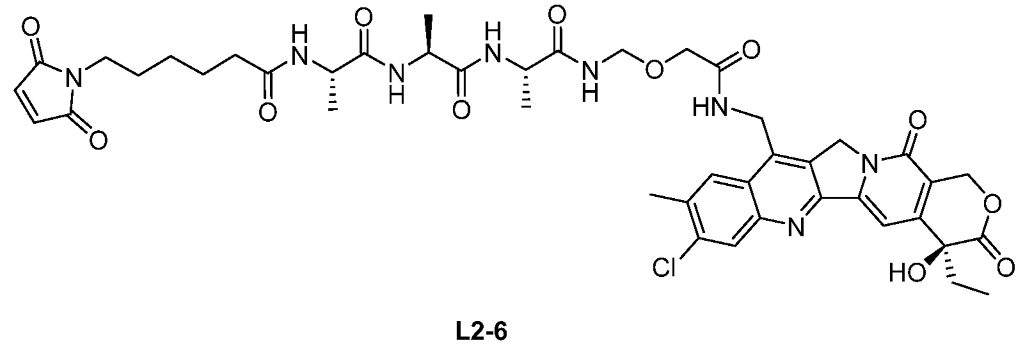

**L2-6**

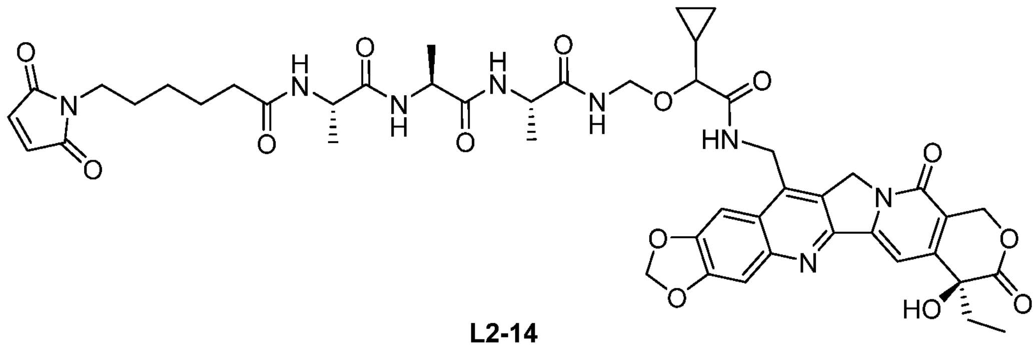

**L2-14**

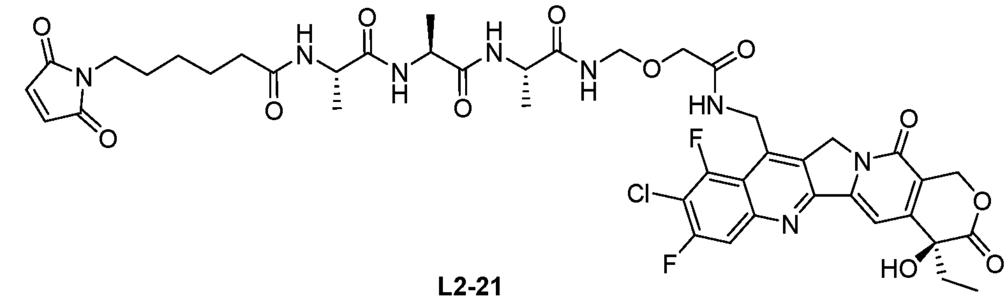

**L2-21**

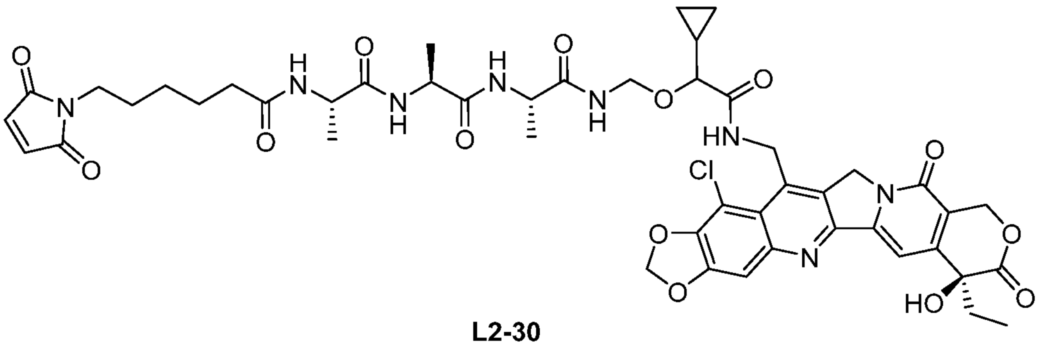

**L2-30**

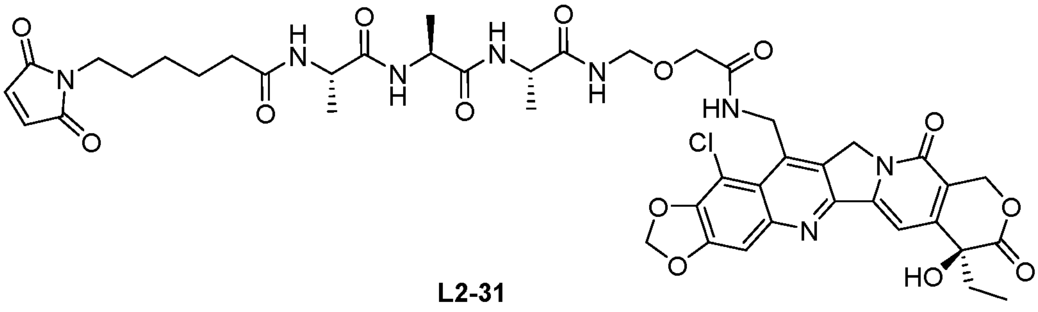

**L2-31**

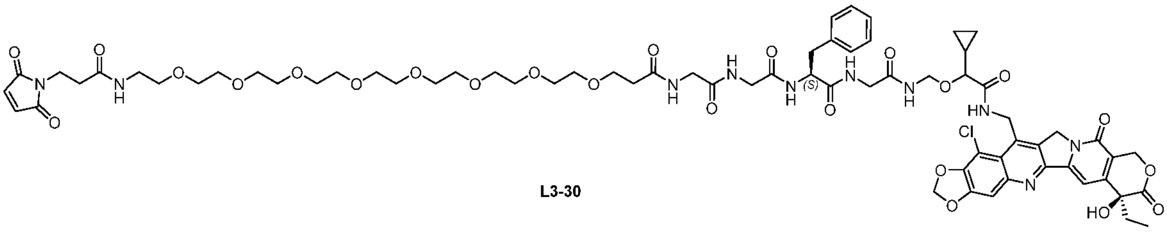

**L3-30**

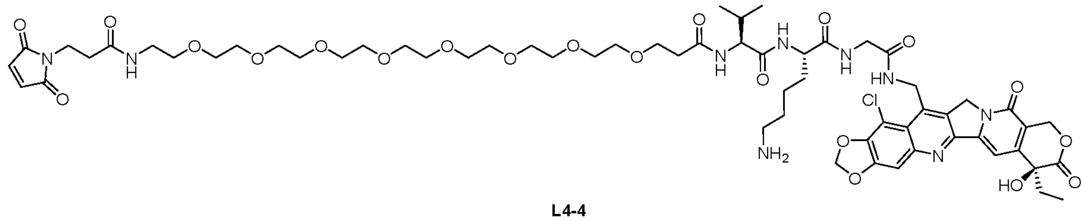

L4-4

or

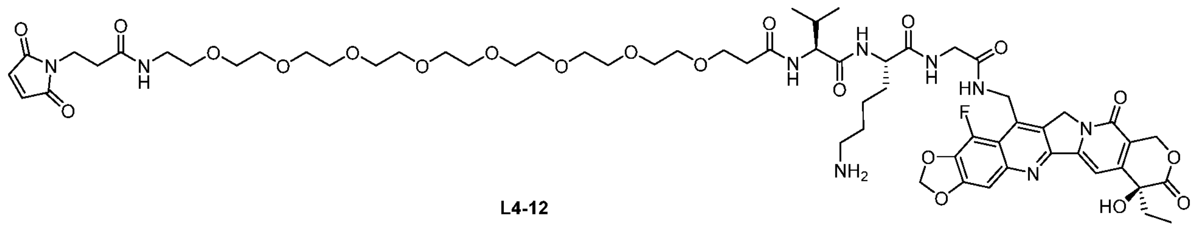

L4-12

.

**29.** A pharmaceutical composition, comprising the ligand-drug conjugate or the pharmaceutically acceptable salt thereof according to any of claims 1 to 23 and a pharmaceutically acceptable excipient.

**30.** Use of the ligand-drug conjugate or the pharmaceutically acceptable salt thereof according to any one of claims 1 to 23, or the pharmaceutical composition according to claim 29 in the manufacture of a medicament for treating a tumor.

**31.** A method for preparing the ligand-drug conjugate or the pharmaceutically acceptable salt thereof according to any one of claims 1 to 23, comprising the step of conjugating the drug-linker compound according to any one of claims 24 to 28 to a ligand, wherein optionally, the ligand is an antibody or an antigen-binding fragment thereof.

**32.** A method for treating a tumor in a subject in need, comprising administering to the subject the ligand-drug conjugate or the pharmaceutically acceptable salt thereof according to any one of claims 1 to 23, or the pharmaceutical composition according to claim 29.

**33.** Use of the ligand-drug conjugate or the pharmaceutically acceptable salt thereof according to any one of claims 1 to 23, or the pharmaceutical composition according to claim 29 for treating a tumor in a subject.

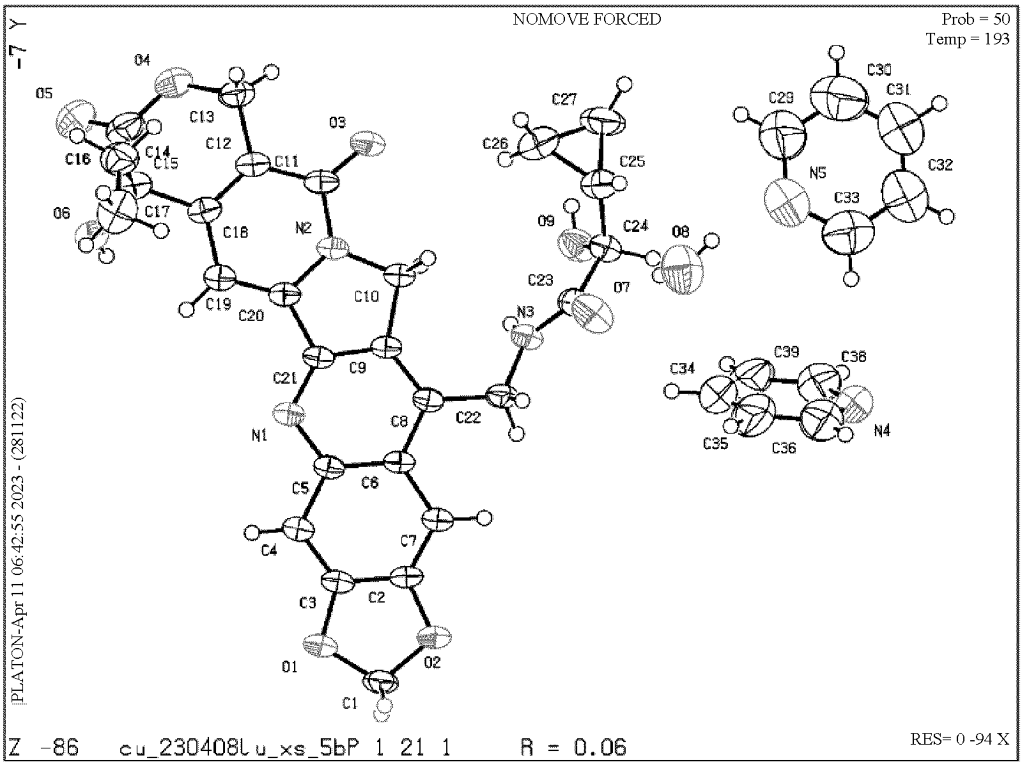
NOMOVE FORCED
Prob = 50
Temp = 193
-7 Y
PLATON-Apr 11 06:42:55 2023 - (281122)
Z -86 cu_230408Lu_xs_5bP 1 21 1 R = 0.06
RES= 0 -94 X

FIG. 1

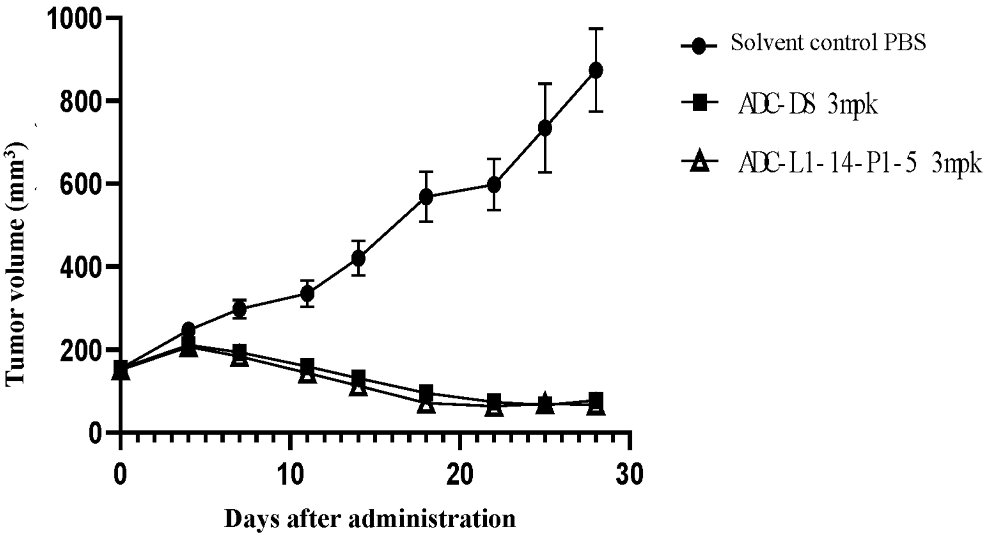
Tumor volume (mm³)
1000
800
600
400
200
0
0
10
20
30
Days after administration
Solvent control PBS
ADC-DS 3mpk
ADC-L1-14-P1-5 3mpk

FIG. 2

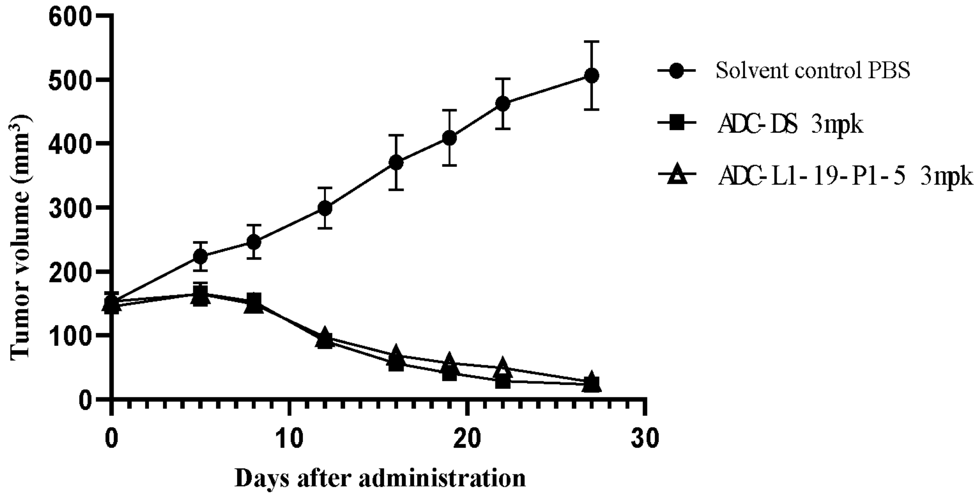
600
500
400
300
200
100
0
Tumor volume (mm3)
0
10
20
30
Days after administration
Solvent control PBS
ADC-DS 3mpk
ADC-L1-19-P1-5 3mpk

FIG. 3

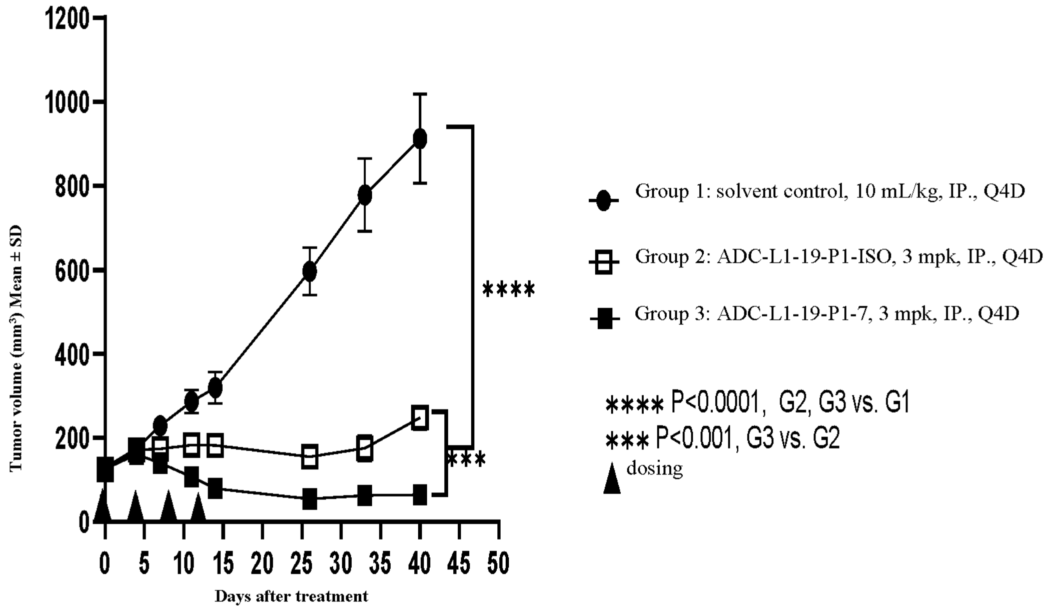
1200
1000
800
600
400
200
0
Tumor volume (mm3) Mean ± SD
0 5 10 15 20 25 30 35 40 45 50
Days after treatment
****
***
Group 1: solvent control, 10 mL/kg, IP., Q4D
Group 2: ADC-L1-19-P1-ISO, 3 mpk, IP., Q4D
Group 3: ADC-L1-19-P1-7, 3 mpk, IP., Q4D
**** P<0.0001, G2, G3 vs. G1
*** P<0.001, G3 vs. G2
dosing

FIG. 4

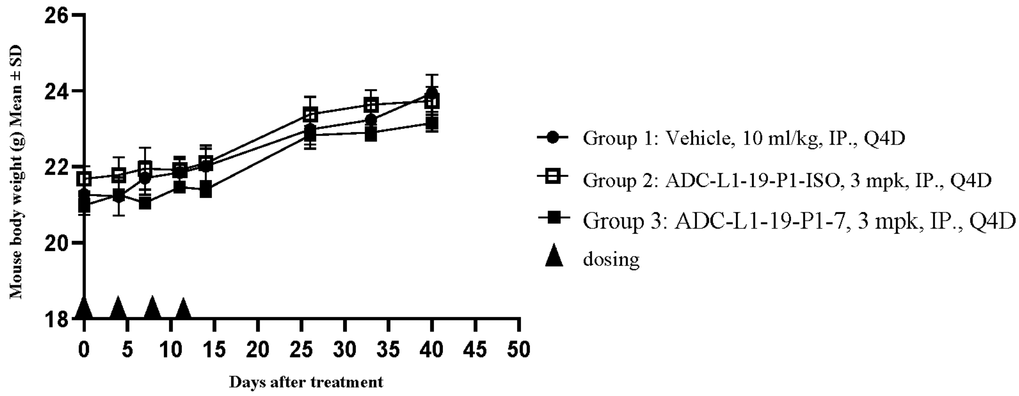


FIG. 5

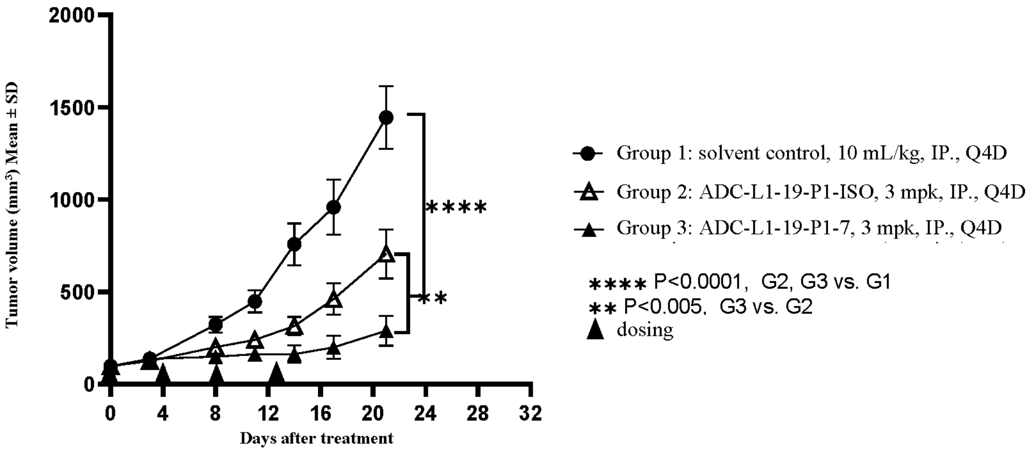


FIG. 6

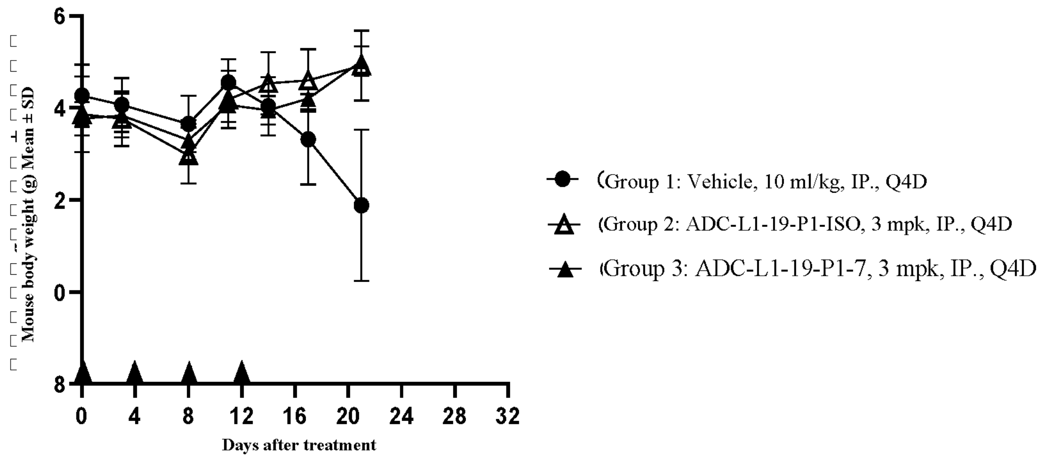


FIG. 7

**INTERNATIONAL SEARCH REPORT**

International application No.
**PCT/CN2023/093503**

**A. CLASSIFICATION OF SUBJECT MATTER**

A61K 47/68(2017.01)i； A61P35/00(2006.01)i； C07D491/22(2006.01)i； C07K16/28(2006.01)i

According to International Patent Classification (IPC) or to both national classification and IPC

**B. FIELDS SEARCHED**

Minimum documentation searched (classification system followed by classification symbols)

IPC： A61K A61P C07D C07K

Documentation searched other than minimum documentation to the extent that such documents are included in the fields searched

Electronic data base consulted during the international search (name of data base and, where practicable, search terms used)

CNABS; CNTXT; DWPI; VEN; ENTXTC; ENTXT; WPABS; CNKI; VCN; CJFD; 万方, WANFANG; ISI Web of Science; 读秀学术, DUXIU ACADEMIC; Springer; STNext; EMBL; NCBI: 先声再明医药有限公司, 喜树碱, 抗体, 偶联物, 结构式检索, search for structural formula, 序列检索, sequence search, Camptothecin, antibody, conjugate, ADC

**C. DOCUMENTS CONSIDERED TO BE RELEVANT**

| Category* | Citation of document, with indication, where appropriate, of the relevant passages | Relevant to claim No. |
|---|---|---|
| X | CN 112512592 A (SEAGEN INC.) 16 March 2021 (2021-03-16) description, paragraphs [0120]-[0124], [0136], [0264], [0265], [0277], [0495], [0507], [1014]-[1016] | 1-9, 11, 12, 17-24, 29-31 |
| A | LI, Wei et al. "Synthesis and Evaluation of Camptothecin Antibody-Drug Conjugates" *ACS Medicinal Chemistry Letters,* 06 September 2019 (2019-09-06), p. 1386, abstract | 1-31 |
| A | WO 2021067820 A1 (SEAGEN INC.) 08 April 2021 (2021-04-08) description, paragraphs [0006]-[0010] | 1-31 |
| A | US 2021101982 A1 (SEATTLE GENETICS INC.) 08 April 2021 (2021-04-08) description, paragraphs [0006]-[0046] | 1-31 |
| A | WO 2021067861 A1 (SEAGEN INC.) 08 April 2021 (2021-04-08) claims 1-75, description, paragraph [0323] | 1-31 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2023** | **24 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/093503**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/093503**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **32, 33**
because they relate to subject matter not required to be searched by this Authority, namely:

The subject matter of claim 32 relates to a method for treating a tumor in a subject in need; and the subject matter of claim 33 relates to the use for the treatment of a tumor in a subject. However, the above-mentioned method or use has an ultimate purpose of treating a disease in a living animal body, which falls within the scope of methods for diagnosing and treating diseases (PCT Rule 39.1(4)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/093503**

| Patent document cited in search report | Publication date (day/month/year) | Patent family member(s) | Publication date (day/month/year) |
|---|---|---|---|
| CN 112512592 A | 16 March 2021 | JP 2021527040 A | 11 October 2021 |
| | | EP 3801633 A1 | 14 April 2021 |
| | | MX 2020013169 A | 29 March 2021 |
| | | IL 278990 A | 31 January 2021 |
| | | CA 3101866 A1 | 12 December 2019 |
| | | AU 2019282767 A1 | 07 January 2021 |
| | | WO 2019236954 A1 | 12 December 2019 |
| | | TW 202015740 A | 01 May 2020 |
| | | BR 112020024915 A2 | 09 March 2021 |
| | | SG 11202011915 TA | 30 December 2020 |
| | | US 2023036256 A1 | 02 February 2023 |
| | | KR 20210053871 A | 12 May 2021 |
| WO 2021067820 A1 | 08 April 2021 | None | |
| US 2021101982 A1 | 08 April 2021 | None | |
| WO 2021067861 A1 | 08 April 2021 | KR 20220079606 A | 13 June 2022 |
| | | JP 2022550851 A | 05 December 2022 |
| | | EP 4037717 A1 | 10 August 2022 |
| | | IL 291686 A | 01 May 2022 |
| | | CA 3152316 A1 | 08 April 2021 |
| | | AU 2020356955 A1 | 14 April 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210515898
- CN 202210999585
- CN 202310406469
- US 4816567 P, Cabilly
- WO 2020219287 A
- WO 2020198531 A2
- CN 113521300 A
- WO 2020074724 A1
- CN 202210094806


### Non-patent literature cited in the description


- **KORNDORFER et al.** *Proteins: Structure, Function, and Bioinformatics*, 2003, vol. 53 (1), 121-129
- **ROQUE et al.** *Biotechnol. Prog*, 2004, vol. 20, 639-654
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **HOLLIGER P. et al.** *Proc. Natl. Acad. Sci. USA*, 1993, vol. 90, 6444-6448
- **POLJAK R.J. et al.** *Structure*, 1994, vol. 2, 1121-1123
- **MORRISON et al.** *Proc. Natl. Acad. Sci. USA*, 1984, vol. 81, 6851-6855
- **KINDT et al.** Kuby Immunology. W. H. Freeman and Co, 2007, 91
- **NEEDLEMA** ; **WUNSCH**. *J. Mol. Biol.*, 1970, vol. 48, 444-453, www.gcg.com
- **MAEHR**. *J. Chem. Ed*, 1985, vol. 62, 114-120
- Greene's Protective Groups in Organic Synthesis. John Wiley & Sons, Inc